# EUROPEAN PATENT SPECIFICATION

(11) **EP 1 443 934 B1**
(45) Date of publication and mention of the grant of the patent: **20.04.2005**
(21) Application number: 02775003.3
(22) Date of filing: 12.11.2002
(51) Int. Cl.: A61K 31/55, C07D 401/04, C07D 255/00, A61P 1/00

(54) **BENZOTRIAZEPINES AS GASTRIN AND CHOLECYSTOKININ RECEPTOR LIGANDS**
BENZOTRIAZEPINE ALS GASTRIN UND CHOLECYSTOKININ REZEPTOR LIGANDEN
BENZOTRIAZEPINES UTILISEES COMME LIGANDS DE RECEPTEURS DE CHOLECYSTOQUININE ET DE GASTRINE

(30) Priority: 13.11.2001 GB 0127262; 15.08.2002 GB 0219051
(43) Date of publication of application: 11.08.2004
(73) Proprietor: JAMES BLACK FOUNDATION LIMITED, London SE24 9JE (GB)
(72) Inventor: MCDONALD, Iain Mair, London SE24 9JE (GB); BUCK, Ildiko Maria, London SE24 9JE (GB); HARPER, Elaine Anne, London SE24 9JE (GB); LINNEY, Ian Duncan, London SE24 9JE (GB); PETHER, Michael John, London SE24 9JE (GB); STEEL, Katherine Isobel Mary, London SE24 9JE (GB); AUSTIN, Carol, London SE24 9JE (GB); DUNSTONE, David John, London SE24 9JE (GB); KALINDJIAN, Sarkis Barret, London SE24 9JE (GB); LOW, Caroline Minli Rachel, London SE24 9JE (GB); SPENCER, John, London SE24 9JE (GB); WRIGHT, Paul Trevor, London SE24 9JE (GB)
(74) Representative: Fisher, Adrian John
(86) International application number: PCT/GB2002/005121
(87) International publication number: WO 2003/041714

(56) References cited:
- US-A- 5 378 838
- AKITO NISHIDA ET AL: "PHARMACOLOGICAL PROFILE OF (R)-1-U2,3-DIHYDRO-1-(2'-METHYLPHENACYL)-2 -OXO-5-PHENYL-1H-1,4-BENZODIAZEPIN-3-YL-3- (3-METHYLPHEN)UREA (YM022), A NEW POTENT AND SELECTIVE GASTRIN/CHOLECYSTOKININ-B RECEPTOR ANTAGONIST, IN VITRO AND IN VIVO" JOURNAL OF PHARMACOLOGY AND EXPERIMENTAL THERAPEUTICS, AMERICAN SOCIETY FOR PHARMACOLOGY AND, US, vol. 269, no. 2, 1994, pages 725-731, XP000562028 ISSN: 0022-3565 cited in the application
- MORGENSTERN, O. ET AL: "Synthesis of 5-benzyl-1,3,4-benzotriazepines from 2- isothiocyanatodeoxybenzoin" PHARMAZIE (1992), 47(1), 25-8 , XP001146151
- ISHIWAKA, TAKUMI ET AL: "O-Aminobenzophenone derivatives. VII. Syntheses of benzo[e]-1,3,4- triazepine derivatives from 2-aminobenzophenones" BULLETIN OF THE CHEMICAL SOCIETY OF JAPAN (1970), 43(1), 135-8 , XP009006705
- VAINIOTALO, P. ET AL: "Mass spectral study of differently substituted 2-oxo- and 2-thioxo-1,3,4-benzotriazepines" JOURNAL OF HETEROCYCLIC CHEMISTRY (1990), 27(2), 259-62 , XP009006707
- MORGENSTERN, O. ET AL: "Preparation of 3,5-dimethyl-2-thioxo-1,2-dihydro-3H-1,3,4 -benzotriazepine" PHARMAZIE (1985), 40(10), 694-6 , XP001146150

## Description

This application claims priority from U.K. patent application Nos. 0127262.4 and 0219051.0, the entire contents of which are hereby incorporated by reference.

This invention relates to gastrin and cholecystokinin (CCK) receptor ligands. (The receptor previously known as the CCK_{B}/gastrin receptor is now termed the CCK₂ receptor). The invention also relates to methods for preparing such ligands and to compounds which are useful intermediates in such methods. The invention further relates to pharmaceutical compositions comprising such ligands and methods for preparing such pharmaceutical compositions.

Gastrin and the cholecystokinins are structurally related neuropeptides which exist in gastrointestinal tissue and the central nervous system (Mutt V., *Gastrointestinal Hormones*, Glass G.B.J., ed., Raven Press, New York, p. 169; Nisson G., *ibid,* p. 127).

Gastrin is one of the three primary stimulants of gastric acid secretion. Several forms of gastrin are found including 34-, 17- and 14-amino acid species with the minimum active fragment being the C-terminal tetrapeptide (TrpMetAspPhe-NH₂) which is reported in the literature to have full pharmacological activity (Tracy H.J. and Gregory R.A., *Nature* (London), 1964, **204,** 935). Much effort has been devoted to the synthesis of analogues of this tetrapeptide (and the N-protected derivative Boc-TrpMetAspPhe-NH₂) in an attempt to elucidate the relationship between structure and activity.

Natural cholecystokinin is a 33 amino acid peptide (CCK-33), the C-terminal 5 amino acids of which are identical to those of gastrin. Also found naturally is the C-terminal octapeptide (CCK-8) of CCK-33.

The cholecystokinins are reported to be important in the regulation of appetite. They stimulate intestinal mobility, gall bladder contraction, pancreatic enzyme secretion and are known to have a trophic action on the pancreas. They also inhibit gastric emptying and have various effects in the central nervous system.

Compounds which bind to cholecystokinin and/or gastrin receptors are important because of their potential pharmaceutical use as antagonists, inverse agonists or partial agonists of the natural peptides. Such compounds are described herein as ligands. The term ligand as used herein means either an antagonist, partial or full agonist, or an inverse agonist. Usually, the term ligand refers to an antagonist.

A number of gastrin ligands have been proposed for various therapeutic applications, including the prevention of gastrin-related disorders including gastrointestinal ulcers, dyspepsia, reflux oesophagitis (gastroesophageal reflux disease (GERD), both erosive and non-erosive) by reduction in gastric acid secretion and/or improving impaired motor activity at the lower oesophageal sphincter, Zollinger-Ellison syndrome, Barrett's oesophagus (specialized intestinal metaplasia of distal oesophagus), ECL cell hyperplasia, rebound hypersecretion (following cessation of anti-secretory therapy), ECL-derived gastric polyps most commonly found in patients with atrophic gastritis both with (pernicious anaemia) or without vitamin B12 deficiency, antral G cell hyperplasia and other conditions in which lower gastrin activity or lower acid secretion is desirable. The hormone has also been shown to have a trophic action on cells and so an antagonist may be expected to be useful in the treatment of cancers, particularly in the GI tract, more particularly in the stomach, oesophagus and colo-rectal areas. Tumours found in other organs such as the pancreas, lung (small cell lung carcinomas) and thyroid (thyroid medullary tumours) may also be treated.

Other possible uses are in the potentiation of opiate (for example morphine) analgesia. Moreover, ligands for cholecystokinin receptors in the brain (so-called CCK₂ receptors) have been claimed to possess anxiolytic activity.

A known antagonist of the CCK₂ receptor is L-365,260 (M.G. *Bock et al*., *J*. *Med Chem*., 1989, 32, 13-16), which is based on a benzodiazepine structure. In the rat stomach assay described hereinbelow, L-365,260 was shown to have an affinity of pK_{B} = 7.61±0.12 for the CCK₂ receptor (S.B. Kalindjian *et al*., *J*. *Med*. *Chem*., 1994, 37, 3671-3).

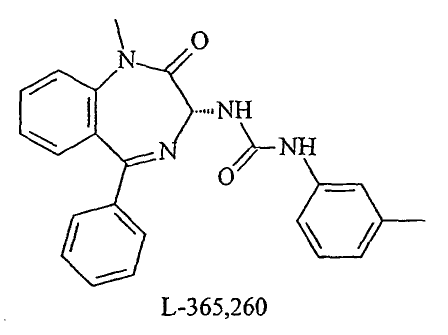

More recently, another benzodiazepine, YF476, was developed as a potent CCK₂ antagonist (A. Nishida *et al*., *Journal of Pharmacology and Experimental Therapeutics,* 1994, *269*, 725-731). In rat cortical membranes, YF476 was found to have an affinity pKᵢ of 10.17±0.03 for the CCK₂ receptor.

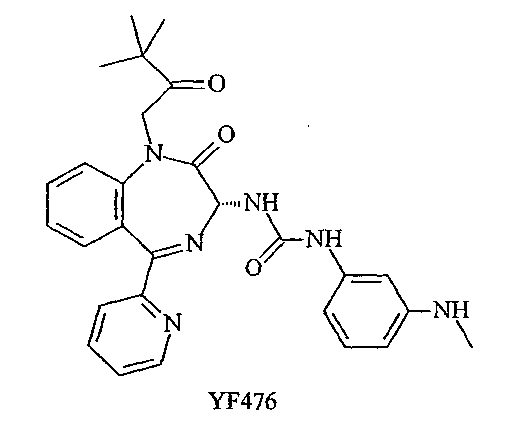

L-365,260 and YF476 are structurally closely related. Both compounds are 1,4-benzodiazepines. The carbon at position three of these 1,4-benzodiazepines is a chiral centre. In both cases, the optimal compounds have an *R*-configuration at this centre. Indeed, all 1,4-benzodiazepines that have found utility as gastrin antagonists have a chiral centre on the diazepine ring, and it has been found that better gastrin receptor antagonism is exhibited by one configuration relative to the other.

The requirement for a single enantiomer of the known 1,4-benzodiazepine gastrin ligands is undesirable. The synthesis of single enantiomers from achiral precursors, as in these cases, is a costly and relatively complex procedure. This generally requires, for example, either a separation step, usually inefficient as one enantiomer is discarded, or the use of an often expensive chiral auxiliary during the synthesis, coupled with an increase in chemical steps. For these reasons, a drug candidate with no stereocentres on the seven-membered ring would offer a distinct advantage over chiral alternatives.

US 5,091,381 describes benzotriazepines which are said to bind to peripheral benzodiazepine receptors.

EP-A-0645378 describes a class of bicyclic compounds which are said to inhibit squalene synthetase.

It is an object of the present invention to provide potent and selective gastrin and CCK receptor ligands. It is a further object of the present invention to provide gastrin and CCK receptor ligands which have no chiral centre on the 7-membered ring and which can therefore be prepared using straightforward synthetic methods.

According to the present invention, there is provided the use of compounds of formula (I): 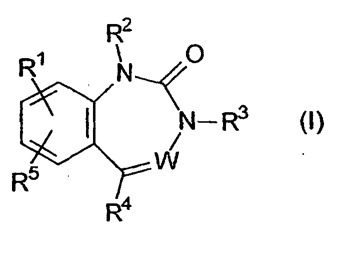 wherein:
W is N or N⁺-O⁻;
R¹ and R⁵ are independently H, C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino, (C₁ to C₆ alkyl)aminocarbonyl, di(C₁ to C₆ alkyl)aminocarbonyl, [*N*-Z](C₁ to C₆ alkyl)carbonylamino, formyloxy, formamido, (C₁ to C₆ alkyl)aminosulfonyl, di(C₁ to C₆ alkyl)aminosulfonyl, [*N*-Z](C₁ to C₆ alkyl)sulfonylamino or cyano; or R¹ and R⁵ together form a methylenedioxy group;
R² is H or an optionally substituted C₁ to C₁₈ hydrocarbyl group wherein up to three C atoms may optionally be replaced by N, O and/or S atoms.
R³ is -(CR¹¹R¹²)ₘ-X-(CR¹³R¹⁴)ₚ-R⁹;
m is 0, 1, 2, 3 or 4;
p is 0, 1 or 2;
X is a bond, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 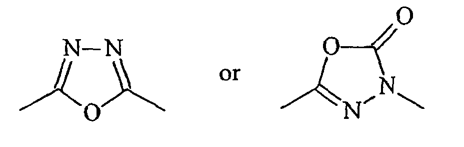
R⁹ is H; C₁ to C₆ alkyl; or phenyl, naphthyl, pyridyl, benzimidazolyl, indazolyl, quinolinyl, isoquinolinyl, tetrahydroisoquinolinyl, indolinyl, isoindolinyl, indolyl, isoindolyl or 2-pyridonyl, all optionally substituted with 1, 2 or 3 groups independently selected from -L-Q
wherein:
L is a bond, or a group of the formula -(CR¹⁷R¹⁸)ᵥ-Y-(CR¹⁷R¹⁸)_{w}, wherein v and w are independently 0, 1, 2 or 3, and Y is a bond, -CR¹⁵=CR¹⁶-, phenyl, furanyl, thiophenyl, pyrrolyl, thiazolyl, imidazolyl, oxazolyl, isoxazolyl, pyrazolyl, isoxazolonyl, piperazinyl, piperidinyl, morpholinyl, pyrrolidinyl, isothiazolyl, triazolyl, oxadiazolyl, thiadiazolyl, pyridyl or pyridazyl; and
Q is H, (C₁ to C₆ alkyl)oxy, [*N*-Z](C₁ to C₆ alkyl)oxy(C₁ to C₆ alkyl)amino, thio, (C₁ to C₆ alkyl)thio, carboxy(C₁ to C₆ alkyl)thio, carboxy, carboxy(C₁ to C₆ alkyl), carboxy(C₁ to C₆ alkenyl), [*N*-Z]carboxy(C₁ to C₆ alkyl)amino, carboxy(C₁ to C₆ alkyl)oxy, formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, amino, [*N*-Z](C₁ to C₆ alkyl)amino, aminocarbonyl, (C₁ to C₆ alkyl)aminocarbonyl, di(C₁ to C₆ alkyl)aminocarbonyl, [*N*-Z](C₁ to C₆ alkyl)carbonylamino, C₅ to C₈ cycloalkyl, [*N*-Z](C₁ to C₆ alkyl)carbonyl(C₁ to C₆ alkyl)amino, halo, halo(C₁ to C₆ alkyl), sulfamoyl, [*N*-Z](C₁ to C₆ alkyl)sulfonylamino, (C₁ to C₆ alkyl)sulfonylaminocarbonyl, carboxy(C₁ to C₆ alkyl)sulfonyl, carboxy(C₁ to C₆ alkyl)sulfinyl, tetrazolyl, [*N*-Z]tetrazolylamino, cyano, amidino, amidinothio, SO₃H, formyloxy, formamido, C₃ to C₈ cycloalkyl, (C₁ to C₆ alkyl)sulphamoyl, di(C₁ to C₆ alkyl)sulphamoyl, (C₁ to C₆ alkyl)carbonylaminosulfonyl, 5-oxo-2,5-dihydro[1,2,4]oxadiazolyl, carboxy(C₁ to C₆ alkyl)carbonylamino, tetrazolyl(C₁ to C₆ alkyl)thio, [*N*-Z]tetrazolyl(C₁ to C₆ alkyl)amino, 5-oxo-2,5-dihydro[1,2,4]thiadiazolyl, 5-oxo-1,2-dihydro[1,2,4]triazolyl, [*N*-Z](C₁ to C₆ alkyl)amino(C₁ to C₆ alkyl)amino, or a group of the formula 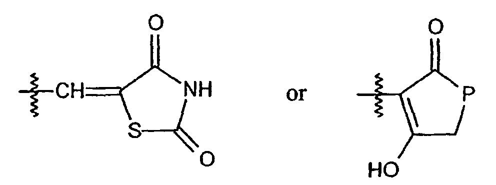
wherein P is O, S or NR¹⁹;
Z is H, C₁ to C₆ alkyl, t-butoxycarbonyl, acetyl, benzoyl or benzyl;
R⁴ is an optionally substituted C₁ to C₁₈ hydrocarbyl group wherein up to three C atoms may optionally be replaced by N, O and/or S atoms;
R¹¹, R¹², R¹³, R¹⁴, R¹⁵, R¹⁷, R¹⁸ and R¹⁹ are independently H or C₁ to C₃ alkyl; and
R¹⁶ is H, C₁ to C₃ alkyl, or acetylamino;
or a pharmaceutically acceptable salt thereof;
for the preparation of a medicament for the treatment of gastrin related disorders.

Preferably, W is N.

Typical gastrin related disorders are gastrointestinal ulcers, dyspepsia, reflux oesophagitis (gastroesophageal reflux disease (GERD), both erosive and non-erosive), Zollinger-Ellison syndrome, Barrett's oesophagus (specialized intestinal metaplasia of distal oesophagus), ECL cell hyperplasia, rebound hypersecretion (following cessation of anti-secretory therapy), ECL-derived gastric polyps, cancers of the GI tract, more particularly in the stomach, oesophagus and colo-rectal areas, as well as tumours found in other organs such as the pancreas, lung (small cell lung carcinomas) and thyroid (thyroid medullary tumours) and anxiety. The potentiation of opiate induced analgesia may also provide a role for the gastrin ligands of the present invention.

Further, the present invention provides compounds of formula (IIa) 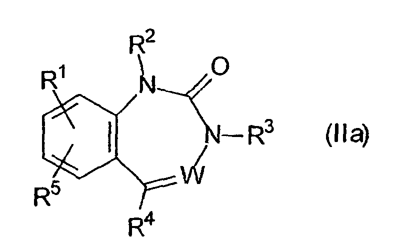 wherein:
W is N or N⁺-O⁻;
R¹ and R⁵ are independently H, C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino, (C₁ to C₆ alkyl)aminocarbonyl, di(C₁ to C₆ alkyl)aminocarbonyl, [*N*-Z](C₁ to C₆ alkyl)carbonylamino, formyloxy, formamido, (C₁ to C₆ alkyl)aminosulfonyl, di(C₁ to C₆ alkyl)aminosulfonyl, [*N*-Z](C₁ to C₆ alkyl)sulfonylamino or cyano; or R¹ and R⁵ together form a methylenedioxy group;
R² is -(CH₂)ₛ-C(O)-(CH₂)ₜ-R⁸
s is 0, 1, 2 or 3;
t is 0, 1, 2 or 3;
R⁸ is selected from H, OH, C₁ to C₁₂ alkyl, (C₁ to C₁₂ alkyl)oxy, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano);
R³ is -(CR¹¹R¹²)ₘ-X-(CR¹³R¹⁴)ₚ-R⁹;
m is 0, 1, 2, 3 or 4 (preferably 1 or 2);
p is 0, 1 or 2;
X is a bond, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 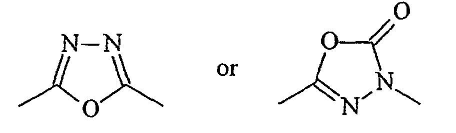
R⁹ is H; C₁ to C₆ alkyl; or phenyl, naphthyl, pyridyl, benzimidazolyl, indazolyl, quinolinyl, isoquinolinyl, tetrahydroisoquinolinyl, indolinyl, isoindolinyl, indolyl, isoindolyl or 2-pyridonyl, all optionally substituted with 1, 2 or 3 groups independently selected from -L-Q
wherein:
L is a bond, or a group of the formula -(CR¹⁷R¹⁸)ᵥ-Y-(CR¹⁷R¹⁸)_{w}, wherein v and w are independently 0, 1, 2 or 3, and Y is a bond, -CR¹⁵=CR¹⁶-, phenyl, furanyl, thiophenyl, pyrrolyl, thiazolyl, imidazolyl, oxazolyl, isoxazolyl, pyrazolyl, isoxazolonyl, piperazinyl, piperidinyl, morpholinyl, pyrrolidinyl, isothiazolyl, triazolyl, oxadiazolyl, thiadiazolyl, pyridyl or pyridazyl; and
Q is H, (C₁ to C₆ alkyl)oxy, [*N*-Z](C₁ to C₆ alkyl)oxy(C₁ to C₆ alkyl)amino, thio, (C₁ to C₆ alkyl)thio, carboxy(C₁ to C₆ alkyl)thio, carboxy, carboxy(C₁ to C₆ alkyl), carboxy(C₁ to C₆ alkenyl), [*N*-Z]carboxy(C₁ to C₆ alkyl)amino, carboxy(C₁ to C₆ alkyl)oxy, formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, amino, [*N*-Z](C₁ to C₆ alkyl)amino, aminocarbonyl, (C₁ to C₆ alkyl)aminocarbonyl, di(C₁ to C₆ alkyl)aminocarbonyl, [*N*-Z](C₁ to C₆ alkyl)carbonylamino, C₅ to C₈ cycloalkyl, [*N*-Z](C₁ to C₆ alkyl)carbonyl(C₁ to C₆ alkyl)amino, halo, halo(C₁ to C₆ alkyl), sulfamoyl, [*N*-Z](C₁ to C₆ alkyl)sulfonylamino, (C₁ to C₆ alkyl)sulfonylaminocarbonyl, carboxy(C₁ to C₆ alkyl)sulfonyl, carboxy(C₁ to C₆ alkyl)sulfinyl, tetrazolyl, [*N*-Z]tetrazolylamino, cyano, amidino, amidinothio, SO₃H, formyloxy, formamido, C₃ to C₈ cycloalkyl, (C₁ to C₆ alkyl)sulphamoyl, di(C₁ to C₆ alkyl)sulphamoyl, (C₁ to C₆ alkyl)carbonylaminosulfonyl, 5-oxo-2,5-dihydro[1,2,4]oxadiazolyl, carboxy(C₁ to C₆ alkyl)carbonylamino, tetrazolyl(C₁ to C₆ alkyl)thio, [*N*-Z]tetrazolyl(C₁ to C₆ alkyl)amino, 5-oxo-2,5-dihydro[1,2,4]thiadiazolyl, 5-oxo-1,2-dihydro[1,2,4]triazolyl, [*N*-Z](C₁ to C₆ alkyl)amino(C₁ to C₆ alkyl)amino, or a group of the formula 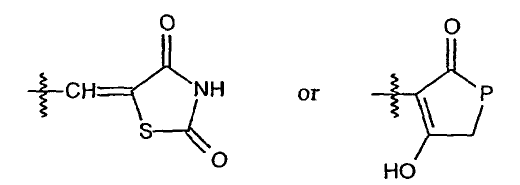
wherein P is O, S or NR¹⁹;
Z is H, C₁ to C₆ alkyl, t-butoxycarbonyl, acetyl, benzoyl or benzyl;
R⁴ is an optionally substituted C₁ to C₁₈ hydrocarbyl group wherein up to three C atoms may optionally be replaced by N, O and/or S atoms;
R¹¹, R¹², R¹³, R¹⁴, R¹⁵, R¹⁷, R¹⁸ and R¹⁹ are independently H or C₁ to C₃ alkyl; and
R¹⁶ is H, C₁ to C₃ alkyl, or acetylamino;
or a pharmaceutically acceptable salt thereof;
with the proviso that R² is not CH₂CO₂H or C(O)CH₃ when R⁴ is phenyl.

Further, the present invention provides compounds of formula (IIb) 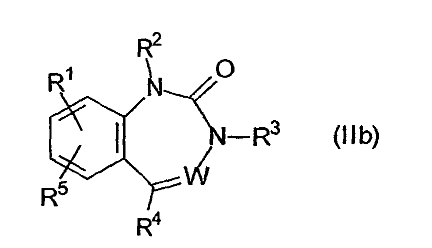 wherein:
W is N or N⁺-O⁻;
R¹ and R⁵ are independently H, C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino, (C₁ to C₆ alkyl)aminocarbonyl, di(C₁ to C₆ alkyl)aminocarbonyl, [*N*-Z](C₁ to C₆ alkyl)carbonylamino, formyloxy, formamido, (C₁ to C₆ alkyl)aminosulfonyl, di(C₁ to C₆ alkyl)aminosulfonyl, [*N*-Z](C₁ to C₆ alkyl)sulfonylamino or cyano; or R¹ and R⁵ together form a methylenedioxy group;
R² is H or an optionally substituted C₁ to C₁₈ hydrocarbyl group wherein up to three C atoms may optionally be replaced by N, O and/or S atoms;
R³ is -(CR¹¹R¹²)ₘ-X-(CR¹³R¹⁴)ₚ-R⁹;
m is 0, 1, 2, 3 or 4 (preferably I or 2);
p is 0, 1 or 2;
X is a bond, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 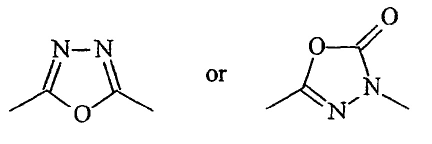
R⁹ is H; C₁ to C₆ alkyl; or phenyl, naphthyl, pyridyl, benzimidazolyl, indazolyl, quinolinyl, isoquinolinyl, tetrahydroisoquinolinyl, indolinyl, isoindolinyl, indolyl, isoindolyl or 2-pyridonyl, all optionally substituted with 1, 2 or 3 groups independently selected from -L-Q
wherein:
L is a bond, or a group of the formula -(CR¹⁷R¹⁸)ᵥ-Y-(CR¹⁷R¹⁸)_{w}, wherein v and w are independently 0, 1, 2 or 3, and Y is a bond, -CR¹⁵=CR¹⁶-, phenyl, furanyl, thiophenyl, pyrrolyl, thiazolyl, imidazolyl, oxazolyl, isoxazolyl, pyrazolyl, isoxazolonyl, piperazinyl, piperidinyl, morpholinyl, pyrrolidinyl, isothiazolyl, triazolyl, oxadiazolyl, thiadiazolyl, pyridyl or pyridazyl; and
Q is H, (C₁ to C₆ alkyl)oxy, [*N*-Z](C₁ to C₆ alkyl)oxy(C₁ to C₆ alkyl)amino, thio, (C₁ to C₆ alkyl)thio, carboxy(C₁ to C₆ alkyl)thio, carboxy, carboxy(C₁ to C₆ alkyl), carboxy(C₁ to C₆ alkenyl), [*N*-Z]carboxy(C₁ to C₆ alkyl)amino, carboxy(C₁ to C₆ alkyl)oxy, formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, amino, [*N*-Z](C₁ to C₆ alkyl)amino, aminocarbonyl, (C₁ to C₆ alkyl)aminocarbonyl, di(C₁ to C₆ alkyl)aminocarbonyl, [*N*-Z](C₁ to C₆ alkyl)carbonylamino, C₅ to C₈ cycloalkyl, [*N*-Z](C₁ to C₆ alkyl)carbonyl(C₁ to C₆ alkyl)amino, halo, halo(C₁ to C₆ alkyl), sulfamoyl, [*N*-Z](C₁ to C₆ alkyl)sulfonylamino, (C₁ to C₆ alkyl)sulfonylaminocarbonyl, carboxy(C₁ to C₆ alkyl)sulfonyl, carboxy(C₁ to C₆ alkyl)sulfinyl, tetrazolyl, [*N*-Z]tetrazolylamino, cyano, amidino, amidinothio, SO₃H, formyloxy, formamido, C₃ to C₈ cycloalkyl, (C₁ to C₆ alkyl)sulphamoyl, di(C₁ to C₆ alkyl)sulphamoyl, (C₁ to C₆ alkyl)carbonylaminosulfonyl, 5-oxo-2,5-dihydro[1,2,4]oxadiazolyl, carboxy(C₁ to C₆ alkyl)carbonylamino, tetrazolyl(C₁ to C₆ alkyl)thio, [*N*-Z]tetrazolyl(C₁ to C₆ alkyl)amino, 5-oxo-2,5-dihydro[1,2,4]thiadiazolyl, 5-oxo-1,2-dihydro[1,2,4]triazolyl, [*N*-Z](C₁ to C₆ alkyl)amino(C₁ to C₆ alkyl)amino, or a group of the fonnula 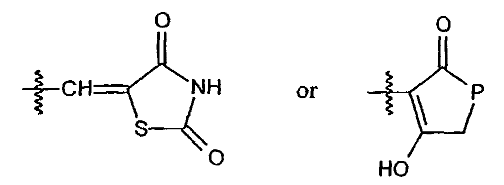
wherein P is O, S or NR¹⁹;
Z is H, C₁ to C₆ alkyl, t-butoxycarbonyl, acetyl, benzoyl or benzyl;
R⁴ is of formula

-(CH₂)_{q}-T-R¹⁰

wherein:
q is 0, 1, 2 or 3;
T is a bond, O, S, NH or N(C₁ to C₆ alkyl); and
R¹⁰ is C₁ to C₁₂ alkyl, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, C₃ to C₈ cycloalkyl, (C₃ to C₈ cycloalkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano);
R¹¹, R¹², R¹³, R¹⁴, R¹⁵, R¹⁷, R¹⁸ and R¹⁹ are independently H or C₁ to C₃ alkyl; and
R¹⁶ is H, C₁ to C₃ alkyl, or acetylamino;
or a pharmaceutically acceptable salt thereof;
with the proviso that R¹⁰ is not phenyl or substituted phenyl when q is 0 and T is a bond.

Preferably R¹ and R⁵ are both H. However, it will be appreciated the benzo-fused ring system may have one or two substituents on the benzene ring as indicated hereinabove. The substituents may have subtle steric and/or electronic effects which modify the activity of the compound at the gastrin receptor. However, the presence or otherwise of certain substituents on the benzene ring is not crucial to the overall pharmacological activity of the present compounds.

Preferably, in the compound of formula (IIa) or (IIb), W is N.

Preferably, in the compound of formula (I) or (IIb) R² is of formula:

-(CH₂)ₛ-C(R⁶R⁷)ₙ-(CH₂)ₜ-R⁸

wherein:
R⁶ and R⁷ are independently selected from H, C₁ to C₆ alkyl or OH; or R⁶ and R⁷ together represent an =O group;
n is 0 or 1;
s is 0, 1, 2 or 3;
t is 0, 1, 2 or 3; and
R⁸ is selected from H, C₁ to C₁₂ alkyl, (C₁ to C₁₂ alkyl)oxy, C₃ to C₁₂ cycloallcyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano).

A preferred group of compounds according to the present invention is where R² is of formula:

-(CH₂)C(O)R⁸

wherein:
R⁸ is a branched C₃ to C₁₂ alkyl group (such as *tert*-butyl, *sec*-butyl, isopropyl, isobutyl or isovaleryl); or R⁸ is a C₃ to C₁₂ cycloalkyl (such as cyclopentyl, cyclohexyl, cycloheptyl or adamantyl) phenyl, pyridyl, pyrrolidinyl or piperidinyl group (all optionally substituted with 1, 2 or 3 C₁₋₆alkyl groups).

Preferably, R¹¹, R¹², R¹³, R¹⁴, R¹⁵, R¹⁶, R¹⁷, R¹⁸ and R¹⁹ are all H.

A preferred group of compounds according to the present invention is where R³ is of formula:

-(CH₂)-X-R⁹

wherein:
X is C(O)NH or NHC(O), more preferably X is C(O)NH.
Preferably, R⁹ is phenyl substituted with a carboxy, carboxy(C₁ to C₆ alkyl), tetrazolyl, tetrazolyl-*N*-(C₁ to C₆ alkyl)amino, carboxy(C₁ to C₆ alkyl)thio, carboxy(C₁ to C₆ alkyl)sulfonyl, (C₁ to C₆ alkyl)amino, or 5-oxo-2,5-dihydro[1,2,4]oxadiazolyl group; or R⁹ is a *N*-[carboxy(C₁ to C₆ alkyl)]indolinyl or *N*-[carboxy(C₁ to C₆ alkyl)]indolyl group.

When R⁹ is a substituted phenyl group, the substituent is preferably at the 3-position of the phenyl group.

Preferably, in compounds according to formula (I) or (IIa), R⁴ is of formula:

-(CH₂)_{q}-T-R¹⁰

wherein:
q is 0, 1, 2 or 3;
T is a bond, O, S, NH or N(C₁ to C₆ alkyl); and
R¹⁰ is C₁ to C₁₂ alkyl, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, C₃ to C₈ cycloalkyl, (C₃ to C₈ cycloalkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano).

More preferably, in compounds according to formula (1) or (IIa), R⁴ is selected from C₁₋₁₂ alkyl (such as *tert-*butyl, *sec*-butyl, isopropyl, isobutyl or isovaleryl), C₃₋₁₂ cycloalkyl (such as cyclopentyl, cyclohexyl, cycloheptyl or adamantyl), pyridyl or phenyl (all of which may be optionally substituted with 1, 2 or 3 groups selected from OMe, NMe₂, CF₃, Me, F, Cl, Br or I).

In all compounds of the present invention, preferably q is 0 and T is a bond. More preferably R⁴ is C₃-C₁₂ cycloalkyl, and more preferably, R⁴ is cyclohexyl.

Certain compounds of the invention exist in various regioisomeric, enantiomeric, tautomeric and diastereomeric forms. It will be understood that the invention comprehends the different regioisomers, enantiomers, tautomers and diastereomers in isolation from each other as well as mixtures.

Compounds of the present invention wherein W is N may be prepared by the representative procedure shown in Reaction Scheme 1.

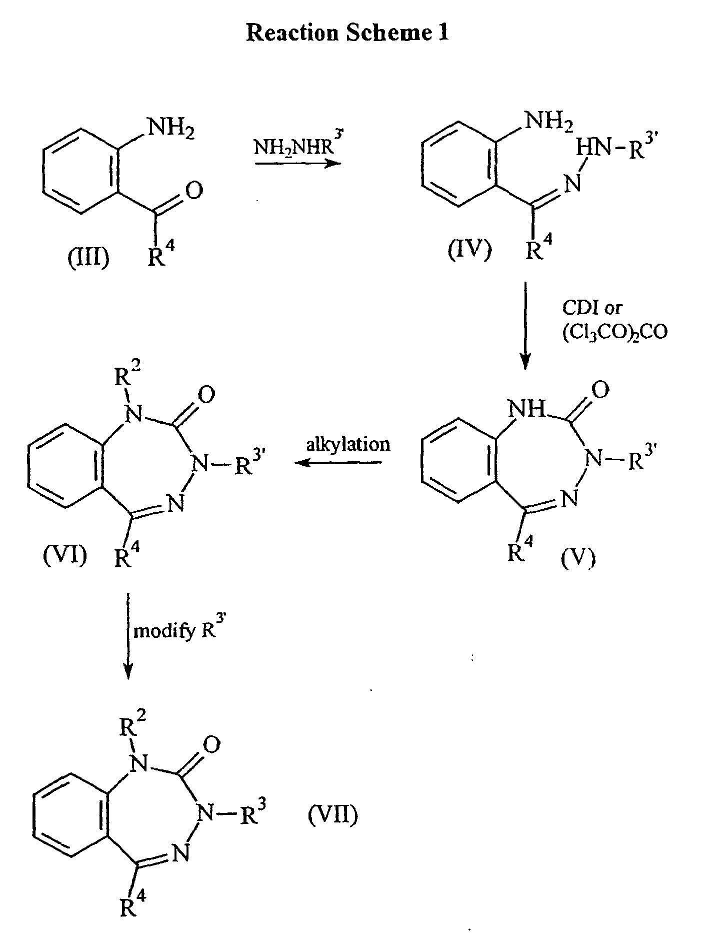

Ketone (III) is reacted with NH₂NHR^{3'} (wherein R^{3'} represents either R³ or a suitable precursor thereof) to form hydrazone (IV). The hydrazone (IV) is then cyclised using a bifunctional carbonyl reagent to form benzotriazepinone (V). Bifunctional carbonyl reagents are well known to the person skilled in the art and include, for example, carbonyldiimidazole (CDI), triphosgene, phosgene or cyanogen bromide. Alkylation under standard conditions followed by modification of R^{3'} affords the desired benzotriazepinone (VII).

Compounds wherein W is N⁺-O⁻ may be prepared by treating compound VII directly or an appropriately protected derivative of compound VI, with an oxidising agent such as MCPBA. Such derivatives of compound VI yield the desired N-oxide following deprotection.

R^{3'} groups which are suitable precursors of R³ will depend on the particular nature of R³. For example, when R³ is -(CH₂)ₘC(O)NH-(CH₂)ₚ-R⁹, a suitable R^{3'} group would be -(CH₂)ₘCO₂(C₁₋₆ alkyl). In this case, the requisite R³ groups may be readily accessed via an ester hydrolysis followed by a simple amide coupling reaction. The skilled person will be aware of many other suitable R^{3'} groups, depending on the nature of R³.

Alkylation may be performed by, for example, displacement of an alkyl halide in the presence of a base. Methods of alkylation will be readily apparent to the person skilled in the art.

Hence, the present invention also provides a method of making compounds according to formula (I), formula (IIa) or fonnula(IIb).

It is an important advantage of the synthesis described hereinabove that no chiral centres are generated in the benzotriazepinone ring system during the synthesis.

The invention also comprehends derivative compounds ("pro-drugs") which are degraded *in vivo* to yield the species of formula (IIa) or (IIb). Pro-drugs are usually (but not always) of lower potency at the target receptor than the species to which they are degraded. Pro-drugs are particularly useful when the desired species has chemical or physical properties which make its administration difficult or inefficient. For example, the desired species may be only poorly soluble, it may be poorly transported across the mucosal epithelium, or it may have an undesirably short plasma half-life. Further discussion of pro-drugs may be found in Stella, V. J. *et al*., "Prodrugs", *Drug Delivery Systems*, 1985, pp. 112-176, and *Drugs,* 1985, **29,** pp. 455-473.

Pro-drug forms of the pharmacologically-active compounds of the invention will generally be compounds according to formula (II) having an acid group which is esterified or amidated. Included in such esterified acid groups are groups of the form ―COOR^{a}, wherein R^{a} is C₁ to C₅ alkyl, phenyl, substituted phenyl, benzyl, substituted benzyl, or one of the following:

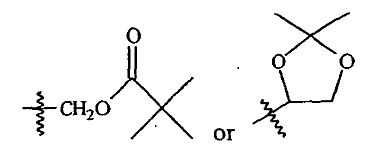

Amidated acid groups include groups of the formula -CONR^{b}R^{c}, wherein R^{b} is H, C₁ to C₅ alkyl, phenyl, substituted phenyl, benzyl, or substituted benzyl, and R^{c} is -OH or one of the groups just recited for R^{b}.

Compounds of formula (II) having an amino group may be derivatised with a ketone or an aldehyde such as formaldehyde to form a Mannich base. This will hydrolyse with first order kinetics in aqueous solution.

Another aspect of the present invention is a pharmaceutical composition comprising a compound of formula (II) substantially as described herein before with a pharmaceutically acceptable diluent or carrier.

Yet another aspect of the present invention is a method of making a pharmaceutical composition comprising a compound of formula (II) substantially as described herein before, comprising mixing said compound with a pharmaceutically acceptable diluent or carrier.

Pharmaceutically acceptable salts of the acidic or basic compounds of the invention can of course be made by conventional procedures, such as by reacting the free base or acid with at least a stoichiometric amount of the desired salt-forming acid or base.

Pharmaceutically acceptable salts of the acidic compounds of the invention include salts with inorganic cations such as sodium, potassium, calcium, magnesium, zinc, and ammonium, and salts with organic bases. Suitable organic bases include N-methyl-D-glucamine, arginine, benzathine, diolamine, olamine, procaine and tromethamine.

Pharmaceutically acceptable salts of the basic compounds of the invention include salts derived from organic or inorganic acids. Suitable anions include acetate, adipate, besylate, bromide, camsylate, chloride, citrate, edisylate, estolate, fumarate, gluceptate, gluconate, glucuronate, hippurate, hyclate, hydrobromide, hydrochloride. iodide, isethionate, lactate, lactobionate, maleate, mesylate, methylbromide, methylsulfate, napsylate, nitrate, oleate, pamoate, phosphate, polygalacturonate, stearate, succinate, sulfate, sulfosalicylate, tannate, tartrate, terephthalate, tosylate and triethiodide.

It is anticipated that the compounds of the invention can be administered by oral or parenteral routes, including intravenous, intramuscular, intraperitoneal, subcutaneous, rectal and topical administration, and inhalation.

For oral administration, the compounds of the invention will generally be provided in the form of tablets or capsules or as an aqueous solution or suspension.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate and lactose. Corn starch and alginic acid are suitable disintegrating agents. Binding agents may include starch and gelatine. The lubricating agent, if present, will generally be magnesium stearate, stearic acid or talc. If desired, the tablets may be coated with a material such as glyceryl monostearate or glyceryl distearate, to delay absorption in the gastrointestinal tract.

Capsules for oral use include hard gelatine capsules in which the active ingredient is mixed with a solid diluent and soft gelatine capsules wherein the active ingredient is mixed with water or an oil such as peanut oil, liquid paraffin or olive oil.

For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate.

Effective doses of the compounds of the present invention may be ascertained be conventional methods. The specific dosage level required for any particular patient will depend on a number of factors, including severity of the condition being treated, the route of administration and the weight of the patient. In general, however, it is anticipated that the daily dose (whether administered as a single dose or as divided doses) will be in the range 0.001 to 5000 mg per day, more usually from 1 to 1000 mg per day, and most usually from 10 to 200 mg per day. Expressed as dosage per unit body weight, a typical dose will be expected to be between 0.01 µg/kg and 50 mg/kg, especially between 10 µg/kg and 10 mg/kg, eg. between 100 µg/kg and 2 mg/kg.

In a further aspect of the present invention there are provided pharmaceutical compositions comprising a compound according to formula (I) and a proton pump inhibitor. Compositions comprising a CCK₂/gastrin antagonist and a proton pump inhibitor are described in International patent application WO93/12817, incorporated herein by reference.

In one aspect of the present invention the proton pump inhibitor is
omeprazole which is 5-methoxy-2-[[(4-methoxy-3,5-dimethyl-2-pyridinyl)-methyl]sulfinyl]-1H-benzimidazole;
BY308;
SK&F 95601 which is 2-[[(3-chloro-4-morpholino-2-pyridyl)methyl]sulfinyl]-5-methoxy-(1H)-benzimidazole;
SK & 96067 which is 3-butyryl-4-(2-methylphenylamino)-8-methoxyquinoline;
5-trifluoromethyl-2-[4-methoxy-3-methyl-2-pyridyl-methyl]-thio-[1H]-benzimidazole;
or pharmaceutically acceptable salts thereof.

These proton pump inhibitors are described and claimed in US Patents 4,472,409 and 4,255,431. These patents are incorporated herein by reference.

In a further aspect of the present invention, the proton pump inhibitor is
lansoprazole which is 2-[[[3-methyl-4-(2,2,2-trifluoroethoxy)-2-pyridinyl]methyl]sulfinyl]-1H-benzimidazole;
pantoprazole which is 5-(difluoromethoxy)-2-[[(3,4-dimethoxy-2-pyridinyl)methyl]sulfinyl]-1H-benzimidazole;
perprazole;
rabeprazole which is 2-[[4-(3-methoxypropoxy)-3-methylpyridin-2-yl]methylsulfinyl]-1H-benzimidazole;
[[4-(2,2,2-trifluoroethoxy)-3-methyl-2-pyridyl]- methyl]sulfenamide;
(Z)-5-methyl-2-[2-(1-naphthyl)ethenyl]-4- piperidinopyridine HCl;
2- (4-cyclohexyloxy-5-methylpyridin-2-yl) -3- (1- naphthyl) -1-propanol;
methyl 2-cyano-3-(ethylthio)-3-(methylthio)-2propenoate;
2-((4-methoxy-2-pyridyl)methylsulphinyl)-5-(1,1,2,2-tetrafluoroethoxy)-1H-benzimidazole sodium;
2-[[[4-(2,2,3,3,4,4,4-heptafluorobutoxy)-2-pyridyl]methyl)sulfinyl]-1H-thieno [3,4-d]imidazole;
2-[[[4-(2,2,2-trifluoroethoxy)-3-methyl-2-pyridyl]methyl]sulfinyl]-1H-benzimidazole;
2-[[[4-(2,2,2-trifluoroethoxy)-3-methyl-2-pyridyl]methyl]sulfinyl]-1H-benzimidazole;
2-methyl-8-(phenylmethoxy)-imidazo(1,2-A)- pyridine-3-acetonitrile;
(2-((2-dimethylaminobenzyl)sulfinyl)-benzimidazole);
4-(N-allyl-N-methylamino)-1-ethyl-8-((5-fluoro-6-methoxy-2-benzimidazolyl) sulfinylmethyl)-1-ethyl 1,2,3,4-tetrahydroquinolone;
2-[[(2-dimethylaminophenyl)methyl]sulfinyl]-4,7-dimethoxy-1H-benz imidazole;
2-[(2-(2-pyridyl)phenyl)sulfinyl)-1H-benzimidazole;
(2-[(2-amino-4-methylbenzyl)sulfinyl]-5-methoxybenzo[d]imidazole;
(4(2-methylpyrrol-3-yl)-2-guanidisothiazole);
4-(4-(3-(imidazole)propoxy)phenyl)-2phenylthiazole;
(E)-2-(2-(4-(3-(dipropylamino)butoxy)phenyl)-ethenyl)benzoxazole;
(E)-2-(2-(4-(3-(dipropylamino)propoxy)phenyl)ethenyl)-benzothiazole;
Benzeneamine, 2-[[(5-methoxy-1H-benzimidazol-2-yl)sulfinyl]methyl)-4-methyl-;
Pumilacidin A;
2,3-dihydro-2-methoxycarbonylamino-1,2-benzisothiazol-3-one;
2-(2-ethylaminophenylmethylsulfinyl)-5,6-dimethoxybenzimidazole;
2-methyl-8-(phenylmethoxy)imidazo[1,2-a)pyridine-3-acetonitrile;
3-amino-2-methyl-8-phenylmethoxyimidazo[1,2-a)-pyrazine HCl;
2-[[(3-chloro-4-morpholino-2-pyridyl)methyl]-sulfinyl)-5-methoxy-(1H)-benzinidazole;
[3-butyryl-4-(2-methylphenylamino)-8-methoxy-quinoline);
2-indanyl 2-(2-pyridyl)-2-thiocarbamoylacetate HCl;
2,3-dihydro-2-(2-pyridinyl)-thiazolo (3,2-a) - benzimidazole;
3-cyanomethyl-2-methyl-8-(3-methyl-2-butenyloxy)- (1,2-a)imidazopyridine;
zinc L-carnosine;
or pharmaceutically acceptable salts thereof.

Rabeprazole is described in US patent 5,045,552. Lansoprazole is described in US patent 4,628,098. Pantoprazole is described in US patent 4,758,579. These patents are incorporated herein by reference.

Preferably, the proton pump inhibitor is selected from (RS)-rabeprazole, (RS)-omeprazole, lansoprazole, pantoprazole, (R)-omeprazole, (S)-omeprazole, perprazole, (R)-rabeprazole, (S)-rabeprazole, or the alkaline salts thereof. The alkaline salts may be, for example, the lithium, sodium, potassium, calcium or magnesium salts.

Compositions of this invention comprising a compound of formula (I) and a proton pump inhibitor may be administered as described above. Preferably the dose of each of the active ingredients in these compositions will be equal to or less than that which is approved or indicated in monotherapy with said active ingredient.

In another aspect of this invention, there is provided a kit comprising a compound of formula (I) and a proton pump inhibitor. The kit is useful as a combined preparation for simultaneous, separate or sequential use in the treatment of patients suffering from gastrointestinal disorders.

In yet a further aspect of the present invention there is provided a method of making a pharmaceutical composition comprising a compound of formula (I) substantially as described herein before and a proton pump inhibitor, comprising mixing said compound and said proton pump inhibitor with a pharmaceutically acceptable carrier or diluent.

The term "hydrocarbyl" is used herein to refer to monovalent groups consisting of carbon and hydrogen. Hydrocarbyl groups thus include alkyl, alkenyl and alkynyl groups (in both straight and branched chain forms), cycloalkyl (including polycycloalkyl groups such as bicyclooctyl and adamantyl), cycloalkenyl and aryl groups, and combinations of the foregoing, such as alkylcycloalkyl, alkylpolycycloalkyl, alkylaryl, alkenylaryl, alkynylaryl, cycloalkylaryl and cycloalkenylaryl groups.

Where reference is made to a carbon atom of a hydrocarbyl group being replaced by a N, O or S atom, what is intended is that 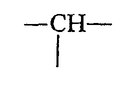 is replaced by 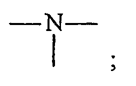 or that -CH₂- is replaced by -O- or -S-.

Where reference is made to an optionally substituted hydrocarbyl group, the hydrocarbyl group is substituted with 1, 2 or 3 groups independently selected from -L-Q
wherein:
L is a bond, or a group of the formula -(CR¹⁷R¹⁸)ᵥ-Y-(CR¹⁷R¹⁸)_{w}, wherein v and w are independently 0, 1, 2 or 3, and Y is a bond, -CR¹⁵=CR¹⁶-, phenyl, furanyl, thiophenyl, pyrrolyl, thiazolyl, imidazolyl, oxazolyl, isoxazolyl, pyrazolyl, isoxazolonyl, piperazinyl, piperidinyl, morpholinyl, pyrrolidinyl, isothiazolyl, triazolyl, oxadiazolyl, thiadiazolyl, pyridyl or pyridazyl;
Q is H, (C₁ to C₆ alkyl)oxy, [*N*-Z](C₁ to C₆ alkyl)oxy(C₁ to C₆ alkyl)amino, thio, (C₁ to C₆ alkyl)thio, carboxy(C₁ to C₆ alkyl)thio, carboxy, carboxy(C₁ to C₆ alkyl), carboxy(C₁ to C₆ alkenyl), [*N*-Z]carboxy(C₁ to C₆ alkyl)amino, carboxy(C₁ to C₆ alkyl)oxy, formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, amino, [*N*-Z](C₁ to C₆ alkyl)amino, aminocarbonyl, (C₁ to C₆ alkyl)aminocarbonyl, di(C₁ to C₆ alkyl)aminocarbonyl, [*N*-Z](C₁ to C₆ alkyl)carbonylamino, C₅ to C₈ cycloalkyl, [*N*-Z](C₁ to C₆ alkyl)carbonyl(C₁ to C₆ alkyl)amino, halo, halo(C₁ to C₆ alkyl), sulfamoyl, [*N*-Z](C₁ to C₆ alkyl)sulfonylamino, (C₁ to C₆ alkyl)sulfonylaminocarbonyl, carboxy(C₁ to C₆ alkyl)sulfonyl, carboxy(C₁ to C₆ alkyl)sulfinyl, tetrazolyl, [*N*-Z]tetrazolylamino, cyano, amidino, amidinothio, SO₃H, formyloxy, formamido, C₃ to C₈ cycloalkyl, (C₁ to C₆ alkyl)sulphamoyl, di(C₁ to C₆ alkyl)sulphamoyl, (C₁ to C₆ alkyl)carbonylaminosulfonyl, 5-oxo-2,5-dihydro[1,2,4]oxadiazolyl, carboxy(C₁ to C₆ alkyl)carbonylamino, tetrazolyl(C₁ to C₆ alkyl)thio, [*N*-Z]tetrazolyl(C₁ to C₆ alkyl)amino, 5-oxo-2,5-dihydro[1,2,4]thiadiazolyl, 5-oxo-1,2-dihydro[1,2,4]triazolyl, [*N*-Z](C₁ to C₆ alkyl)amino(C₁ to C₆ alkyl)amino, or a group of the formula 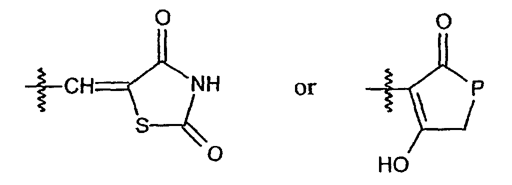
wherein P is O, S or NR¹⁹;
and
Z is H, C₁ to C₆ alkyl, t-butoxycarbonyl, acetyl, benzoyl or benzyl.

The term "alkyl" is used herein to refer to both straight and branched chain forms. Further, the alkyl chain may include multiple bonds. Hence, the term "alkyl" also encompasses alkenyl and alkynyl groups. Likewise, the term "cycloalkyl" also encompasses cycloalkenyl groups. Preferably, alkyl and cycloalkyl groups as used in the present invention do not contain multiple bonds. Where there are preferred alkenyl groups, these are specified as alkenyl groups. However, specific reference to alkenyl groups is not to be construed as any limitation on the definition of alkyl groups as described above.

Where reference is made to dialkyl groups [e.g. di(C₁ to C₆ alkyl)amino groups], it is understood that the two alkyl groups may be the same or different.

In the interests of simplicity, terms which are normally used to refer to monovalent groups (such as "alkyl" or "phenyl") are also used herein to refer to divalent bridging groups which are formed from the corresponding monovalent group by the loss of one hydrogen atom. Whether such a term refers to a monovalent group or to a divalent group will be clear from the context. For example, when L is -(CR¹⁷R¹⁸)ᵥ-Y-(CR¹⁷R¹⁸)_{w}-, it is clear that Y must be a divalent group. Thus, when Y is defined as thiazolyl, for example, this refers to a divalent group having the structure

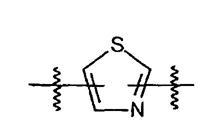

Where, as in this example, a divalent bridging group is formed from a cyclic moiety, the linking bonds may be on any suitable ring atom, subject to the normal rules of valency.
Accordingly, by way of further example, the term pyrrolyl in the definition of Y includes all of the following groups:

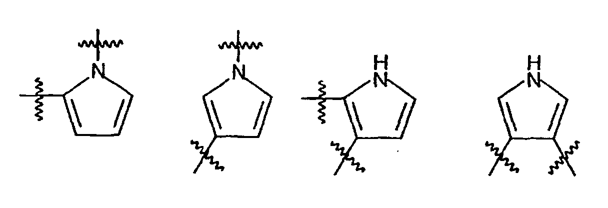

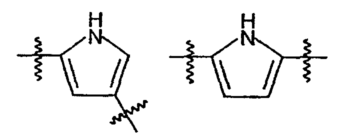

The term "halogen" or "halo" is used herein to refer to any of fluorine, chlorine, bromine and iodine. Most usually, however, halogen substituents in the compounds of the invention are chlorine and fluorine substituents. Groups such as halo(C₁ to C₆ alkyl) includes mono-, di- or tri-halo substituted C₁ to C₆ alkyl groups. Moreover, the halo substitution may be at any position in the alkyl chain.

The prefix [*N*-Z] refers to possible substitution of an amino group in the following compound or substituent name. For example, [*N*-Z]alkylamino refers to groups of the form

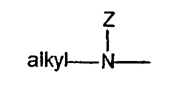

Similarly, [*N*-Z]tetrazolylamino, wherein Z is C₁ to C₆ alkyl, includes groups such as tetrazolyl[*N*-methyl]amino and tetrazolyl[*N*-ethyl]amino. Of course, when Z is H, no substitution is present.

In case there is any doubt, the group named as 5-oxo-2,5-dihydro[1,2,4]oxadiazolyl has the following formula 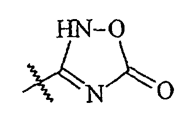 and comprehends tautomeric forms.

The invention is now further illustrated by means of the following Examples.

### Experimental

All reactions were performed under an atmosphere of dry argon unless otherwise stated. Commercially available dichloromethane (DCM), tetrahydrofuran (THF) and *N,N*-dimethylformamide (DMF) were used. In reactions in which anilines were used, where necessary un-reacted aniline was removed either by chromatography or by stirring with excess methylisocyanate polystyrene HL resin (200-400 mesh, 2mmol/g) in DCM (R. J. Booth, *et. al., J. Am. Chem. Soc.,* (1997), **119**, 4882). Flash column chromatography was performed on Merck silica gel 60 (40-63µm) using the reported solvent systems. ¹H NMR spectra were recorded on a Bruker DRX-300 instrument at 300MHz and the chemical shifts (δ_{H}) were recorded relative to an internal standard. (2-Amino-phenyl)-cyclohexyl-methanone was prepared by a published method (M. S. Chambers, *et. al., Bioorg. Med*. *Chem. Lett.* (1993), **3**, 1919), and (2-amino-phenyl)-cyclopentyl-methanone and 1-(2-amino-phenyl)-3-methyl-butan-1-one were prepared by a modification of this method. 2-Bromo-1-cyclopentyl-ethanone and 2-bromo-1-cyclohexyl-ethanone were prepared by a published method (M. Gaudry, A. Marquet, *Org. Synth.*, (1976), **55**, 24), and 2-bromo-1-cyclopropyl-ethanone was prepared by a modification of this method. 2-Bromo-1-(1-methyl-cyclopentyl)-ethanone was prepared by a published method (T. S. Sorensen, *J*. *Am. Chem. Soc.,* (1969), **91,** 6398). Substituted anilines were either obtained commercially, synthesized by the literature method indicated where first mentioned or prepared in a number of steps and from the starting material as indicated, using standard chemical transformations.

### Example 1. 3-{2-[5-Cyclohexyl-1-(3,3-dimethyl-2-oxo-butyl)-2-oxo-1,2-dihydro-3H-1,3,4-benzotriazepin-3-yl]-acetylamino}-benzoic acid methyl ester.

**Step a.** *{*N*'-[(2-Amino-phenyl)-cyclohexyl-methylene]-hydrazino}-acetic acid ethyl ester.* A mixture of (2-amino-phenyl)-cyclohexyl-methanone (20.3g, 0.1mol), ethyl hydrazinoacetate hydrochloride (23.25g, 0.15mol) and pyridine (12.1ml, 0.15mol) was heated at reflux in EtOH (400ml) for 72h. On cooling, un-reacted ethyl hydrazinoacetate hydrochloride crystallised from the solution and was removed by filtration. The filtrate was evaporated and the residue was partitioned between saturated NaHCO₃ (250ml) and EtOAc (250ml). The organic phase was washed with brine (250ml), dried over MgSO₄ then the solvent was evaporated under reduced pressure. The residue was purified by flash column chromatography (EtOAc-hexane (1:4)) to afford {*N'*-[(2-amino-phenyl)-cyclohexyl-methylene]-hydrazino}-acetic acid ethyl ester as a pale yellow foam (21.2g, 71%) and un-reacted (2-amino-phenyl)-cyclohexyl-methanone (2.40g). ¹H NMR (CDCl₃) 7.17 (1H, dt), 6.98 (1H, dd), 6.80 (1H, dt), 6.73 (1H, dd), 5.32 (1H, t), 4.16 (2H, m), 3.95 (2H, br s), 3.89 (2H, m), 2.37 (1H, m), 1.80 (1H, m), 1.75-1.61 (4H, m), 1.33-1.19 (8H, m).

**Step b.** *(5-Cyclohexyl-2-oxo-1,2-dihydro-3*H*-1,3,4-benzotriazepin-3-yl)-acetic acid ethyl ester.* To a solution of the product of step a (23.39g, 77.0mmol) and triethylamine (26.8ml, 0.19mol) in DCM (300ml) at 0°C, a solution of triphosgene (11.4g, 39mmol) in DCM (100ml) was added drop-wise over 1h. The reaction mixture was stirred at this temperature for 1h, washed with H₂O (300ml), saturated NaHCO₃ (300ml) and brine (300ml). The organic phase was dried over MgSO₄, filtered and the solvent was evaporated under reduced pressure. The crude product was re-crystallised from Et₂O-hexane (1:3) to afford the main product, (5-cyclohexyl-2-oxo-1,2-dihydro-3*H*-1,3,4-benzotriazepin-3-yl)-acetic acid ethyl ester (15.8g, 62%), as a yellow solid. Concentration of the mother liquors and re-crystallisation of the residue from EtOAc-hexane (1:9) yielded the minor product, (1-chlorocarbonyl-5-cyclohexyl-2-oxo-1,2-dihydro-3*H*-1,3,4-benzotriazepin-3-yl)-acetic acid ethyl ester (2.1g, 7.0%), as a pale yellow solid.

Major product: *(5-Cyclohexyl-2-oxo-1,2-dihydro-3H-1,3,4-benzotriazepin-3-yl)-acetic acid ethyl ester.* ¹H NMR (CDCl₃) 7.35 (2H, m), 7.12 (1H, t), 6.85 (2H, m), 4.32 (2H, s), 4.18 (2H, m), 2.68 (1H, m), 1.81-1.68 (5H, m), 1.49-1.22 (8H, m).

Minor product: *(1-Chlorocarbonyl-5-cyclohexyl-2-oxo-1,2-dihydro-3*H*-1,3,4-benzotriazepin-3-yl)-acetic acid ethyl ester.* ¹H NMR (CDCl₃) 7.65-7.49 (4H, m), 4.61-4.35 (2H, m), 4.15 (2H, m), 2.86 (1H, m), 2.00-1.22 (10H, m), 1.18 (3H, t).

**Step c.** *[5-Cyclohexyl-1-(3,3-dimethyl-2-oxo-butyl)-2-oxo-1,2-dihydro-3*H*-1,3,4-benzotriazepin-3-yl]-acetic acid ethyl ester.* To an ice cooled solution of the major product of step b (3.29g, 10.0mml) in DMF (30ml) was added sodium hydride (60% dispersion in mineral oil, 480mg, 12.0mmol) in small portions. The mixture was stirred at room temperature for 30min then 1-bromo-3,3-dimethyl-butan-2-one (1.60ml, 12.0mmol) was added. The reaction mixture was stirred at room temperature for 2h, diluted with H₂O (200ml) and extracted with EtOAc (30ml × 3). The combined organic extracts were washed with brine (50ml), dried over MgSO₄, filtered and the solvent was evaporated under reduced pressure. The residue was purified by flash column chromatography (EtOAc-DCM (1:9)) to afford the product as a yellow foam (3.59g, 84%). ¹H NMR (CDCl₃) 7.37 (2H, m), 7.17 (1H, dt), 6.93 (1H, d), 4.66 (2H, s), 4.35 (1H, m), 4.13 (3H, m), 2.74 (1H, m), 1.90-1.70 (6H, m), 1.31-1.16 (16H, m).

**Step d.** *[5-Cyclohexyl-1-(3,3-dimethyl-2-oxo-butyl)-2-oxo-1,2-dihydro-3*H*-1,3,4-benzotriazepin-3-yl]-acetic acid*. A solution of the product of step c (3.57g, 8.20mmol), and 1.0M NaOH (8.70ml, 8.70mmol) in EtOH (30ml) was stirred at room temperature for 16h. The EtOH was evaporated under reduced pressure, the residue was diluted with H₂O (30ml) and acidified to pH 3 with 1N HCl. The mixture was extracted with DCM (30ml × 2), the combined extracts were dried over MgSO₄, filtered and the solvent was evaporated under reduced pressure to afford the product as a pale yellow foam (3.10g, 95%). ¹H NMR (CDCl₃) 11.00 (1H, br s), 7.45 (2H, m), 7.25 (1H, m), 6.97 (1H, dd), 4.68 (2H, m), 4.25 (1H, d), 3.90 (1H, d), 2.80 (1H, m), 2.08-1.61 (6H, m), 1.44-1.18 (13H, m).

**Step e.** To a solution of the product of step d (1.60g, 4.00mmol), and 3-amino-benzoic acid methyl ester (600mg, 4.00mmol) in DMF (20ml) was added 1-hydroxybenzotriazole (HOBt) (810mg, 6.00mmol), 4-dimethylaminopyridine (DMAP) (50mg, 0.40mmol) and 1-(3-dimethylaminopropyl)-3-ethylcarbodiimide hydrochloride (EDC) (1.15g, 6.00mmol). The solution was maintained at room temperature for 16h, diluted with H₂O (100ml) and the reaction mixture was extracted with EtOAc (50ml × 2). The combined extracts were washed with 5% KHSO₄ (60ml), saturated NaHCO₃ (60ml) and brine (60ml). The organic phase was dried over MgSO₄, filtered and the solvent was evaporated under reduced pressure. The residue was triturated with Et₂O to afford the title compound as an off-white solid (1.51g, 71%). ¹H NMR (CDCl₃) 8.49 (1H, s), 7.92 (1H, m), 7.84 (1H, t), 7.74 (1H, dt), 7.48 (2H, m), 7.39-7.27 (2H, m), 7.03 (1H, d), 4.77 (1H, d), 4.60 (1H, d), 4.25 (2H, s), 3.91 (3H, s), 2.80 (1H, m), 2.05-1.73 (6H, m), 1.34-1.17 (13H, m). Found: C 67.56, H 6.83, N, 10.36%; C₃₀H₃₆N₄O₅ requires: C 67.56, H 6.81, N 10.52%.

### Example 2. 3-{2-[5-Cyclohexyl-1-(3,3-dimethyl-2-oxo-butyl)-2-oxo-1,2-dihydro-3H-1,3,4-benzotriazepin-3-yl]-acetylamino}-benzoic acid.

To a solution of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) (1.33g, 2.49mmol) in THF-H₂O (2:1 / 45ml) was added lithium hydroxide monohydrate (318mg, 7.58mmol) and the mixture was stirred at room temperature for 16h. The THF was evaporated under reduced pressure, the aqueous solution was diluted with H₂O (50ml) and acidified to pH 3 with 1N HCl. The reaction mixture was extracted with DCM (30ml × 2), the combined extracts were washed with brine (50ml), dried over MgSO₄, filtered and the solvent was evaporated under reduced pressure to afford the product as an off-white solid (1.28g, 99%). ¹H NMR (DMSO-*d*_{*6*}) 12.89 (1H, br s), 9.99 (1H, s), 8.16 (1H, s), 7.70 (1H, dd), 7.60-7.36 (4H, m), 7.26-7.15 (2H, m), 4.78 (2H, d), 4.30 (1H, d), 3.98 (1H, d), 2.87 (1H, m), 1.80-1.50 (6H, m), 1.34-1.13 (13H, m). The compound was further characterised as the *N*-methyl-D-glucamine salt. Found: C 60.39, H 7.31, N, 9.78%; C₂₉H₃₄N₄O₅•C₇H₁₇NO₅ requires: C 60.57, H 7.20, N 9.81%.

### Example 3. 2-[5-Cyclohexyl-1-(3,3-dimethyl-2-oxo-butyl)-2-oxo-1,2-dihydro-3H-1,3,4-benzotriazepin-3-yl]-N-(3-methylamino-phenyl)-acetamide.

**Step a.** *(3-Nitro-phenyl)-carbamic acid* tert*-butyl ester*. A solution of 3-nitrophenyl isocyanate (14.44g, 88.0mmol) in *tert*-butanol (80ml) was heated at reflux for 2h. After cooling the solvent was evaporated and the residue was dried under high vacuum, washed thoroughly with Et₂O to afford the product as a yellow solid (19.96g, 95%). ¹H NMR (CDCl₃) 8.30 (1H, s), 7.88 (1H, d), 7.71 (1H, d), 7.45 (1H, t), 6.68 (1H, br s), 1.55 (9H, s).

**Step b.** *Methyl-(3-nitro-phenyl)-carbamic acid* tert-*butyl ester*. To an ice-cooled solution of the product of step a (3.57g, 15.0mmol), in DMF (30ml) was added sodium hydride (60% dispersion in mineral oil, 720mg, 18.0mmol) in small portions. After stirring at room temperature for 1h, the reaction mixture was cooled externally with ice and iodomethane (1.4ml, 22.5mmol) was added. The reaction mixture was stirred at room temperature for 2h, H₂O (150ml) was added and extracted with EtOAc (50ml × 2). The combined extracts were washed with brine, dried (MgSO₄), filtered and the solvent was evaporated. The residue was purified by flash column chromatography (EtOAc-DCM (1:9)) to afford the product as a yellow foam (3.34g, 88%). ¹H NMR (CDCl₃) 8.16 (1H, t), 8.00 (1H, m), 7.63 (1H, m), 7.48 (1H, t), 3.34 (3H, s), 1.49 (9H, s).

**Step c.** *(3-Amino-phenyl)-methyl-carbamic acid tert-butyl ester*. A round bottom flask containing the product of step b (3.30g, 13.1mmol), 10% palladium on charcoal (300 mg) and THF-MeOH (1:1 / 50 ml) was evacuated and flushed with hydrogen three times. The mixture was stirred vigorously overnight under an atmosphere of hydrogen. The catalyst was removed by filtration through a pad of celite and the filtrate evaporated to afford the product as a white solid (2.90g, 99%). ¹H NMR (CDCl₃) 7.10 ( 1 H, t), 6.62 (2H, m), 6.50 (1H, m), 3.66 (2H, br s), 3.22 (3H, s), 1.46 (9H, s).

**Step d.** *(3-{2-[5-Cyclohexyl-1-(3,3-dimethyl-2-oxo-butyl)-3-oxo-1,2-dihydro-3*H-*1,3,4-benzotriazepin-3-yl]-acetylamino}-phenyl)-methyl-carbamic acid* tert-*butyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-phenyl)-methyl-carbamic acid *tert*-butyl ester (Example 3, step c) was used in place of 3-amino-benzoic acid methyl ester in step e. ¹H NMR (CDCl₃) 8.29 (1H, s), 7.44 (3H, m), 7.29-7.11 (4H, m), 7.03-6.94 (2H, m), 4.67 (2H, m), 4.23 (2H, m), 3.23 (3H, s), 2.79 (1H, m), 2.05-1.52 (6H, m), 1.45 (9H, s), 1.37-1.23 (13H, m).

**Step e.** A solution of the product of step d (270mg, 0.45mmol) in trifluoroacetic acid (3ml) was stirred at room temperature for 1h. The trifluoroacetic acid was evaporated under reduced pressure, the residue was partitioned between saturated NaHCO₃ (20ml) and EtOAc (20ml). The organic phase was separated and dried over MgSO₄. Filtration and evaporation of the solvent gave the crude product, which was purified by flash column chromatography (EtOAc-DCM (1:9)) to afford the title compound, as a colourless foam (154mg, 68%). ¹H NMR (CDCl₃) 8.07 (1H, s), 7.48 (2H, m), 7.27 (1H, dt), 7.04 (2H, m), 6.91 (1H, t), 6.46(1H, dd), 6.31(1H, dd), 4.68 (2H, m), 4.35 (1H, d), 4.13 (1H, d), 3.72 (1H, br s), 2.80 (4H, m), 2.05-1.72 (6H, m), 1.27 (13H, m). Found: C 68.77, H 7.64, N 13.69%; C₂₉H₃₇N₅O₃ requires: C 69.16, H 7.40, N 13.91%.

### Example 4. 2-[1-(3,3-Dimethyl-2-oxo-butyl)-2-oxo-5-phenyl-1,2-dihydro-3H-1,3,4-benzotriazepin-3-yl]-N-(3-methylamino-phenyl)-acetamide.

**Step a.** *[1-(3,3-Dimethyl-2-oxo-butyl)-2-oxo-5-phenyl-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d), except that 2-amino-benzophenone was used in step a instead of (2-amino-phenyl)-cyclohexyl-methanone. ¹H NMR (CDCl₃) 7.61 (2H, dd), 7.49-7.42 (4H, m), 7.17 (2H, m), 7.05 (1H, d), 4.76 (2H, br m), 4.28 (2H, br d), 1.25 (9H, s).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that [1-(3,3-dimethyl-2-oxo-butyl)-2-oxo-5-phenyl-1,2-dihydro-3*H*-1,3,4-benzotriazepin-3-yl]-acetic acid (Example 4, step a) and (3-amino-phenyl)-methyl-carbamic acid *tert*-butyl ester (Example 3, step c) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.01 (1H, s), 7.63 (2H, m), 7.55 (1H, m), 7.42 (3H, m), 7.25 (2H, m), 7.14 (1H, d), 7.00 (1H, t), 6.90 (1H, t), 6.40 (1H, dd), 6.29 (1H, dd), 4.76 (2H, m), 4.52 (1H, d), 4.34 (1H, d), 3.25 (1H, br s), 2.77 (3H, s), 1.26 (9H, s). Found: C 68.44, H 6.46, N 13.80%; C₂₉H₃₁N₅O₃•0.6H₂O requires: C 68.41, H 6.39, N 13.76%.

### Example 5. 2-[1-(3,3-Dimethyl-2-oxo-butyl)-2-oxo-5-pyridin-2-yl-1,2-dihydro-3H-1,3,4-benzotriazepin-3-yl]-N-(3-methylamino-phenyl)-acetamide.

**Step a.** *[1-(3,3-Dimethyl-2-oxo-butyl)-2-oxo-5-pyridin-2-yl-1,2-dihydro-3*H*-1,3,4-benzotriazepin-3-yl]-acetic acid* was prepared using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d), except that (2-amino-phenyl)-pyridin-2-yl-methanone (G. Semple, *et. al. Synth. Commun*., (1996), **26**, 721) was used in step a instead of (2-amino-phenyl)-cyclohexyl-methanone. ¹H NMR (DMSO-*d*_{*6*}) 12.50 (1H, br s), 8.57 (1H, m), 7.94 (2H, m), 7.50 (2H, m), 7.16 (3H, m), 4.78 (2H, s), 4.25 (2H, br s), 1.15 (9H, s).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that [1-(3,3-dimethyl-2-oxo-butyl)-2-oxo-5-pyridin-2-yl-1,2-dihydro-3*H*-1,3,4-benzotriazepin-3-yl]-acetic acid (Example 5, step a) and (3-amino-phenyl)-methyl-carbamic acid *tert*-butyl ester (Example 3, step c) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.62 (1H, d), 8.10 (1H, d), 8.00 (1H, br s), 7.79 (1H, dt), 7.55 (1H, m), 7.29 (3H, m), 7.13 (1H, d), 7.01 (1H, t), 6.92 (1H, t), 6.48 (1H, dd), 6.30 (1H, dd), 4.68 (2H, br d), 4.45 (2H, br d), 2.80 (1H, br s), 2.78 (3H, s), 1.27 (9H, s). Found: C 67.18, H 6.28, N 16.63%; C₂₈H₃₀N₆O₃ requires: C 67.45, H 6.06, N 16.86%.

### Example 6. N-(3-Methylamino-phenyl)-2-[2-oxo-1-(2-oxo-2-pyrrolidin-1-yl-ethyl)-5-pyridin-2-yl-1,2-dihydro-3H-1,3,4-benzotriazepin-3-yl]-acetamide.

**Step a.** *[2-Oxo-1-(2-oxo-2-pyrrolidin-1-yl-ethyl)-5-pyridin-2-yl-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d), except that (2-amimo-phenyl)-pyridin-2-yl-methanone was used in step a instead of (2-amino-phenyl)-cyclohexyl-methanone and 2-bromo-1-pyrrolidin-1-yl-ethanone (prepared from pyrrolidine and bromoacetyl bromide) replaced 1-bromo-3,3-dimethyl-butan-2-one in step c.

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that [2-oxo-1-(2-oxo-2-pyrrolidin-1-yl-ethyl)-5-pyridin-2-yl-1,2-dihydro-3*H*-1,3,4-benzotriazepin-3-yl]-acetic acid (Example 6, step a) and (3-amino-phenyl)-methyl-carbamic acid *tert*-butyl ester (Example 3, step c) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.61 (1H, d), 8.07 (2H, m), 7.77 (1H, dt), 7.54 (2H, m), 7.36-7.24 (3H, m), 6.98 (1H, t), 6.88 (1H, s), 6.48 (1H, d), 6.28 (1H, d), 4.53-4.34 (4H, br m), 3.52-3.20 (5H, br m), 2.75 (3H, s), 1.97 (2H, m), 1.87 (2H, m). Found: C 65.44, H 6.00, N 18.79%; C₂₈H₂₉N₇O₃ requires: C 65.74, H 5.71, N 19.17%.

### Example 7. N-(3-Methylamino-phenyl)-2-[2-oxo-1-(2-oxo-2-o-tolyl-ethyl)-5-pyridin-2-yl-1,2-dihydro-3H-1,3,4-benzotriazepin-3-yl]- acetamide.

**Step a.** *[2-Oxo-1-(2-oxo-2-*o*-tolyl-ethyl)-5-pyridin-2-yl-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d), except (2-amino-phenyl)-pyridin-2-yl-methanone was used in step a instead of (2-amino-phenyl)-cyclohexyl-methanone and 2-bromo-1-*o*-tolyl-ethanone (H. Shinagawa, *et*. *al*. *Bioorg*. *Med*. *Chem.,* (1997), 5, 601) replaced 1-bromo-3,3-dimethyl-butan-2-one in step c.

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that [2-oxo-1-(2-oxo-2-*o*-tolyl-ethyl)-5-pyridin-2-yl-1,2-dihydro-3*H*-1,3,4-benzotriazepin-3-yl]-acetic acid (Example 7, step a) and (3-amino-phenyl)-methyl-carbamic acid *tert*-butyl ester (Example 3, step c) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.64 (1H, d), 8.08 (1H, d), 8.03 (1H, s), 7.79 (1H, dt), 7.59 (2H, m), 7.39-7.26 (5H, m), 7.19 (2H, m), 7.01 (1H, t), 6.90 (1H, s), 6.47 (1H, dd), 6.30 (1H, dd), 4.98 (2H, s), 4.45 (2H, br m), 3.30 (1H, br s), 2.76 (3H, s), 2.47 (3H, s). Found: C 69.63, H 5.45, N 15.59%; C₃₁H₂₈N₆O₃ requires: C 69.91, H 5.30, N 15.78%.

### Example 8. 1-[1-(3,3-Dimethyl-2-oxo-butyl)-2-oxo-5-pyridin-2-yl-1,2-dihydro-3H-1,3,4-benzotriazepin-3-ylmethyl]-3-(3-methylamino-phenyl)-urea.

**Step a.** *(3-{3-[1-(3,3-Dimethyl-2-oxo-butyl)-2-oxo-5-pyridin-2-yl-1,2-dihydro-3H-1,3,4-benzotriazepin-3-ylmethyl]-ureido}-phenyl)-methyl-carbamic acid tert-butyl ester.* To an ice-cooled solution of [1-(3,3-dimethyl-2-oxo-butyl)-2-oxo-5-pyridin-2-yl-1,2-dihydro-3H-1,3,4-benzotriazepin-3-yl]-acetic acid (Example 5, step a) (390mg, 1.00mmol) and triethylamine (180µl, 1.30mmol) in acetone (3ml) was added ethyl chloroformate (120µl, 1.30mmol). The mixture was stirred for 20min, and a solution of sodium azide (100mg, 1.50mmol) in H₂O (3ml) was added. The reaction mixture was stirred at 0°C for 1h then poured into PhCH₃-H₂O (1:1 / 20ml). The organic phase was separated, dried over MgSO₄, filtered and the filtrate heated at reflux for 1h. After cooling to room temperature the solvent was evaporated under reduced pressure. The residue was dissolved in DCM (5ml) and (3-amino-phenyl)-methyl-carbamic acid tert-butyl ester (Example 3, step c) (220mg, 1.00mmol) was added. The solution was stirred at room temperature for 1h, diluted with DCM (15ml), washed with saturated NaHCO₃ (20ml) and dried over MgSO₄. Filtration and evaporation of the solvent gave the crude product which was purified by flash column chromatography (EtOAc-DCM (1:1)) to afford the product as a colourless foam (166mg, 27%). ¹H NMR (CDCl₃) 8.51 (1H, d), 8.05 (1H, d), 7.85 (1H, d), 7.67 (1H, t), 7.39 (1H, m), 7.25 (2H, m), 7.13-7.05 (4H, m), 6.95 (1H, d), 6.84 (1H, d), 6.18 (1H, br t), 5.04 (1H, br s), 4.86 (1H, br s), 4.65 (1H, br s), 4.32 (1H, br s), 3.15 (3H, s), 1.49 (9H, s), 1.18 (9H, s).

**Step b.** The title compound was obtained by reaction of (3-{3-[1-(3,3-dimethyl-2-oxo-butyl)-2-oxo-5-pyridin-2-yl-1,2-dihydro-3*H*-1,3,4-benzotriazepin-3-ylmethyl]-ureido}-phenyl)-methyl-carbamic acid *tert*-butyl ester (Example 8, step a), in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.56 (1H, d), 8.00 (1H, d), 7.71 (1H, dt), 7.43 (1H, m), 7.30-7.15 (4H, m), 7.02 (2H, m), 6.72 (1H, t), 6.52 (1H, dd), 6.29 (1H, dd), 6.09 (1H, br t), 4.97 (2H, br d), 4.70 (1H, br s), 4.44 (1H, br s), 3.90 (1H, br s), 2.76 (3H, s), 1.23 (9H, s). Found: C 65.55, H 6.27, N 19.00%; C₂₈H₃₁N₇O₃ requires: C 65.48, H 6.08, N 19.09%.

### Example 9. 3-{2-[5-Cyclopentyl-1-(3,3-dimethyl-2-oxo-butyl)-2-oxo-1,2-dihydro-3H-1,3,4-benzotriazepin-3-yl]-acetylamino}-benzoic acid.

**Step a.** *[5-Cyclopentyl-1-(3,3-dimethyl-2-oxo-butyl)-2-oxo-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d), except that (2-amino-phenyl)-cyclopentyl-methanone was used in step a instead of (2-amino-phenyl)-cyclohexyl-methanone. ¹H NMR (DMSO-*d*_{*6*}) 12.30 (1H, br s), 7.51-7.10 (4H, m), 4.77 (2H, s), 4.05 (2H, m), 3.39 (1H, m), 1.74-1.56 (8H, m), 1.12 (9H, s).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that [5-cyclopentyl-1-(3,3-dimethyl-2-oxo-butyl)-2-oxo-1,2-dihydro-3*H*-1,3,4-benzotriazepin-3-yl]-acetic acid (Example 9, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.89 (1H, br s), 9.98 (1H, s), 8.16 (1H, s), 7.71-7.14 (7H, m), 4.79 (2H, m), 4.22 (1H, m), 4.00 (1H, m), 3.42 (1H, m), 1.75-1.54 (8H, m), 1.32-1.19 (9H, s). The compound was further characterised as the *N*-methyl-D-glucamine salt. Found: C 57.08, H 7.45, N 9.60 %; C₂₈H₃₂N₄O₅•C₇H₁₇NO₅•2.0H₂O requires: C 57.13, H 7.26, N 9.52%.

### Example 10. 2-[5-Cyclopentyl-1-(3,3-dimethyl-2-oxo-butyl)-2-oxo-1,2-dihydro-3H-1,3,4-benzotriazepin-3-yl]-[3-(2H-tetrazol-5-yl)-phenyl]-acetamide.

**Step a.** *2,2-Dimethyl-propionic acid 5-(3-{2-[5-cyclopentyl-1-(3,3-dimethyl-2-oxo-butyl)-2-oxo-1,2-dihydro-*3H*-1,3,4-benzotriazepin-3-yl]-acetylamino}-phenyl)-tetrazol-2-ylmethyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclopentyl-1-(3,3-dimethyl-2-oxo-butyl)-2-oxo-1,2-dihydro-3*H*-1,3,4-benzotriazepin-3-yl]-acetic acid (Example 9, step a) and 5-(3-amino-phenyl)-tetrazol-2-ylmethyl ester were used in place of [5-cyclohexyl-1-(3,3-dimethyl-2-oxo-butyl)-2-oxo-1,2-dihydro-3*H*-1,3,4-henzotriazepin-3-yl]-acetic acid (Example 1, step d) and 3-amino-benzoic acid methyl ester respectively in step e. ¹H NMR (CDCl₃) 8.00 (1H, s), 7.55-7.44 (3H, m), 7.27-7.22 (3H, m), 7.00 (2H, m), 6.29 (2H, s), 4.75 (1H, d), 4.65 (1H, d), 4.25 (1H, m), 4.11 (1H, m), 3.29 (1H, m), 2.00-1.30 (8H, m), 1.26-1.19 (18H, m).

**Step b.** 2,2-Dimethyl-propionic acid 5-(3-{2-[5-cyclopentyl-1-(3,3-dimethyl-2-oxo-butyl)-2-oxo-1,2-dihydro-3*H*-1,3,4-benzotriazepin-3-yl]-acetylamino}-phenyl)-tetrazol-2-ylmethyl ester (Example 10, step a) (120mg, 0.22mmol) was stirred overnight in saturated methanolic ammonia solution (10ml) at room temperature. After concentration *in vacuo*, the residue was dissolved in H₂O-MeOH (10:1 / 22ml) and acidified to pH 3 by the addition of 5% KHSO₄ solution. The title compound was isolated as a light pink solid by filtration of the reaction mixture and dried *in vacuo* (81 mg, 68%). ¹H NMR (CDCl₃) 8.61 (1H, s), 7.50-7.44 (3H, m), 7.31-7.24 (2H, m), 7.14 (1H, m), 6.97 (2H, m), 4.95 (1H, d), 4.65 (1H, d), 4.17 (2H, m), 3.30 (1H, m), 1.80-1.58 (8H, m), 1.15 (9H, m). The compound was further characterised as the *N*-methyl-D-glucamine salt. Found: C 54.46, H 7.13, N 16.28%; C₂₈H₃₂N₈O₃•C₇H₁₇NO₅•2.5H₂O requires: C 54.67, H 7.07, N 16.39%.

### Example 11. 2-[5-Cyclopentyl-1-(3,3-dimethyl-2-oxo-butyl)-2-oxo-1,2-dihydro-3H-1,3,4-benzotriazepin-3-yl]-N-(3-methylaminophenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclopentyl-1-(3,3-dimethyl-2-oxo-butyl)-2-oxo-1,2-dihydro-3*H*-1,3,4-benzotriazepin-3-yl]-acetic acid (Example 9, step a) and (3-amino-phenyl)-methyl-carbamic acid *tert*-butyl ester (Example 3, step c) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. The product was characterised as the hydrochloride salt. ¹H NMR (DMSO-*d*_{*6*}) 9.89 (1H, br s), 7.84-7.40 (6H, m), 7.20 (1H, m), 6.66 (1H, m), 4.78 (2H, m), 3.98 (1H, m), 3.68 (1H, m), 3.35 (1H, m), 2.73 (3H, s), 1.85-1.33 (8H, m), 1.13 (9H, s).

### Example 12. 3-{2-[1-(3,3-Dimethyl-2-oxo-butyl)-5-isobutyl-2-oxo-1,2-dihydro-3H-1,3,4-benzotriazepin-3-yl]-acetylamino}-benzoic acid.

**Step a.** *[1-(3,3-Dimethyl-2-oxo-butyl)-5-isobutyl-2-oxo-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d), except that 1-(2-amino-phenyl)-3-methyl-butan-1-one was used in step a instead of (2-amino-phenyl)-cyclohexyl-methanone. ¹H NMR (CDCl₃) 7.47-7.39 (2H, m), 7.24 (1H, m), 6.98 (1H, d), 4.60 (2H, br m), 4.10 (2H, br m), 2.60 (2H, br m), 1.93 (1H, m), 1.25 (9H, s), 0.95 (6H, br m).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [1-(3,3-dimethyl-2-oxo-butyl)-5-isobutyl-2-oxo-1,2-dihydro-3*H*-1,3,4-benzotriazepin-3-yl]-acetic acid (Example 12, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 13.0 (1H, br), 10.01 (1H, s), 8.17 (1H, t), 7.70 (1H, d), 7.60-7.49 (3H, m), 7.38 (1H, t), 7.24 (1H, t), 7.13 (1H, d), 4.74 (2H, br m), 4.35 (1H, br m), 4.02 (1H, br m), 2.85 (1H, br m), 2.35 (1H, br m), 1.74 (1H, m), 1.15 (9H, s), 0.85 (6H, br m). The compound was further characterised as the *N*-methyl-D-glucamine salt. Found: C 56.58, H 7.55, N 9.54%; C₂₇H₃₂N₄O₅•C₇H₁₇NO₅•2.0H₂O requires C 56.42, H 7.38, N 9.68%.

### Example 13. 2-[1-(3,3-Dimethyl-2-oxo-butyl)-5-isobutyl-2-oxo-1,2-dihydro-3H-1,3,4-benzotriazepin-3-yl]-N-(3-methylaminophenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [1-(3,3-dimethyl-2-oxo-butyl)-5-isobutyl-2-oxo-1,2-dihydro-3*H*-1,3,4-benzotriazepin-3-yl]-acetic acid (Example 11, step a) and (3-amino-phenyl)-methyl-carbamic acid *tert*-butyl ester (Example 3, step c) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. The compound was further characterised as the hydrochloride salt. ¹H NMR (DMSO-*d*_{*6*}) 10.03 (1H, br s), 7.56-7.46 (3H, m), 7.28-7.20 (3H, m), 7.13 (1H, d), 6.89 (1H, d), 4.73 (2H, br s), 4.10 (2H, br), 3.85 (1H, br m), 2.78 (3H, s), 2.30 (1H, br m), 1.74 (1H, m), 1.15 (9H, s), 0.86 (6H, br m). Found: C 61.02, H 7.35, N 13.18%; C₂₇H₃₅N₅O₃•H₂O•HCl requires C 60.95, H 7.20, N 13.16%.

### Example 14. 3-{2-[5-Cyclohexyl-1-methyl-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that iodomethane was used in step c instead of 1-bromo-3,3-dimethyl-butan-2-one, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.90 (1H, br s), 10.03 (1H, s), 8.16 (1H, s), 7.69 (1H, d), 7.60-7.50 (3H, m), 7.38 (1H, t), 7.31-7.22 (2H, m), 4.20 (2H, br d), 3.14 (3H, s), 2.85 (1H, m), 1.64-1.04 (10H, m). The compound was further characterised as the *N*-methyl-D-glucamine salt. Found: C 56.92, H 7.12, N, 10.58%; C₂₄H₂₆N₄O₄•C₇H₁₇NO₅•1.4H₂O requires: C 56.82, H 7.05, N 10.69%.

### Example 15. 3-{2-[5-Cyclohexyl-2-oxo-1-(2-oxo-2-o-tolyl-ethyl)-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2-bromo-1-*o*-tolyl-ethanone was used in step c instead of 1-bromo-3,3-dimethyl-butan-2-one, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 13.00 (1H, br s), 9.98 (1H, s), 8.15 (1H, s), 7.77 (1H, d), 7.70 (1H, dd), 7.60-7.51 (3H, m), 7.39 (2H, m), 7.26 (4H, m), 5.06 (2H, m), 4.32 (1H, d), 3.98 (1H, d), 2.85 (1H, m), 2.29 (3H, s), 1.65-1.54 (6H, m), 1.36-1.14 (4H, m). The compound was further characterised as the N-methyl-D-glucamine salt. Found: C 60.58, H 6.86, N, 8.86%; C₃₂H₃₂N₄O₅•C₇H₁₇NO₅•1.4H₂O requires: C 60.45, H 6.76, N 9.04%.

### Example 16. 3-{2-[5-Cyclohexyl-1-(3-methyl-butyl)-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 1-bromo-3-methyl-butane was used in step c instead of 1-bromo-3,3-dimethyl-butan-2-one, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*₆) 13.00 (1H, br s), 9.99 (1H, s), 8.14 (1H, s), 7.68 (1H, dd), 7.56 (3H, m), 7.40 (2H, m), 7.27 (1H, d), 4.33 (1H, d), 3.98 (2H, m), 3.60 (1H, m), 2.85 (1H, m), 1.65-1.06 (13H, m), 0.77 (6H, m). The compound was further characterised as the *N*-methyl-D-glucamine salt. Found: C 59.05, H 7.81, N, 10.02%; C₂₈H₃₄N₄O₄•C₇H₁₇NO₅•1.3H₂O requires: C 59.23, H 7.62, N 9.87%.

### Example 17. 3-{2-[1-(2-Adamantan-1-yl-2-oxo-ethyl)-5-cyclohexyl-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 1-adamantan-1-yl-2-bromo-ethanone was used in step c instead of 1-bromo-3,3-dimethyl-butan-2-one, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.51 (1H, s), 8.04 (1H, d), 7.84 (1H, s), 7.80 (1H, d), 7.52-7.23 (4H, m), 7.03 (1H, d), 4.75 (1H, d), 4.59 (1H, d), 4.26 (2H, m), 2.80 (1H, m), 2.07-1.70 (21H, m), 1.27 (4H, m). The compound was further characterised as the *N*-methyl-D-glucamine salt. Found: C 60.07, H 7.58, N, 8.37%; C₃₅H₄₀N₄O₅•C₇H₁₇NO₅•2.7H₂O requires: C 60.05, H 7.48, N 8.34%.

### Example 18. 3-{2-[5-Cyclohexyl-1-(2-ethoxy-ethyl)-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that bromoethyl-ethyl ether was used in step c instead of 1-bromo-3,3-dimethyl-butan-2-one, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.45 (1H, s), 7.97 (1H, dd), 7.80 (1H, d), 7.63 (1H, t), 7.57 (1H, dt), 7.48 (1H, d), 7.37 (3H, m), 4.44 (1H, d), 4.34 (1H, m), 4.16 (1H, d), 3.75 (1H, m), 3.55 (2H, m), 3.38 (2H, m), 2.81 (1H, m), 2.02-1.67 (6H, m), 1.28 (4H, m), 1.08 (3H, t). The compound was further characterised as the *N*-methyl-D-glucamine salt. Found: C 56.72, H 7.46, N, 9.69%; C₂₇H₃₂N₄O₅•C₇H₁₇NO₅•1.8H₂O requires: C 56.72, H 7.36, N 9.73%.

### Example 19. 3-{2-[5-Cyclohexyl-2-oxo-1-(terahydro-pyran-2-ylmethyl)-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2-bromomethyl-tetrahydro-pyran was used in step c instead of 1-bromo-3,3-dimethyl-butan-2-one, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.45 (1H, d), 7.98 (1H, d), 7.79 (1H, d), 7.64-7.54 (2H, m), 7.49-7.33 (3H, m), 7.25 (1H, m), 4.47-4.12 (3H, m), 3.80 (1H, m), 3.55-3.30 (2H, m), 3.23 (1H, m), 2.82 (1H, m), 2.05-1.20 (16H, m). The compound was further characterised as the *N*-methyl-D-glucamine salt. Found: C 57.71, H 7.53, N, 9.14%; C₂₉H₃₄N₄O₅•C₇H₁₇NO₅•2.1H₂O requires: C 57.54, H 7.40, N 9.32%.

### Example 20. 3-{2-[5-Cyclohexyl-2-oxo-1-(2-oxo-2-pyrrolidin-1-yl-ethyl)-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2-bromo-1-pyrrolidin-1-yl-ethanone was used in step c instead of 1-bromo-3,3-dimethyl-butan-2-one, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.59 (1H, s), 8.02 (1H, d), 7.85 (1H, s), 7.75 (1H, d), 7.48 (2H, m), 7.33 (3H, m), 4.50 (1H, d), 4.35 (1H, d), 4.80 (2H, s), 3.53 (4H, m), 2.78 (1H, m), 2.05-1.70 (10H, m), 1.26 (4H, m). The compound was further characterised as the *N*-methyl-D-glucamine salt. Found: C 56.43, H 7.30, N, 10.86%; C₂₉H₃₃N₅O₅•C₇H₁₇NO₅•2.3H₂O requires: C 56.34, H 7.16, N 10.95%.

### Example 21. 3-{2-[5-Cyclohexyl-2-oxo-1-(2-oxo-propyl)-1,2-dihydro-3H-1,3,4-benzotriazepin-3-yl]-acetylamino}-benzoic acid.

**Step a.** *[5-Cyclohexyl-2-oxo-1-(2-oxo-propyl)-1,2-dihydro-3*H*-1,3,4-benzotriazepin-3-yl]-acetic acid ethyl ester.* To a solution of (5-cyclohexyl-2-oxo-1,2-dihydro-3*H*-1,3,4-benzotriazepin-3-yl)-acetic acid ethyl ester (Example 1, major product of step b) (300mg, 1.00mmol in MeCN (5ml) were added K₂CO₃ (166mg, 1.20mmol), KI (20mg) and 1-chloro-propan-2-one (90µl, 1.10mmol). The reaction mixture was heated at reflux for 48h, then 1-chloro-propan-2-one (180µl, 2.20mmol) was added and heating continued for 16h. The reaction mixture was filtered and the filtrate was evaporated. The residue was partitioned between saturated NaHCO₃ (30ml) and EtOAc (30ml). The organic phase was dried (MgSO₄), filtered and the solvent was evaporated. The crude product was purified by flash column chromatography (EtOAc-DCM (1:19)) to afford the product as a colourless foam (297mg, 77%). ¹H NMR (CDCl₃) 7.40 (2H, m), 7.19 (1H, t), 6.98 (1H, d), 4.50-4.11 (6H, m), 2.73 (1H, m), 2.19 (3H, s), 1.98-1.60 (6H, m), 1.26 (7H, m).

**Step b.** The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-2-oxo-1-(2-oxo-propyl)-1,2-dihydro-3*H*-1,3,4-benzotriazepin-3-yl]-acetic acid ethyl ester (Example 21, step a) was used in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c) in step d, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.55 (1H, s), 7.99 (1H, d), 7.81 (2H, m), 7.54-7.31 (4H, m), 7.09 (1H, d), 4.66 (1H, d), 4.43 (1H, d), 4.27 (2H, m), 2.82 (1H, m), 2.21 (3H, s), 2.07-1.73 (6H, m), 1.24 (4H, m). The compound was further characterised as the *N*-methyl-D-glucamine salt. Found: C 54.51, H 7.23, N, 9.88%; C₂₆H₂₈N₄O₅•C₇H₁₇NO₅•2.9H₂O requires: C 54.72, H 7.07, N 9.67%.

### Example 22. 3-{2-[5-Cyclohexyl-1-(2-cyclopentyl-2-oxo-ethyl)-2-oxo-1,2-dihydro-3H-1,3,4-benzotriazepin-3-yl]-acetylamino}-benzoic acid.

**Step a.** *[5-Cyclohexyl-1-(2-cyclopentyl-2-oxo-ethyl)-2-oxo-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that 2-bromo-cyclopentyl-ethanone was used in step c instead of 1-bromo-3,3-dimethyl-butan-2-one. ¹H NMR (CDCl₃) 11.00 (1H, br s), 7.45 (2H, m), 7.25 (1H, m), 7.01 (1H, d), 4.56 (2H, d), 4.23 and 3.95 (2H, 2xd), 2.97 (1H, m), 2.81 (1H, m), 2.03-1.58 (13H, m), 1.30 (5H, m).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.52 (1H, s), 8.02 (1H, dd), 7.85 (1H, s), 7.81 (1H, d), 7.49 (2H, m), 7.41 (1H, t), 7.32 (1H, t), 7.08 (1H, d), 4.69 (1H, d), 4.46 (1H, d), 4.27 (2H, m), 2.96 (1H, m), 2.81 (1H, m), 2.05-1.61 (13H, m), 1.29 (5H, m). The compound was further characterised as the *N*-methyl-D-glucamine salt. Found: C 58.44, H 7.42, N, 9.18%; C₃₀H₃₄N₄O₅•C₇H₁₇NO₅•1.9H₂O requires: C 58.43, H 7.27, N 9.21 %.

### Example 23. 2-[5-Cyclohexyl-1-(3,3-dimethyl-2-oxo-butyl)-2-oxo-1,2-dihydro-3H-1,3,4-benzotriazepin-3-yl]-N-[3-(2H-tetrazol-5-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2,2-dimethyl-propionic acid 5-(3-amino-phenyl)-tetrazol-2-ylmethyl ester was used in place of 3-amino-benzoic acid methyl ester in step e, followed by reaction of the product obtained, in place of 2,2-dimethyl-propionic acid 5-(3-{2-[5-cyclopentyl-1-(3,3-dimethyl-2-oxo-butyl)-2-oxo-1,2-dihydro-3*H*-1,3,4-benzotriazepin-3-yl]-acetylamino}-phenyl)-tetrazol-2-ylmethyl ester (Example 10, step a), according to the method of Example 10, step b. ¹H NMR (CDCl₃) 8.86 (1H, s), 8.18 (1H, m), 7.79 (1H, m), 7.46-7.24 (6H, m), 6.97 (1H, m), 4.73 (1H, d), 4.63 (1H, d), 4.27 (1H, d), 4.22 (1H, d), 2.77 (1H, m), 1.97-1.68 (6H, m), 1.24-1.11 (13H, m). The compound was further characterised as the *N*-methyl-D-glucamine salt. Found: C 55.36, H 7.36, N, 16.01%; C₂₉H₃₄N₈O₃•C₇H₁₇NO₅•2.5H₂O requires: C 55.23, H 7.21, N 16.10%.

### Exauiple 24. 2-[5-Cyclohexyl-1-(3,3-dimethyl-2-oxo-butyl)-2-oxo-1,2-dihydro-3H-1,3,4-benzotriazepin-3-yl]-N-m-tolyl-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *m*-toluidine was used instead of 3-amino-benzoic acid methyl ester in step e. ¹H NMR (CDCl₃) 8.19 (1H, s), 7.48 (2H, m), 7.46 (2H, m), 7.28 (2H, m), 7.15 (1H, d), 6.87 (1H, m), 4.72 (1H, d), 4.65 (1H, d), 4.28 (1H, d), 4.20 (1H, d), 2.80 (1H, m), 2.30 (3H, s), 1.76-1.70 (6H, m), 1.29-1.19 (13H, m). Found: C 70.23, H 7.63, N 11.09 %; C₂₉H₃₆N₄O₃•0.5H₂O requires: C 69.99, H 7.49, N 11.26%.

### Example 25. 2-[5-Cyclohexyl-1-(3,3-dimethyl-2-oxo-butyl)-2-oxo-1,2-dihydro-3H-1,3,4-benzotriazepin-3-yl]-N-phenyl-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that aniline was used instead of 3-amino-benzoic acid methyl ester in step e. ¹H NMR (CDCl₃) 8.20 (1H, s), 7.48-7.41 (5H, m), 7.38-7.27 (2H, m), 7.03 (2H, m), 4.70 (1H, d), 4.64 (1H, d), 4.32 (1H, d), 4.18 (1H, d), 2.78 (1H, m), 2.00-1.53 (6H, m), 1.32-1.23 (13H, m). Found: C 69.45, H 7.37, N 11.39 %; C₂₈H₃₄N₄O₃•0.5H₂O requires: C 69.54, H 7.29, N 11.58%.

### Example 26. 2-[5-Cyclohexyl-1-(3,3-dimethyl-2-oxo-butyl)-2-oxo-1,2-dihydro-3H-1,3,4-benzotriazepin-3-yl]-N-(3-methanesulfonylamino-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *N*-(3-amino-phenyl)-methanesulfonamide was used instead of 3-amino-benzoic acid methyl ester in step e. ¹H NMR (CDCl₃) 8.51 (1H, s), 7.61 (1H, s), 7.45 (2H, m), 7.29 (2H, m), 7.06 (4H, m), 4.75 (1H, d), 4.62 (1H, d), 4.26 (2H, m), 2.98 (3H, s), 2.79 (1H, m), 2.00-1.50 (6H, m), 1.32-1.23 (13H, m). Found: C 56.14, H 6.86, N 10.97 %; C₂₉H₃₇N₅O₅S•3.0H₂O requires: C 56.02, H 6.97, N 11.26%.

### Example 27. 2-[5-Cyclohexyl-1-(3,3-dimethyl-2-oxo-butyl)-2-oxo-1,2-dihydro-3H-1,3,4-benzotriazepin-3-yl]-N-(3-pyrrolidin-1-yl-phenyl)-acetamide.

The title compound was obtained by the method used in the preparation of 3-{2-(5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-pyrrolidin-1-yl-phenylamine (prepared in two steps from 3-nitro-aniline) was used instead of 3-amino-benzoic acid methyl ester in step e. ¹H NMR (CDCl₃) 8.01 (1H, s), 7.49 (2H, m), 7.24 (1H, m), 7.04 (2H, m), 6.72 (1H, s), 6.51 (1H, d), 6.26 (1H, d), 4.69 (2H, m), 4.42 (1H, d), 4.12 (1H, d), 3.25 (4H, m), 2.77 (1H, m), 2.00-1.69 (10H, m), 1.28-1.21 (13H, m). The compound was further characterised as the hydrochloride salt. Found: C 57.06, H 6.38, N 10.59 %; C₃₂H₄₁N₅O₉•HCl requires: C 56.82, H 6.26, N 10.36%.

### Example 28. 4-{2-[5-Cyclohexyl-1-(3,3-dimethyl-2-oxo-butyl)-2-oxo-1,2-dihydro-3H-1,3,4-benzotriazepin-3-yl]-acetylamino}-benzoic acid methyl ester.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 4-amino-benzoic acid methyl ester was used instead of 3-amino-benzoic acid methyl ester in step e. ¹H NMR (CDCl₃) 8.57 (1H, s), 7.98 (2H, d), 7.50 (4H, m), 7.24 (1H, m), 7.03 (1H, d), 4.69 (1H, d), 4.59 (1H, d), 4.22 (2H, m), 3.89 (3H, s), 2.79 (1H, m), 2.00-1.69 (6H, m), 1.28-1.21 (13H, m). Found: C 66.21, H 6.98, N 10.42%; C₃₀H₃₆N₄O₅•5.0H₂O requires: C 66.52, H 6.88, N 10.34%.

### Example 29. (3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-phenyl)-acetic acid methyl ester was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.28 (1H, s), 7.48 (2H, m), 7.34-7.22 (4H, m), 7.00 (2H, m), 4.72 (1H, d), 4.64 (1H, d), 4.28 (1H, d), 4.15 (1H, d), 3.48 (2H, s), 2.70 (1H, m), 1.79-1.36 (6H, m), 1.22-1.05 (13H, m). The compound was further characterised as the *N*-methyl-D-glucamine salt. Found: C 58.04, H 7.73, N 9.14%; C₃₀H₃₆N₄O₅•C₇H₁₇NO₅•2.0H₂O requires: C 58.18, H, 7.52, N 9.17%.

### Example 30. 2-[5-Cyclohexyl-1-(3,3-dimethyl-2-oxo-butyl)-2-oxo-1,2-dihydro-3H-1,3,4-benzotriazepin-3-yl]-N-(3-methoxy-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *m*-anisidine was used instead of 3-amino-benzoic acid methyl ester in step e. ¹H NMR (CDCl₃) 8.22 (1H, m), 7.45-7.30 (2H, m), 7.27 (1H, m), 7.20-7.12 (2H, m), 7.03 (1H, d), 6.84 (1H, d), 6.63 (1H, d), 4.73 (1H, d), 4.64 (1H, d), 4.28 (1H, d). 4.19 (1H, d), 3.79 (3H, s), 2.80 (1H, m), 1.76-1.55 (6H, m), 1.36-1.24 (13H, m). Found C 68.01, H 7.48, N 11.01; C₂₉H₃₆N₄O₄•0.5H₂O requires C 67.81, H, 7.26, N 10.91%

### Example 31. 2-[5-Cyclohexyl-1-(3,3-dimethyl-2-oxo-butyl)-2-oxo-1,2-dihydro-3H-1,3,4-benzotriazepin-3-yl]-N-p-tolyl-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *p*-toluidine was used instead of 3-amino-benzoic acid methyl ester in step e. ¹H NMR (CDCl₃) 8.11 (1H, s), 7.47 (2H, m), 7.29-7.25 (2H, m), 7.08-7.00 (4H, m), 4.71 (1H, d), 4.64 (1H, d), 4.32 (1H, d), 4.16 (1H, d), 2.77 (1H, m), 2.29 (3H, s), 1.86-1.53 (6H, m), 1.33-1.21 (13H, m). Found C 71.49, H 7.54, N 11.25; C₂₉H₃₆N₄O₃ requires C 71.28, H, 7.43, N 11.47%

### Example 32. 2-[5-Cyclohexyl-1-(3,3-dimethyl-2-oxo-butyl)-2-oxo-1,2-dihydro-3H-1,3,4-benzotriazepin-3-yl]-N-(4-methoxy-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *p*-anisidine was used instead of 3-amino-benzoic acid methyl ester in step e. ¹H NMR (CDCl₃) 8.11 (1H, m), 7.47 (2H, m), 7.32-7.24 (3H, m), 7.01 (1H, d), 6.82 (2H, m), 4.74 (1H, d), 4.60 (1H, d), 4.31 (1H, d), 4.16 (1H, d), 3.77 (3H, s), 2.78 (1H, m), 2.03-1.71 (6H, m), 1.36-1.13 (13H, m). Found C 67.74, H 7.53, N 10.67; C₂₉H₃₆N₄O₄•0.5H₂O requires C 67.81, H, 7.26, N 10.91%

### Example 33. 2-[5-Cyclohexyl-1-(3,3-dimethyl-2-oxo-butyl)-2-oxo-1,2-dihydro-3H-1,3,4-benzotriazepin-3-yl]-N-(3-dimethylamino-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *m-N,N*-dimethylaminoaniline was used instead of 3-amino-benzoic acid methyl ester in step e. ¹H NMR (CDCl₃) 8.08 (1H, s), 7.50 (2H, m), 7.28 (1H, m), 7.10 (2H, m), 6.88 (1H, m), 6.64 (1H, m), 6.45 (1H, m), 4.69 (1H, d), 4.67 (1H, d), 4.36 (1H, d), 4.14 (1H, d), 2.95 (6H, m), 2.74 (1H, m), 2.00-1.71 (6H, m), 1.36-1.17 (13H, m). The compound was further characterised as the hydrochloride salt. Found C 61.26, H 7.58, N 11.82; C₃₀H₃₉N₅O₃•HCl•2.0H₂O requires C 61.05, H, 7.51, N 11.86%

### Example 34. (6-{2-[5-Cyclohexyl-1-(3,3-dimethyl-2-oxo-butyl)-2-oxo-1,2-dihydro-3H-1,3,4-benzotriazepin-3-yl]-acetylamino}-2,3-dihydro-indol-1-yl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (6-amino-2,3-dihydro-indol-1-yl)-acetic acid methyl ester (prepared in two steps from 6-nitro-2,3-dihydro-1*H*-indole), was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.40 (1H, br s), 9.38 (1H, s), 7.53 (2H, m), 7.18 (2H, m), 6.86 (1H, d), 6.65 (1H, d), 6.54 (1H, s), 4.78 (2H, m), 4.25 (1H, d), 3.90 (1H, d), 3.78 (2H, s), 3.46 (2H, m), 2.84 (3H, m), 1.86-1.55 (6H, m), 1.25-1.17 (13H, m). The compound was further characterised as the N-methyl-D-glucamine salt. Found C 56.11, H 7.64, N 9.87; C₃₂H₃₉N₅O₅•C₇H₁₇NO₅•3.5H₂O requires C 56.30, H, 7.63, N 10.10%.

### Example 35. 2-[5-Cyclohexyl-1-(3,3-dimethyl-2-oxo-butyl)-2-oxo-1,2-dihydro-3H-1,3,4-benzotriazepin-3-yl]-N-(4-fluoro-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 4-fluoroaniline was used instead of 3-amino-benzoic acid methyl ester in step e. ¹H NMR (CDCl₃) 8.31 (1H, s), 7.48-7.28 (5H, m), 6.96 (3H, m), 4.75 (1H, d), 4.55 (1H, d), 4.21 (2H, m), 2.74 (1H, m), 1.90-1.65 (6H, m), 1.34-1.15 (13H, m). Found C 66.98, H 7.09, N 10.88; C₂₈H₃₃FN₄O₃•0.5H₂O requires C 67.04, H, 6.82, N 11.16%.

### Example 36. 2-[5-Cyclohexyl-1-(3,3-dimethyl-2-oxo-butyl)-2-oxo-1,2-dihydro-3H-1,3,4-benzotriazepin-3-yl]-N-{4-(methyl-(2H-tetrazol-5-yl)-amino]-phenyl}-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2,2-dimethyl-propionic acid 5-[(3-amino-phenyl)-methyl-amino]-tetrazol-2-ylmethyl ester (J. L. Castro *et al. J. Med. Chem*. (1996), **39**, 842) was used in place of 3-amino-benzoic acid methyl ester in step e, followed by reaction of the product obtained, in place of 2,2-dimethyl-propionic acid 5-(3-{2-[5-cyclopentyl-1-(3,3-dimethyl-2-oxo-butyl)-2-oxo-1,2-dihydro-3*H*-1,3,4-benzotriazepin-3-yl]-acetylamino}-phenyl)-tetrazol-2-ylmethyl ester (Example 10, step a), according to the method of Example 10, step b. ¹H NMR (DMSO-*d*_{*6*}) 9.84 (1H, s), 7.55-7.05 (8H, m), 4.78 (2H, m), 3.96 (1H, m), 3.55 (1H, m), 3.39 (3H, s), 2.86 (1H, m), 1.85-1.65 (6H, m), 1.33-1.10 (13H, m). The compound was further characterised as the *N*-methyl-D-glucamine salt. Found C 57.86, H 7.31, N 18.31; C₃₀H₃₇N₉O₃•C₇H₁₇NO₅ requires C 57.94, H, 7.10, N 18.26%.

### Example 37. N-(3-Cyano-phenyl)-2-[5-cyclohexyl-1-(3,3-dimethyl-2-oxo-butyl)-2-oxo-1,2-dihydro-3H-1,3,4-benzotriazepin-3-yl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-aminobenzonitrile was used instead of 3-amino-benzoic acid methyl ester in step e. ¹H NMR (CDCl₃) 8.76 (1H, s), 7.83 (2H, m), 7.50-7.29 (5H, m), 7.02 (1H, m), 4.81 (1H, d), 4.48 (1H, d), 4.28 (1H, d), 4.14 (1H, d), 2.79 (1H, m), 1.91-1.65 (6H, m), 1.36-1.17 (13H, m). Found C 66.32, H 6.89, N 13.39; C₂₉H₃₃N₅O₃•1.5H₂O requires C 66.14, H, 6.89, N 13.29%.

### Example 38. (3-{2-[5-Cyclohexyl-1-(3,3-dimethyl-2-oxo-butyl)-2-oxo-1,2-dihydro-3H-1,3,4-benzotriazepin-3-yl]-acetylamino}-phenylsulfanyl)-acetic acid.

The title compound was obtained by the method used in the preparation of 3-{2-[5-cyclohexyl-1-(3,3-dimethyl-2-oxo-butyl)-3-oxo-1,2-dihydro-3*H*-1,3,4-benzotriazepin-3-yl]-acetylamino}-benzoic acid methyl ester (Example 1) except that (3-amino-phenylsulfanyl)-acetic acid ethyl ester (S. Hagishita *et al*. *Bioorg. Med*. *Chem*. (1997), **5**, 1433) was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (DMSO-*d*_{*6*}) 12.60 ( 1 H, br s), 9.81 (1H, s), 7.50 (3H, m), 7.27-7.21 (4H, m), 6.97 (1H, m), 4.78 (2H, m), 4.27 (1H, d), 3.96 (1H, d), 3.72 (2H, s), 2.86 (1H, m), 1.83-1.65 (6H, m), 1.29-1.17 (13H, m). The compound was further characterised as the *N*-methyl-D-glucamine salt. Found C 54.50, H 7.36, N 8.44; C₃₀H₃₆N₄O₅S•C₇H₁₇NO₅•3.0H₂O requires C 54.59, H, 7.30, N 8.60%.

### Example 39. 5-{2-[5-Cyclohexyl-1-(3,3-dimethyl-2-oxo-butyl)-2-oxo-1,2-dihydro-3H-1,3,4-benzotriazepin-3-yl]-acetylamino}-isophthalic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 5-amino-isophthalic acid dimethyl ester was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 10.19 (1H, s), 8.33 (2H, s), 8.13 (1H, s), 7.53 (1H, d), 7.48 (1H, t), 7.23 (1H, t), 7.16 (1H, d), 4.80 (2H, br), 4.30 (1H, br), 4.00 (1H, br), 2.85 (1H, m), 1.90-1.50 (6H, m), 1.45-1.13 (13H, m). The compound was further characterised as the *N*-methyl-D-glucamine salt. Found: C 54.69, H 7.11, N 8.70%; C₃₀H₃₄N₄O₇•C₇H₁₇NO₅•3.0H₂O requires C 54.74, H 7.08, N 8.63%.

### Example 40. 2-[5-Cyclohexyl-1-(3,3-dimethyl-2-oxo-butyl)-2-oxo-1,2-dihydro-3H-1,3,4-benzotriazepin-3-yl]-N-(3-methanesulfonylaminocarbonyl-phenyl)-acetamide.

Methanesulfonamide (96mg, 1.00mmol) was added to a solution of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid (Example 2) (409mg, 0.80mmol), EDC (207mg, 1.08mmol) and DMAP (122mg, 1.00mmol) in DCM (20ml) at room temperature. After stirring for 17h, the mixture was washed with 5% KHSO₄ (50ml), brine (50ml) and dried (MgSO₄). Filtration and evaporation of the solvent gave the crude product which was purified by flash column chromatography (MeOH-DCM (1:10) to afford the title compound as a white crystalline solid (392mg, 83%). ¹H NMR (DMSO-*d*_{*6*}) 9.99 (1H, s), 8.02 (1H, s), 7.63-7.36 (5H, m), 7.23 (1H, t), 7.13 (1H, d), 4.73 (2H, dd), 4.29 and 4.25 (2H, d × 2), 3.24 (3H, s), 2.87 (1H, m), 1.80-1.48 (6H, m), 1.30-1.09 (13H, m). The compound was further characterised as the *N*-methyl-D-glucamine salt. Found: C 53.93, H 7.07, N, 10.17%; C₃₀H₃₇N₅O₆S•C₇H₁₇NO₅•2.0H₂O requires: C 53.74, H 7.07, N 10.16%.

### Example 41. 2-[5-Cyclohexyl-1-(3,3-dimethyl-2-oxo-butyl)-2-oxo-1,2-dihydro-3H-1,3,4-benzotriazepin-3-yl]-N-(3-propylamino-phenyl)-acetamide.

**Step a.** *(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-propyl-carbamic acid tert-butyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-phenyl)-propyl-carbamic acid *tert*-butyl ester (obtained using steps b and c of the method employed in the preparation of (3-amino-phenyl)-methyl-carbamic acid *tert*-butyl ester (Example 3, step c) except that 1-bromopropane was used in step b instead of iodomethane), was used instead of 3-amino-benzoic acid methyl ester in step e.

**Step b.** 4.0M HCl in dioxan (5ml, 20.0mmol) was added to a solution of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-propyl-carbamic acid *tert*-butyl ester (Example 41, step a) (524mg,0.83mmol) in dioxan (15ml) at room temperature. The mixture was stirred for 2h during which time a white precipitate formed. The solvent was evaporated, and the solid obtained was washed with Et₂O, isolated by filtration and dried, to afford the hydrochloride salt of the title compound (430mg, 91%). ¹H NMR (CDCl₃) 8.39 (1H, s), 7.61-7.27 (7H, m), 7.01 (1H, d), 4.69 (2H, br s), 4.60 (1H, d), 4.29 (2H, dd), 3.21 (2H, br s), 2.80 (1H, br s), 2.01-1.45 (6H, m), 1.43-1.19 (15H, m), 0.95 (3H, t). Found: C 63.68, H 7.65, N, 11.98%; C₃₁H₄₁N₅O₃•HCl•H₂O requires: C 63.52, H 7.57, N 11.95%.

### Example 42. 2-[5-Cyclohexyl-1-(3,3-dimethyl-2-oxo-butyl)-2-oxo-1,2-dihydro-3H-1,3,4-benzotriazepin-3-yl]-N-[3-(2-ethoxy-ethylamino)-phenyl)-acetamide.

The title compound was obtained as the hydrochloride salt by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that (3-amino-phenyl)-2-ethoxyethyl-carbamic acid *tert*-butyl ester (obtained using the method employed in the preparation of (3-amino-phenyl)-methyl-carbamic acid tert-butyl ester (Example 3, step c) except that 2-bromoethyl-ethyl ether was used in step b instead of iodomethane) was used instead of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-propyl-carbamic acid *tert*-butyl ester (Example 41, step a) according to the method of Example 41 step b. ¹H NMR (CDCl₃) 8.45 (1H, s), 7.61 (1H, s), 7.56-7.47 (2H, m), 7.36 (4H, m), 7.03 (1H, d), 4.70 (2H, dd), 4.26 (2H, dd), 3.73 (2H, br s), 3.51 (4H, m), 2.82 (1H, br s), 2.05-1.65 (6H, m), 1.30-1.17 (16H, m). Found: C 63.90 H 7.66, N, 11.58%; C₃₂H₄₃N₅O₄•HCl•0.25H₂O requires: C 63.77, H 7.44, N 11.62%.

### Example 43. 5-Cyclohexyl-1-(3,3-dimethyl-2-oxo-butyl)-3-(2-oxo-2-m-tolyl-ethyl)-1,3-dihydro-3H-1,3,4-benzotriazepin-2-one.

**Step a.** *2-[5-Cyclohexyl-1-(3,3-dimethyl-2-oxo-butyl)-2-oxo-1,2-dihydro-3*H*-1,3,4-benzotriazepin-3-yl]-*N*-methoxy-*N*-methyl-acetamide* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *N,O*-dimethylhydroxylamine hydrochloride and triethylamine were used instead of 3-amino-benzoic acid methyl ester in step e. ¹H NMR (CDCl₃) 7.86 (2H, m), 7.17 (1H, m), 6.94 (1H, d), 4.70-4.14 (3H, m), 4.09 (1H, m), 3.68 (3H, s), 3.17 (3H, s), 2.72 (1H, m), 1.93-1.55 (6H, m), 1.43-1.22 (13H, m).

**Step b.** A 1.0M solution of *m*-tolylmagnesium chloride in THF (0.6ml, 0.6mmol) was added drop-wise to a solution of 2-[5-cyclohexyl-1-(3,3-dimethyl-2-oxo-butyl)-2-oxo-1,2-dihydro-3*H*-1,3,4-benzotriazepin-3-yl]-*N*-methoxy-*N*-methyl-acetamide (Example 43, step a) (220mg, 0.5mmol) in Et₂O (10ml) at -78 °C. The reaction mixture was allowed to warm to room temperature and stirred overnight. After solvent concentration, the reaction mixture was extracted with Et₂O, washed with saturated NH₄Cl solution, brine and dried (MgSO₄). Flash column chromatography (EtOAc-hexane (1:4)) afforded the title compound as a white solid (36 mg, 15%). ¹H NMR (CDCl₃) 7.69 (2H, m), 7.39-7.31 (4H, m), 7.26 (1H, m), 6.95 (1H, dd), 5.20 (1H, m), 4.68 (3H, m), 2.79 (1H, m), 2.30 (3H, s), 1.80-1.62 (6H, m), 1.31-1.24 (13H, m). Found C 73.14, H 7.82, N 8.51; C₂₉H₃₅N₃O₃ requires C 73.54, H 7.45, N 8.87%

### Example 44. 3-{5-[5-Cyclohexyl-1-(3,3-dimethyl-2-oxo-butyl)-2-oxo-1,2-dihydro-3H-1,3,4-benzotriazepin-3-ylmethyl]-[1,3,4]oxadiazol-2-yl}-benzoic acid.

**Step a.** *3-(*N'*-{2-[5-Cyclohexyl-1-(3,3-dimethyl-2-oxo-butyl)-2-oxo-1,2-dihydro-3*H*-1,3,4-benzotriazepin-3-yl]-acetyl}-hydrazinocarbonyl)-benzoic acid methyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-hydrazinocarbonyl-benzoic acid methyl ester (prepared in two steps from isophthalic acid dimethyl ester) was used instead of 3-amino-benzoic acid methyl ester in step e.

**Step b.** *3-{5-[5-Cyclohexyl-1-(3,3-dimethyl-2-oxo-butyl)-2-oxo-1,2-dihydro-3*H*-1,3,4-benzotriazepin-3-ylmethyl]-[1,3,4]oxadiazol-2-yl}-benzoic acid methyl ester.* A solution of the 3-(*N*'-{2-[5-cyclohexyl-1-(3,3-dimethyl-2-oxo-butyl)-2-oxo-1,2-dihydro-3*H*-1,3,4-benzotriazepin-3-yl]-acetyl}-hydrazinocarbonyl)-benzoic acid methyl ester (Example 44, step a) (94mg, 0.16mmol), triphenylphosphine (64mg, 0.24mmol) and 1,8-diazabicyclo[5.4.0]undec-7-ene (75mg, 0.49mmol) in CCl₄-MeCN (1:1 / 2ml) was stirred at room temperature for 5h. A further quantity of triphenylphosphine (43mg, 0.16mmol) was added after 3h. The solvent was evaporated under reduced pressure and the residue purified by flash column chromatography (EtOAc-hexane (1:1)) to afford the product as a off-white solid (52mg, 57%). ¹H NMR (CDCl₃) 8.62 (1H, s), 8.19 (2H, m), 7.56 (1H, t), 7.39-7.35 (2H, m), 7.19 (1H, t), 6.97 (1H, d), 5.05 (1H, br d), 4.85 (1H, br d), 4.68 (2H, s), 3.96 (3H, s), 2.70 (1H, m), 1.80-1.60 (6H, br), 1.25 (13H, m).

**Step c.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid (Example 2) except that 3-{5-[5-cyclohexyl-1-(3,3-dimethyl-2-oxo-butyl)-2-oxo-1,2-dihydro-3*H*-1,3,4-benzotriazepin-3-ylmethyl]-[1,3,4]oxadiazol-2-yl}-benzoic acid methyl ester (Example 44, step b) was used in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1). ¹H NMR (CDCl₃) 8.69 (1H, s), 8.27 (2H, m), 7.61 (1H, t), 7.42-7.38 (2H, m), 7.20 (1H, t), 6.98 (1H, d), 5.10 (1H, br d), 4.90 (1H, br d), 4.70 (2H, s), 2.73 (1H, m), 1.90-1.60 (6H, m), 1.30-1.25 (13H, m). The compound was further characterised as the *N*-methyl-D-glucamine salt. Found: C 56.75, H 7.08, N, 10.61%; C₃₀H₃₃N₅O₅•C₇H₁₇NO₅•2.5H₂O requires: C 56.69, H 7.07, N 10.72%.

### Example 45. 3-({2-[5-Cyclohexyl-1-(3,3-dimethyl-2-oxo-butyl)-2-oxo-1,2-dihydro-3H-1,3,4-benzotriazepin-3-yl]-acetylamino}-methyl)-benzoic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-aminomethyl-benzoic acid methyl ester was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.02 (1H, d), 7.94 (1H, s), 7.51 (1H, d), 7.45-7.35 (2H, m), 7.21 (1H, dd), 7.14 (1H, d), 6.93 (1H, d), 6.75 (1H, br t), 4.66-4.53 (3H, m), 4.38-4.19 (3H, m), 2.64 (1H, br m), 1.88-1.49 (6H, m), 1.31-1.20 (13H, m). The compound was further characterised as the *N-*methyl-D-glucamine salt. Found: C 58.33, H 7.64, N 8.95%; C₃₀H₃₆N₄O₅•C₇H₁₇NO₅•2.0H₂O requires C 58.18, H 7.52, N 9.17%.

### Example 46. [5-Cyclohexyl-1-(3,3-dimethyl-2-oxo-butyl)-2-oxo-1,2-dihydro-3H-1,3,4-benzotriazepin-3-yl]-acetic acid N'-m-tolyl-hydrazide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *meta*-tolyl-hydrazine was used instead of 3-amino-benzoic acid methyl ester in step e. ¹H NMR (CDCl₃) 8.12 (1H, d), 7.43-7.41 (2H, m), 7.25 (1H, t), 7.09 (1H, m), 6.99 (1H, d), 6.70 (1H, d), 6.65-6.31 (2H, m), 6.01 (1H, d), 4.71 (1H, d), 4.63 (1H, d), 4.24 (2H, s), 2.80 (1H, m), 2.29 (3H, s), 2.10-1.60 (6H, m), 1.35-1.24 (13H, m). Found: C 68.00, H 7.69, N 13.66%; C₂₉H₃₇N₅O₃•0.5H₂O requires C 67.94, H 7.47, N 13.66%.

### Example 47. 5-Cyclohexyl-1-(3,3-dimethyl-2-oxo-butyl)-3-(5-oxo-4-m-tolyl-4,5-dihydro-[1,3,4]oxadiazol-2-ylmethyl)-1,3-dihydro-3H-1,3,4-benzotriazepin-2-one.

A solution of [5-cyclohexyl-1-(3,3-dimethyl-2-oxo-butyl)-2-oxo-1,2-dihydro-3*H*-1,3,4-benzotriazepin-3-yl]-acetic acid *N*'-*m*-tolyl-hydrazide (Example 46) (300mg, 0.60mmol), 1,1'-carbonyldiimidzole (483mg, 2.98mmol) and triethylamine (181mg, 1.79mmol) in THF (6ml) was stirred at room temperature for 19h, then heated at reflux for 6h. The solvent was evaporated under reduced pressure and the residue was purified by flash column chromatography (EtOAc-hexane (2:3-7:3)) to afford the title compound as an off-white solid (83mg, 26%). ¹H NMR (CDCl₃) 7.63-7.60 (2H, m), 7.41-7.37 (2H, m), 7.32 (1H, m), 7.20 (1H, t), 7.05 (1H, d), 6.96 (1H, d), 4.75 (1H, br d), 4.68 (2H, d), 4.55 (1H, br d), 2.75 (1H, m), 2.38 (3H, s), 1.95-1.60 (6H, m), 1.35-1.20 (13H, m). Found: C 67.93, H 6.78, N 13.23%; C₃₀H₃₅N₅O₄ requires C 68.03, H 6.66, N 13.22%.

### Example 48. 2-[5-Cyclohexyl-1-(3,3-dimethyl-2-oxo-butyl)-2-oxo-1,2-dihydro-3H-1,3,4-benzotriazepin-3-yl]-N-(3-hydroxymethyl-phenyl)-acetamide.

**Step a.** Carbonic acid 3-{2-[5-cyclohexyl-1-{3,3-dimethyl-2-oxo-butyl)-2-oxo-1,2-dihydro-3H-1,3,4-benzotriazepin-3-yl]-acetylamino}-benzyl ester methyl ester was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that carbonic acid 3-amino-benzyl ester methyl ester was used instead of 3-amino-benzoic acid methyl ester in step e. ¹H NMR (CDCl₃) 8.30 (1H, s), 7.51-7.39 (4H, m), 7.31-7.24 (2H, m), 5.11 (2H, s), 4.72 (1H, d), 4.64 (1H, d), 4.23 (1H, d), 4.14 (1H, d), 3.80 (3H, s), 2.89 (1H, m), 1.76-1.67 (6H, m), 1.29-1.24 (13H, m).

**Step b.** Carbonic acid 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzyl ester methyl ester (Example 48, step a) was dissolved in THF-MeOH (1:1 / 40 ml) and 1% K₂CO₃ solution (30ml) was added. The mixture was stirred at room temperature for 2h. After concentration of the organic solvents, a precipitate formed, which was collected by filtration and dried *in vacuo* to afford the title compound as a yellow solid (790 mg, 89%). ¹H NMR (CDCl₃) 8.31 (1H, s), 7.51-7.45 (3H, m), 7.31-7.28 (3H, m), 7.26-7.10 (2H, m), 4.70 (1H, d), 4.67 (2H, s), 4.64 (1H, d), 4.26 (1H, d), 4.22 (1H, d), 2.89 (1H, m), 1.85-1.55 (6H, m), 1.30-1.24 (13H, m). Found: C 67.70, H 7.40, N 10.79 %; C₂₉H₃₆N₄O₄•0.5H₂O requires: C 67.81, H 7.26, N 10.91%.

### Example 49. N-(3-Carbamimidoylsulfanylmethyl-phenyl)-2-[5-cyclohexyl-1-(3,3-dimethyl-2-oxo-butyl)-2-oxo-1,2-dihydro-3H-1,3,4-benzotriazepin-3-yl]-acetamide.

**Step a.** *N*-(3-Chloromethyl-phenyl)-2-[5-cyclohexyl-1-(3,3-dimethyl-2-oxo-butyl)-2-oxo-1,2-dihydro-3H-1,3,4-benzotriazepin-3-yl]-acetamide. 2-[5-Cyclohexyl-1-(3,3-dimethyl-2-oxo-butyl)-2-oxo-1,2-dihydro-3H-1,3,4-benzotriazepin-3-yl]-N-(3-hydroxymethyl-phenyl)-acetamide (Example 48) (750mg, 1.49mmol) and triphenylphosphine polystyrene resin (1.20mmol/g; 2.50g, 3.00mmol) (P. Hodge, G. Richardson, *J. C. S. Chem. Commun*., (1983), 622) were heated at reflux in CCl₄ (30ml) for 3h. After filtration and evaporation of the solvent the product was obtained as a pale pink solid (220 mg, 29%). ¹H NMR (CDCl₃) 8.36 (1H, s), 7.50-7.28 (4H, m), 7.10-7.01 (4H, m), 4.75 (1H, d), 4.60 (1H, d), 4.54 (2H, s), 4.25 (1H, d), 4.22 (1H, d), 2.79 (1H, m), 2.05-1.62 (6H, m), 1.30-1.22 (13H, m).

**Step b.** *N*-(3-Chloromethyl-phenyl)-2-[5-cyclohexyl-1-(3,3-dimethyl-2-oxo-butyl)-2-oxo-1,2-dihydro-3H-1,3,4-benzotriazepin-3-yl]-acetamide (Example 49, step a) (52mg, 0.10mmol) was reacted with thiourea (7.6mg, 0.10mmol) and NaI (2 mg) in refluxing acetone (10ml) for 3h. After cooling, the solvents were concentrated in vacuo, affording the title compound as a yellow solid after trituration with Et₂O (18 mg, 30%). ¹H NMR (DMSO-d₆) 9.89 (1H, br s), 8.99 (3H, br s), 7.75-7.46 (3H, m), 7.29-7.04 (5H, m), 4.77 (2H, m), 4.40 (2H, m), 4.38 (1H, m), 3.93 (1H, m), 2.86 (1H, m), 1.73-1.64 (6H, m), 1.21-1.08 (13H, m).

### Example 50. (3-{2-[5-Cyclohexyl-1-(3,3-dimethyl-2-oxo-butyl)-2-oxo-1,2-dihydro-3H-1,3,4-benzotriazepin-3-yl]-acetylamino}-benzenesulfonyl)-acetic acid ethyl ester.

The title compound was obtained by the method used in the preparation of 3-{2-[5-cyclohexyl-1-{3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-benzenesulfonyl)-acetic acid ethyl ester (prepared in one step from (3-amino-phenylsulfanyl)-acetic acid ethyl ester) was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (DMSO-d₆) 13.08 (1H, br s), 10.26 (1H, s), 8.16 (1H, s), 7.77-7.45 (5H, m), 7.26-7.15 (2H, m), 4.80 (2H, m), 4.39 (2H, s), 4.38 (1H, d), 3.98 (1H, d), 2.86 (1H, m), 1.65-1.34 (6H, m), 1.25-1.13 (13H, m). The compound was further characterised as the N-methyl-D-glucamine salt. Found: C 53.55, H 6.90, N 8.48 %; C₃₀H₃₆N₄O₇S•C₇H₁₇NO₅•2.0H₂O requires: C 53.67, H 6.94, N 8.46%.

### Example 51. [tert-Butoxycarbonyl-(3-{2-[5-cyclohexyl-1-(3,3-dimethyl-2-oxo-butyl)-2-oxo-1,2-dihydro-3H-1,3,4-benzotriazepin-3-yl]-acetylamino}-phenyl)-amino]-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [(3-amino-phenyl)-*tert*-butoxycarbonyl-amino]-acetic acid methyl ester (prepared in three steps from 1-isocyanato-3-nitro-benzene) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.60 (1H, br s), 9.81 (1H, s), 7.52-7.47 (3H, m), 7.23-7.17 (4H, m), 6.89 (1H, d), 4.78 (2H, m), 4.25 (1H, d), 4.14 (2H, s), 3.92 (1H, d), 2.86 (1H, m), 1.86-1.51 (6H, m), 1.36-1.13 (22H, m). The compound was further characterised as the *N*-methyl-D-glucamine salt. Found: C 55.18, H 7.90, N 9.20 %; C₃₅H₄₅N₅O₇•C₇H₁₇NO₅•4.0H₂O requires: C 55.12, H 7.71, N 9.18%.

### Example 52. (3-{2-[5-Cyclohexyl-1-(3,3-dimethyl-2-oxo-butyl)-2-oxo-1,2-dihydro-3H-1,3,4-benzotriazepin-3-yl]-acetylamino}-phenylamino)-acetic acid.

[*tert*-Butoxycarbonyl-(3-{2-[5-cyclohexyl-1-(3,3-dimethyl-2-oxo-butyl)-2-oxo-1,2-dihydro-3H-1,3,4-benzotriazepin-3-yl]-acetylamino}-phenyl)-amino]-acetic acid (Example 51) (137mg, 0.21mmol) was stirred in trifluoroacetic acid for 2h at room temperature. After concentration, the resulting gum was dissolved in DCM, washed with saturated NaHCO₃ and then with 1N HCl. The organic phase was dried over MgSO₄, filtered and the solvent was evaporated to afford the title compound as a yellow solid (85 mg, 73%). ¹H NMR (DMSO-d₆) 9.47 (1H, s), 7.55-7.27 (2H, m), 7.23-7.15 (2H, m), 6.97 (1H, t), 6.92-6.68 (2H, m), 6.25(1H, m), 5.80 (2H, br s), 4.78 (2H, m), 4.25 (1H, d), 3.95 (1H, d), 3.72 (2H, s), 2.80 (1H, m), 1.86-1.51 (6H, m), 1.26-1.09 (13H, m). The compound was further characterised as the N-methyl-D-glucamine salt. Found: C 57.66, H 7.45, N 9.07 %; C₃₀H₃₆N₄O₆•C₇H₁₇NO₅•1.5H₂O requires: C 57.65, H 7.32, N 9.09%.

### Example 53. (3-{2-[5-Cyclohexyl-1-(3,3-dimethyl-2-oxo-butyl)-2-oxo-1,2-dihydro-3H-1,3,4-benzotriazepin-3-yl]-acetylamino}-phenoxy)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-phenoxy)-acetic acid methyl ester was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 13.00 (1H, br s), 9.75 (1H, s), 7.50 (2H, m), 7.26-7.13 (4H, m), 7.04 (1H, d), 6.55 (1H, d), 4.78 (2H, s), 4.58 (2H, s), 4.27 (1H, d), 3.97 (1H, d), 2.85 (1H, m), 1.90-1.20 (10H, m), 1.13 (9H, s). The compound was further characterised as the N-methyl-D-glucamine salt. Found: C 57.66, H 7.45, N 9.07%; C₃₀H₃₆N₄O₆•C₇H₁₇NO₅•1.5H₂O requires: C 57.65, H 7.32, N 9.09%.

### Example 54. 3-{2-[5-Adamantan-1-yl-1-(3,3-dimethyl-2-oxo-butyl)2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that adamantan-1-yl-(2-amino-phenyl)-methanone (A. Cappelli, *et*. *al*., *J. Med*. *Chem*., (1999), **42**, 1556) was used in step a instead of (2-amino-phenyl)-cyclohexyl-methanone, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 13.0 (1H, br s), 9.89 (1H, s), 8.14 (1H, s), 7.67 (1H, m), 7.59 (2H, d), 7.42 (2H, m), 7.21 (2H, m), 4.79 (2H, m), 4.29 (1H, d), 3.93 (1H, d), 2.04-1.66 (15H, m), 1.13 (9H, s). The compound was further characterised as the *N*-methyl-D-glucamine salt. Found: C 59.82, H 7.66, N 8.67%; C₃₃H₃₈N₄O₅•C₇H₁₇NO₅•2.0H₂O requires C 59.91, H 7.41, N 8.73%.

### Example 55. 3-{2-[5-Cycloheptyl-1-(3,3-dimethyl-2-oxo-butyl)2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-phenyl)-cycloheptyl-methanone (prepared according to the method of A. Cappelli, *et. al*., *J. Med. Chem*., (1999), **42,** 1556) was used in step a instead of (2-amino-phenyl)-cyclohexyl-methanone, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.49 (1H, s), 8.04 (1H, m), 7.87 (1H, m), 7.81 (1H, m), 7.50-7.26 (4H, m), 7.04 (1H, d), 4.78 (1H, d), 4.60 (1H, d), 4.27 (2H, s), 3.00 (1H, m), 2.05-1.44 (12H, m), 1.25 (9H, s). The compound was further characterised as the *N*-methyl-D-glucamine salt. Found: C 57.03, H 7.58, N 8.77 %; C₃₀H₃₆N₄O₅•C₇H₁₇NO₅•3.5H₂O requires: C 56.84, H 7.61, N 8.95%.

### Example 56. (3-{2-[5-Cyclohexy-1-(2-cyclopentyl-2-oxo-ethyl)-2-oxo-1,2-dihydro-3H-1,3,4-benzotriazepin-3-yl]-acetylamino}-phenyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and (3-amino-phenyl)-acetic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 9.20 (1H, br s), 8.29 (1H, s), 7.46 (2H, m), 7.36-7.19 (4H, m), 7.06 (1H, d), 6.99 (1H, d), 4.66 (1H, d), 4.46 (1H, d), 4.32 (1H, d), 4.17 (1H, d), 3.58 (2H, s), 2.93 (1H, m), 2.78 (1H, m), 1.99-1.51 (13H, m), 1.27 (5H, m). The compound was further characterised as the *N*-methyl-D-glucamine salt. Found: C 58.65, H 7.44, N 9.06%; C₃₁H₃₆N₄O₅•C₇H₁₇NO₅•2.1H₂O requires: C 58.70, H 7.41, N 9.01%.

### Example 57. 2-[5-Cyclohexyl-1-(2-cyclopentyl-2-oxo-ethyl)-2-oxo-1,2-dihydro-3H-1,3,4-benzotriazepin-3-yl]-N-(3-methylaminophenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and (3-amino-phenyl)-methyl-carbamic acid *tert*-butyl ester (Example 3, step c) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.07 (1H, s), 7.47 (2H, m), 7.27 (1H, m), 7.04 (2H, m), 6.92 (1H, t), 6.44 (1H, dd), 6.31 (1H, dd), 4.64 (1H, d), 4.48 (1H, d), 4.36 (1H, d), 4.13 (1H, d), 3.71 (1H, br s), 2.95 (1H, m), 2.80 (4H, m), 2.05-1.56 (13H, m), 1.27 (5H, m). The compound was further characterised as the hydrochloride salt. Found: C 64.90, H 7.02, N 12.57%; C₃₀H₃₇N₄O₃•HCl requires: C 65.26, H 6.94, N 12.68%.

### Example 58. (3-{2-[5-Cyclohexyl-1-(2-cyclopentyl-2-oxo-ethyl)-2-oxo-1,2-dihydro-3H-1,3,4-benzotriazepin-3-yl]-acetylamino}-phenylsulfanyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and (3-amino-phenylsulfanyl)-acetic acid ethyl ester were used instead of [5-cyclohexyl-1-(3,3-dimethyl-2-oxo-butyl)-2-oxo-1,2-dihydro-3*H*-1,3,4-benzotriazepin-3-yl]-acetic acid (Example I, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.41 (1H, s), 7.45 (2H, m), 7.31-7.17 (4H, m), 7.08 (2H, m), 6.12 (1H, br s), 4.66 (1H, d), 4.21 (1H, d), 4.23 (1H, d), 4.19 (1H, d), 3.65 (2H, s), 2.96 (1H, m), 2.79 (1H, m), 1.82-1.62 (13H, m), 1.29 (5H, m). The compound was further characterised as the N-methyl-D-glucamine salt. Found: C 52.82, H 7.43, N 8.12 %; C₃₁H₃₆N₄O₅S•C₇H₁₇NO₅•5.0H₂O requires: C 52.95, H 7.36, N 8.04%.

### Example 59. 2-[5-Cyclohexyl-1-(3,3-dimethyl-2-oxo-butyl)-2-oxo-1,2-dihydro-3H-1,3,4-benzotriazepin-3-yl]-N-[3-(5-oxo-2,5-dihydro-[1,2,4]oxadiazol-3-yl)-phenyl]-acetamide.

A solution of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) (200mg, 0.5mmol), EDC (140mg, 0.74mmol), HOBT (100mg, 0.74mmol) and DMAP (20mg) in DMF (10ml) was stirred at room temperature for 2h. Thereafter, a solution of 3-(3-amino-phenyl)-2*H*-[1,2,4]oxadiazol-5-one trifluoroacetic acid salt (WO 93/19063) (170mg, 0.58mmol) and triethylamine (120µl, 1.00mmol) in DMF (2ml) was added and the reaction mixture was stirred for 16h. The mixture was diluted with EtOAc (20ml), and the organic layer washed with aqueous 5% KHSO₄, brine and dried over MgSO₄. Filtration and evaporation of the solvent afforded an oil which was purified by flash column chromatography (acetone-DCM (1:6)) to give the title compound as an orange foam (62 mg, 22%). ¹H NMR (CDCl₃) 8.83 (1H, s), 7.90 (1H, m), 7.53-7.25 (7H, m), 7.02 (1H, d), 4.76 (1H, d), 4.57 (1H, d), 4.22 (1H, d), 4.20 (1H, d), 2.80 (1H, m), 1.95-1.55 (6H, m), 1.28-1.20 (13H, m). The compound was further characterised as the *N*-methyl-D-glucamine salt. Found: C 55.99, H 7.15, N 12.14 %; C₃₀H₃₄N₆O₅•C₇H₁₇NO₅•2.0H₂O requires: C 56.26, H 7.01, N 12.41%.

### Example 60. 3-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-acrylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-(3-amino-phenyl)-acrylic acid methyl ester was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.43 (1H, s), 7.75-7.45 (5H, m), 7.35-7.23 (3H, m), 7.04 (1H, d), 6.42 (1H, d), 4.76 (1H, d), 4.61 (1H, d), 4.27 (2H, m), 2.79 (1H, m), 2.01-1.65 (6H, m), 1.28-1.23 (13H, m). Found: C 60.70, H 7.50, N 9.60 %; C₃₁H₃₆N₄O₅•C₇H₁₇NO₅•0.5H₂O requires: C 60.49, H 7.26, N 9.35%.

### Example 61. 3-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-propionic acid.

The title compound was obtained by the method used in the preparation of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1) except that 3-(3-amino-phenyl)-propionic acid methyl ester (D. F. Biggs, *et. al*., *J. Med*. *Chem*. (1976), **19**, 472) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1), according to the method of Example 2. ¹H NMR (CDCl₃) 9.05 (1H, br s), 8.35 (1H, s), 7.45 (2H, m), 7.31-7.18 (4H, m), 7.03 (1H, d), 6.92 (1H, d), 4.73 (1H, d), 4.63 (1H, d), 4.27 (1H, d), 4.20 (1H, d), 2.91 (2H, m), 2.79 (1H, m), 2.63 (2H, m), 1.90-1.35 (6H, m), 1.29-1.21 (13H, m). The compound was further characterised as the N-methyl-D-glucamine salt. Found: C 57.25, H 7.81, N 9.02 %; C₃₁H₃₈N₄O₅•C₇H₁₇NO₅•3.0H₂O requires: C 57.34, H 7.72, N 8.80%.

### Example 62. N-(3,5-Bis-hydroxymethyl-phenyl)-2-[5-cyclohexyl-1-(3,3-dimethyl-2-oxo-butyl)-2-oxo-1,2-dihydro-3H-1,3,4-benzotriazepin-3-yl]-acetamide.

The title compound was prepared by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that carbonic acid 3-amino-5-methoxycarbonyloxymethyl-benzyl ester methyl ester was used instead of 3-amino-benzoic acid methyl ester in step e, followed by reaction of the product obtained, in place carbonic acid 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzyl ester methyl ester (Example 48, step a), according to the method of Example 48, step b. ¹H NMR (CDCl₃) 8.37 (1H, s), 7.47 (2H, m), 7.30 (3H, m), 7.09 (1H, s), 7.02 (1H, d), 4.78-4.58 (6H, m), 4.23 (2H, m), 2.79 (1H, m), 2.04-1.44 (8H, m), 1.26 (13H, m). Found: C 64.88, H 7.45, N 10.15%; C₃₀H₃₈N₄O₅•1.2H₂O requires: C 64.75, H 7.32, N 10.07%.

### Example 63. 2-[5-Cyclohexyl-1-(3,3-dimethyl-2-oxo-butyl)-2-oxo-1,2-dihydro-3H-1,3,4-benzotriazepin-3-yl]-N-pyridin-2-yl-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2-aminopyridine was used instead of 3-amino-benzoic acid methyl ester in step e. ¹H NMR (CDCl₃) 8.83 (1H, s), 8.20-8.14 (2H, m), 7.62 (1H, t), 7.46-7.40 (2H, m), 7.25 (1H, m), 6.98-6.93 (2H, m), 4.70 (1H, d), 4.66 (1H, d), 4.30 (1H, d), 4.20 (1H, m), 2.78 (1H, m), 2.03-1.60 (6H, m), 1.38-1.22 (13H, m). The compound was further characterised as the hydrochloride salt. Found: C 61.20, H 6.90, N 13.04%; C₂₇H₃₃N₅O₃•HCl•H₂O requires: C 61.18, H 6.85, N 13.21%.

### Example 64. 2-[5-Cyclohexyl-1-(3,3-dimethyl-2-oxo-butyl)-2-oxo-1,2-dihydro-3H-1,3,4-benzotriazepin-3-yl]-N-pyridin-3-yl-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-aminopyridine was used instead of 3-amino-benzoic acid methyl ester in step e. ¹H NMR (CDCl₃) 8.52 (1H, br s), 8.36 (1H, d), 8.31-8.24 (2H, m), 7.48-7.44 (2H, m), 7.31-7.22 (2H, m), 7.02 (1H, d), 4.79 (1H, d), 4.56 (1H, d), 4.23 (2H, s), 2.80 (1H, br m), 2.01 (1H, br m), 1.87-1.62 (5H, m), 1.33-1.23 (13H, m). The compound was further characterised as the hydrochloride salt. Found: C 60.81, H 7.01, N 12.93%; C₂₇H₃₃N₅O₃•HCl•H₂O requires: C 61.18, H 6.85, N 13.21%.

### Example 65. [Acetyl-(3-{2-[5-cyclohexyl-1-(3,3-dimethyl-2-oxo-butyl)-2-oxo-1,2-dihydro-3H-1,3,4-benzotriazepin-3-yl]-acetylamino}-phenyl)-amino]-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [acetyl-(3-amino-phenyl)-amino]-acetic acid methyl ester (prepared in three steps from 3-nitroaniline) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 13.00 (1H, br s), 10.01 (1H, s), 7.50 (4H, m), 7.33 (1H, t), 7.23 (1H, m), 7.16 (1H, d), 7.03 (1H, d), 4.79 (2H, s), 4.28 (1H, d), 4.17 (2H, s), 3.96 (1H, d), 2.86 (1H, m), 2.01 (1H, br m), 1.90-1.10 (10H, m), 1.76 (3H, s), 1.16 (9H, s). The compound was further characterised as the *N*-methyl-D-glucamine salt. Found: C 55.77, H 7.44, N 9.81%; C₃₂H₃₉N₅O₆•C₇H₁₇NO₅•3.0H₂O requires: C 55.83, H 7.49, N 10.02%.

### Example 66. N-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-succinamic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *N-*(3-amino-phenyl)-succinamic acid methyl ester (prepared in three steps from 3-nitroaniline) was used instead of 3-amino-benzoic acid methyl ester in step e, followed by reaction of the product obtained, in place of 3-{2-[5-cyclohexyl-1-(3,3-dimethyl-3-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.00 (1H, br s), 9.91 (1H, s), 9.75 (1H, s), 7.88 (1H, s), 7.50 (2H, m), 7.25-7.13 (5H, m), 4.78 (2H, s), 4.25 (1H, d), 3.96 (1H, d), 2.86 (1H, m), 2.48 (4H, s), 1.98-1.17 (10H, m), 1.13 (9H, s). The compound was further characterised as the *N*-methyl-D-glucamine salt. Found: C 55.74, H 7.69, N 9.91%; C₃₂H₃₉N₅O₆•C₇H₁₇NO₅•3.0H₂O requires: C 55.84, H 7.49, N 10.02%.

### Example 67. 2-[5-Cyclohexyl-1-(3,3-dimethyl-2-oxo-butyl)-2-oxo-1,2-dihydro-3H-1,3,4-benzotriazepin-3-yl]-N-[3-(1H-tetrazol-5-ylmethyl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-(1*H*-tetrazol-5-yl-methyl)-aniline was used instead of 3-amino-benzoic acid methyl ester in step e. ¹H NMR (CDCl₃) 8.61 (1H, s), 7.44 (4H, m), 7.27 (4H, m), 6.98 (2H, m), 4.70 (2H, q), 4.25 (2H, d), 2.80 (1H, m), 2.04 (1H, br m), 1.84 (1H, br d), 1.67 (6H, m), 1.28 (2H, m), 1.19 (9H, s). The compound was further characterised as the *N*-methyl-D-glucamine salt. Found C 56.69, H 7.43, N 16.08, C₃₀H₃₆N₈O₃•C₇H₁₇NO₅•1.9H₂O requires C 56.57, H 7.43, N 16.77%.

### Example 68. 3-{2-[5-Cyclohexyl-1-(2-morpholin-4-yl-2-oxo-ethyl)-2-oxo-1,2-dihydro-3H-1,3,4-benzotriazepin-3-yl]-acetylamino}-benzoic acid.

**Step a.** *(1-*tert*-Butoxycarbonylmethyl-5-cyclohexyl-2-oxo-1,2-dihydro-3*H*-1,3,4-benzotriazepin-3-yl)-acetic acid ethyl ester* was obtained by the method used in the preparation of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c) except that *tert*-butyl-bromoacetate was used in place of 1-bromo-3,3-dimethyl-butan-2-one. ¹H NMR (CDCl₃) 7.40 (2H, m), 7.18 (1H, dt), 7.10 (1H, d), 4.51-4.10 (6H, m), 2.72 (1H, m), 1.66 (6H, m), 1.45 (9H, s), 1.23 (7H, m).

**Step b.** *(5-Cyclohexyl-3-ethoxycarbonylmethyl-2-oxo-2,3-dihydro-3*H*-1,3,4-benzotriazepin-1-yl)-acetic acid*. (1-*tert*-Butoxycarbonylmethyl-5-cyclohexyl-2-oxo-1,2-dihydro-3*H*-1,3,4-benzotriazepin-3-yl)-acetic acid ethyl ester (Example 68, step a) (860mg, 1.94mmol) was dissolved in trifluoroacetic acid (5ml) and the solution was stirred at room temperature for 2h. The trifluoroacetic acid was evaporated, the residue was dissolved in DCM (20ml) and washed with H₂O (20ml × 2). The organic phase was separated and dried over MgSO₄. Filtration and evaporation of the solvent afford the product (630mg, 84%). ¹H NMR (CDCl₃) 9.50 (1H, br s), 7.43 (2H, m), 7.25 (1H, m), 7.14 (1H, d), 4.40 (4H, m), 4.15 (2H, m), 2.73 (1H, m), 1.75 (6H, m), 1.24 (7H, m).

**Step c.** *[5-Cyclohexyl-1-(2-morpholin-3-yl-2-oxo-ethyl)-2-oxo-1,2-dihydro-3*H*-1,3,4-benzotriazepin-3-yl]-acetic acid ethyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that (5-cyclohexyl-3-ethoxycarbonylmethyl-2-oxo-2,3-dihydro-3*H*-1,3,4-benzotriazepin-1-yl)-acetic acid (Example 68, step b) and morpholine were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively. ¹H NMR (CDCl₃) 7.40 (2H, m), 7.18 (2H, m), 4.51 (2H, br d), 4.26 (2H, br d), 4.14 (2H, m), 3.69-3.50 (8H, m), 2.74 (1H, m), 1.74 (6H, m), 1.24 (7H, m).

**Step d.** The title compound was obtained using steps d and e of the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [5-cyclohexyl-1-(2-morpholin-3-yl-2-oxo-ethyl)-2-oxo-1,2-dihydro-3*H*-1,3,4-benzotriazepin-3-yl]-acetic acid ethyl ester (Example 68, step c) was used in step d instead of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.60 (1H, s), 8.09 (1H, dd), 7.90 (1H, t), 7.78 (1H, d), 7.52 (2H, m), 7.42-7.29 (2H, m), 7.20 (1H, d), 4.64 (1H, d), 4.41 (1H, d), 4.26 (2H, m), 3.70 (6H, br s), 3.50 (2H, br s), 2.80 (1H, m), 2.05-1.71 (6H, m), 1.25 (4H, m). The compound was further characterised as the *N*-methyl-D-glucamine salt. Found: C 55.33, H 7.19, N 10.70%; C₂₉H₃₃N₅O₆•C₇H₁₇NO₅•2.2H₂O requires: C 55.22, H 7.01, N 10.73%.

### Example 69. (3-{2-[5-(4-tert-Butyl-cyclohexyl)-1-(3,3-dimethyl-2-oxo-butyl)-2-oxo-1,2-dihydro-3H-1,3,4-benzotriazepin-3-yl]-acetylamino}-phenyl)-acetic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-phenyl)-(4-*tert*-butyl-cyclohexyl)-methanone (prepared from 1-bromo-4-*tert*-butyl-cyclohexane (A. L. J. Beckwith, *et. al*., *J. Chem. Soc. Perkin Trans. II*, (1983), 661) and 2-aminobenzonitrile) was used in step a instead of (2-amino-phenyl)-cyclohexyl-methanone, and (3-amino-phenyl)-acetic acid methyl ester replaced 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.28 (1H, s), 7.50-7.44 (2H, m), 7.33-7.21 (4H, m), 7.03-6.98 (2H, m), 4.76 (1H, d), 4.60 (1H, d), 4.31 (1H, d), 4.17 (1H, d), 3.61 (2H, s), 2.71 (1H, m), 2.11-1.66 (5H, m), 1.34-1.24 (10H, m), 1.21-1.02 (3H, br m), 0.86 (9H, s). The compound was further characterised as the *N*-methyl-D-glucamine salt. Found: C 58.81, H 8.24, N 8.49%; C₃₄H₄₄N₄O₅•C₇H₁₇NO₅•3.0H₂O requires C 58.76, H 8.06, N 8.36%.

### Example 70. (6-{2-[5-Cyclohexyl-1-(3,3-dimethyl-2-oxo-butyl)-2-oxo-1,2-dihydro-3H-1,3,4-benzotriazepin-3-yl]-acetylamino}-indol-1-yl)-acetic acid.

The title compound was obtained by the method used in the preparation of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1) except that (6-amino-indol-1-yl)-acetic acid ethyl ester (prepared in two steps from 6-nitroindole) was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.33 (1H, s), 7.92 (1H, s), 7.45 (3H, m), 7.28 (1H, m), 7.02 (2H, m), 6.58 (1H, dd), 6.47 (1H, t), 4.84 (2H, s), 4.68 (2H, m), 4.40 (1H, d), 4.18 (1H, d), 2.78 (1H, m), 2.02-1.15 (19H, m). The compound was further characterised as the N-methyl-D-glucamine salt. Found: C 58.25, H 7.41, N 10.61%; C₃₂H₃₇N₅O₅•C₇H₁₇NO₅•2.0H₂O requires: C 58.38, H 7.28, N 10.47%.

### Example 71. (3-{2-[5-Cyclohexyl-1-(3,3-dimethyl-2-oxo-butyl)-2-oxo-1,2-dihydro-3H-1,3,4-benzotriazepin-3-yl]-acetylamino}-benzylsulfanyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-benzyl-sulfanyl)-acetic acid methyl ester (prepared in two steps from 3-bromomethyl nitrobenzene) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.37 (1H, s), 7.51-7.45 (2H, m), 7.31-7.20 (4H, m), 7.01 (1H, d), 6.80 (1H, br s), 4.79 (1H, d), 4.60 (1H, d), 4.31 (1H, d), 4.17 (1H, d), 3.78 (2H, s), 3.10 (2H, s), 2.79 (1H, m), 1.99-1.71 (6H, m), 1.29-1.19 (13H, m). The compound was further characterised as the *N*-methyl-D-glucamine salt. Found: C 52.84, H 7.53, N, 8.10%; C₃₁H₃₈N₄O₅S•C₇H₁₇NO₅•5.0H₂O requires: C 52.82, H 7.58, N 8.10%.

### Example 72. 2-{5-Cyclohexyl-1-[2-(4-methyl-piperazin-1-yl)-2-oxo-ethyl]-2-oxo-1,2-dihydro-3H-1,3,4-benzotriazepin-3-yl}-N-(3-methylamino-phenyl)-acetamide.

**Step a.** *{5-Cyclohexyl-1-[2-(4-methyl-piperazin-1-yl)-2-oxo-ethyl]-2-oxo-1,2-dihydro-3*H*-1,3,4-benzotriazepin-3-yl}-acetic acid ethyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example I, step e) except that (5-cyclohexyl-3-ethoxycarbonylmethyl-2-oxo-2,3-dihydro-3*H*-1,3,4-benzotriazepin-1-yl)-acetic acid (Example 68, step b) and 1-methylpiperazine were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively. ¹H NMR (CDCl₃) 7.40 (2H, m), 7.19 (2H, m), 4.48 (2H, br d), 4.25-4.10 (4H, m), 3.59 (4H, br m), 2.77 (1H, m), 2.60 (4H, m), 2.41 (3H, s), 1.72 (6H, m), 1.24 (7H, m).

**Step b.** *{5-Cyclohexyl-1-[2-(4-methyl-piperazin-1-yl)-2-oxo-ethyl]-2-oxo-1,2-dihydro-3*H*-1,3,4-benzotriazepin-3-yl)-acetic acid* was obtained by the method used in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that {5-cyclohexyl-1-[2-(4-methyl-piperazin-1-yl)-2-oxo-ethyl]-2-oxo-1,2-dihydro-3*H*-1,3,4-benzotriazepin-3-yl}-acetic acid ethyl ester (Example 72, step a) was used in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c). ¹H NMR (CDCl₃) 9.30 (1H, br s), 7.42 (2H, m), 7.21 (2H, m), 4.45 (2H, br s), 4.08-3.78 (6H, br m), 2.92 (4H, m), 2.76 (1H, m), 2.62 (3H, s), 1.98-1.44 (6H, m), 1.23 (4H, m).

**Step c.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that {5-cyclohexyl-1-[2-(4-methyl-piperazin-1-yl)-2-oxo-ethyl]-2-oxo-1,2-dihydro-3*H*-1,3,4-benzotriazepin-3-yl)-acetic acid (Example 72, step b) and (3-amino-phenyl)-methyl-carbamic acid *tert*-butyl ester (Example 3, step c) were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.12 (1H, s), 7.46 (2H, m), 7.27 (2H, m), 7.03 (1H, t), 6.91 (1H, t), 6.46 (1H, dd), 6.30 (1H, dd), 4.52 (2H, m), 4.37 (1H, d), 4.14 (1H, d), 3.62 (2H, m), 3.49 (2H, m), 2.79 (5H, m), 2.40 (4H, m), 2.30 (3H, s), 2.00-1.75 (6H, m), 1.26 (4H, m). The compound was further characterised as the di-hydrochloride salt. Found: C 53.60, H 7.08, N, 14.50%; C₃₀H₃₉N₇O₃•2.0HCl•3.0H₂O requires: C 53.54, H 7.05, N 14.57%.

### Examples 73/74

*3-{2-[5-Cyclohexyl-1-(3,3-dimethyl-2-oxo-butyl)-2-oxo-1,2-dihydro-3H-1,3,4-benzotriazepin-3-yl]-acetylamino}-5-methylamino-benzoic acid methyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that with 3-amino-5-[methyl-(2,2,2-trifluoro-acetyl)-amino]-benzoic acid methyl ester (prepared in four steps from 3-amino-5-nitrobenzoic acid) was used in place of 3-amino-benzoic acid methyl ester in step e, and following reaction of the product according to the method of Example 2, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), two compounds were isolated by chromatography.

### Example 73. 3-{2-[5-Cyclohexyl-1-(3,3-dimethyl-2-oxo-butyl)-2-oxo-1,2-dihydro-3H-1,3,4-benzotriazepin-3-yl]-acetylamino}-5-methylamino-benzoic acid.

¹H NMR (CDCl₃) 8.30 (1H, s), 7.52-7.46 (3H, m), 7.35-7.29 (1H, m), 7.05-6.92 (3H, m), 4.77 (1H, d, 17.7), 4.68 (1H, d, 17.7), 4.36-4.12 (2H, m), 2.88-2.76 (4H, m), 1.88-1.69 (5H, m), 1.31-1.23 (14H, m). The compound was further characterised as the *N*-methyl-D-glucamine salt. Found: C 56.60, H 7.65, N 10.44%; C₃₀H₃₇N₅O₅•C₇H₁₇NO₅•3.5H₂O requires: C 56.40, H 7.55, N 10.67%.

### Example 74. 3-{2-[5-Cyclohexyl-1-(3,3-dimethyl-2-oxo-butyl)-2-oxo-1,2-dihydro-3H-1,3,4-benzotriazepin-3-yl]-acetylamino}-5-methylamino-benzoic acid methyl ester.

¹H NMR (CDCl₃) 8.31 (1H, s), 7.61-7.41 (3H, m), 7.33-7.27 (1H, m), 7.04-6.92 (3H, m), 4.77-4.61 (2H, m), 4.33 (1H, d, 13.2), 4.19 (1H, d, 13.2), 3.88 (4H, br s), 2.86-2.76 (4H, m), 2.05-1.73 (5H, m), 1.31-1.19 (14H, m). Found: C 65.98, H 7.25, N 12.20%; C₃₁H₃₉N₅O₅ requires: C 66.29, H 7.00, N 12.47%.

### Example 75. 2-(3{2-[5-Cyclohexyl-1-(3,3-dimethyl-2-oxo-butyl)-2-oxo-1,2-dihydro-3H-1,3,4-benzotriazepin-3-yl]-acetylamino}-phenylamino)-propionic acid.

The title compound was obtained as the hydrochloride salt by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2-[(3-amino-phenyl)-*tert*-butoxycarbonyl-amino]-propionic acid methyl ester (prepared in two steps from 3-nitro-*N-tert*-butoxycarbonylaniline) was used instead of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-propyl-carbamic acid *tert*-butyl ester (Example 41, step a) according to the method of Example 41, step b. ¹NMR (DMSO-*d*_{*6*}) 9.53 (1H,s), 7.50 (2H, m), 7.25 (2H, m), 6.98 (2H, m), 6.73 (1H, d), 6.30 (1H, d), 5.00 (3H, br s), 4.79 (2H, s), 4.25 (1H, m), 3.91 (2H, m), 2.86 (1H, m), 2.00-1.00 (10H, m), 1.33 (3H, d), 1.13 (9H, s). Found: C 60.48, H 6.77, N 11.18%; C₃₁H₃₉N₅O₅•HCl•H₂O requires: C 60.43, H 6.87, N 11.37%.

### Example 76. (R)-1-(3-{2-[5-Cyclohexyl-1-(3,3-dimethyl-2-oxobutyl)-2-oxo-1,2-dihydro-3H-1,3,4-benzotriazepin-3-yl]-acetylamino}-phenyl)-pyrrolidine-2-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (*R*)-1-(3-amino-phenyl)-pyrrolidine-2-carboxylic acid methyl ester (prepared in three steps from 3-nitro-iodobenzene and L-proline) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.40 (1H, br s), 9.48 (1H, s), 7.50 (2H, m), 7.24 (2H, m), 7.01 (1H, t), 6.78 (1H, d), 6.68 (1H, s), 6.13 (1H, d), 4.78 (2H, s), 4.20 (1H, d), 4.06 (1H, d), 3.90 (1H, d), 3.21 (2H, m), 2.90 (1H, m), 2.20-1.20 (14H, m), 1.13 (9H, s). The compound was further characterised as the *N*-methyl-D-glucamine salt. Found: C 60.09, H 7.50, N 10.41%. C₃₃H₄₁N₅O₅•C₇H₁₇NO₅•H₂O requires: C 59.98, H 7.55, N 10.49%.

### Example 77. 2-[5-Cyclohexyl-1-(3,3-dimethyl-2-oxo-butyl)-2-oxo-1,2-dihydro-3H-1,3,4-benzotriazepin-3-yl]-N-(3-(1H-imidazol-4-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 4-(3-amino-phenyl)-imidazole-1-carboxylic acid *tert*-butyl ester (prepared in three steps from 2-bromo-3'-nitroacetophenone) was used in place of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.35 (1H, s), 7.82 (1H, s), 7.68 (1H, s), 7.51-7.46 (3H, m), 7.33-7.19 (5H, m), 7.04 (1H, d, 8.1), 4.78 (1H, d, 17.1), 4.66 (1H, d, 17.1), 4.35 (1H, d, 16.5), 4.22 (1H, d, 16.5), 2.80 (1H, m), 2.05-1.62 (5H, m), 1.31-1.18 (14H, m). Found C 62.46, H 7.01, N 14.35%; C₃₁H₃₆N₆O₃•3.0H₂O requires C 62.61, H 7.12, N 14.13%.

### Example 78. 3-{2-[5-Cyclohexyl-1(-2-hydroxy-3,3-dimethyl-butyl)-2-oxo-1,2-dihydro-3H-1,3,4-benzotriazepin-3-yl]-acetylamino}-5-methylamino-benzoic acid methyl ester.

Sodium borohydride (5mg, 0.13mmol) was added to an ice-cooled solution of 3-{2-[5-cyclohexyl-1-(3,3-dimethyl-2-oxo-butyl)-2-oxo-1,2-dihydro-3*H*-1,3,4-benzotriazepin-3-yl]-acetylamino}-5-methylamino-benzoic acid methyl ester (Example 74) (64mg, 0.11mmol) in MeOH (2ml). The reaction mixture was stirred at 0-5°C for 40mins, then at room temperature for 2h. Saturated NH₄Cl solution (10ml) was added and the product was extracted with CHCl₃ (15ml). The organic phase was dried (MgSO₄), filtered and the filtrate was evaporated to afford the title compound (60mg, 97%). ¹H NMR (CDCl₃) 8.10-8.79 (1H, m), 7.55 (2H, m), 7.36 (2H, m), 7.24 (1H, m), 6.95 (1H, s), 6.70 (1H, m), 4.46 (1H, m), 4.29-4.04 (2H, m), 3.89 (4H, m), 3.82-3.60 (1H. m), 3.43-3.23 (1H, m), 2.81 (4H, m), 2.15-1.66 (7H, m), 1.33-1.24 (3H, m), 0.97 and 0.93 (9H, s × 2). Found C 63.17, H 7.59, N 11.85%; C₃₁H₄₁N₅O₅•1.5H₂O requires C 63.03, H 7.51, N 11.86%.

### Example 79. 3-{2-[5-Cyclohexyl-1-(2-cyclohexyl-2-oxo-ethyl)-2-oxo-1,2-dihydro-3H-1,3,4-benzotriazepin-3-yl]-acetylamino}-benzoic acid.

**Step a.** *[5-Cyclohexyl-1-(2-cyclohexyl-2-oxo-ethyl)-2-oxo-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that 2-bromo-cyclohexyl-ethanone was used in step c instead of 1-bromo-3,3-dimethyl-butan-2-one. ¹H NMR (CDCl₃) 7.45 (2H, m), 7.23 (1H, m), 7.01 (1H, d), 4.56 (2H, d), 4.25 (1H, d), 3.89 (1H, d), 2.82 (1H, m), 2.47 (1H, m), 2.08-1.61 (11H, m), 1.46-1.19 (9H, m).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [5-cyclohexyl-1-(2-cyclohexyl-2-oxo-ethyl)-2-oxo-1,2-dihydro-3*H*-1,3,4-benzotriazepin-3-yl]-acetic acid (Example 79, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.52 (1H, s), 8.03 (1H, d), 7.82 (2H, m), 7.49 (2H, m), 7.41 (1H, t), 7.32 (1H, t), 7.06 (1H, d), 4.69 (1H, d), 4.47 (1H, d), 4.27 (2H, m), 2.81 (1H, m) 2.48 (1H, m), 2.05-1.69 (11H, m), 1.48-1.23 (9H, m). The compound was further characterised as the *N*-methyl-D-glucamine salt. Found: C 57.41, H 7.60, N 9.02%; C₃₁H₃₆N₄O₅•C₇H₁₇NO₅•2.9H₂O requires: C 57.59, H 7.48, N 8.84%.

### Example 80. (3-{2-[5-Cyclohexyl-1-(2-cyclohexyl-2-oxo-ethyl)-2-oxo-1,2-dihydro-3H-1,3,4-benzotriazepin-3-yl]-acetylamino}-phenyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclohexyl-2-oxo-ethyl)-2-oxo-1,2-dihydro-3*H*-1,3,4-benzotriazepin-3-yl]-acetic acid (Example 79, step a) and (3-amino-phenyl)-acetic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.27 (1H, s), 7.47 (2H, m), 7.27 (4H, m), 7.04 (1H, d), 6.99 (1H, d), 4.64 (1H, d), 4.46 (1H, d), 4.31 (1H, d), 4.17 (1 H, d), 3.60 (2H, s), 2.78 (1H, m) 2.46 (1H, m), 2.04-1.66 (11H, m), 1.44-1.23 (9H, m). The compound was further characterised as the *N*-methyl-D-glucamine salt. Found: C 56.73, H 7.67, N 8.65%; C₃₂H₃₈N₄O₅•C₇H₁₇NO₅•3.9H₂O requires: C 56.87, H 7.68, N 8.50%.

### Example 81. (3-{2-[1-(2-Cyclopentyl-2-oxo-ethyl)-2-oxo-5-pyridin-2-yl-1,2-dihydro-3H-1,3,4-benzotriazepin-3-yl]-phenyl)-acetic acid.

**Step a.** *[1-(2-Cyclopentyl-2-oxo-ethyl)-2-oxo-5-pyridin-2-yl-1,2-dihydro-3*H*-1,3,4-benzotriazapin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that (2-amino-phenyl)-pyridin-2-yl-methanone was used in step a instead of (2-amino-phenyl)-cyclohexyl-methanone and 2-bromo-1-cyclopentyl-ethanone replaced 1-bromo-3,3-dimethyl-butan-2-one in step c.

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [1-(2-cyclopentyl-2-oxo-ethyl)-2-oxo-5-pyridin-2-yl-1,2-dihydro-3*H*-1,3,4-benzotriazapin-3-yl]-acetic acid and (3-amino-phenyl)-acetic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.20 (1H, br s), 9.99 (1H, s), 8.57 (1H, d), 7.91 (2H, m), 7.43 (4H, m), 7.20 (4H, m), 6.91 (1H, d), 4.66 (2H, br s), 4.40 (2H, br m), 3.50 (2H, s), 3.00 (1H, m), 1.75-1.47 (8H, m). The compound was further characterised as the *N*-methyl-D-glucamine salt. Found: C 56.91, H 6.61, N 10.65%; C₃₀H₂₉N₅O₅•C₇H₁₇NO₅•2.5H₂O requires: C 56.99, H 6.59, N 10.78%.

### Example 82. [3-(2-{5-Cyclohexyl-1-[2-(1-methyl-cyclopentyl)-2-oxo-ethyl]-2-oxo-1,2-dihydro-3H-1,3,4-benzotriazepin-3-yl}-acetylamino)-phenyl]-acetic acid.

**Step a.** *{5-Cyclohexyl-1-[2-(1-methyl-cyclopentyl)-2-oxo-ethyl]-2-oxo-1,2-dihydro-3*H*-1,3,4-benzotriazepin-3-yl}-acetic acid* was obtained using a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that 2-bromo-1-(1-methyl-cyclopentyl)-ethanone was used in step c instead of 1-bromo-3,3-dimethyl-butan-2-one. ¹H NMR (CDCl₃) 11.00 91H, br s), 7.45 (2H, m), 7.25 (1H, m), 6.99 (1H, d), 4.67 (2H, m), 4.24 (1H, d), 3.86 (1H, d), 2.83 (1H, m), 2.17-1.22 (21H, m).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that {5-cyclohexyl-1-[2-(1-methyl-cyclopentyl)-2-oxo-ethyl]-2-oxo-1,2-dihydro-3*H*-1,3,4-benzotriazepin-3-yl}-acetic acid (Example 82, step a) and (3-amino-phenyl)-acetic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.27 (1H, s), 7.46 (2H, m), 7.35-7.20 (4H, m), 7.03 (1H, d), 6.99 (1H, d), 4.75 (1H, d), 4.58 (1H, d), 4.30 (1H, d), 4.18 (1H, d), 3.61 (2H, s), 2.78 (1H, m), 2.15-1.23 (21H, m). The compound was further characterised as the *N*-methyl-D-glucamine salt. Found: C 56.76, H 7.92, N 8.59%; C₃₂H₃₈N₄O₅•C₇H₁₇NO₅•4.0H₂O requires: C 56.69, H 7.69, N 8.48%.

### Example 83. 3-({2-[5-Cyclohexyl-1-(3,3-dimethyl-2-oxo-butyl)-2-oxo-1,2-dihydro-3H-1,3,4-benzotriazepin-3-yl]-acetyl)-methyl-amino)-benzoic acid.

**Step a.** *3-({2-[5-Cyclohexyl-1-(3,3-dimethyl-2-oxo-butyl)-2-oxo-1,2-dihydro-3*H*-1,3,4-benzotriazepin-3-yl]-acetyl}-methyl-amino)-benzoic acid methyl ester*. Methyl trifluoromethane sulfonate (329mg, 2.0mmol) was added drop-wise to an ice-cooled solution of 1,1'-carbonyldiimidazole (162mg, 1.0mml) in nitromethane (2ml). The solution was stirred at this temperature for 5 min, then added to a suspension of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) (400mg, 1.0mmol) in nitromethane (2ml). The resulting mixture was stirred for 10 min at room temperature, then a solution of 3-methylamino-benzoic acid methyl ester (165mg, 1.0mmol) in nitromethane (2ml) was added. The mixture was stirred at room temperature for 19h, diluted with H₂O (30ml) and extracted with EtOAc (15ml × 3). The combined extracts were washed with H₂O (15ml × 2), and dried (MgSO₄). Filtration and evaporation of the solvent gave the crude product which was purified by flash column chromatography (EtOAc-hexane (4:1)) to give the product as an off-white solid (330mg, 60%). ¹H NMR (CDCl₃) 7.95 (1H, d), 7.85 (1H, s), 7.40-7.34 (4H, m), 7.14 (1H, t), 6.88 (1H, d), 4.65 (2H, br d), 4.25 (1 H, br), 3.98 (1H, br), 3.94 (3H, s), 3.25 (3H, *s),* 2.75 (1H, brm), 2.00-1.60 (5H, m), 1.35-1.20 (14H, m).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid (Example 2) except that 3-({2-[5-cyclohexyl-1-(3,3-dimethyl-2-oxo-butyl)-2-oxo-1,2-dihydro-3*H*-1,3,4-benzotriazepin-3-yl]-acetyl}-methyl-amino)-benzoic acid methyl ester (Example 83, step a) was used in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1). ¹H NMR (DMSO*-d*_{*6*}) 13.10 (1H, br), 7.85 (1H, m), 7.79 (1H, s), 7.51-7.41 (4H, m), 7.21 (1H, t), 7.11 (1H, d), 4.71 (2H, d), 4.15 (1H, br), 3.70 (1H, br), 3.12 (3H, s), 2.83 (1H, m), 1.85-1.00 (19H, m). The compound was further characterised as the *N*-methyl-D-glucamine salt. Found: C 57.48, H 7.61, N 9.25%; C₃₀H₃₆N₄O₅•C₇H₁₇NO₅•2.5H₂O requires C 57.50, H 7.56, N 9.06%.

### Example 84. 2-[5-Cyclohexyl-1-(3,3-dimethyl-2-oxo-butyl)-2-oxo-1,2-dihydro-3H-1,3,4-benzotriazepin-3-yl]-N-(3-hydroxy-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-aminophenol was used instead of 3-amino-benzoic acid methyl ester in step e. ¹H NMR (CDCl₃) 8.31 (1H, s), 7.68 (1H, s), 7.30 (2H, m), 7.26 (1H, m), 7.08 (2H, m), 6.60 (1H, m), 6.57 (1H, m), 6.41 (1H, m), 4.72 (1H, d), 4.65 (1H, d), 4.32 (1H, d), 4.22 (1H, d), 2.77 (1H, m), 1.98-1.69 (6H, m), 1.27-1.24 (13H, m). Found: C 67.78, H 7.28, N11.27%; C₂₈H₃₄N₄O₄•0.4H₂O requires: C 67.56, H 7.04, N 11.25%.

### Example 85. N-(3-Amino-phenyl)-2-[5-cyclohexyl-1-(3,3-dimethyl-2-oxo-butyl)-2-oxo-1,2-dihydro-3H-1,3,4-benzotriazepin-3-yl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-phenyl)-carbamic acid *tert*-butyl ester was used in place of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 11.28 (1H, br s), 8.85 (1H, s), 7.69 (1H, s), 7.57-7.27 (6H, m), 7.04 (1H, m), 4.76 (1H, d), 4.63 (1H, d), 4.21 (2H, m), 2.80 (1H, m), 2.18-1.35 (6H, m), 1.32-1.22 (13H, m). The product was further characterised as the hydrochloride salt. Found: C 59.71, H 7.33, N, 12.48%; C₂₈H₃₅N₅O₃•HCl•2.0H₂O requires: C 59.83, H 7.17, N 12.46%.

### Example 86. 2-[5-Cyclohexyl-1-(3,3-dimethyl-2-oxo-butyl)-2-oxo-1,2-dihydro-3H-1,3,4-benzotriazepin-3-yl]-N-[3-(1H-tetrazol-5-ylmethylsulfanyl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-(1*H*-tetrazol-5-ylmethylsulfanyl)-aniline was used instead of 3-amino-benzoic acid methyl ester in step e. ¹H NMR (CDCl₃) 8.82 (1H, s), 7.72 (1H, br s), 7.40 (3H, m), 7.27 (1H, m), 7.15 (1H, t), 6.96 (3H, m), 4.70 (2H, q), 4.41 (2H, s), 4.24 (2H, s), 2.82 (1H, m), 2.1-1.6 (6H, m), 1.28 (4H, m), 1.22 (9H, s).

### Example 87. 2-{5-Cyclohexyl-1-[2-(1-methyl-cyclopentyl)-2-oxo-ethyl]-2-oxo-1,2-dihydro-3H-1,3,4-benzotriazepin-3-yl}-N-[3-(5-oxo-2,5-dihydro-[1,2,4]oxadiazol-3-yl)-phenyl]-acetamide.

The title compound was obtained by the method used in the preparation of 2-[5-cyclohexyl-1-(3,3-dimethyl-2-oxo-butyl)-2-oxo-1,2-dihydro-3*H*-1,3,4-benzotriazepin-3-yl]-*N*-[3-(5-oxo-2,5-dihydro-[1,2,4]oxadiazol-3-yl)-phenyl]-acetamide (Example 59) except that {5-cyclohexyl-1-[2-(1-methyl-cyclopentyl)-2-oxo-ethyl]-2-oxo-1,2-dihydro-3*H*-1,3,4-benzotriazepin-3-yl}-acetic acid (Example 82, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d). ¹H NMR (CDCl₃) 11.00 (1H, br s), 8.84 (1H, s), 7.91 (1H, s), 7.58 (2H, m), 7.44 (3H, m), 7.28 (1H, t), 7.04 (1H, d), 4.81 (1H, d), 4.55 (1H, d), 4.21 (2H, s), 2.80 (1H, m), 2.09-1.24 (21H, m). The compound was further characterised as the *N*-methyl-D-glucamine salt. Found: C 56.28, H 6.94, N 11.95%; C₃₂H₃₆N₆O₅•C₇H₁₇NO₅•2.8H₂O requires: C 56.45, H 7.11, N 11.82%.

### Example 88. 3-(2-{5-Cyclohexyl-1-[2-(1-methyl-cyclopentyl)-2-oxo-ethyl]-2-oxo-1,2-dihydro-3H-1,3,4-benzotriazepin-3-yl}-acetylamino)-benzoic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that {5-cyclohexyl-1-[2-(1-methyl-eyelopentyl)-2-oxo-ethyl]-2-oxo-1,2-dihydro-3*H*-1,3,4-benzotriazepin-3-yl}-acetic acid (Example 82, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example I, step d) in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.51 (1H, s), 8.03 (1H, d), 7.87 (1H, s), 7.81 (1H, d), 7.53-7.27 (4H, m), 7.05 (1H, d), 4.78 (1H, d), 4.57 (1H, d), 4.26 (2H, m), 2.81 (1H, m), 2.16-1.23 (21H, m). The compound was further characterised as the *N*-methyl-D-glucamine salt. Found: C 57.94, H 7.53, N 8.71%; C₃₁H₃₆N₄O₅•C₇H₁₇NO₅•2.8H₂O requires: C 57.80 H 7.47, N 8.87%.

### Example 89. 2-[5-Cyclohexyl-1-(3,3-dimethyl-2-oxo-butyl)-2-oxo-1,2-dihydro-3H-1,3,4-benzotriazepin-3-yl]-N-[3-(1H-imidazol-1-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-imidazol-1-yl-phenylamine (prepared in two steps from 1-fluoro-nitrobenzene and imidazole) was used instead of 3-amino-benzoic acid methyl ester in step e. ¹H NMR (CDCl₃) 8.57 (1H, s), 7.86 (1H, s), 7.71 (1H, t, 1.8), 7.52-7.28 (6H, m), 7.19 (1H, s), 7.11-7.08 (1H, m), 7.04-7.01 (1H, m), 4.84 (1H, d, 17.7), 4.58 (1H, d, 17.7), 4.24-4.23 (2H, m), 2.83-2.77 (1H, m), 2.05-1.67 (6H, m), 1.38-1.19 (13H, m). Found: C 68.06, H 6.75, N 15.09%; C₃₁H₃₆N₆O₃•0.4H₂O requires: C 67.87, H 6.78, N 15.32%.

### Example 90. N-(3H-Benzimidazol-5-yl)-2-[5-cyclohexyl-1-(3,3-dimethyl-2-oxo-butyl)-2-oxo-1,2-dihydro-3H-1,3,4-benzotriazepin-3-yl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 6-amino-benzimidazole-1-carboxylic acid *tert*-butyl ester (prepared in two steps from 5(6)-nitro-benzimidazole) was used in place of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.44 (1H, s), 8.24 (1H, br s), 7.98 (1H, s), 7.60-7.47 (3H, m), 7.32-7.27 (1H, m), 7.04 (1H, d, 8.1), 6.84 (1H, br s), 4.77 (1H, d, 18), 4.67 (1H, d, 18), 4.40 (1H, d, 16.5), 4.25 (1H, d, 16.5), 2.84-2.76 (1H, m), 2.05-1.65 (6H, m), 1.45-1.24 (13H, m). Found C 65.40, H 6.89, N 15.79%; C₂₉H₃₄N₆O₃•H₂O requires C 65.39, H 6.81, N 15.78%.

### Example 91. 3-{2-[5-Cyclohexyl-1-(3,3-dimethyl-2-oxo-butyl)-2-oxo-1,2-dihydro-3H-1,3,4-benzotriazepin-3-yl]-acetylamino}-5-methyl-benzoic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-amino-5-methyl-benzoic acid methyl ester (prepared in three steps from 4-bromo-3-methylbenzoic acid) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.44 (1H, *s),* 7.87 (1H, *s),* 7.62 (2H, m), 7.53-7.47 (2H, m), 7.34-7.28 (1H, m), 7.05 (1H, d, 8.1), 4.81-4.58 (2H, m), 4.26-4.19 (2H, m), 2.84-2.80 (1H, m), 2.39 (3H, s), 2.05-173 (6H, m), 1.41-1.19 (13H, m). The compound was further characterised as the *N*-methyl-D-glucamine salt. Found C 58.77, H 7.70, N 9.33%; C₃₀H₃₆N₄O₅•C₇H₁₇NO₅•1.5H₂O requires C 58.87, H 7.48, N 9.28%.

### Example 92. N-[3-(Acetyl-methyl-amino)-5-methylamino-phenyl]-2-[5-cyclohexyl-1-(3,3-dimethyl-2-oxo-butyl)-2-oxo-1,2-dihydro-3H-1,3,4-benzotriazepin-3-yl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *N*-(3-amino-5-methylamino-phenyl)-*N*-methyl-acetamide (prepared in four steps from 3,5-difluoronitrobenzene) was used instead of 3-amino-benzoic acid methyl ester in step e. ¹H NMR (CDCl₃) 8.29 (1H, s), 7.51-7.43 (2H, m), 7.30-7.24 (1H, m), 7.03 (1H, d, 8.4), 6.92 (1H, s), 6.39 (1H, s), 6.10 (1H, s), 4.79 (1H, d, 18), 4.62 (1H, d, 18), 4.29 (1H, d, 16.5), 4.21 (1H, d, 16.5), 3.21 (3H, s), 2.83-2.75 (4H, m), 2.04-1.67 (9H, m), 1.33-1.17 (13H, m).

### Example 93. 3-[2-(1-Carboxymethyl-5-cyclohexyl-2-oxo-1,2-dihydro-3H-1,3,4-benzotriazepin-3-yl)-acetylamino]-benzoic acid.

Step a. *(1*-tert-*Butoxycarbonylmethyl-5-cyclohexyl-2-oxo-1,2-dihydro-3*H*-1,3,4-benzotriazepin-3-yl)-acetic acid* was obtained by the method used in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that (1-*tert*-butoxycarbonylmethyl-5-cyclohexyl-2-oxo-1,2-dihydro-3*H*-1,3,4-benzotriazepin-3-yl)-acetic acid ethyl ester (Example 68, step a) was used in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c). ¹H NMR (CDCl₃) 7.45 (2H, m), 7.27 (1H. m), 7.14 (1H, d), 4.41-3.93 (4H, br m), 2.80 (1H, m), 2.02-1.45 (6H, m), 1.46 (9H, m), 1.21 (4H, m).

**Step b.** 3-[2-(1-*tert*-Butoxycarbonylmethyl-5-cyclohexyl-2-oxo-1,2-dihydro-3*H*-1,3,4-benzotriazepin-3-yl)-acetylamino]-benzoic acid methyl ester was obtained by the method used in the preparation of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1, step e) except that (1-tert-butoxycarbonylmethyl-5-cyclohexyl-2-oxo-1,2-dihydro-3H-1,3,4-benzotriazepin-3-yl)-acetic acid was used instead of [5-cyclohexyl-1-(3,3-dimethyl-2-oxo-butyl)-2-oxo-1,2-dihydro-3H-1,3,4-benzotriazepin-3-yl]-acetic acid (Example 1, step d). ¹H NMR (CDCl₃) 8.51 (1H, s), 7.89 (1H, d), 7.80 (1H, t), 7.74 (1H, d), 7.50 (2H, m), 7.35 (2H, m), 7.18 (1H, d), 4.43-4.09 (4H, m), 3.91 (3H, s), 2.80 (1H, m), 2.05-1.60 (6H, m), 1.43 (9H, s), 1.24 (4H, m).

**Step c.** The title compound was obtained by the method used in the preparation of (5-cyclohexyl-3-ethoxycarbonylmethyl-2-oxo-2,3-dihydro-3H-1,3,4-benzotriazepin-1-yl)-acetic acid (Example 68, step b) except that 3-[2-(1-tert-butoxycarbonylmethyl-5-cyclohexyl-2-oxo-1,2-dihydro-3H-1,3,4-benzotriazepin-3-yl)-acetylamino]-benzoic acid methyl ester (Example 93, step b) was used instead of (1-tert-butoxycarbonylmethyl-5-cyclohexyl-2-oxo-1,2-dihydro-3H-1,3,4-benzotriazepin-3-yl)-acetic acid ethyl ester (Example 68, step a), followed by reaction of the product obtained, in place of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1), according to the method of Example 2. ¹H NMR (CDCl₃) 9.15 (2H, br s), 8.84 (1H, *s),* 8.03 (1H, d), 7.73 (1H, s), 7.67 (1H, d), 7.48 (2H, m), 7.30 (2H, m), 7.14 (1H, d), 4.62 (1H, d), 4.29 (3H, m), 2.81 (1H, m), 2.07-1.74 (6H, m), 1.27 (4H, m). The compound was further characterised as the bis(N-methyl-D-glucamine) salt. Found: C 50.94, H 7.29, N 8.05%; C₂₅H₂₆N₄O₆•C₁₄H₃₄N₂O₁₀•3.0H₂O•1.2C₄H₈O₂ requires: C 51.05 H 7.41, N 8.16%.

### Example 94. (6-{2-[5-Cyclohexyl-1-(3,3-dimethyl-2-oxo-butyl)-2-oxo-1,2-dihydro-3H-1,3,4-benzotriazepin-3-yl]-acetylamino}-benzimidazol-1-yl)-acetic acid.

The of title compound was obtained as the hydrochloride salt by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (5-amino-benzimidazol-1-yl)-acetic acid *tert*-butyl ester (prepared in two steps from 5(6)-nitro-benzimidazole) was used instead of 3-amino-benzoic acid methyl ester in step e, followed by reaction of the product obtained, in place of (1-*tert*-butoxycarbonylmethyl-5-cyclohexyl-2-oxo-1,2-dihydro-3*H*-1,3,4-benzotriazepin-3-yl)-acetic acid ethyl ester (Example 68, step a) according to the method of Example 68, step b. ¹H NMR (DMSO-*d*_{*6*}) 10.27-10.23 (1H, br s), 9.23 and 9.10 (1H, s), 8.19 (1H, s), 7.77-7.73 (1H, m), 7.55-7.40 (3H, m), 7.26-7.15 (2H, m), 5.40 and 5.32 (2H, m), 4.79 (2H, s), 4.41 (1H, br d, 15), 3.99 (1H, br d, 15), 2.87 (1H, m), 1.83-1.65 (6H, m), 1.30-1.06 (13H, m). Found: C 61.13, H 6.08, N 13.77%; C₃₁H₃₆N₆O₅•HCl requires: C 61.13, H 6.12, N 13.77%.

### Example 95. 3-[2-(1-tert-Butoxycarbonylmethyl-5-cyclohexyl-2-oxo-1,2-dihydro-3H-1,3,4-benzotriazepin-3-yl)-acetylamino]-benzoic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid (Example 2) except that 3-[2-(1-*tert*-butoxycarbonylmethyl-5-cyclohexyl-2-oxo-1,2-dihydro-3*H*-1,3,4-benzotriazepin-3-yl)-acetylamino]-benzoic acid methyl ester (Example 93, step b) was used in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1). ¹H NMR (CDCl₃) 8.53 (1H, s), 8.02 (1H, d), 7.81 (2H, m), 7.50 (2H, m), 7.37 (2H, m), 7.18 (1H, d), 4.45-4.12 (4H, m), 2.81 (1H, m), 2.05-1.65 (6H, m), 1.44 (9H, s), 1.27 (4H, m). The compound was further characterised as the *N*-methyl-D-glucamine salt. Found: C 56.81, H 7.01, N 8.72%; C₂₉H₃₄N₄O₆•C₇H₁₇NO₅•1.5H₂O•0.3C₄H₈O₂ requires: C 57.04, H 7.26, N 8.94%.

### Example 96. [(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-methyl-amino]-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [(3-amino-phenyl)-methyl-amino]-acetic acid methyl ester (prepared in three steps from sarcosine and 3-nitro-iodobenzene) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.40 (1H, br s), 9.47 (1H, s), 7.50 (2H, m), 7.20 (2H, m), 7.02 (1H, t), 6.80 (2H, m), 6.33 (1H, m), 4.78 (2H, s), 4.25-3.75 (2H, m), 4.00 (2H, s) 2.91 (3H, s), 2.90 (1H, m), 2.00-1.16 (10H, m), 1.13 (9H, s). The compound was further characterised as the *N*-methyl-D-glucamine salt. Found: C 58.95, H 7.81, N 10.76%. C₃₁H₃₉N₆O₅•C₇H₁₇NO₅•H₂O requires: C 58.90, H 7.55, N 10.85%.

### Example 97. [4-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-imidazol-1-yl]-acetic acid.

The of title compound was obtained as the hydrochloride salt by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [4-(3-amino-phenyl)-imidazol-1-yl]-acetic acid *tert*-butyl ester (prepared in three steps from 2-bromo-3'-nitro-acetophenone) was used instead of 3-amino-benzoic acid methyl ester in step e, followed by reaction of the product obtained, in place of (1-*tert*-butoxycarbonylmethyl-5-cyclohexyl-2-oxo-1,2-dihydro-3*H*-1,3,4-benzotriazepin-3-yl)-acetic acid ethyl ester (Example 68, step a) according to the method of Example 68, step b. ¹H NMR (DMSO-*d*_{*6*/}/D₂O) 10.02 (1H, s), 8.89 and 8.79 (1H, s), 8.04 (1H, s), 7.95 and 7.91 (1H, s), 7.53-7.34 (5H, m), 7.26-7.12 (2H, m), 5.14 and 5.08 (2H, s), 4.76-4.74 (2H, m), 4.31 (1H, m), 4.01-3.96 (1H, m), 2.87-2.84 (1H, m), 1.82-1.64 (6H, m), 1.32-1.04 (13H, m). Found: C 59.97, H 6.48, N 12.22%; C₃₃H₃₈N₆O₅•1.6HCl•0.5C₄H₈O₂ requires: C *59.96, H* 6.27, N 11.99%.

### Example 98. 3-[2-(Carbamoylmethyl-5-cyclohexyl-2-oxo-1,2-dihydro-3H-1,3,4-benzotriazepin-3-yl)-acetylamino]-benzoic acid.

**Step a.** *(1-Carbamoylmethyl-5-cyclohexyl-2-oxo-1,2-dihydro-3H-1,3,4-benzotriazepin-3-yl)-acetic acid ethyl ester.* To a solution of (5-cyclohexyl-3-ethoxycarbonylmethyl-2-oxo-2,3-dihydro-3H-1,3,4-benzotriazepin-1-yl)-acetic acid (Example 68, step b) (200mg, 0.52mmol) in DMF-THF (1:1 / 10ml) were added EDC (150mg, 0.77mmol), HOBt (100mg, 0.77mmol) and DMAP (20mg). The solution was stirred at room temperature for 1h, and then ammonia was bubbled into the reaction mixture for 5min. The reaction mixture was stirred at room temperature for 16h, H₂O (30ml) was added and the mixture was extracted with EtOAc (20ml × 2). The extracts were washed with brine, dried (MgSO₄) and the solvent was evaporated. The residue was purified by flash column chromatography (DCM-EtOAc (1:1)) to afford the product (141mg, 71%). ¹H NMR (300MHz, CDCl₃) 7.41 (2H, m), 7.22 (2H, m), 6.61 (1H, br s), 5.66 (1H, br s), 4.31-4.11 (6H, m), 2.75 (1H, m), 1.75 (6H, m), 1.23 (7H, m).

**Step b.** The title compound was obtained using steps d and e of the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that (1-carbamoylmethyl-5-cyclohexyl-2-oxo-1,2-dihydro-3*H*-1,3,4-benzotriazepin-3-yl)-acetic acid ethyl ester (Example 98, step a) was used in step d instead of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.88 (1H, br s), 11.12 (1H, s), 9.97 (1H, s), 8.19 (1H, s), 7.80 (1H, d), 7.61 (1H, d), 7.55 (1H, m), 7.45 (1H, m), 7.25 (1H, dd), 5.60 (1H, m), 4.27 (2H, m), 3.80 (2H, m), 2.15 (1H, m), 1.67-0.87 (9H, m). The compound was further characterised as the *N*-methyl-D-glucamine salt. Found: C 55.13, H 6.93, N 11.11%; C₂₅H₂₇N₅O₅•C₇H₁₇NO₅•1.3H₂O•0.7C₄H₈O₂ requires: C 55.16, H 6.94, N 11.09%.

### Example 99. (6-{2-[5-Cyclohexyl-1-(3,3-dimethyl-2-oxo-butyl)-2-oxo-1,2-dihydro-3H-1,3,4-benzotriazepin-3-yl]-acetylamino}-indazol-1-yl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (6-amino-indazol-1-yl)-acetic acid methyl ester (prepared in two steps from 6-nitro-indazole) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 13.0 (1H, br s), 9.97 (1H, s), 7.95-7.94 (2H, m), 7.66-7.46 (3H, m), 7.27-7.03 (3H, m), 5.11 (2H, s), 4.80-4.79 (2H, m), 4.34 (1H, m), 4.03 (1H, m), 2.88-2.84 (1H, m), 1.90-1.65 (6H, m), 1.34-1.08 (13H, m).

### Example 100. (3-{2-[5-Cyclohexyl-1-(2-cyclohexyl-2-oxo-ethyl)-2-oxo-1,2-dihydro-3H-1,3,4-benzotriazepin-3-yl]-acetylamino}-phenylsulfanyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [5-cyclohexyl-1-(2-cyclohexyl-2-oxo-ethyl)-2-oxo-1,2-dihydro-3*H*-1,3,4-benzotriazepin-3-yl]-acetic acid (Example 79, step a) and (3-amino-phenylsulfanyl)-acetic acid ethyl ester were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.44 (1H, s), 7.45 (3H, m), 7.38 (1H, d), 7.24 (2H, m), 7.11 (1H, d), 7.05 (1H, d), 6.70 (1H, br s), 4.70 (1H, d), 4.44 (1H, d), 4.21 (2H, m), 3.66 (2H, s), 2.80 (1H, m), 2.47 (1H, m), 2.18-1.66 (11H, m), 1.44-1.22 (9H, m). The compound was further characterised as the *N*-methyl-D-glucamine salt. Found: C 56.91, H 6.96, N 8.36%; C₃₂H₃₈N₄O₅S•C₇H₁₇NO₅•2.0H₂O requires: C 56.99, H 7.24, N 8.52%.

### Example 101. 2-[5-Cyclohexyl-1-(3,3-dimethyl-2-oxo-butyl)-2-oxo-1,2-dihydro-3H-1,3,4-benzotriazepin-3-yl]-N-(2-methylamino-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that *N*-methyl-benzene-1,2-diamine (prepared in two steps from 2-fluoro-nitrobenzene) was used instead of 3-amino-benzoic acid methyl ester in step e. ¹H NMR (CDCl₃) 8.26 (1H, s), 7.48-7.41 (2H, m), 7.35-7.32 (1H, m), 7.26-7.20 (1H, m), 7.17-7.11 (1H, m), 7.00 (1H, d,), 6.74-6.68 (2H, m), 4.78 (1H, d), 4.60 (1H, d), 4.20-4.19 (3H, m), 2.84-2.76 (4H, m), 2.07-1.71 (6H, m), 1.44-1.02 (13H, m). Found: C 66.60, H 7.02, N 13.15%; C₂₉H₃₇N₅O₃•0.3CH₂Cl₂ requires: C 66.51, H 7.16, N 13.24%.

### Example 102. 3-{2-[1-(3,3-Dimethyl-2-oxo-butyl)-5-(4-methyl-cyclohexyl)-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-phenyl)-(4-methyl-cyclohexyl)-methanone (prepared from 4-methyl-cyclohexanol by bromination using phosphorus pentabromide (A. L. J. Beckwith et al.: *J. Chem. Soc. Perkin Trans. II*, 1983, 661) and reaction of the corresponding Grignard reagent with 2-aminobenzonitrile (J. A. Robl, *Synthesis*, 1991, 56-58)) was used in step a instead of (2-amino-phenyl)-cyclohexyl-methanone, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.51 (1H, br s), 8.02 (1H, d), 7.85-7.79 (2H, m), 7.53-7.29 (4H, m), 7.03 (1H, d), 4.78 (1H, d), 4.60 (1H, d), 4.30 (1H, d), 4.22 (1H, d), 2.75 (1H, m), 2.00 (1H, br m), 1.90-1.65 (4H, br m), 1.50-1.20 (11H, m), 1.10-0.85 (5H, m). The compound was further characterised as the *N*-methyl-D-glucamine salt. Found: C 59.10, H 7.20, N 9.07%; C₃₀H₃₆N₄O₅•C₇H₁₇NO₅•H₂O requires C 59.58, H 7.43, N 9.39%.

### Example 103. (3-{2-[1-(3,3-Dimethyl-2-oxo-butyl)-5-(4-methyl-cyclohexyl)-2-oxo-1,2-dihydro-3H-1,3,4-benzotriazepin-3-yl]-acetylamino}-phenyl)-acetic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-phenyl)-(4-methyl-cyclohexyl)-methanone was used in step a instead of (2-amino-phenyl)-cyclohexyl-methanone, and (3-amino-phenyl)-acetic acid methyl ester replaced 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.27 (1H, br), 7.47-7.44 (2H, m), 7.33-7.23 (4H, m), 7.03-6.98 (2H, m), 4.75 (1H, d), 4.61 (1H, d), 4.31 (1H, d), 4.17 (1H, d), 3.60 (2H, s), 2.70 (1H, m), 2.00 (1H, br), 1.85-1.65 (4H, m), 1.45-1.25 (2H, m), 1.23 (9H, s), 1.05-0.80 (5H, m). The compound was further characterised as the *N*-methyl-D-glucamine salt. Found: C 59.28, H 7.36, N 8.90%; C₃₁H₃₈N₄O₅•C₇H₁₇NO₅•1.5H₂O requires C 59.36, H 7.60, N 9.11%.

### Example 104. N-(3,5-Bis-methylamino-phenyl)-2-[5-cyclohexyl-1-(3,3-dimethyl-2-oxo-butyl)-2-oxo-1,2-dihydro-3H-1,3,4-benzotriazepin-3-yl]-acetamide.

The title compound was obtained as the tri-hydrochloride salt by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-5-methylamino-phenyl)-methyl-carbamic acid *tert*-butyl ester (prepared in four steps from 3,5-difluoro-nitrobenzene) was used instead of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-propyl-carbamic acid *tert*-butyl ester (Example 41, step a) according to the method of Example 41, step b. ¹H NMR (DMSO-*d*_{*6*}) 9.83 (1H, br s), 7.55-7.45 (2H, m), 7.26-7.14 (2H, m), 6.79 (2H, br s), 6.26 (1H, s), 4.77 (2H, s), 4.31-4.26 (1H, m), 3.96-3.91 (1H, m), 3.65 (4H, br s), 2.86 (1H, m), 2.72 (6H, m), 1.84-1.64 (6H, m), 1.34-1.10 (13H, m). Found: C 55.92, H 6.79, N 12.84%; C₃₀H₄₀N₆O₃•3.0HCl requires: C 56.11, H 6.75, N 13.09%.

### Example 105. 2-[5-Cyclohexyl-1-(3,3-dimethyl-2-oxo-butyl)-2-oxo-1,2-dihydro-3H-1,3,4-benzotriazepin-3-yl]-N-(4-methoxy-3-methylamino-phenyl)-acetamide.

The title compound was obtained as the hydrochloride salt by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (5-amino-2-methoxy-phenyl)-methyl-carbamic acid *tert*-butyl ester (prepared in two steps from 2-methoxy-5-nitrophenylisocyanate) was used instead of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-propyl-carbamic acid *tert*-butyl ester (Example 41, step a) according to the method of Example 41, step b. ¹H NMR (CDCl₃) 11.00-10.00 (1H, br s), 8.16-8.12 (1H, m), 7.61-7.27 (5H, m), 7.02-6.89 (2H, m), 4.74-4.68 (2H, m), 4.37 (1H, d, 16.8), 4.18 (1H, d), 3.90 (3H, s), 2.99 (3H, s), 2.84 (1H, m), 2.05-1.70 (6H, m), 1.36-1.12 (13H, m). Found: C 61.98, H 7.12, N 11.65%; C₃₀H₃₉N₅O₄•1.4HCl requires: C 61.66, H 6.97, N 11.99%.

### Example 106. 2-[5-Cyclohexyl-1-(3,3-dimethyl-2-oxo-butyl)-2-oxo-1,2-dihydro-3H-1,3,4-benzotriazepin-3-yl]-N-(6-hydroxymethyl-pyridin-2-yl)-acetamide.

The title compound was obtained by the method used in the preparation 3-({2-[5-cyclohexyl-1-(3,3-dimethyl-2-oxo-butyl)-2-oxo-1,2-dihydro-3*H*-1,3,4-benzotriazepin-3-yl]-acetyl}-methyl-amino)-benzoic acid methyl ester (Example 83, step a), except that (6-amino-pyridin-2-yl)-methanol (prepared from 6-amino-pyridine-2-carboxylic acid methyl ester (T. R. Kelly *et al*.: *J. Org. Chem*., (1996), **61,** 4633) by reaction with lithium aluminium hydride) was used instead of 3-amino-benzoic acid methyl ester. ¹H NMR (CDCl₃) 7.38-7.33 (3H, m), 7.16 (1H, t), 6.93 (1H, d), 6.59 (1H, d), 6.39 (1H, d), 5.04 (2H, s), 4.67 (2H, s), 4.45 (1H, br), 4.40 (2H, br), 4.30 (1H, br), 2.70 (1H, m), 1.90-1.55 (7H, m), 1.40-1.15 (12H, m). Found: C 66.28, H 7.03, N 13.60%; C₂₈H₃₅N₅O₄ requires C 66.51, H 6.98, N 13.85%.

### Example 107. 2-[5-Cyclohexyl-1-(3,3-dimethyl-2-oxo-butyl)-2-oxo-1,2-dihydro-3H-1,3,4-benzotriazepin-3-yl]-N-[3-(2-methyl-thiazol-4-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-(2-methyl-thiazol-4-yl)-phenylamine (prepared in two steps from 2-bromo-3'-nitroacetophenone) was used instead of 3-amino-benzoic acid methyl ester in step e. ¹H NMR (CDCl₃) 8.38 (1H, br s), 7.85 (1H, d), 7.62-7.59 (1H, m), 7.50-7.46 (3H, m), 7.35-7.27 (3H, m), 7.04-7.01 (1H, m), 4.77 (1H, d), 4.67 (1H, d), 4.35 (1H, d), 4.22 (1H, d), 2.84-2.78 (4H, m), 2.05-1.71 (6H, m), 1.31-1.22 (13H, m). Found: C 66.74, H 6.49, N 12.05%; C₃₂H₃₇N₅O₃S•0.3H₂O requires: C 66.62, H 6.57, N 12.14%.

### Example 108. 4-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-thiazole-2-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 4-(3-amino-phenyl)-thiazole-2-carboxylic acid ethyl ester (prepared in two steps from 2-bromo-3'-nitroacetophenone) was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.49 (1H, br s), 8.09-8.07 (1H, m), 7.89 (1H, s), 7.63-7.61 (1H, m), 7.56-7.46 (3H, m), 7.40-7.28 (2H, m), 7.05 (1H, d, 7.8), 4.82 (1H, d), 4.62 (1H, d), 4.33-4.20 (2H, m), 2.81-2.77 (1H, m), 2.05-1.69 (6H, m), 1.44-1.16 (13H, m). The compound was further characterised as the *N*-methyl-D-glucamine salt. Found: C 56.05, H 6.47, N 9.82%; C₃₂H₃₅N₅O₅S•C₇H₁₇NO₅•2.0H₂O requires: C 56.24, H 6.78, N 10.09%.

### Example 109. (3-{2-[5-Cyclohexyl-1-(3,3-dimethyl-2-oxo-butyl)-2-oxo-1,2-dihydro-3H-1,3,4-benzotriazepin-3-yl]-acetylamino}-2-oxo-2H-pyridin-1-yl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (3-amino-2-oxo-2*H*-pyridin-1-yl)-acetic acid ethyl ester (prepared in two steps from 3-nitro-1*H*-pyridin-2-one) was used in place of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 9.04(1H, s), 8.50 (1H, dd), 7.48-7.41 (2H, m), 7.25 (1H, m), 6.99-6.96 (2H, m), 6.37 (1H, t), 4.70-4.60 (4H, m), 4.46 (1H, d), 4.15 (1H, d), 2.75 (1H, m), 2.00-1.65 (6H, m), 1.40-1.23 (14H, m). The compound was further characterised as the *N*-methyl-D-glucamine salt. Found: C 55.03, H 6.91, N 10.29%; C₂₉H₃₅N₅O₆•C₇H₁₇NO₅•2.5H₂O requires C 54.74, H 7.27, N 10.64%.

### Example 110. (3-{2-[5-Cyclohexyl-1-(4-hydroxy-3,3-dimethyl-2-oxo-butyl)-2-oxo-1,2-dihydro-3H-1,3,4-benzotriazepin-3-yl]-acetylamino}-phenyl)-acetic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that benzoic acid 4-bromo-2,2-dimethyl-3-oxo-butyl ester (prepared in four steps from 2,2-dimethyl-3-oxo-butyric acid ethyl ester) was used in step c instead 1-bromo-3,3-dimethyl-butan-2-one, and (3-amino-phenyl)-acetic acid methyl ester replaced 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.20 (1H, br s), 9.75 (1H, s), 7.55-7.11 (7H, m), 6.90 (1H, d), 4.90 (1H, br s), 4.79 (2H, s), 4.30-3.90 (2H, m), 3.48 (4H, s), 2.82 (1H, m), 2.00-1.10 (10H, m) 1.05 (6H, s). The compound was further characterised as the *N*-methyl-D-glucamine salt. Found: C 56.88, H 7.22, N 8.66%. C₃₀H₃₆N₄O₆•C₇H₁₇NO₅•2.0H₂O requires: C 56.98, H 7.36, N 8.98%.

### Example 111. 2-[5-Cyclohexyl-1-(2-cyclohexyl-2-oxo-ethyl)-3-oxo-1,2-dihydro-3H-1,3,4-benzotriazepin-3-yl]-N-[3-(5-oxo-2,5-dihydro-[1,2,4]oxadiazol-3-yl)-phenyl]-acetamide.

The title compound was obtained by the method used in the preparation of 2-[5-cyclohexyl-1-(3,3-dimethyl-2-oxo-butyl)-2-oxo-1,2-dihydro-3*H*-1,3,4-benzotriazepin-3-yl]-*N*-[3-(5-oxo-2,5-dihydro-[1,2,4]oxadiazol-3-yl)-phenyl]-acetamide (Example 59) except that [5-cyclohexyl-1-(2-cyclohexyl-2-oxo-ethyl)-2-oxo-1,2-dihydro-3*H*-1,3,4-benzotriazepin-3-yl]-acetic acid (Example 79, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d). ¹H NMR (CDCl₃) 10.9 (1H, br s), 8.82 (1H, s), 7.85 (1H, s), 7.60 (2H, m), 7.45 (3H, m), 7.28 (1H, t), 7.04 (1H, d),), 4.73 (1H, d), 4.45 (1H, d), 4.20 (2H, s), 2.80 (1H, m), 2.47 (1H, m), 2.01-1.64 (11H, m), 1.47-1.17 (9H, m). The compound was further characterised as the *N*-methyl-D-glucamine salt. Found: C 56.12, H 6.88, N 11.10%; C₃₂H₃₆N₆O₅•C₇H₁₇NO₅•2.8H₂O•0.4C₄H₈O₂ requires: C 56.34, H 7.20, N 11.33%.

### Example 112. 3-{2-[5-Cyclohexyl-1-(3,3-dimethyl-2-oxo-butyl)-8-methyl-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-4-methyl-phenyl)-cyclohexyl-methanone was used in step a instead of (2-amino-phenyl)-cyclohexyl-methanone, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.54 (1H, s), 8.05 (1H, d), 7.82 (1H, s), 7.79 (1H, d), 7.40 (2H, m), 7.11 (1H, d), 6.82 (1H, s), 4.65 (2H, q), 4.25 (2H, q), 2.78 (1H, m), 2.41 (3H, s), 2.00 (1H, br m), 1.75 (4H, m), 1.31 (5H, m), 1.24 (9H, s). The compound was further characterised as the *N*-methyl-D-glucamine salt. Found C 58.49, H 7.20, N 8.34; C₃₀H₃₆N₄O₅•C₇H₁₇NO₅•1.3H₂O•0.6C₄H₈O₂ requires C 58.85, H 7.57, N 8.71%.

### Example 113. 3-{2-[5-Cyclohexyl-1-(3,3-dimethyl-2-oxo-butyl)-6-fluoro-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-6-fluoro-phenyl)-cyclohexyl-methanone was used in step a instead of (2-amino-phenyl)-cyclohexyl-methanone, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.60 (1H, s), 8.07 (1H, d), 7.98 (1H, s), 7.82 (1H, d), 7.42 (2H, m), 7.02 (1H, t), 6.81 (1H, d), 4.81 (1H, d), 4.56 (1H, d), 4.25 (2H, q), 2.91 (1H, m), 2.10 (1H, br m), 1.9-1.6 (4H, m), 1.28 (5H, m), 1.29 (9H, s). The compound was further characterised as the *N*-methyl-D-glucamine salt. Found C 56.33, H 7.07, N 8.60; C₂₉H₃₃FN₄O₅•C₇H₁₇NO₅•1.9H₂O•0.5C₄H₈O₂ requires C 56.34, H 7.19, N 8.64%.

### Example 114. 3-{2-[5-Cyclohexyl-1-(3,3-dimethyl-2-oxo-butyl)-6-methyl-2-oxo-1,2-dihydro-3H-1,3,4-benzotriazepin-3-yl]-acetylamino}-benzoic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-6-methyl-phenyl)-cyclohexyl-methanone was used in step a instead of (2-amino-phenyl)-cyclohexyl-methanone, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.41 (1H, s), 8.11 (1H, d), 7.82 (1H, d), 7.75 (1H, s), 7.39 (2H, m), 7.17 (1H, d), 6.86 (1H, d), 4.76 (1H, d), 4.57 (1H, d), 4.37 (1H, d), 4.21 (1H, d), 2.66 (1 H, m), 2.40 (3H, s), 2.00 (1H, br m), 1.75 (5H, m), 1.27 (4H, m), 1.26 (9H, s). The compound was further characterised as the *N*-methyl-D-glucamine. Found C 57.70, H 7.27, N 8.30; C₃₀H₃₆N₄O₅•C₇H₁₇NO₅•2.5 H₂O•0.6C₄H₈O₂ requires C 57.85, H 7.62, N 8.65%.

### Example 115. 2-[5-Cyclohexyl-2-oxo-1-(pyrrolidine-1-carbonyl)1,2-dihyhdro-3H-1,3,4-benzotriazepin-3-yl]-N-m-tolyl-acetamide.

**Step a.** *[5-Cyclohexyl-2-oxo-1-(pyrrolidine-1-carbonyl)-1,2-dihydro-3*H*-1,3,4-benzotriazepin-3-yl]-acetic acid ethyl ester.* A mixture of (1-chlorocarbonyl-5-cyclohexyl-2-oxo-1,2-dihydro-3*H*-1,3,4-benzotriazepin-3-yl)-acetic acid ethyl ester (Example 1, minor product of step b) (391mg, 1.0mmol), and pyrrolidine (156mg, 2.2mmol) in DCM (5ml) was stirred at room temperature for 30min. The solution was washed with dilute HCl, dried (MgSO₄), and the solvent evaporated under reduced pressure to afford the product as a white solid (417mg, 98%). ¹H NMR (CDCl₃) 8.12 (1H, d), 7.50 (1H, t), 7.39-7.31 (2H, m), 4.31 (2H, s), 4.16 (2H, q), 3.45 (4H, m), 2.81 (1H, m), 1.92-1.27 (14H, m), 1.22 (3H, t).

**Step b.** *[5-Cyclohexyl-2-oxo-1-(pyrrolidine-1-carbonyl)-1,2-dihydro-3*H-*1,3,4-benzotriazepin-3-yl]-acetic acid* was obtained by the method used in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that [5-cyclohexyl-2-oxo-1-(pyrrolidine-1-carbonyl)-1,2-dihydro-3*H*-1,3,4-benzotriazepin-3-yl]-acetic acid ethyl ester (Example 115, step a) was used in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c). ¹H NMR (CDCl₃) 8.11 (1H, d), 7.50 (1H, t), 7.42-7.33 (2H, m), 4.26 (2H, s), 3.42 (4H, m), 2.85 (1H, m), 1.91-1.19 (10H, m).

**Step c.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that [5-cyclohexyl-2-oxo-1-(pyrrolidine-1-carbonyl)-1,2-dihydro-3*H*-1,3,4-benzotriazepin-3-yl]-acetic acid (Example 115, step b) and *m*-toluidine were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively. ¹H NMR (CDCl₃) 8.12 (1H, d), 8.10 (1H, br s), 7.59 (1H, t), 7.43 (2H, m), 7.16 (2H, m), 7.07 (1H, d), 6.88 (1H, d), 4.34 (2H, s), 3.45-3.35 (4H, m), 2.88 (1H, m), 2.31 (3H, s), 2.00-1.31 (14H, m). Found: C 68.00, H 6.73, N 14.07%; C₂₈H₃₃N₅O₃•0.5H₂O requires: C 67.72, H 6.90, N 14.10%.

### Example 116. (7-{2-[5-Cyclohexyl-1-(3,3-dimethyl-2-oxo-butyl)-2-oxo-1,2-dihydro-3H-1,3,4-benzotriazepin-3-yl]-acetylamino}-3,4-dihydro-1H-isoquinolin-2-yl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (7-amino-3,4-dihydro-1*H*-isoquinolin-2-yl)-acetic acid ethyl ester was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (DMSO-*d*_{*6*}) 9.90 (1H, brs), 7.54-7.42 (3H, m), 7.28-7.11 (4H, m), 4.78 (2H, m), 4.33 (2H, m), 4.09 (2H, m), 3.39 (3H, m), 3.00 (3H, m), 1.65-1.21 (6H, m), 1.12-1.04 (13H, m). The product was further characterised as the hydrochloride salt. Found: C 47.05, H 5.34, N, 7.72%; C₃₃H₄₁N₅O₅•HCl•3.4CH₂Cl₂ requires: C 47.14, H 5.48, N 7.55%.

### Example 117. (5-{2-[5-Cyclohexyl-1-(3,3-dimethyl-2-oxo-butyl)-2-oxo-1,2-dihydro-3H-1,3,4-benzotriazepin-3-yl]-acetylamino}-indol-1-yl)-acetic acid.

The title compound was obtained by the method used in the preparation of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1) except that (5-amino-indol-1-yl)-acetic acid ethyl ester (prepared in two steps from 5-nitroindole) was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3H-1,3,4-benzotriazepin-3-yl]-acetic acid ethyl ester (Example 1, step c), according to the method of Example 1, step d. ¹H NMR (CDCl₃) 8.21 (1H, s), 7.70 (1H, s), 7.47 (2H, m), 7.27 (1H, m), 7.05 (4H, m), 6.47 (1H, d), 4.79 (2H, s), 4.68 (2H, m), 4.39 (1H, d), 4.20 (1H, d), 2.78 (1H, m), 2.18-1.07 (19H, m). The compound was further characterised as the N-methyl-D-glucamine salt. Found: C 58.02, H 7.08, N 9.62%; C₃₂H₃₇N₅O₅•C₇H₁₇NO₅•1.9H₂O•0.6C₄H₈O₂ requires: C 58.23, H 7.39, N 9.84%.

### Example 118. (5-{2-[5-Cyclohexyl-1-(3,3-dimethyl-2-oxo-butyl)-2-oxo-1,2-dihydro-3H-1,3,4-benzotriazepin-3-yl]-acetylamino}-indazol-1-yl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (5-amino-indazol-1-yl)-acetic acid methyl ester (prepared in two steps from 5-nitro-indazole) was used in place of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.43 (1H, s), 8.05 (1H, s), 7.52-7.45 (2H, m), 7.32-7.23 (3H, m), 7.04 (1H, d, 8.1), 5.12 (2H, s), 4.82 (1H, d, 17.4), 4.61 (1H, d, 17.4), 4.27-4.26 (2H, m), 2.79 (1H, m), 2.05-1.72 (6H, m), 1.48-1.19 (13H, m). The compound was further characterised as the *N*-methyl-D-glucamine salt. Found: C 56.75, H 6.90, N 11.81%; C₃₁H₃₆N₆O₅•C₇H₁₇NO₅•2.3H₂O requires: C 56.46, H 7.17, N 12.13%.

### Example 119. (3-{2-[5-Cyclohexyll-1-(2-cyclopropyl-2-oxo-ethyl)-2-oxo-1,2-dihydro-3H-1,3,4-benzotriazepin-3-yl]-acetylamino}-phenyl)-acetic acid.

**Step a.** *[5-Cyclohexyl-1-(2-cyclopropyl-2-oxo-ethyl)-2-oxo-1,2-dihydro-3*H*-1,3,4-benzotriazepin-3-yl]-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that 2-bromo-1-cyclopropyl-ethanone was used in step c instead of 1-bromo-3,3-dimethyl-butan-2-one. ¹H NMR (CDCl₃) 10.80 (1H, br s), 7.46 (2H, m), 7.27 (1H, m), 7.04 (1H, d), 4.66 (2H, m), 4.28 (1H, d), 3.93 (1H, d), 2.85 (1H, m), 1.99-0.92 (15H, m).

**Step b.** The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e) except that [5-cyclohexyl-1-(2-cyclopropyl-2-oxo-ethyl)-2-oxo-1,2-dihydro-3*H*-1,3,4-benzotriazepin-3-yl]-acetic acid (Example 119, step a) and (3-amino-phenyl)-acetic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 10.80 (1H, br s), 8.30 (1H, s), 7.46 (2H, m), 7.26 (4H, m), 7.08 (1H, d), 6.98 (1H, d), 4.78 (1H, d), 4.57 (1H, d), 4.32 (1H, d), 4.18 (1H, d), 3.59 (2H, s), 2.78 (1H, m), 2.17-1.68 (7H, m), 1.24 (4H, m), 1.08 (2H, m), 0.93 (2H, m). The compound was further characterised as the *N*-methyl-D-glucamine salt. Found: C 58.57, H 7.22, N 8.57%; C₂₉H₃₂N₄O₅•C₇H₁₇NO₅•1.2H₂O•0.9C₄H₈O₂ requires: C 58.52, H 7.27, N 8.62%.

### Example 120. [3-(2-{5-Cyclohexyl-1-(2-cyclopropyl-2-oxo-ethyl)-2-oxo-1,2-dihydro-3H-1,3,4-benzotriazepin-3-yl]-acetylamino}-phenylsulfanyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except [5-cyclohexyl-1-(2-cyclopropyl-2-oxo-ethyl)-2-oxo-1,2-dihydro-3*H*-1,3,4-benzotriazepin-3-yl]-acetic acid (Example 119, step a) and (3-amino-phenylsulfanyl)-acetic acid ethyl ester were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.90 (1H, br s), 8.44 (1H, s), 7.49 (3H, m), 7.29 (2H, m), 7.19 (1H, t), 7.08 (2H, t), 4.81 (1H, d), 4.56 (1H, d), 4.22 (2H, m), 3.65 (2H, s), 2.79 (1H, m), 2.01-1.68 (7H, m), 1.26 (4H, m), 1.09 (2H, m), 0.93 (2H, m). The compound was further characterised as the *N*-methyl-D-glucamine salt. Found: C 55.42, H 6.74, N 8.42%; C₂₉H₃₂N₄O₅S•C₇H₁₇NO₅•1.8H₂O•0.4C₄H₈O₂ requires: C 55.65, H 6.93, N 8.63%.

### Example 121. 2-[5-Cyclohexyl-1-(2-cyclopentyl-2-oxo-ethyl)-2-oxo-1,2-dihydro-3H-1,3,4-benzotriazepin-3-yl]-N-[3-(5-oxo-2,5-dihydro-[1,2,4]oxadiazol-3-yl)-phenyl]-acetamide.

The title compound was obtained by the method used in the preparation of 2-[5-cyclohexyl-1-(3,3-dimethyl-2-oxo-butyl)-2-oxo-1,2-dihydro-3*H*-1,3,4-benzotriazepin-3-yl]-*N*-[3-(5-oxo-2,5-dihydro-[1,2,4]oxadiazol-3-yl)-phenyl]-acetamide (Example 59) except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) was used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d). ¹H NMR (CDCl₃) 10.80 (1H, br s), 8.85 (1H, s), 7.85 (1H, s), 7.59 (2H, m), 7.43 (3H, m), 7.28 (1H, t), 7.05 (1H, d),), 4.73 (1H, d), 4.45 (1H, d), 4.22 (2H, s), 2.93 (1H, m), 2.80 (1H, m), 2.18-1.59 (13H, m), 1.28 (5H, m). The compound was further characterised as the *N*-methyl-D-glucamine salt. Found: C 57.52, H 6.80, N 12.01%; C₃₁H₃₄N₆O₅•C₇H₁₇NO₅•1.7H₂O requires: C 57.25, H 6.89, N 12.30%.

### Example 122. 2-[5-Cyclohexyl-1-(3,3-dimethyl-2-oxo-butyl)-2-oxo-1,2-dihydro-3H-1,3,4-benzotriazepin-3-yl]-N-[3-(5-methyl-[1,3,4]oxadiazol-2-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-(5-methyl-[1,3,4]oxadiazol-2-yl)-phenylamine (prepared in three steps from 3-nitrobenzoic acid) was used instead of 3-amino-benzoic acid methyl ester in step e. ¹H NMR (CDCl₃) 8.57 (1H, s), 8.04 (1H, s), 7.78-7.70 (2H, m), 7.50-7.30 (4H, m), 7.02 (1H, m), 4.80-4.56 (2H, m), 4.25 (2H, s), 2.81 (1H, m), 2.61 (3H, s), 2.00-1.27 (10H, m), 1.24 (9H, s). Found: C 66.70, H 6.71, N 14.98%; C₃₁H₃₆N₆O₄ requires: C 66.89, H 6.52, N 15.10%.

### Example 123. 2-[5-Cyclohexyl-1-(3,3-dimethyl-2-oxo-butyl)-2-oxo-1,2-dihydro-3H-1,3,4-benzotriazepin-3-yl]-N-(3-morpholin-4-yl-phenyl)-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 3-morpholin-4-yl-phenylamine (prepared in two steps from 3-fluoro-1-nitrobenzene) was used instead of 3-amino-benzoic acid methyl ester in step e. ¹H NMR (CDCl₃) 8.22 (1H, br s), 7.50-7.44 (2H, m), 7.29-7.12 (3H, m), 7.03 (1H, d, 8.1), 6.75 (1H, d, 8.1), 6.63-6.60 (1H, m), 4.77 (1H, d, 17.7), 4.65 (1H, d, 17.7), 4.34 (1H, d, 16.8), 4.19 (1H, d, 16.8), 3.84 (4H, t, 4.8), 3.15 (4H, t, 4.8), 2.82-2.75 (1H, m), 2.05-1.74 (6H, m), 1.36-1.24 (13H, m). Found: C 68.18, H 7.42, N 12.29%; C₃₂H₄₁N₅O₄•0.3H₂O requires: C 68.10, H 7.42, N 12.41%.

### Example 124. 2-[5-Cyclohexyl-1-(2-cyclopentyl-2-oxo-ethyl)-2-oxo-1,2-dihyduo-3H-1,3,4-benzotriazepin-3-yl]-N-{3-[methyl-(2H-tetrazol-5-yl)-amino]-phenyl}-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 2,2-dimethyl-propionic acid 5-[(3-amino-phenyl)-methyl-amino]-tetrazol-2-ylmethyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 2,2-dimethyl-propionic acid 5-(3-{2-[5-cyclopentyl-1-(3,3-dimethyl-2-oxo-butyl)-2-oxo-1,2-dihydro-3*H*-1,3,4-benzotriazepin-3-yl]-acetylamino}-phenyl)-tetrazol-2-ylmethyl ester (Example 10, step a), according to the method of Example 10, step b. ¹H NMR (DMSO-*d*_{*6*}) 9.85 (1H, s), 7.56-7.48 (3H, m), 7.30-7.20 (3H, m), 7.15 (1H, d), 7.06 (1H, d), 7.00 (1H, br m), 6.65 (1H, br m), 4.65 (2H, br m), 4.25 (1H, br d), 3.95 (1H, br d), 3.40 (3H, s), 3.05-2.85 (2H, m), 1.90-1.40 (9H, m), 1.40-0.95 (9H, m). The compound was further characterised as the *N*-methyl-D-glucamine salt. Found: C 56.42, H 7.05, N 16.27%; C₃₁H₃₇N₉O₃•C₇H₁₇NO₅•1.5CH₃CO₂H requires C 56.67, H 6.96, N 16.12%.

### Example 125. 2-[5-Cyclohexyl-1-(2-cyclopentyl-2-oxo-ethyl)-2-oxo-1,2-dihydro-3H-1,3,4-benzotriazepin-3-yl]-N-[3-(2H-tetrazol-5-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 2,2-dimethyl-propionic acid 5-(3-amino-phenyl)-tetrazol-2-ylmethyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 2,2-dimethyl-propionic acid 5-(3-{2-[5-cyclopentyl-1-(3,3-dimethyl-2-oxo-butyl)-2-oxo-1,2-dihydro-3*H*-1,3,4-benzotriazepin-3-yl]-acetylamino}-phenyl)-tetrazol-2-ylmethyl ester (Example 10, step a), according to the method of Example 10, step b. ¹H NMR (*d*_{*6*}-DMSO) 10.11 (1H, s), 8.36 (1H, s), 7.68-7.46 (5H, m), 7.22 (1H, m), 7.16 (1H, d), 6.95 (1H, br), 6.65 (1H, br), 4.64 (2H, m br), 4.35 (1H, m br), 4.00 (1H, m br), 3.00-2.80 (2H, m), 1.95-1.45 (9H, m), 1.40-1.00 (9H, m). The compound was further characterised as the *N*-methyl-D-glucamine salt. Found: C 56.11, H 7.00, N 14.82%; C₃₀H₃₄N₈O₃•C₇H₁₇NO₅•0.8CH₃CO₂H•2.2CH₃OH requires C 56.43, H 7.31, N 14.52%.

### Example 126. (6-{2-[5-Cyclohexyl-1-(2-cyclopentyl-2-oxo-ethyl)-2-oxo-1,2-dihydro-3H-1,3,4-benzotriazepin-3-yl]-acetylamino}-indol-1-yl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and (6-amino-indol-1-yl)-acetic acid ethyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (DMSO-*d*_{*6*}) 9.65 (1H, s), 7.67 (1H, s), 7.58-7.47 (2H, m), 7.39 (1H, d), 7.28-7.15 (3H, m), 6.97 (1H, d), 6.31 (1H, d), 4.75-4.55 (4H, m), 4.30 (1H, br d), 3.95 (1H, br d), 3.00-2.88 (2H, m), 1.98-1.40 (14H, m), 1.35-1.10 (4H, m). The compound was further characterised as the *N*-methyl-D-glucamine salt. Found: C 58.53, H 6.80, N 10.01%; C₃₃H₃₇N₅O₅•C₇H₁₇NO₅•2.0H₂O requires C 58.95, H 7.17, N 10.31%.

### Example 127. (4-{2-[5-Cyclohexyl-1-(3,3-dimethyl-2-oxo-butyl)-2-oxo-1,2-dihydro-3H-1,3,4-benzotriazepin-3-yl]-acetylamino}-indol-1-yl)-acetic acid.

The title compound was obtained by the method used in the preparation of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1) except that (4-amino-indol-1-yl)-acetic acid ethyl ester (prepared in two steps from 4-nitroindole) was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3H-1,3,4-benzotriazepin-3-yl]-acetic acid ethyl ester (Example 1, step c), according to the method of Example 1, step d. ¹H NMR (CDCl₃) 8.47 (1H, s), 7.84 (1H, d), 7.47 (2H, m), 7.27 (1H, m), 7.15 (1H, t), 7.05 (1H, d), 6.96 (1H, d), 6.87 (1H, t), 5.69 (1H, d), 4.75 (4H, m), 4.57 (1H, d), 4.25 (1H, d), 2.79 (1H, m), 1.99-1.09 (19H, m). The compound was further characterised as the N-methyl-D-glucamine salt. Found: C 58.46, H 7.08, N 9.78%; C₃₂H₃₇N₅O₅•C₇H₁₇NO₅•1.6H₂O•0.5C₄H₈O₂ requires: C 58.64, H 7.35, N 10.01%.

### Example 128. 3-(3-{2-[5-Cyclohexyl-1-(2-cyclopentyl-2-oxo-ethyl)-2-oxo-1,2-dihydro-3H-1,3,4-benzotriazepin-3-yl]-acetylamino}-phenyl)-propionic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 3-(3-amino-phenyl)-propionic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.28 (1H, s), 7.47 (2H, m), 7.32-7.16 (4H, m), 7.07 (1H, d), 6.92 (1H, d), 4.67 (1H, d), 4.45 (1H, d), 4.32 (1H, d), 4.18 (1H, d), 2.95 (3H, m), 2.80 (1H, m), 2.66 (2H, m), 1.84-1.57 (14H, m), 1.30 (4H, m). The compound was further characterised as the *N*-methyl-D-glucamine salt. Found: C 59.30, H 7.18, N 8.70%; C₃₂H₃₈N₄O₅•C₇H₁₇NO₅•1.8H₂O requires: C 59.63, H 7.51, N 8.91%.

### Example 129. 2-[5-Cyclohexyl-1-(3,3-dimethyl-2-oxo-butyl)-2-oxo-1,2-dihydro-3H-1,3,4-benzotriazepin-3-yl]-N-[3-(4-methyl-piperazin-1-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 3-(4-methyl-piperazin-1-yl)-phenylamine (prepared in two steps from 3-fluoro-1-nitrobenzene) was used instead of 3-amino-benzoic acid methyl ester in step e. ¹H NMR (CDCl₃) 8.17 (1H, br s), 7.50-7.44 (2H, m), 7.29-7.27 (1H, m), 7.16-7.09 (2H, m), 7.04 (1H, d, 8.1), 6.78-6.75 (1H, m), 6.64-6.61 (1H, m), 4.76-4.60 (2H, m), 4.36-4.12 (2H, m), 3.21-3.18 (4H, m), 2.82-2.74 (1H, m), 2.57-2.54 (4H, m), 2.35 (3H, s), 2.04-1.60 (6H, m), 1.45-1.23 (13H, m). Found: C 68.32, H 7.55, N 14.16%; C₃₃H₄₄N₆O₃•0.5H₂O requires: C 68.19, H 7.79, N 14.46%.

### Example 130. (4-{2-[5-Cyclohexyl-1-(3,3-dimethyl-2-oxo-butyl)-2-oxo-1,2-dihydro-3H-1,3,4-benzotriazepin-3-yl]-acetylamino}-indazol-1-yl)-acetic acid.

**Step a.** *(4-{2-[5-Cyclohexyl-1-(3,3-dimethyl-2-oxo-butyl)-2-oxo-1,2-dihydro-3*H*-1,3,4-benzotriazepin-3-yl]-acetylamino}-indazol-1-yl)-acetic acid* tert-*butyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (4-amino-indazol-1-yl)-acetic acid *tert*-butyl ester (prepared in two steps from 4-nitro-indazole) was used in place of 3-amino-benzoic acid methyl ester in step e.

**Step b.** *(4-{2-[5-Cyclohexyl-1-(3,3-dimethyl-2-oxo-butyl)-2-oxo-1,2-dihydro-3*H*-1,3,4-benzotriazepin-3-yl]-acetylamino}-indazol-1-yl)-acetic acid* tert-*butyl ester* (Example 130, step a) (522mg, 0.82mmol) was dissolved in trifluoroacetic acid (5ml) and the solution was stirred at room temperature for 2h. The solvent was evaporated, and the solid obtained was washed with Et₂O, isolated by filtration and dried to afford the trifluoroacetate salt of the title compound (430mg, 91%). ¹H NMR (CDCl₃) 8.98 (1H, s), 8.00-7.86 (3H, br m), 7.57-7.29 (5H, m), 7.10-7.05 (2H, m), 5.21 (2H, s), 4.77-4.62 (2H, m), 4.47-4.30 (2H, m), 2.80-2.76 (1H, m), 2.02-1.61 (6H, m), 1.30-1.16 (13H, m). Found: C 57.90, H 5.54, N 12.51%; C₃₁H₃₆N₆O₅•CF₃CO₂H requires: C 57.72, H 5.43, N 12.24%.

### Example 131. 2-[5-Cyclohexyl-1-(3,3-dimethyl-2-oxo-butyl)-2-oxo-1,2-dihydro-3H-1,3,4-benzotriazepin-3-yl]-N-[1-(1H-tetrazol-5-ylmethyl)-1H-indol-6-yl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 2,2-dimethyl-propionic acid 5-(6-amino-indol-1-ylmethyl)-tetrazol-1-ylmethyl ester (prepared in four steps from 6-nitro-indole) was used in place of 3-amino-benzoic acid methyl ester in step e, followed by reaction of the product obtained, in place of 2,2-dimethyl-propionic acid 5-(3-{2-[5-cyclopentyl-1-(3,3-dimethyl-2-oxo-butyl)-1-oxo-1,2-dihydro-3*H*-1,3,4-benzotriazepin-3-yl]-acetylamino}-phenyl)-tetrazol-2-ylmethyl ester (Example 10, step a), according to the method of Example 10, step b. ¹H NMR (CDCl₃) 8.66 (1H, s), 7.87 (1H, s), 7.38 (3H, m), 7.24 (1H, m), 7.14 (1H, d), 6.93 (1H, d), 6.66 (1H, d), 6.45 (1H, d), 5.39 (2H, s), 4.60 (2H, q), 4.13 (2H, q), 2.78 (1H, m), 2.00 (1H, m), 1.70 (5H, m), 1.25 (4H, m), 1.15 (9H, s).

### Example 132. (3-{2-[5-Cyclohexyl-1-(3,3-dimethyl-2-oxo-butyl)-6-methyl-2-oxo-1,2-dihydro-3H-1,3,4-benzotriazepin-3-yl]-acetylamino}-phenyl)-acetic acid.

The title compound was obtained using the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that (2-amino-6-methyl-phenyl)-cyclohexyl-methanone was used in step a instead of (2-amino-phenyl)-cyclohexyl-methanone, and (3-amino-phenyl)-acetic acid methyl ester replaced 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.22 (1H, s), 7.31 (4H, m), 7.10 (1H, d), 7.00 (1H, d), 6.84 (1H, d), 4.73 (1H, d), 4.56 (1H, d), 4.34 (1H, d), 4.16 (1H, d), 3.61 (2H, s), 2.63 (1H, m), 2.35 (3H, s), 2.00 (1H, br m), 1.75 (5H, m), 1.24 (13H, m).

### Example 133. [3-(2-{5-Cyclohexyl-1-[2-(1-methyl-cyclohexyl)-2-oxo-ethyl]-2-oxo-1,2-dihydro-3H-1,3,4-benzotriazepin-3-yl}-acetylamino)-phenyl]-acetic acid.

**Step a.** *{5-Cyclohexyl-1-[2-(1-methyl-cyclohexyl)-2-oxo-ethyl]-2-oxo-1,2-dihydro-3*H*-1,3,4-benzotriazepin-3-yl}-acetic acid* was obtained using steps a-d of the method employed in the preparation of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) except that 2-bromo-1-(1-methyl-cyclohexyl)-ethanone (prepared from 1-methyl-cyclohexane-carboxylic acid in two steps) was used in step c instead of 1-bromo-3,3-dimethyl-butan-2-one. ¹H NMR (CDCl₃) 10.80 (1H, br s), 7.45 (2H, m), 7.24 (1H, t), 6.96 (1H, d), 4.67 (2H, m), 4.22 (1H, d), 3.90 (1H, d), 2.82 (1H, m), 2.01-1.17 (23H, m).

Step b. The title compound was obtained by the method used in the preparation of 3-{2-[5-cyctohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that {5-cyclohexyl-1-[2-(1-methyl-cyclohexyl)-2-oxo-ethyl]-2-oxo-1,2-dihydro-3*H*-1,3,4-benzotriazepin-3-yl}-acetic acid (Example 133, step a) and (3-amino-phenyl)-acetic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.28 (1H, s), 7.47 (2H, m), 7.37-7.20 (4H, m), 7.00 (2H, t), 4.66 (2H, m), 4.25 (2H, m), 3.60 (2H, s), 2.78 (1H, m), 2.18-1.23 (20H, m), 1.19 (3H, s). The compound was further characterised as the *N*-methyl-D-glucamine salt. Found: C 60.87, H 7.66, N 8.38%; C₃₃H₄₀N₄O₅•C₇H₁₇NO₅•0.9H₂O•0.6C₄H₈O₂ requires: C 60.84, H 7.66, N 8.37%.

### Example 134. [3-(2-{5-Cyclohexyl-1-[2-(1-methyl-cyclohexyl)-2-oxo-ethyl]-2-oxo-1,2-dihydro-3H-1,3,4-benzotriazepin-3-yl}-acetylamino)-phenylsulfanyl]-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that {5-cyclohexyl-1-[2-(1-methyl-cyclohexyl)-2-oxo-ethyl]-2-oxo-1,2-dihydro-3*H*-1,3,4-benzotriazepin-3-yl}-acetic acid (Example 133, step a) and (3-amino-phenylsulfanyl)-acetic acid ethyl ester were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.40 (1H, s), 7.48 (3H, m), 7.31 (2H, m), 7.20 (1H, t), 7.10 (1H, d), 7.02 (1H, d), 4.76 (1H, d), 4.58 (1H, d), 4.23 (2H, m), 3.67 (2H, s), 2.79 (1H, m), 1.99-1.25 (20H, m), 1.19 (3H, s). The compound was further characterised as the *N*-methyl-D-glucamine salt. Found: C 58.41, H 7.21, N 8.18%; C₃₃H₄₀N₄O₅S•C₇H₁₇NO₅•1.4H₂O requires: C 58.17, H 7.31, N 8.48%.

### Example 135. 5-{2-[5-Cyclohexyl-1-(3,3-dimethyl-2-oxo-butyl)-2-oxo-1,2-dihydro-3H-1,3,4-benzotriazepin-3-yl]-acetylamino}-1H-indole-2-carboxylic acid ethyl ester.

The title compound was obtained by the method used in the preparation of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1) except that 5-amino-1H-indole-2-carboxylic acid ethyl ester was used instead of 3-amino-benzoic acid methyl ester in step e. ¹H NMR (CDCl₃) 8.75 (1H, s), 8.24 (1H, s), 7.90 (1H, s), 7.48-7.19 (6H, m), 7.04 (1H, d), 4.68 (1H, d), 4.45 (1H, d), 4.41-4.25 (4H, m), 2.79 (1H, m), 2.05-1.39 (6H, m), 1.28-1.23 (16H, m). Found: C 67.04, H 6.98, N 11.19 %; C₃₃H₃₉N₅O₅•0.4CH₃CO₂C₂H₅ requires: C 66.93, H 6.85, N 11.28 %.

### Example 136. 2-[5-Cyclohexyl-1-(2-cyclopentyl-2-oxo-ethyl)-2-oxo-1,2-dihydro-3H-1,3,4-benzotriazepin-3-yl]-N-[3-(2-methyl-thiazol-4-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 3-(2-methyl-thiazol-4-yl)-phenylamine were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e. ¹H NMR (CDCl₃) 8.39 (1H, s), 7.84 (1H, t), 7.61 (1H, d), 7.47 (3H, t), 7.30 (3H, m), 7.08 (1H, d), 4.57 (2H, m), 4.26 (2H, m), 2.95 (1H, m), 2.82 (1H, m), 2.77 (3H, s), 1.82-1.26 (18H, m). Found: C 67.52, H 6.42, N 11.83%; C₃₃H₃₇N₅O₃S•0.2H₂O requires: C 67.44, H 6.42, N 11.92%.

### Example 137. 4-(3-{2-[5-Cyclohexyl-1-(2-cyclopentyl-2-oxo-ethyl)-2-oxo-1,2-dihydro-3H-1,3,4-benzotriazepin-3-yl]-acetylamino}-phenyl)-thiazole-2-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 4-(3-amino-phenyl)-thiazole-2-carboxylic acid ethyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.51 (1H, s), 8.05 (1H, s), 7.88 (1H, s), 7.63 (1H, d), 7.49 (3H, m), 7.40-7.27 (2H, m), 7.07 (1H, d), 4.70 (1H, d), 4.45 (1H, d), 4.27 (2H, m), 2.95 (1H, m), 2.81 (1H, m), 2.06-1.56 (14H, m), 1.28 (4H, m). The compound was further characterised the *N*-methyl-D-glucamine salt. Found: C 57.65, H 6.41, N 9.83%; C₃₃H₃₅N₅O₅S•C₇H₁₇NO₅•1.5H₂O requires: C 57.51, H 6.63, N 10.06%.

### Example 138. (3-{2-[1-(3,3-Dimethyl-2-oxo-butyl)-2-oxo-5-pyridin-2-yl-1,2-dihydro-3H-1,3,4-benzotriazepin-3-yl]-acetylamino}-phenylsulfanyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [1-(3,3-dimethyl-2-oxo-butyl)-2-oxo-5-pyridin-2-yl-1,2-dihydro-3*H*-1,3,4-benzotriazepin-3-yl]-acetic acid (Example 5, step a) and (3-amino-phenylsulfanyl)-acetic acid ethyl ester were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.63 (1H, m), 8.36 (1H, m), 8.02 (1H, m), 7.81 (111, t), 7.36 (2H, m), 7.23 (3H, m), 7.11 (4H, m), 5.05 (1H, br s), 4.72 (2H, m), 4.36 (2H, m), 3.61 (2H, s), 1.27 (9H, s).

### Example 139. (3-{2-[1-(3,3-Dimethyl-2-oxo-butyl)-2-oxo-5-phenyl-1,2-dihydro-3H-1,3,4-benzotriazepin-3-yl]-acetylamino}-phenylsulfanyl)-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that [1-(3,3-dimethyl-2-oxo-butyl)-2-oxo-5-phenyl-1,2-dihydro-3*H*-1,3,4-benzotriazepin-3-yl]-acetic acid (Example 4, step a) and (3-amino-phenylsulfanyl)-acetic acid ethyl ester were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.60 (1H, br s), 8.40 (1H, s), 7.63-7.40 (7H, m), 7.25-7.14 (6H, m), 4.76 (2H, m), 4.43 (2H, s), 3.58 (2H, s), 1.25 (9H, s). The compound was further characterised as the *N*-methyl-D-glucamine salt. Found: C 50.02, H 6.04, N 7.59%; C₃₀H₃₀N₅O₅S•C₇H₁₇NO₅•1.8C₄H₈O₂•2.2CH₂Cl₂ requires: C 50.06, H 5.96, N 7.55%.

### Example 140. 2-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-oxazole-4-carboxylic acid methyl ester.

The title compound was obtained by the method used in the preparation of 3-{2-[5-cyclohexyl-1-(3,3-dimethyl-2-oxo-butyl)-3-oxo-1,2-dihydro-3*H*-1,3,4-benzotriazepin-3-yl]-acetylamino}-benzoic acid methyl ester (Example 1), except that 2-(3-amino-phenyl)-oxazole-4-carboxylic acid methyl ester (prepared in three steps from serine methyl ester and 3-nitrobenzoic acid) was used instead of 3-amino-benzoic acid methyl ester in step e. ¹H NMR (CDCl₃) 8.51 (1H, br s), 8.28 (1H, s), 8.00 (1H, t, 1.8), 7.86-7.83 (1H, m), 7.76-7.73 (1H, m), 7.54-7.34 (4H, m), 7.05 (1H, d, 1.8), 4.81 (1H, d, 17.7), 4.64 (1H, d, 17.7), 4.26-4.25 (2H, m), 3.96 (3H, s), 2.83-2.77 (1H, m), 2.05-1.70 (6H, m), 1.38-1.18 (13H, m). Found: C 64.43, H 6.10, N 11.25%; C₃₃H₃₇N₆O₅•0.8H₂O requires: C 64.56, H 6.33, N 11.41%.

### Example 141. 2-[5-Cyclohexyl-1-(3,3-dimethyl-2-oxo-butyl)-2-oxo-1,2-dihydro-3H-1,3,4-benzotriazepin-3-yl]-N-[3-(1-methyl-1H-imidazol-4-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 3-(1-methyl-1*H*-imidazol-4-yl)-phenylamine (prepared in three steps from 2-bromo-3'-nitroacetophenone) was used instead of 3-amino-benzoic acid methyl ester in step e. ¹H NMR (CDCl₃) 8.25 (1H, br s), 7.85 (1H, t, 1.8), 7.54-7.44 (4H, m), 7.30-7.24 (2H, m), 7.19 (2H, m), 7.04 (1H, d, 7.8), 4.71-4.68 (2H, m), 4.37 (1H, d, 16.2), 4.21 (1H, d, 16.2), 3.72 (3H, s), 2.79 (1H, m), 2.05-1.72 (6H, m), 1.32-1.24 (13H, m). Found: C 68.00, H 6.90, N 14.55%; C₃₂H₃₈N₆O₃•0.7H₂O requires: C 67.77, H 7.00, N 14.82%.

### Example 142. (4-{2-[5-Cyclohexyl-1-(3,3-dimethyl-2-oxo-butyl)-2-oxo-1,2-dihydro-3H-1,3,4-benzotriazepin-3-yl]-acetylamino}-indazol-2-yl)-acetic acid.

The title compound was obtained as the trifluoroacetate salt by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that (4-amino-indazol-2-yl)-acetic acid *tert*-butyl ester (prepared in two steps from 4-nitro-indazole) was used instead of 3-amino-benzoic acid methyl ester in step e, followed by reaction of the product obtained, in place of (4-{2-[5-cyclohexyl-1-(3,3-dimethyl-2-oxo-butyl)-2-oxo-1,2-dihydro-3*H*-1,3,4-benzotriazepin-3-yl]-acetylamino}-indazol-1-yl)-acetic acid *tert*-butyl ester (Example 130, step a), according to the method of Example 130, step b. ¹H NMR (CDCl₃) 10.04-9.95 (2H, br s), 9.06 (1H, s), 8.13 (1H, s), 7.53-7.20 (6H, m), 7.06 (1H, d, 6), 5.26 (2H, s), 4.80 (1H, d, 18), 4.63 (1H, d, 18), 4.29 (2H, s), 2.81-2.78 (1H, m), 2.07-1.67 (6H, m), 1.37-1.19 (13H, m). Found: C 56.82, H 5.42, N 11.88%; C₃₁H₃₆N₆O₅•CF₃CO₂H•0.7H₂O requires: C 56.72, H 5.53, N 12.03%.

### Example 143. 2-[5-Cyclohexyl-1-(3,3-dimethyl-2-oxo-butyl)-2-oxo-1,2-dihydro-3H-1,3,4-benzotriazepin-3-yl]-N-{3-[methyl-(2-methylamino-ethyl)-amino]-phenyl}-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that {2-[(3-amino-phenyl)-methyl-amino]-ethyl}-methyl-carbamic acid *tert*-butyl ester (prepared in three steps from 3-fluoro-1-nitrobenzene and *N,N'*-dimethylethylenediamine) was used instead of 3-amino-benzoic acid methyl ester in step e, followed by reaction of the product obtained, in place of (3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-methyl-carbamic acid *tert*-butyl ester (Example 3, step d), according to the method of Example 3, step e. ¹H NMR (CDCl₃) 8.09 (1H, s), 7.50-7.45 (2H, m), 7.29-7.24 (1H, m), 7.12-7.01 (3H, m), 6.53-6.46 (2H, m), 4.69-4.67 (2H, m), 4.38 (1H, d, 13.8), 4.17 (1H, d, 13.8), 3.50-3.44 (2H, m), 2.93 (3H, s), 2.86-2.74 (3H, m), 2.49 (3H, s), 2.04-1.58 (7H, m), 1.31-1.19 (13H, m). Found: C 65.52, H 7.53, N 14.12%; C₃₂H₄₄N₆O₅•0.4CH₂Cl₂ requires: C 65.58, H 7.61, N 14.17%.

### Example 144. 2-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-oxazole-4-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid (Example 2) except that 2-(3-{2-[5-cyclohexyl-1-(3,3-dimethyl-2-oxo-butyl)-2-oxo-1,2-dihydro-3*H*-1,3,4-benzotriazepin-3-yl]-acetylamino}-phenyl)-oxazole-4-carboxylic acid methyl ester (Example 140) was used instead of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1). ¹H NMR (CDCl₃) 8.58 (1H, m), 8.36 (1H, s), 8.08 (1H, t, 1.2), 7.83-7.81 (1H, m), 7.76-7.73 (1H, m), 7.54-7.32 (4H, m), 7.05 (1H, d, 8.1), 4.83 (1H, d, 17.4), 4.63 (1H, d, 17.4), 4.26 (2H, s), 2.84-2.78 (1H, m), 2.06-1.73 (6H, m), 1.36-1.18 (13H, m). The compound was further characterised as the *N*-methyl-D-glucamine salt. Found: C 57.95, H 6.68, N 10.18%; C₃₂H₃₅N₆O₅•C₇H₁₇NO₅•1.6H₂O requires: C 57.85, H 6.87, N 10.38%.

### Example 145. 5-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-furan-2-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 5-(3-amino-phenyl)-furan-2-carboxylic acid methyl ester (prepared in two steps from 5-(3-nitrophenyl)-2-furoic acid) was used in place of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.34 (1H, br s), 7.63-7.50 (5H, m), 7.40-7.27 (3H, m), 7.05 (1H, d), 6.77 (1H, m), 4.79 (1H, d), 4.65 (1H, d), 4.39 (1H, d), 2.81 (1H, m), 2.05-1.72 (6H, m), 1.36-1.23 (13H, m). The compound was further characterised as the *N*-methyl-D-glucamine salt. Found: C 59.03, H 6.69, N 8.48%; C₃₃H₃₆N₄O₆•C₇H₁₇NO₅•1.7H₂O requires: C 59.34, H 7.01, N 8.65%.

### Example 146. 3 '-{2-[5-Cyclohexyl-1-(3,3-dimethyl-2-oxo-butyl)-2-oxo-1,2-dihydro-3H-1,3,4-benzotriazepin-3-yl]-acetylamino}-biphenyl-4-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 3'-amino-biphenyl-4-carboxylic acid methyl ester (prepared by catalytic hydrogenation of 3'-nitro-biphenyl-4-carboxylic acid methyl ester (Y. Matsushita, *et. al*., *Syn*. *Comm*., (1994), **24**, 3307)) was used in place of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.9 (1H, br s), 9.90 (1H, s), 8.02 (2H, d), 7.88 (1H, s), 7.71 (2H, d), 7.57-7.38 (5H, m), 7.26-7.16 (2H, m), 4.80-4.79 (2H, m), 4.37-4.31 (1H, m), 4.01 (1H, m), 2.87 (1H, m), 1.75-1.65 (6H, m), 1.34-1.13 (13H, m). The compound was further characterised as the *N*-methyl-D-glucamine salt. Found: C 61.82, H 7.05, N 8.19%; C₃₅H₃₈N₄O₅•C₇H₁₇NO₅•1.7H₂O requires: C 61.51, H 7.17, N 8.54%.

### Example 147. 2-[5-Cyclohexyl-1-(3,3-dimethyl-2-oxo-butyl)-2-oxo-1,2-dihydro-3H-1,3,4-benzotriazepin-3-yl]-N-[3-(2,4-dioxo-thiazolidin-5-ylidenemethyl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 5-(3-amino-benzylidene)-thiazolidine-2,4-dione (prepared in two steps from 3-nitro-benzaldehyde) was used in place of 3-amino-benzoic acid methyl ester in step e. ¹H NMR (DMSO-*d*_{*6*}) 9.84 (1H, br s), 7.62-7.45 (4H, m), 7.33-7.15 (4H, m), 4.80-4.76 (2H, m), 4.32-4.30 (1H, br m), 4.03-3.94 (1H, br m), 2.87 (1H, m), 1.86-1.53 (6H, m), 1.34-1.10 (13H, m). Found: C 61.16, H 5.89, N 11.08%; C₃₂H₄₄N₆O₅S•1.4H₂O requires: C 61.34, H 6.07, N 11.18%.

### Example 148. 2-(3-{2-[5-Cyclohexyl-1-(2-cyclopentyl-2-oxo-ethyl)-2-oxo-1,2-dihydro-3H-1,3,4-benzotriazepin-3-yl]-acetylamino}-phenyl)-oxazole-4-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 2-(3-amino-phenyl)-oxazole-4-carboxylic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.60 (1H, s), 8.35 (1H, s), 8.08 (1H, s), 7.83-7.81 (1H, m), 7.74-7.72 (1H, m), 7.61-7.32 (4H, m), 7.09 (1H, d), 4.73 (1H, d), 4.49 (1H, d), 4.26 (2H, s), 3.01-2.91 (1H, m), 2.81 (1H, m), 2.13-1.58 (14H, m), 1.31-1.25 (4H, m). The compound was further characterised as the *N*-methyl-D-glucamine salt. Found: C 57.97, H 6.50, N 9.95%; C₃₃H₃₅N₅O₆•C₇H₁₇NO₅•1.8H₂O requires: C 58.20, H 6.79, N 10.18%.

### Example 149. 5-(3-{2-[5-Cyclohexyl-1-(2-cyclopentyl-2-oxo-ethyl)-2-oxo-1,2-dihydro-3H-1,3,4-benzotriazepin-3-yl]-acetylamino}-phenyl)-furan-2-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 5-(3-amino-phenyl)-furan-2-carboxylic acid methyl ester were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.35 (1H, s), 7.63-7.50 (5H, m), 7.41-7.32 (3H, m), 7.11 (1H, d), 6.80 (1H, d), 4.71 (1H, d), 4.51 (1H, d), 4.45 (1H, d), 4.24 (1H, d), 3.01-2.91 (1H, m), 2.85-2.79 (1H, m), 2.06-1.60 (14H, m), 1.31-1.30 (4H, m). The compound was further characterised as the *N*-methyl-D-glucamine salt. Found: C 60.14, H 6.70, N 8.49%; C₃₄H₃₆N₄O₆•C₇H₁₇NO₅•1.5H₂O requires: C 60.16, H 6.89, N 8.56%.

### Example 150. 2-[5-Cyclohexyl-1-(3,3-dimethyl-2-oxo-butyl)-2-oxo-1,2-dihydro-3H-1,3,4-benzotriazepin-3-yl]-N-[3-(2-methylamino-thiazol-4-yl)-phenyl]-acetamide.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [4-(3-amino-phenyl)-thiazol-2-yl]-methyl-amine (prepared in two steps from 2-bromo-3'-nitroacetophenone) was used in place of 3-amino-benzoic acid methyl ester in step e. ¹H NMR (CDCl₃) 8.28 (1H, s), 7.77 (1H, s), 7.55-7.25 (7H, m), 7.04 (1H, d), 6.71 (1H, s), 5.12 (1H, d), 4.77 (1H, d), 4.67 (1H,d), 4.36 (1H, d), 4.22 (1H, d), 3.04 (3H, d), 2.79 (1H, m), 2.05-1.73 (6H, m), 1.45-1.19 (13H, m). Found: C 64.64, H 6.57, N 14.05%; C₃₂H₃₈N₆O₃S•0.5H₂O requires: C 64.57, H 6.59 N 14.12%.

### Example 151. 3'-{2-[5-Cyclohexyl-1-(3,3-dimethyl-2-oxo-butyl)-2-oxo-1,2-dihydro-3H-1,3,4-benzotriazepin-3-yl]-acetylamino}-biphenyl-3-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 3'-amino-biphenyl-3-carboxylic acid ethyl ester (prepared in two steps from 3-nitrophenylboronic acid) was used instead of 3-amino-benzoic acid methyl ester in step e, followed by reaction of the product obtained, in place of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), according to the method of Example 1, step d. ¹H NMR (CDCl₃) 8.40 (1H, s), 8.29 (1H, t), 8.12-8.08 (1H, m), 7.85-7.81 (1H, m), 7.64-7.61 (1H, m), 7.57-7.47 (4H, m), 7.41-7.27 (3H, m), 7.06 (1H, d), 4.80 (1H, d), 4.65 (1H, d), 4.39 (1H, d), 4.25 (1H, d), 2.84-2.77 (1H, m), 2.02-1.70 (6H, m), 1.44-1.16 (13H, m). The compound was further characterised as the *N*-methyl-D-glucamine salt. Found: C 61.19, H 7.07, N 8.48%; C₃₅H₃₈N₄O₅•C₇H₁₇NO₅•1.9H₂O requires: C 61.21, H 7.19, N 8.50%.

### Example 152. (3-{2-[1-(2-tert-Butyl-allyl)-5-cyclohexyl-2-oxo-1,2-dihydro-3H-1,3,4-benzotriazepin-3-yl]-acetylamino}-phenyl)-acetic acid.

The title compound was obtained by using the method employed in the preparation of 3-{2-[5-cyclohexyl-1-(3,3-dimethyl-2-oxo-butyl)-2-oxo-1,2-dihydro-3H-1,3,4-benzotriazepin-3-yl]-acetylamino}-benzoic acid methyl ester (Example 1) except that 2-bromomethyl-3,3-dimethyl-but-1-ene (E. Lee, *et al*., *J Org. Chem*. (1994), **59**, 1444) was used instead of 1-bromo-3,3-dimethyl-butan-2-one in step c, and (3-amino-phenyl)-acetic acid methyl ester replaced 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 12.10 (1H, br s), 9.79 (1H, s), 7.58-7.34 (5H, m), 7 21 (2H, m), 6.90 (1H, d), 4.79 (1H, s), 4.68 (2H, m), 4.32 (2H, br t), 3.95 (1H, d), 3.48 (2H, s), 2.80 (1H, m), 2.00-1.10 (10H, m), 1.04 (9H, s). The compound was further characterised as the *N*-methyl-D-glucamine salt. Found: C 58.80, H 7.60, N 8.73%; C₃₁H₃₈N₄O₄•C₇H₁₇NO₅•3.0H₂O requires: 58.52, H 7.88, N 8.97%.

### Example 153. 5-(3-{2-[1-(2-tert-Butyl-allyl)-5-cyclohexyl-2-oxo-1,2-dihydro-3H-1,3,4-benzotriazepin-3-yl]-acetylamino]-phenyl)-furan-2-carboxylic acid.

The title compound was obtained by the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1) except that 2-bromomethyl-3,3-dimethyl-but-1-ene was used instead of 1-bromo-3,3-dimethyl-butan-2-one in step c, and 5-(3-amino-phenyl)-furan-2-carboxylic acid methyl ester replaced 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (DMSO-*d*_{*6*}) 13.00 (1H, br s), 10.01 (1H, s), 7.59-7.34 (7H, m), 7.25 (2H, m), 7.03 (1H, d), 4.80 (1H, s), 4.68 (1H, s), 4.60-4.04 (2H, br m), 4.30 (2H, br m), 2.90 (1H, m), 2.00-1.20 (10H, m), 1.04 (9H, s). The compound was further characterised as the *N*-Methyl-D-glucamine salt. Found: C 58.88, H 7.03, N 8.13%; C₃₄H₃₈N₄O₆•C₇H₁₇NO₅•3.0H₂O requires: C 59.19, H 7.39, N 8.41%.

### Example 154. 3'-{2-[5-Cyclohexyl-1-(3,3-dimethyl-2-oxo-butyl)-2-oxo-1,2-dihydro-3H-1,3,4-benzotriazepin-3-yl]-acetylamino}-biphenyl-2-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 3'-amino-biphenyl-2-carboxylic acid methyl ester (prepared in two steps from 3-nitrophenylboronic acid) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.52 (1H, s), 7.91 (1H, d), 7.63-7.20 (9H, m), 7.11 (1H, d), 7.03 (1H, d), 4.82 (1H, d), 4.61 (1H, d), 4.30 (1H, d), 4.16 (1H, d), 2.82-2.75 (1H, m), 2.05-1.57 (6H, m), 1.44-1.00 (13H, m). The compound was further characterised as the *N*-methyl-D-glucamine salt. Found: C 61.11, H 6.88, N 8.29%; C₃₅H₃₈N₄O₅•C₇H₁₇NO₅•1.8H₂O requires: C 61.40, H 7.18, N 8.53%.

### Example 155. 2-Acetylamino-3-(3-{2-[5-cyclohexyl-1-(3,3-dimethyl-2-oxo-butyl)-2-oxo-1,2-dihydro-3H-1,3,4-benzotriazepin-3-yl]-acetylamino}-phenyl)-acrylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 2-acetylamino-3-(3-amino-phenyl)-acrylic acid methyl ester (prepared in three steps from 3-nitro-benzaldehyde and *N*-acetyl-glycine) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.43 (1H, s), 7.99 (1H, s), 7.61-7.00 (9H, m), 4.80 (1H, d), 4.61 (1H, d), 4.22-4.16 (2H, m), 2.79 (1H, m), 2.19-1.65 (9H, m), 1.44-1.19 (13H, m). The compound was further characterised as the *N*-methyl-D-glucamine salt. Found: C 56.61, H 6.99, N 9.44%; C₃₃H₃₉N₅O₆•C₇H₁₇NO₅•3.2H₂O requires: C 56.22, H 7.36, N 9.83%.

### Example 156. 3-{2-[1-(tert-Butylcarbamoyl-methyl)-5-cyclohexyl-2-oxo-1,2-dihydro-3H-1,3,4-benzotriazepin-3-yl]-acetylamino}-benzoic acid.

**Step a.** *[1-(*tert*-Butylcarbamoyl-methyl)-5-cyclohexyl-2-oxo-1,2-dihydro-3*H*-1,3,4-benzotriazepin-3-yl]-acetic acid ethyl ester* was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1, step e), except that (5-cyclohexyl-3-ethoxycarbonylmethyl-2-oxo-2,3-dihydro-3*H*-1,3,4-benzotriazepin-1-yl)-acetic acid (Example 68, step b) and *tert*-butylamine were used instead of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively. ¹H NMR (CDCl₃) 7.40 (2H, m), 7.22 (2H, m), 6.63 (1H, s), 4.27-4.09 (6H, m), 2.75 (1H, m), 2.00-1.55 (6H, m), 1.30-1.20 (16H, m).

**Step b.** The title compound was obtained using steps d and e of the method employed in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [1-(*tert*-butylcarbamoyl-methyl)-5-cyclohexyl-2-oxo-1,2-dihydro-3*H*-1,3,4-benzotriazepin-3-yl]-acetic acid ethyl ester (Example 156, step a) was used in step d instead of [5-cyclohexyl-1-(3,3-dimethyl-2-oxo-butyl)-2-oxo-1,2-dihydro-3*H*-1,3,4-benzotriazepin-3-yl]-acetic acid ethyl ester (Example 1, step c), followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.59 (1H, s), 7.95 (1H, d), 7.78-7.75 (2H, m), 7.54-7.45 (2H, m), 7.40-7.28 (3H, m), 6.49 (1H, s), 4.35-4.09 (4H, m), 2.81 (1H, m br), 2.05 (1H, br), 1.88 (1H, br), 1.80-1.60 (4H, m), 1.35-1.20 (13H, m). The compound was further characterised as the *N*-methyl-D-glucamine salt. Found: C 55.95, H 7.01, N 10.29%; C₂₉H₃₅N₅O₅•C₇H₁₇NO₅•0.7CH₂Cl₂ requires C 55.92, H 6.83, N 10.66%.

### Example 157. 5-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-thiophene-2-carboxylic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that 5-(3-amino-phenyl)-thiophene-2-carboxylic acid methyl ester (prepared in three steps from 5-bromo-2-thiophenecarboxylic acid) was used instead of 3-amino-benzoic acid methyl ester in step e, followed by reaction of the product obtained, in place of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), according to the method of Example 2. ¹H NMR (CDCl₃) 8.47 (1H, s), 7.85 (1H, d), 7.62-7.47 (4H, m), 7.37-7.27 (4H, m), 7.06 (1H, d), 4.81 (1H, d), 4.64 (1H, d), 4.36 (1H, d), 4.25 (1H, d), 2.84-2.78 (1H, m), 2.07-1.70 (6H, m), 1.30-1.19 (13H, m). The compound was further characterised as the *N*-methyl-D-glucamine salt. Found: C 58.29, H 6.63, N 8.22%; C₃₃H₃₆N₄O₅S•C₇H₁₇NO₅•1.7H₂O requires: C 58.10, H 6.88, N 8.46%.

### Example 158. [2-(3-{2-[5-Cyclohexyl-1-(3,3-dimethyl-2-oxo-butyl)-2-oxo-1,2-dihydro-3H-1,3,4-benzotriazepin-3-yl]-acetylamino}-phenyl)-pyrrol-1-yl]-acetic acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [2-(3-amino-phenyl)-pyrrol-1-yl]-acetic acid methyl ester (prepared in four steps from 1-(*tert*-butoxycarbonyl)-pyrrole-2-boronic acid and 1-bromo-3-nitrobenzene) was used instead of 3-amino-benzoic acid methyl ester in step e, followed by reaction of the product obtained, in place of 3-{2-[5-cyclohexyl-1-(3,3-dimethyl-2-oxo-butyl)-2-oxa-1,2-dihydro-3*H*-1,3,4-benzotriazepin-3-yl]-acetylamino}-benzoic acid methyl ester (Example 1), according to the method of Example 2. ¹H NMR (CDCl₃) 8.46 (1H, s), 7.47-7.43 (3H, m), 7.32-7.23 (3h, m), 7.09-7.00 (2H, m), 6.77-6.75 (1H, m), 6.27-6.21 (2H, m), 4.79-4.56 (4H, m), 4.31 (1H, d), 4.21 (1H, d), 2.81-2.75 (1H, m), 2.05-1.71 (6H, m), 1.36-1.19 (13H, m). The compound was further characterised as the *N*-methyl-D-glucamine salt. Found: C 59.36, H 7.02, N 9.65%; C₃₄H₃₉N₅O₅•C₇H₁₇NO₅•2.2H₂O requires: C 59.12, H 7.31, N 10.09%.

### Example 159. 4-(3-{2-[5-Cyclohexyl-1-(2-cyclopentyl-2-oxo-ethyl)-2-oxo-1,2-dihydro-3H-1,3,4-benzotriazepin-3-yl]-acetylamino}-phenyl-butyric acid.

The title compound was obtained by the method used in the preparation of 3-{2-[5-cyclohexyl-1-(3,3-dimethyl-2-oxo-butyl)-2-oxo-1,2-dihydro-3*H*-1,3,4-benzotriazepin-3-yl]-acetylamino}-benzoic acid methyl ester (Example 1), except that [5-cyclohexyl-1-(2-cyclopentyl-2-oxo-ethyl)-2-oxo-1,2-dihydro-3*H*-1,3,4-benzotriazepin-3-yl]-acetic acid (Example 22, step a) and 4-(3-amino-phenyl)-butyric acid methyl ester (J. P. Weichert, *et. al., J*. *Med. Chem*. (1995), **38**, 636) were used in place of [5-cyclohexyl-1-(3,3-dimethyl-2-oxo-butyl)-2-oxo-1,2-dihydro-3*H*-1,3,4-benzotriazepin-3-yl]-acetic acid (Example 1, step d) and 3-amino-benzoic acid methyl ester respectively in step e, followed by reaction of the product obtained, in place of 3-{2-[5-cyclohexyl-1-(3,3-dimethyl-2-oxo-butyl)-2-oxo-1,2-dihydro-3*H-*1,3,4-benzotriazepin-3-yl]-acetylamino}-benzoic acid methyl ester (Example 1), according to the method of Example 2. ¹H NMR (DMSO-*d*_{*6*}) 12.00 (1H, br s), 9.67 (1H, s), 7.50 (2H, m), 7.32-7.15 (5H, m), 6.84 (1H, d), 4.63 (2H, m), 4.25 (1H, br d), 3.99 (1H, br d), 3.00 (1H, m), 2.88 (1H, m), 2.52 (2H, t), 2.19 (2H, t), 2.00-1.00 (20H, m). The compound was further characterised as the *N*-methyl-D-glucamine salt. Found: C 59.52, H 7.41, N 8.62%; C₃₃H₄₀N₄O₅•C₇H₁₇NO₅•2.0H₂O requires: C 59.76, H 7.65, N 8.71%.

### Example 160. 2-(5-Cyclohexyl-1-methyl-2-oxo-4-oxy-1,2-dihydro-3H-1,3,4-benzotriazepin-3-yl)-N-phenyl-acetamide.

**Step a.** *2-(5-Cyclohexyl-1-methyl-2-oxo -1,2-dihydro-3*H*-1,3,4-benzotriazepin-3-yl)-*N*-phenyl-acetamide* was obtained by the method used in the preparation of 3-{2-[5-cyclohexyl-1-(3,3-dimethyl-2-oxo-butyl)-2-ox0-1,2-dihydro-3*H*-1,3,4-benzotriazepin-3-yl]-acetylamino}-benzoic acid methyl ester (Example 1) except that iodomethane was used in step c instead of 1-bromo-3,3-dimethyl-butane-2-one, and aniline was used in step e instead of 3-amino-benzoic acid methyl ester. ¹H NMR (CDCl₃) 8.25 (1H, s), 7.55 (1H, t), 7.44 (1H, d), 7.32-7.21 (6H, m), 7.06 (1H, t), 4.47 (1H, d), 4.14 (1H, d), 3.29 (3H, s), 2.76 (1H, m), 2.00-1.00 (10H, m).

**Step b.** To a solution of the product of step a (390mg, 1.00mmol) in DCM (10ml) was added 3-chloroperoxybenzoic acid (1.23 g of 70%, 5.00mmol) and the solution stirred at room temperature for 16hr. After dilution with DCM (50ml) the solution was washed with 5% Na₂CO₃ (2 x 50ml), then brine (50ml). The organic phase was dried over MgSO₄, and the solvent evaporated under reduced pressure. The residue was purified by flash column chromatography (EtOAc-DCM (1:9) to afford the product as a white solid (94mg, 23%) ¹H NMR (CDCl₃) 8.17 (1H br.s), 7.51 (2H, d), 7.39-7.22 (6H, m), 7.06 (1H, m), 4.60 (1H, d), 4.23 (1H, d), 3.39 (3H, s), 3.15 (1H, m), 2.00-1.00 (10H, m). Found: C 67.73, H 6.46, N 13.71%; C₂₃H₂₆N₄O₃ requires: C 67.96, H 6.45, N 13.78%

### Gastrin (CCK₂) Antagonist Activity

The compounds of the examples were tested for gastrin (CCK₂) antagonist activity in an immature rat stomach assay. The procedure was as follows:
The oesophagus of immature rats (33-50 g, ca. 21 days old) was ligated at the level of the cardiac sphincter and the duodenal sphincter was cannulated. The stomach was excised and flushed with ca. 1 ml of unbuffered physiological saline solution. The fundus was punctured and cannulated. A further 4-5 ml of unbuffered solution was flushed through the stomach to ensure the preparation was not leaking. The stomach was lowered into a jacketed organ bath containing 40 ml of buffered solution containing 3 x 10⁻⁸ M 5-methylfurmethide, maintained at 37° and gassed vigorously with 95% O₂ / 5% CO₂. The stomach was continuously perfused at a rate of 1 ml min⁻¹ with unbuffered solution gassed with 100% O₂ with the perfusate passing over an internally referenced pH-electrode fixed 12 cm above the stomach.

After 120 min of stabilisation the drugs were added directly to the serosal solution in the organ bath and after a further 60 min cumulative pentagastrin dose-response curves were started. Changes in acid secretion were monitored and the curves analysed according to Black *et al*., *Br. J Pharmacol*., 1985, **86**, 581.

The results obtained at gastrin (CCK₂) receptors are set out in the following Table.

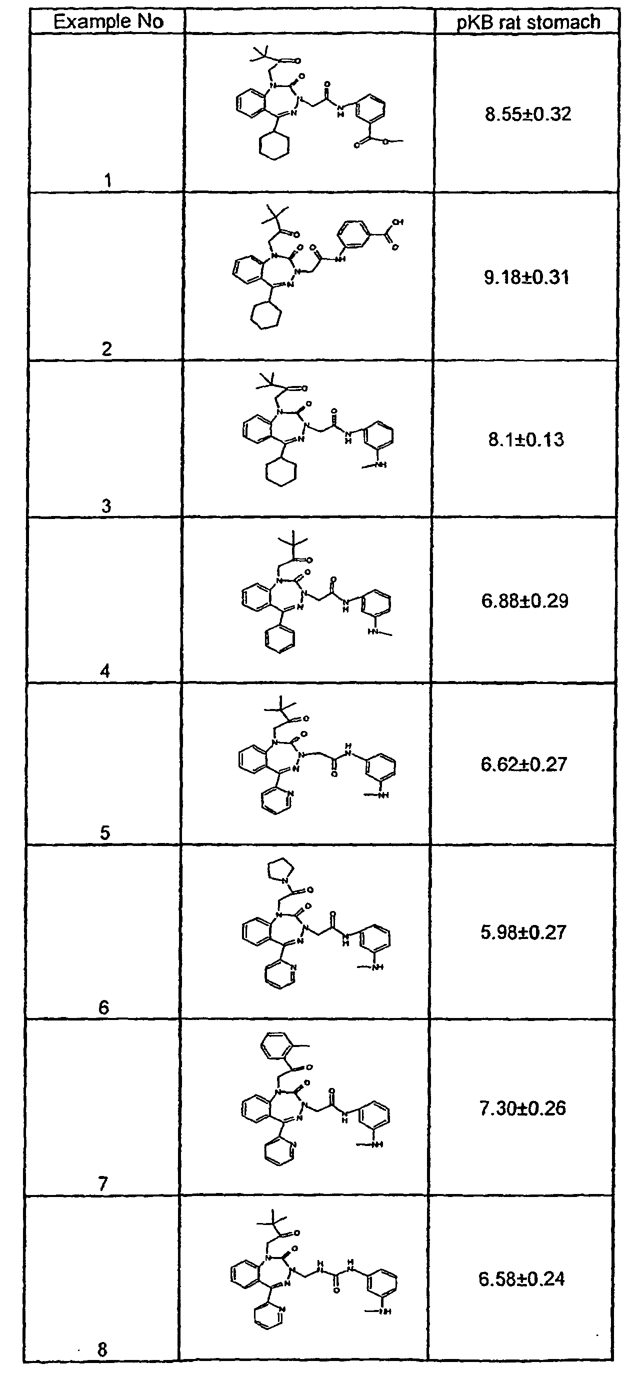

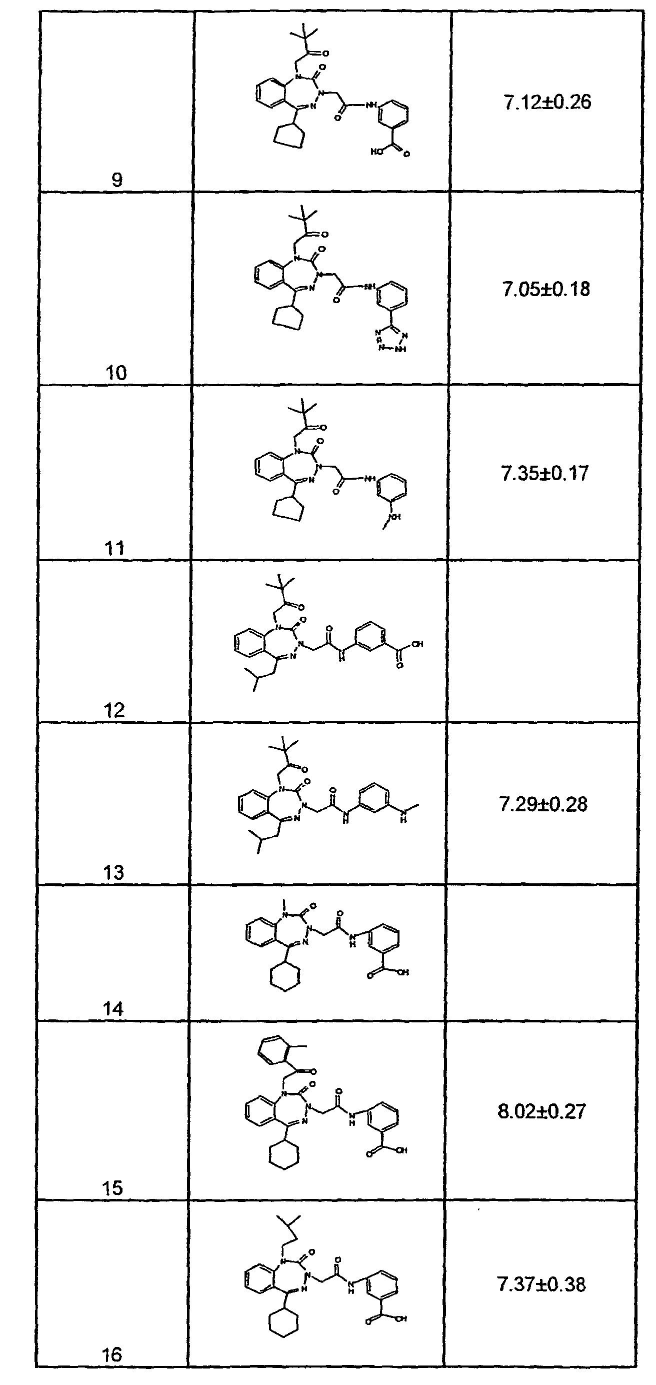

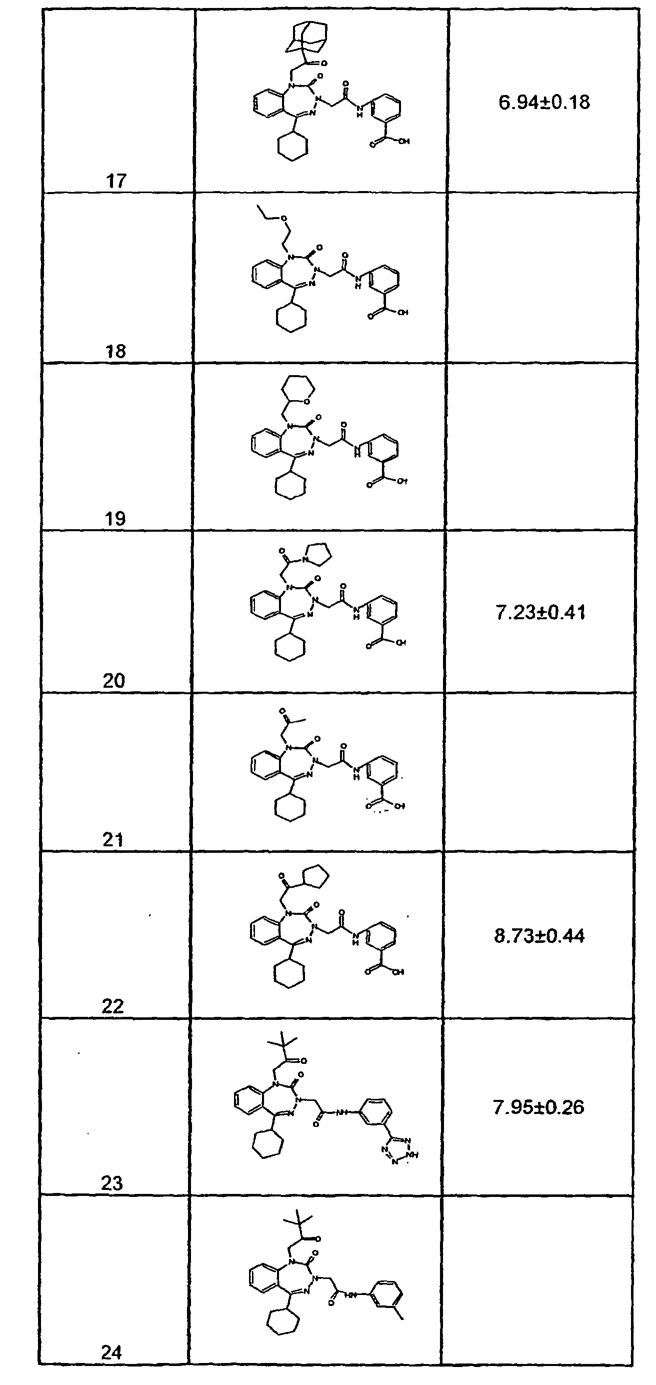

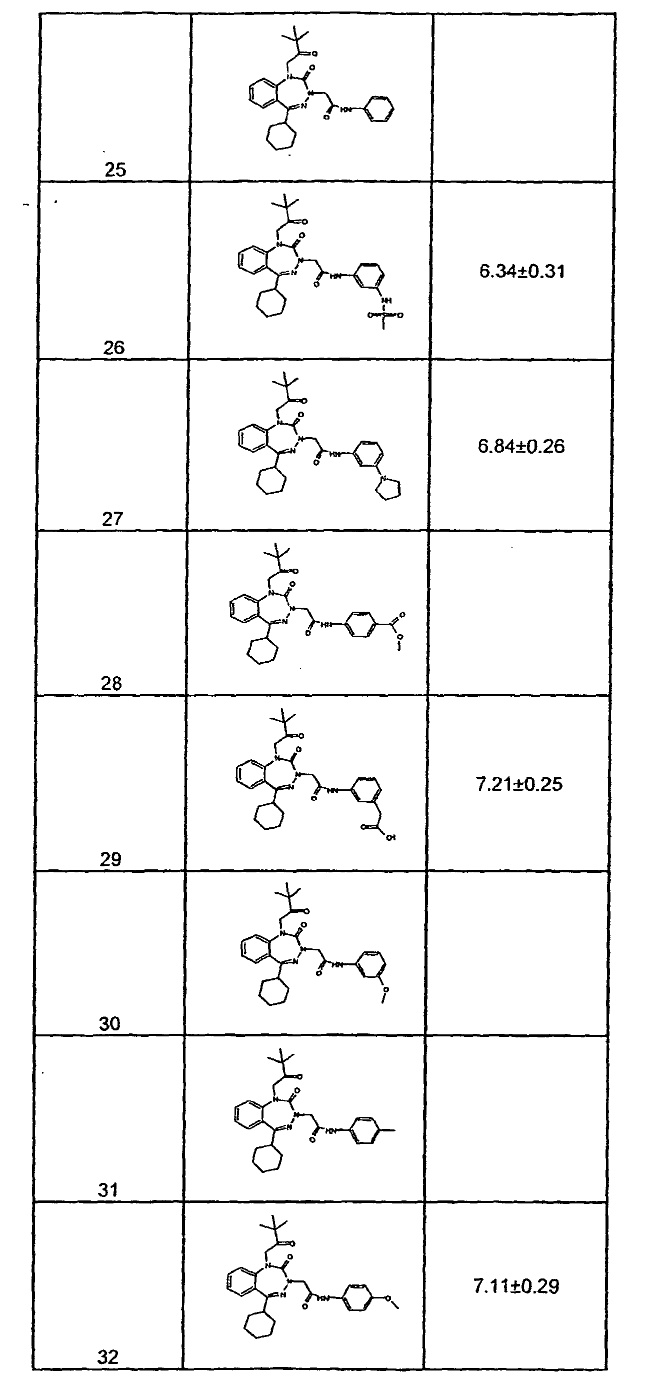

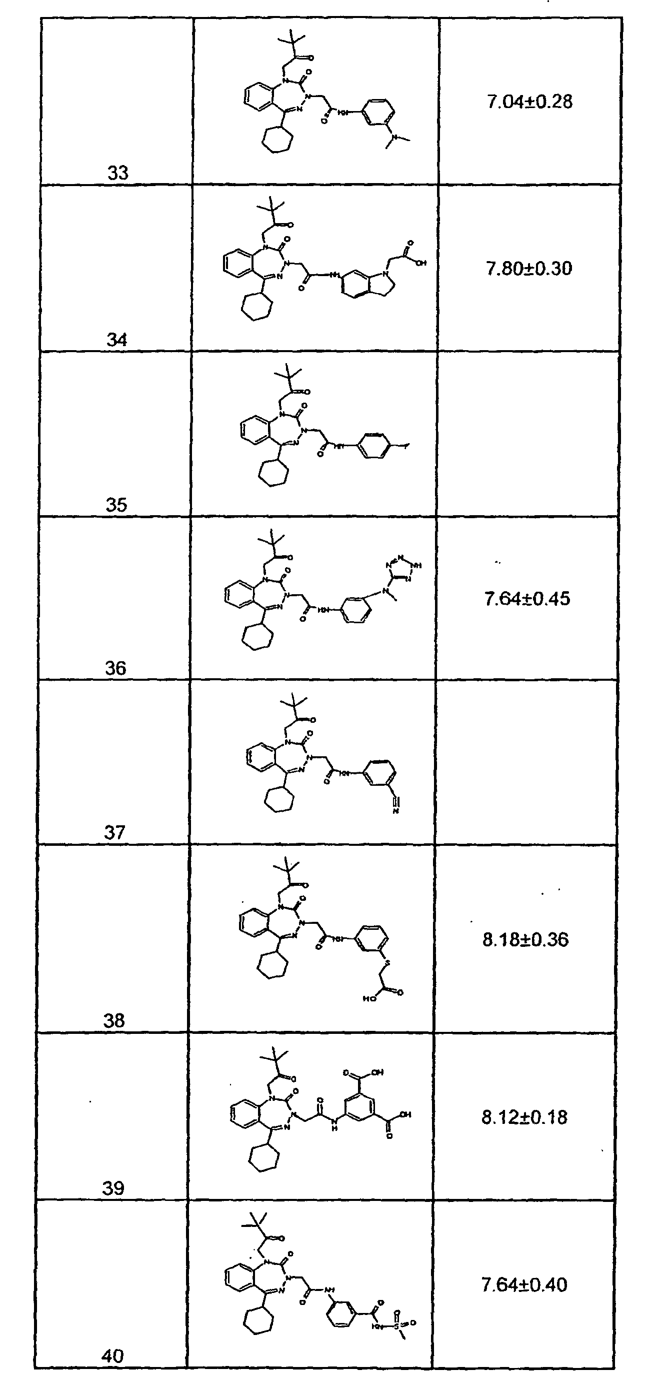

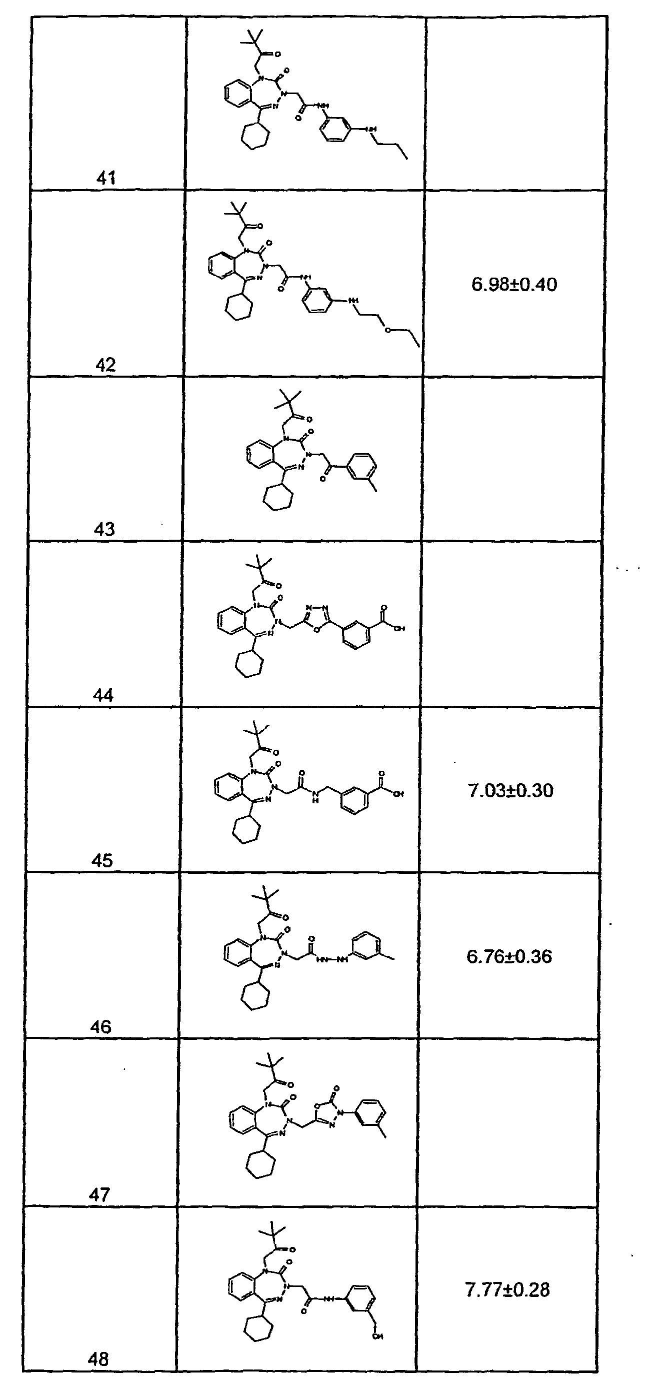

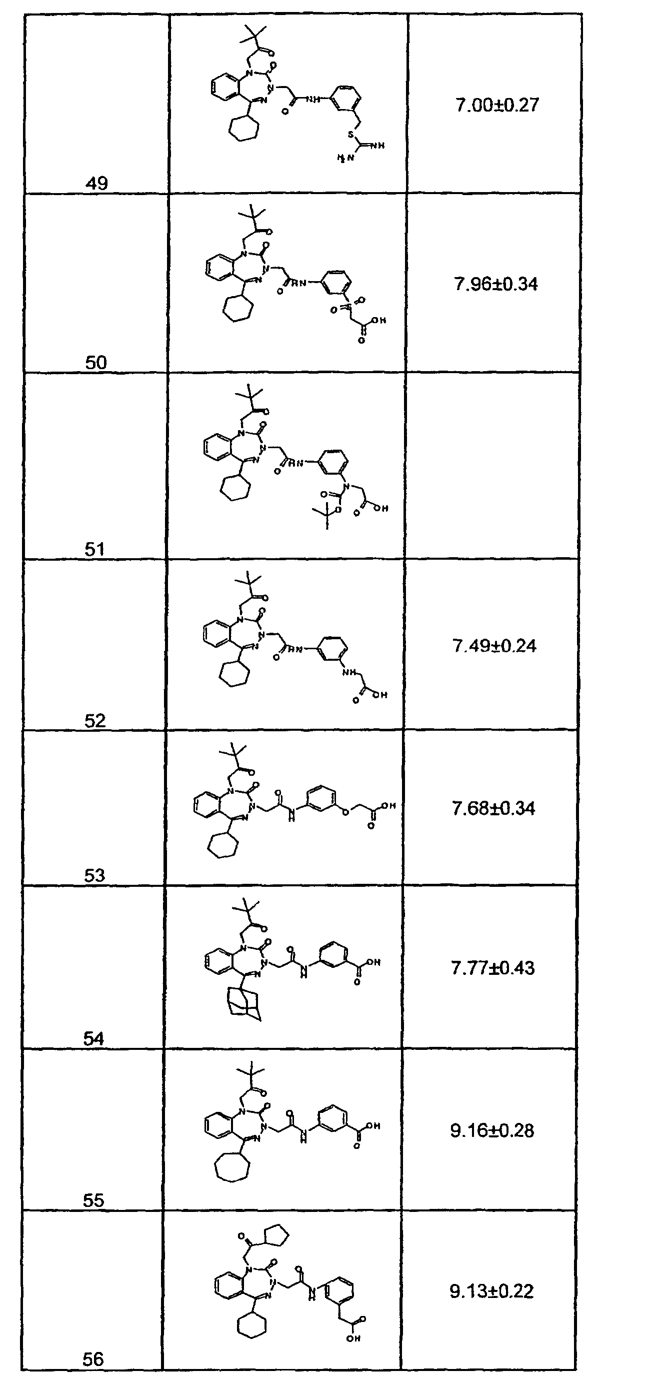

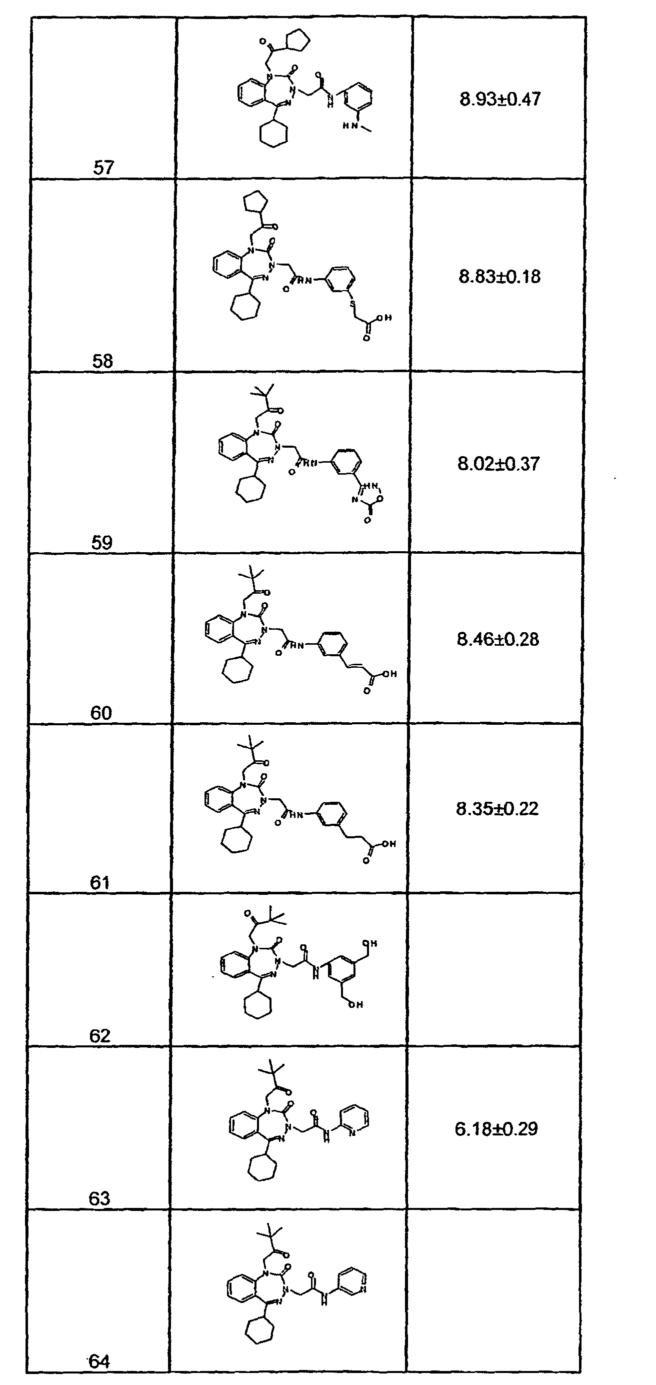

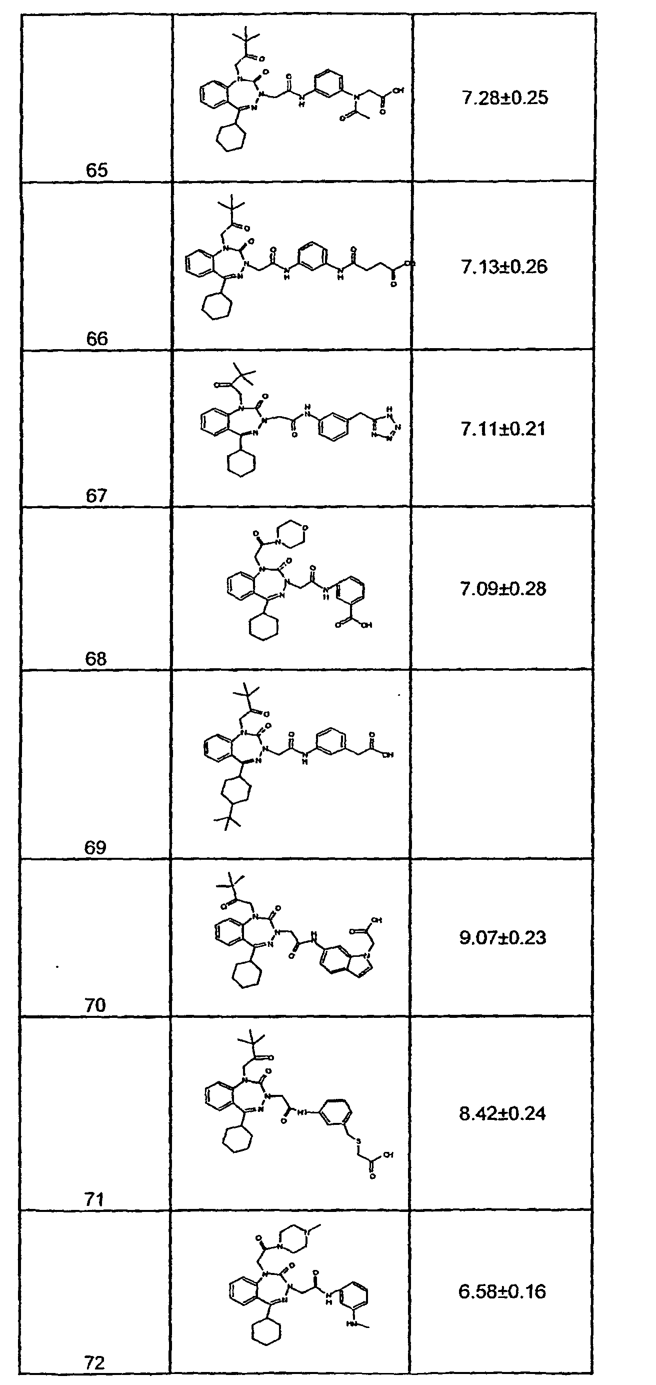

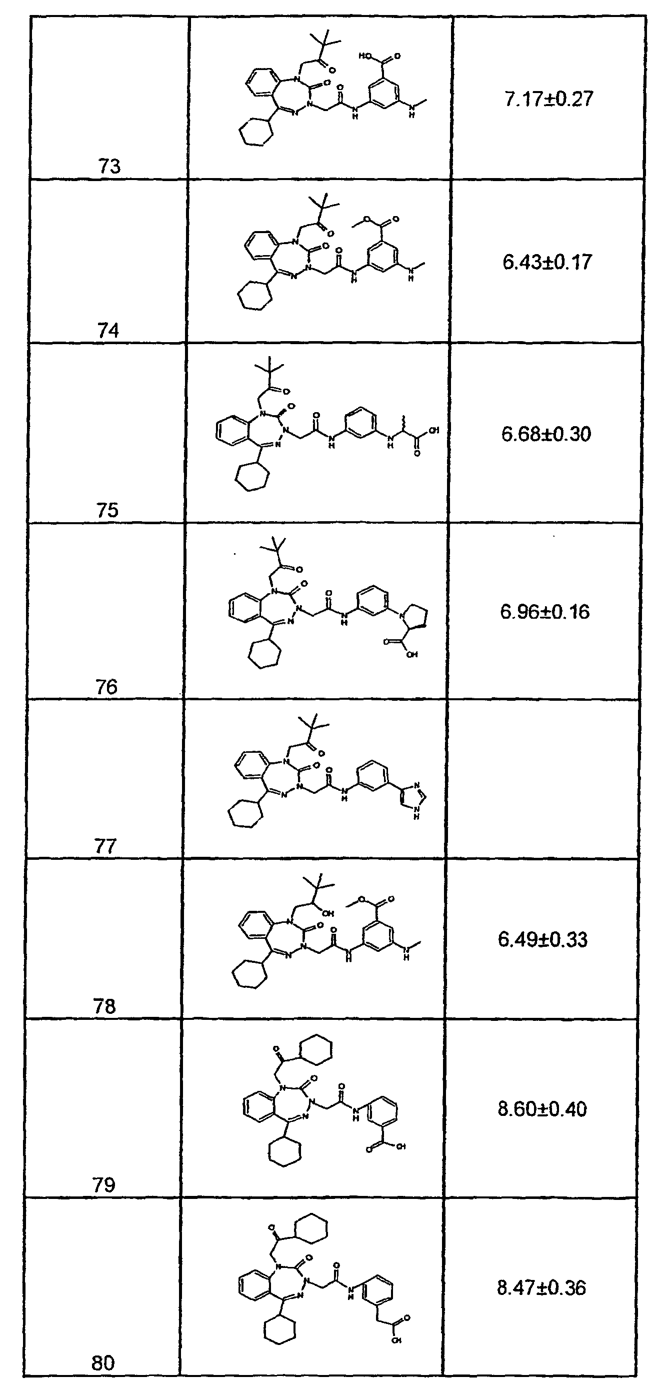

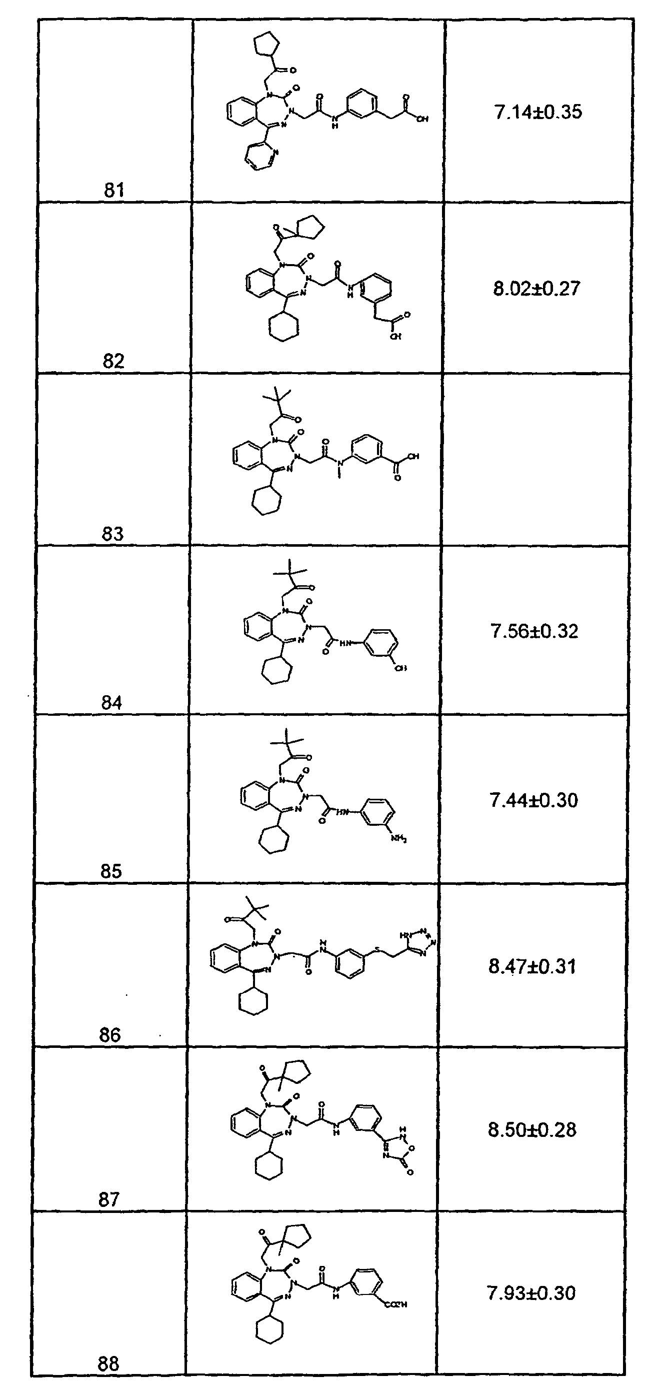

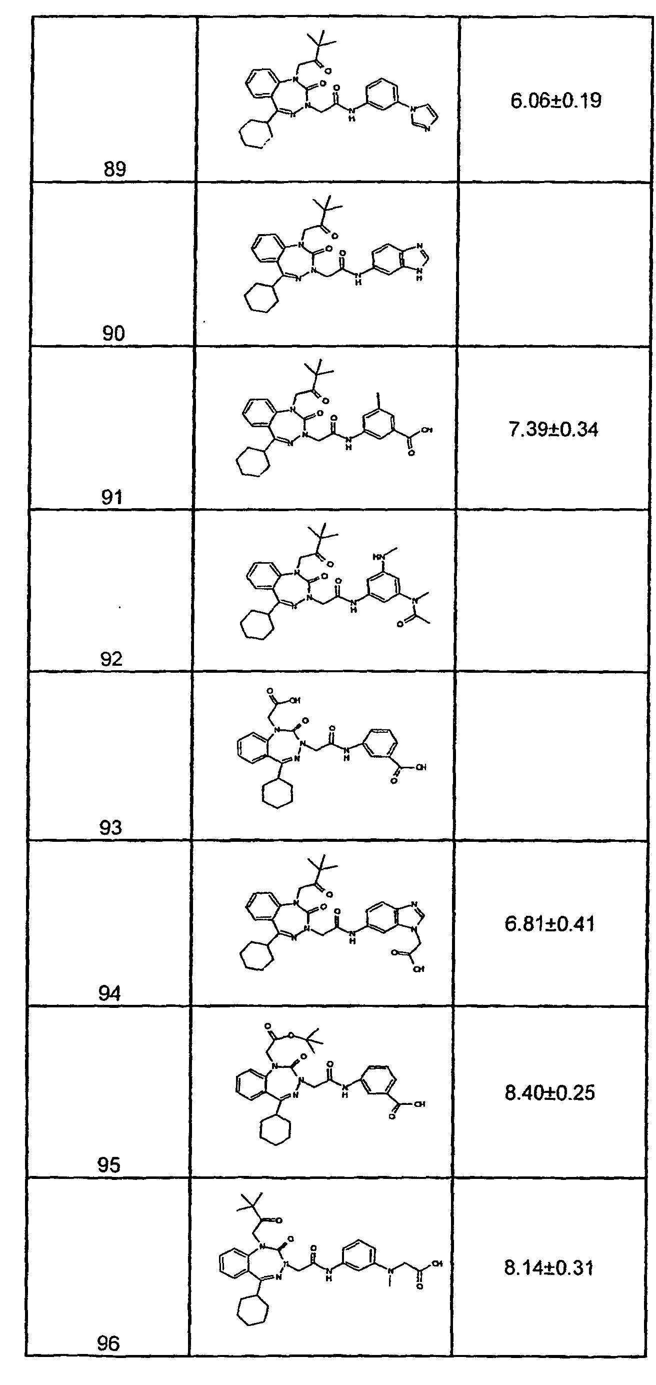

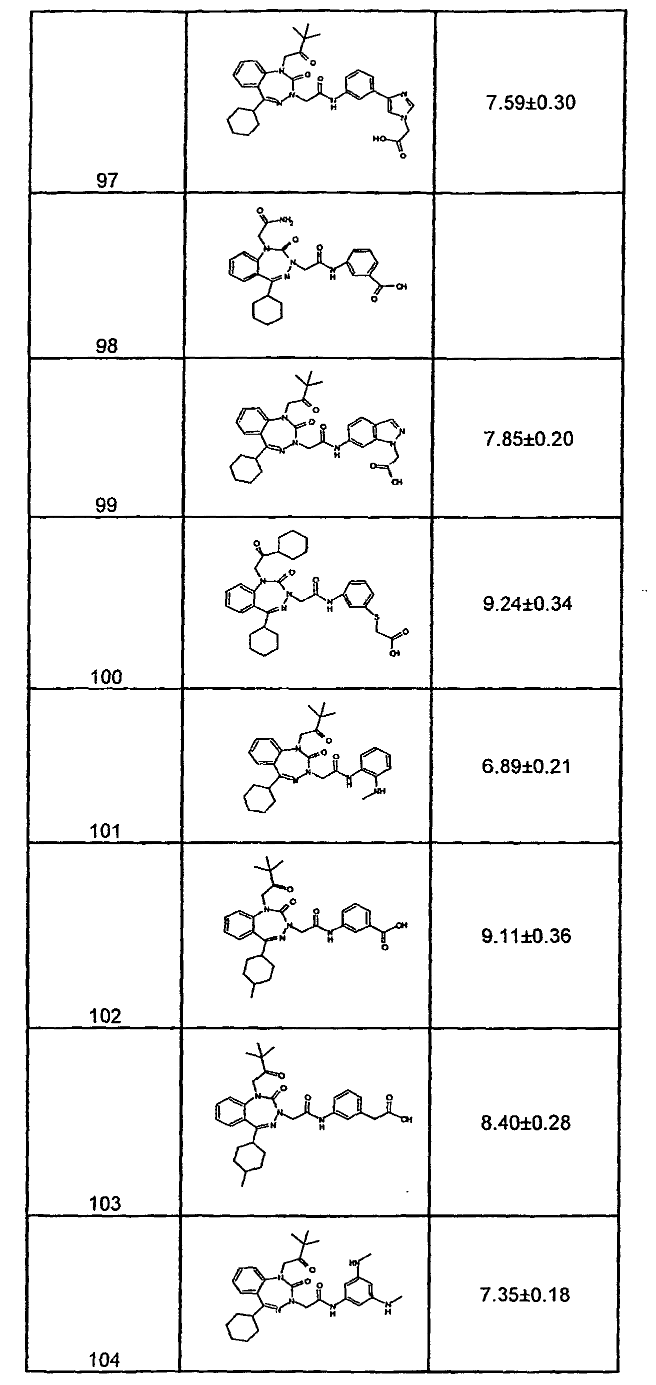

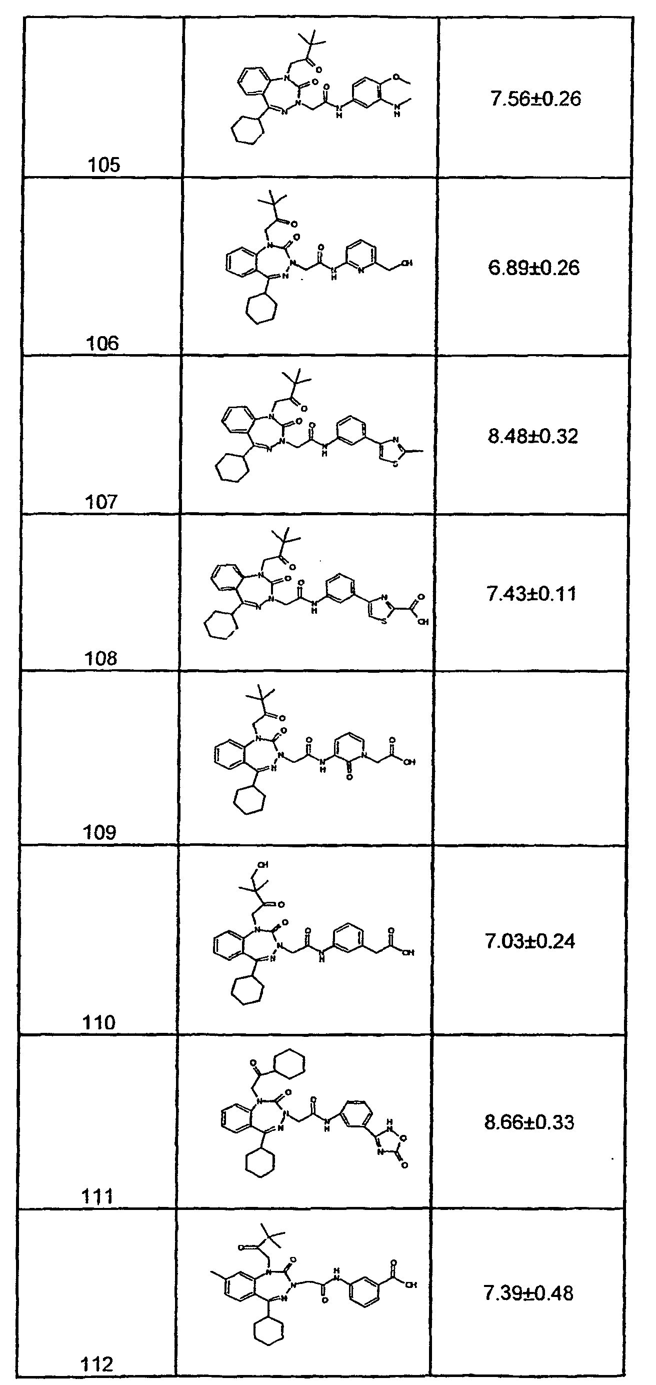

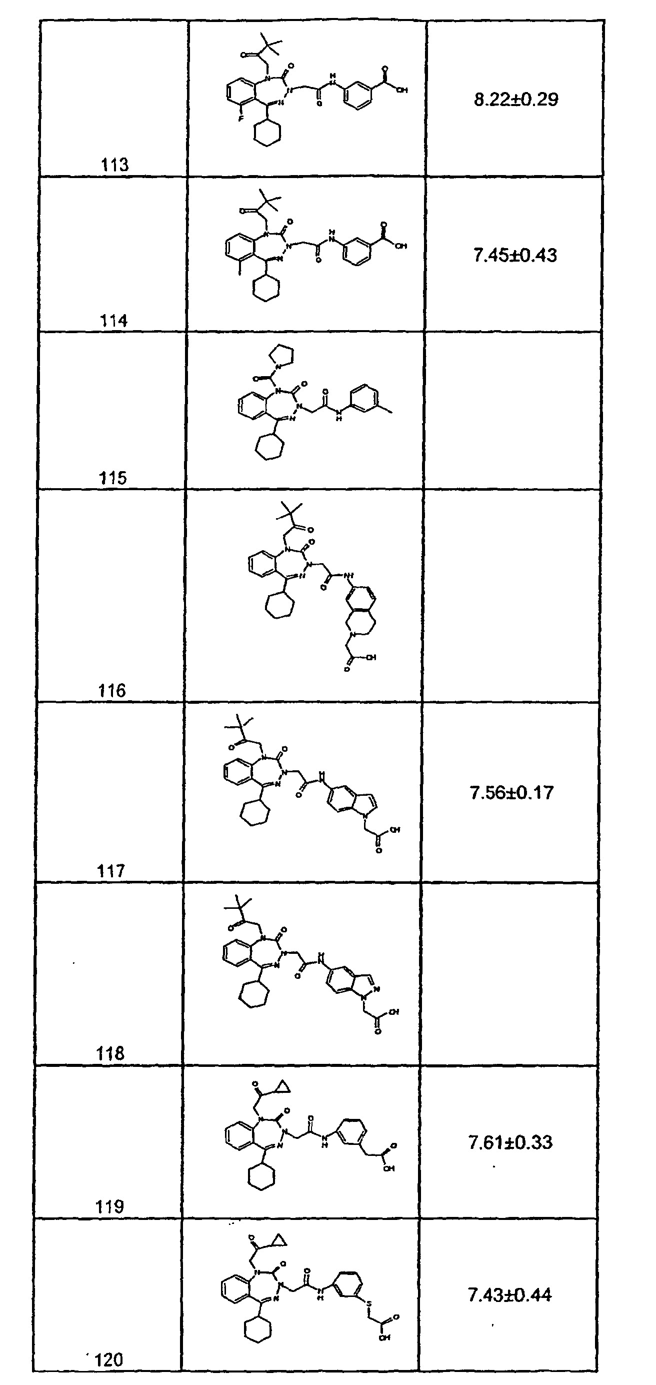

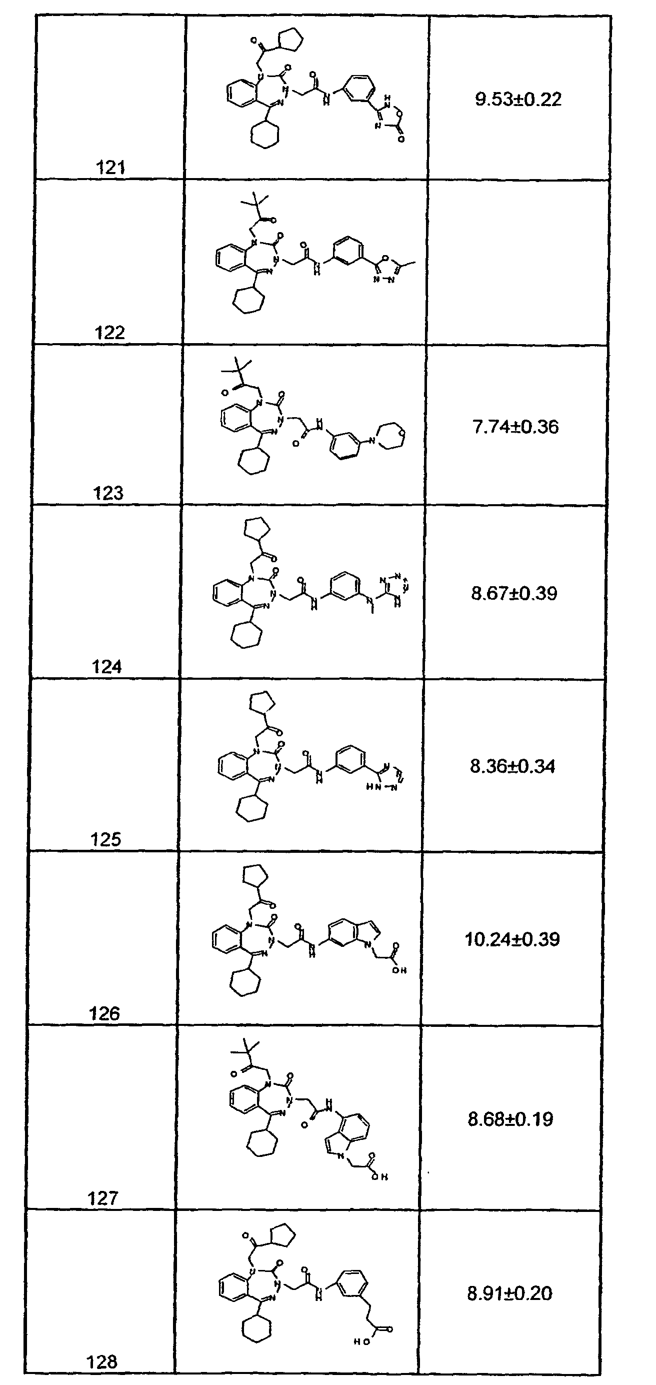

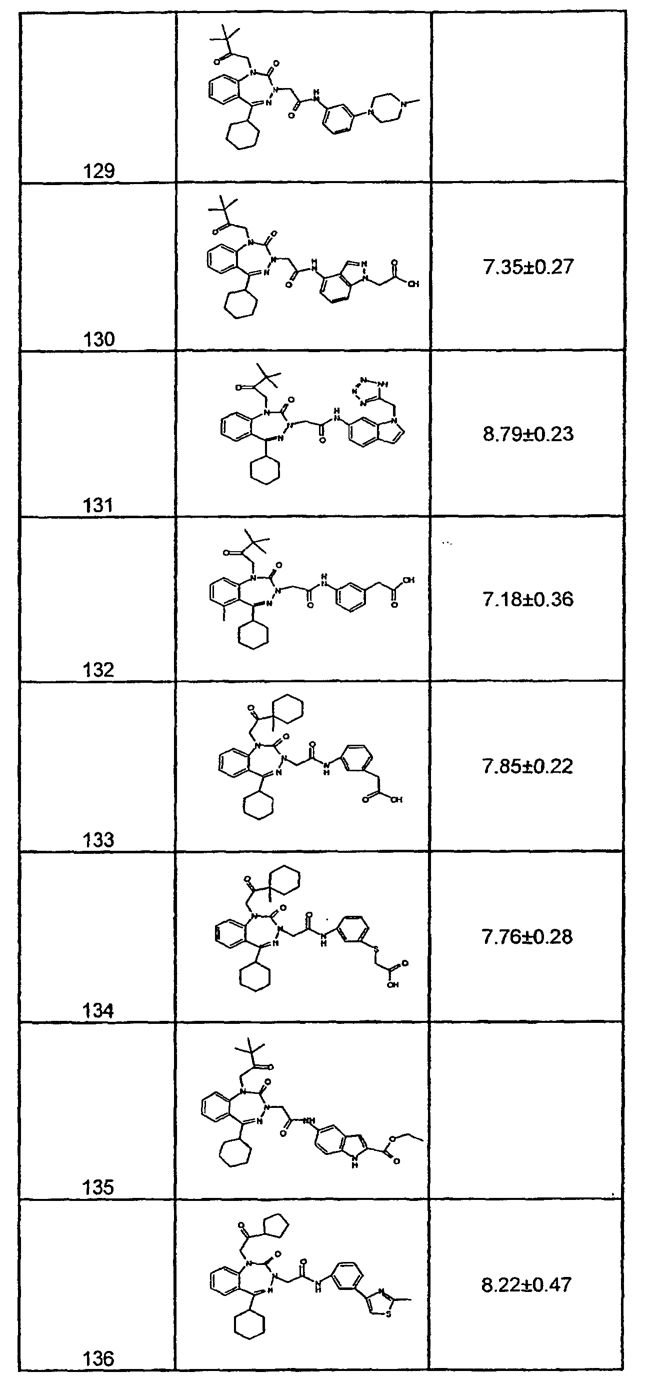

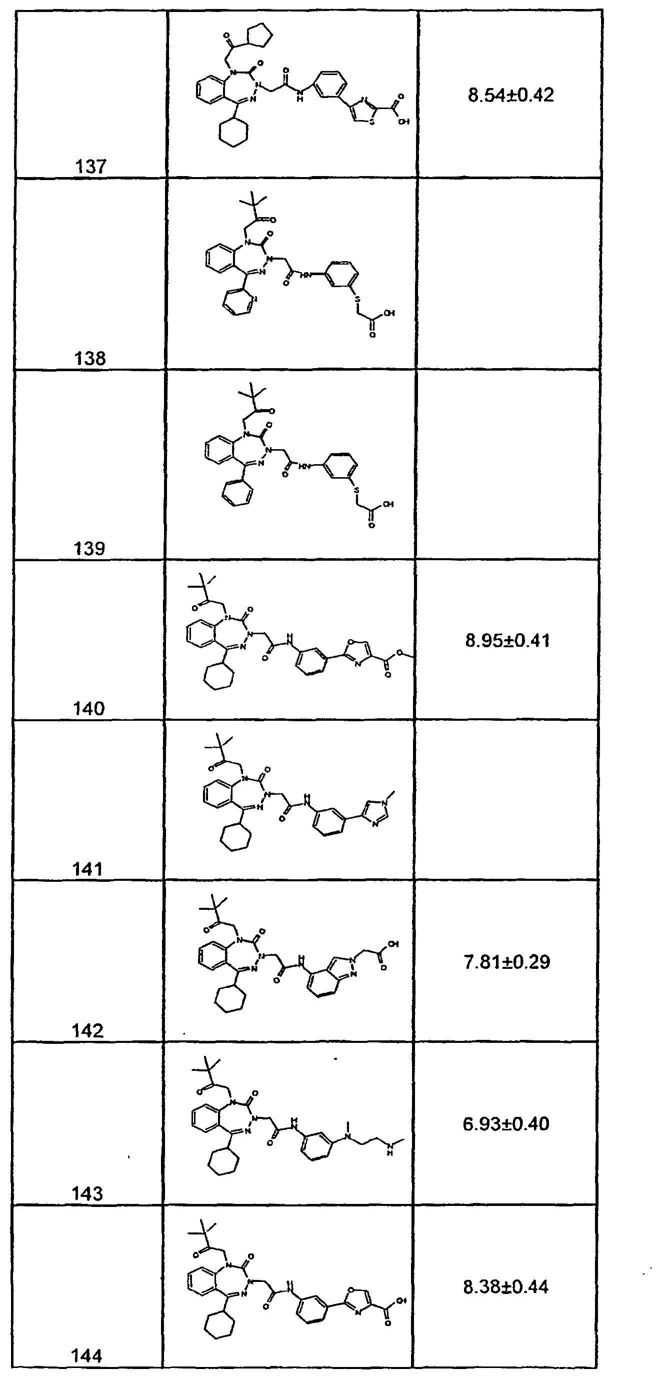

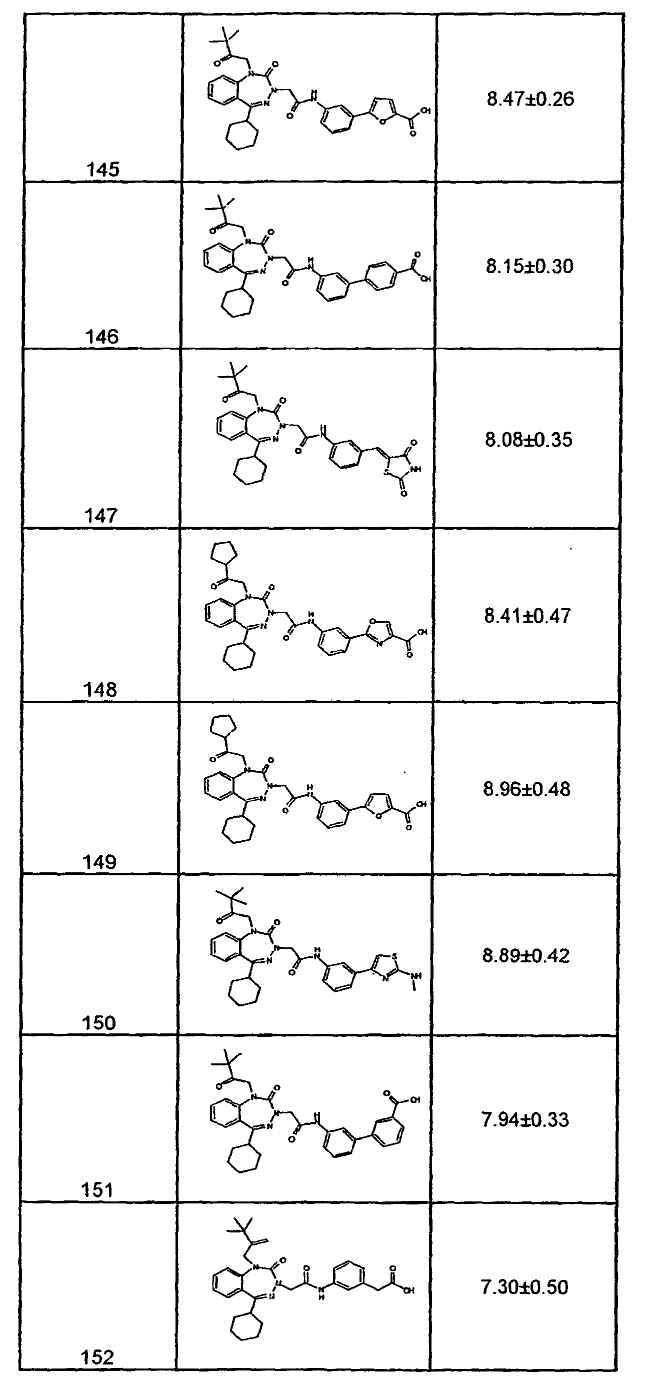

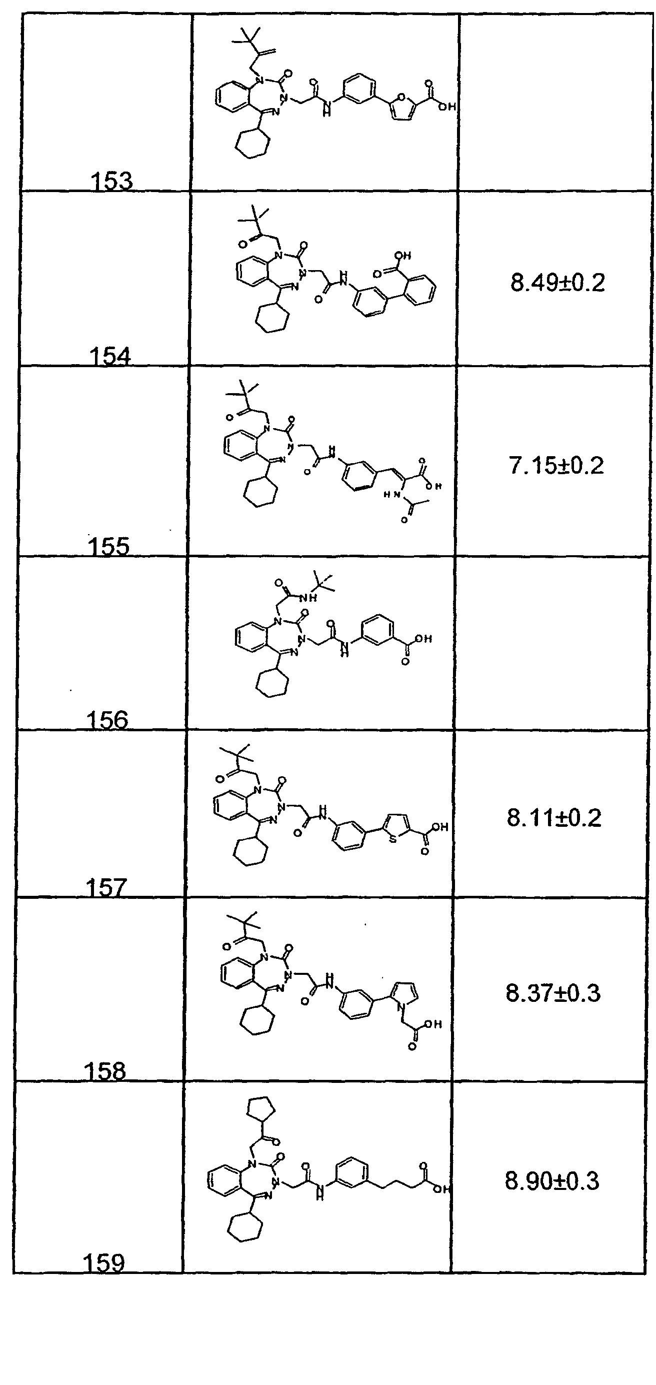

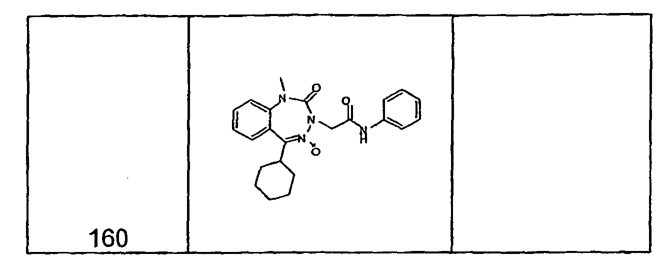

A number of compounds were tested at human gastrin (CCK₂) receptors which have been cloned into an NIH3T3 cell line as follows:

### Step a: Subcloning of IMAGE clone encoding the human CCK₂R into a mammalian expression vector

I.M.A.G.E. (Integrated Molecular Analysis of Genomes and their Expression) clone number 3504160 (Lennon et al. Genomics **33**, 151-152 (1996)) was purchased from the HGMP (Human Genome Mapping Project, Cambridge). The cDNA encoding the mRNA for the human CCK₂R, corresponding to accession number BC000740, was present in vector pOTB7 in host cell DH10B. The cells, initially streaked on to LB-Agar plates containing 20µg/ml chloramphenicol, were then grown in LB containing 20µg/ml chloramphenicol with shaking at 37°C according to standard techniques (Current Protocols in Molecular Biology, Wiley). DNA was prepared using the QIAGEN® EndoFree™ plasmid Maxi kit (Qiagen Ltd.) according to the manufacturer's protocol. The DNA was then amplified by PCR (polymerase chain reaction) from the start codon to the stop codon using primers containing restriction sites, *Eco* R1 and *Xba* I respectively, to facilitate unidirectional cloning. The start codon primer also contained a Kozak consensus site (Kozak M, *Nucleic Acids Res.* 1984 Jan 25;12(2):857-72) for optimal initiation of translation. Primers 1 and 2 (see Table 1) were synthesised to HPLC grade by Invitrogen. The PCR was performed in 20mM Tris-HCl (pH 8.4), 50mM KCl containing 2mM MgCl₂, 0.2mM dNTP (Invitrogen) and 0.1µM of each primer. A hot start PCR was used: the samples were denatured for 2min at 95°C, cooled to 75°C, then 1U *Taq* Polymerase (Invitrogen) was added and the reactions were cycled 30 times at 95°C for 1min, 60°C for 30sec and 72°C for 3min. The samples were cooled to 4°C, after a final extension at 72°C for 5min.

The PCR product was purified using the QIAGEN® MinElute™ PCR purification kit, according to the manufacturer's instructions. The PCR product and a mammalian expression vector were digested using both *Eco* RI (Promega Corp.) and *Xba* I (Promega Corp.) in 1x Buffer H (90mM Tris-HCl, 10mM MgCl₂, 50mM NaCl, pH 7.5) (Promega Corp.) and 0.1µg/ml BSA. The digested DNA bands of the correct size, analysed by ethidium bromide stained agarose/TBE gels, were excised and purified using QIAGEN® MinElute™ gel extraction kit, according to the manufacturer's instructions. The PCR product was then ligated into the vector using the Lightning™ DNA Ligation kit (Bioline) and transformed into *Escherichia coli,* strain XL1-Blue cells (Stratagene) according to the manufacturer's instructions.

Colonies were selected and screened using restriction digestion of DNA prepared from small-scale cultures (5ml) using QIAGEN® plasmid Mini-prep columns. One positive clone was cultured on a larger scale (100ml) using standard techniques (Current Protocols in Molecular Biology, Wiley) and DNA was prepared using QIAGEN® plasmid Maxi-prep columns. The DNA was then custom sequenced by MWG Biotech AG using primers 3, 4 and 5 (**Table A**). The sequence contained the correct sequence of primers 1 and 2 and the sequence of the coding region exactly matched that of accession number BC000740.

### Step b: Generation of Stable Cell Line

NIH3T3 cells from (ECACC) were cultured in Dulbecco's modified Eagle's medium (DMEM) (Invitrogen), containing 2mM Glutamax I (Invitrogen), 10% heat inactivated newborn calf serum (Invitrogen). Cells (4 x 10⁵) were seeded into 35mm x 10 mm dishes (Coming) and transfected using the Transfast™ reagent (Promega Corp.) according to the manufacturers instructions using 10µg of the hCCK₂R plasmid DNA at a ratio of 1:1 (DNA:Transfast™ reagent). Untransfected cells and cells transfected with vector only were also prepared as controls. After 48 h the cells were trypsinised using standard techniques (Culture of Animal Cells, A Manual of Basic Techniques 4^{th} Ed, R. Ian Freshney) and dilutions were plated on 35mm x 10mm dishes in media containing 800µg/ml G-418 (Invitrogen). Cells were selected for 2 weeks until individual, separate colonies appeared. In the untransfected cells all cells had died after this time, confirming adequate selection. Using cloning rings and trypsinisation, according to standard techniques (Culture of Animal Cells, A Manual of Basic Techniques 4^{th} Ed, R. Ian Freshney), individual colonies were picked from plates containing cells transfected with the vector only and cells transfected with the hCCK₂R plasmid construct. The cells were expanded and analysed by radioligand binding analysis (see below).

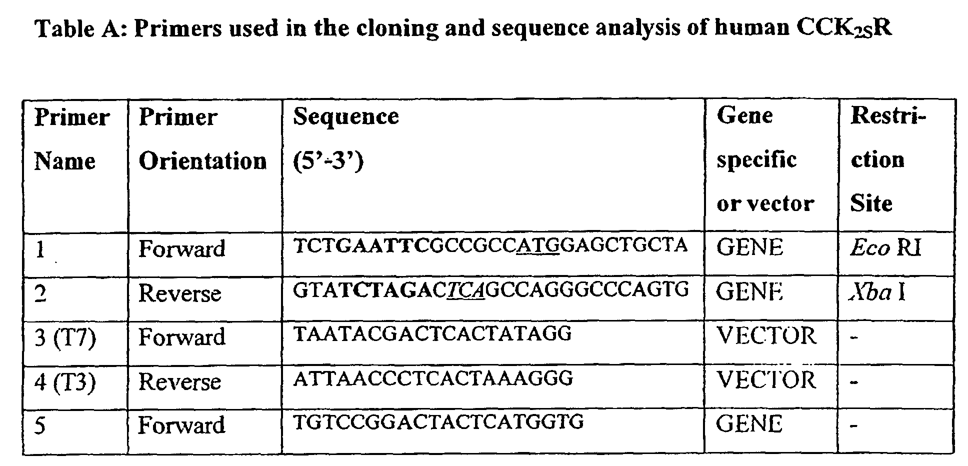

Restrictions site in bold. Start codon underlined. Stop codon italic and underlined.

### Step c: Clonal Selection

Stable clones expressing hCCK₂R were screened for their ability to specifically bind [¹²⁵I]-BH-CCK-8S in tissue concentration curve studies (0.3x10⁴ - 1x10⁶ cells per tube) using the assay conditions described below. Of those tested, clone 7 gave the highest amount of specifically bound and % specific bound label whilst also meeting the criteria that the amount of total bound label did not exceed 10% of the total added radio label (e.g. 4.2%). In addition there was a direct linear correlation between the amount of specific bound label and the cell concentration up to and including 2.5x10⁵ cells per ml. Based on the above, this clone was chosen for expansion and full binding characterisation.

### Step d: Membrane Preparation

Cultured clone 7 cells were stored as frozen pellets at -70°C until required. Cell pellets were thawed in CCK₂ assay buffer ( (mM): 10 Hepes; 130 NaCl; 5 MgCl_{2;} 4.7 KCl; 1 EGTA (pH7.2 at 21°C) with 0.125g Bacitracin added to each litre), and homogenised using a Polytron (4 x 1s). The resulting membrane preparation was centrifuged at 39,800g for 15min at 4°C. Each cell pellet was re-suspended in fresh buffer and re-centrifuged as above. The final pellet was re-suspended by homogenisation (Teflon-in-glass), to the appropriate membrane concentration.

### Step e: Incubation Conditions

For saturation and competition studies, the cell membranes prepared as in step d (3x 10⁴ cells per 400µl) were incubated for 150min at 21°C in a final volume of 0.5ml with CCK₂ assay buffer containing [¹²⁵I]-BH-CCK-8S (50µl; 200pM). Total and non-specific binding of [¹²⁵I]-BH-CCK-8S were defined, respectively using 50µl of buffer and 50µl of 10µM YM022. The assays were terminated by rapid filtration through pre-soaked Whatman GF/B filters which were washed (3 x3ml) with ice-cold 50mM Tris HCl (pH7.4 @ 4°C). Filters were transferred to plastic gamma counter vials and bound radioactivity determined by counting (1 min) in a Clini-gamma counter.

### Step f: Saturation analysis

The binding of [¹²⁵I]-BH-CCK-8S to the hCCK₂ receptor isoform was saturable. Scatchard plots appeared linear and the mean slope of the corresponding Hill plots was not significantly different from unity (0.97 ± 0.08; n = 4). The equilibrium dissociation constant (pK_{D}) and Bmax values were 10.75 ± 0.08 and 1.1 ± 0.3 finol per 1x10⁵ cells, respectively (n = 4 ± s.e. mean). Saturation data were analysed using the curve-fitting programmes, Radlig and Ligand.

### Step g: Competition studies

A number of compounds of the invention as well as reference compounds were tested for their ability to displace [¹²⁵I]-BH-CCK-8S from the receptors prepared as above. Briefly, dilution and addition of test compounds, radioligand and cell membranes were performed using a Beckman Biomek 2000. The ability of compounds to inhibit the specific binding of to hCCK₂ receptors was determined in triplicate over a range of concentrations at half-log intervals. Total and non-specific binding was determined for each compound. Each compound was tested in a minimum of three experiments. Competition data were fitted to the Hill equation using Graph-pad Prism software to obtain estimates of the IC₅₀ (mid-point location parameter) and n_{H} (mid-point slope parameter). Dissociation constants (K_{I}) were determined using the Cheng & Prusoff equation (1973) to correct for the receptor occupancy by the radioligand. All compounds were dissolved in DMF to give a stock concentration of either 1 or 10mM and subsequent dilutions were made in assay buffer. The pK_{I} for representative examples together with a number of reference compounds are shown in the table below. All Hill slopes were not significantly different from unity.

| Example no or reference | pKi±s.e.mean |
|---|---|
| Ex 22 | 9.45±0.04 |
| Ex 56 | 9.79±0.03 |
| Ex 58 | 9.48±0.08 |
| Ex 59 | 9.66±0.05 |
| Ex 121 | 9.96±0.01 |
| Ex 128 | 9.69±0.03 |
| Ex 160 | 5.49±0.05 |
| L-365, 260 | 8.45±0.09 |
| YM022 | 10.19±0.03 |

Compounds of certain examples were also tested in a CCK₁ binding assay. All the examples tested were found to have a CCK₁ pKᵢ not exceeding 6.5.

It is found that the compositions and products of the present invention comprising a compound of formula (I) and a proton pump inhibitor reduce hyperplasia, associated with administration of proton pump inhibitors. This was measured according to the following experimental protocol.

### Animals and treatment:

40 male SPF Wistar rats (200 g) were divided into 4 treatment groups and 2 strata. The treatment of the 20 rats in the second stratum started 2 weeks after the treatment of the first stratum. The design of the study was completely randomised double blind with individual blinding; all rats were placed in a separate cage. Animals had continuous access to water and food.

Animals were treated once daily during 14 days:
- Control group: 1 ml gastrin test drug vehicle + 1 ml p.o.(gavage) 0,25% Methocel (Dow Corning)
- PPI group: 1 ml gastrin test drug vehicle +1 ml p.o.(gavage) 25 mg/kg Rabeprazole in 0.25% Methocel.
- GRA group: 1 ml gastrin test drug + 1 ml p.o. (gavage) 0,25% Methocel
- GRA-PPI group: 1 ml gastrin test drug + 1 ml p.o.(gavage) 25 mg/kg Rabeprazole in 0.25% Methocel.

Gastrin test drug made up to an appropriate dose in physiologically compatible solvent.

### Preparation of tissue:

After removal of the fundus, the stomach were rinsed with phosphate buffered saline prior to fixation with 4% formalin in Millonig buffer. After 4 hours immersion in fixative solutions at room temperature, tissue was rinsed in phosphate buffered saline (PBS), dehydrated and embedded in paraffin using the Leitz paraffin embedding station (Leitz TP 1050; Germany) dehydration module and paraffin embedding module (Leitz EG 1160; Germany).

Cross sections (3 µm thick) of the oxyntic part of the stomach were made at 3 levels, each separated by a distance of 400 µm.

### Immunostaining

The following indirect immunofluorescence labeling method was used:
- removal of paraffin and rehydratation of the sections followed by a blocking step
- primary antibodies: polyclonal guinea pig anti-histidine decarboxylase, 1/2000 (from Euro-Diagnostica) and monoclonal mouse anti PCNA 1/2500 (Clone PC10 from Sigma). All antibodies were diluted in a 0.2% BSA solution. Sections were incubated overnight at 4°C and then washed with a BSA solution. secondary antibodies: goat anti guinea pig coupled to CY5, 1/500 (from Jackson Laboratories) and goat anti-mouse coupled to Cy3, 1/250 (from Jackson Laboratories); incubation for 4 hours at 37°C. After rinsing with BSA and PBS solutions, sections were mounted with slowfade (Molecular Probes Europe BV), and stored at 4°C.

### Imaging

Fluorescence labelling was observed with an epifluorescence microscope or a Zeiss LSM510 (Carl Zeiss Jena GmbH) confocal microscope.

By using CY5- and CY3-coupled antibodies, the high autofluorescence properties of the oxyntic mucosa were circumvented when sections are illuminated by a 488 nm (FITC channel) light source. Negative controls, by omitting the primary antibodies, and an isotype control staining for PCNA showed complete absence of staining. The specific labelling of PCNA was checked using double staining with TOPRO-3® (Molecular Probes Europe BV), a nuclear stain. Only in the most luminal located epithelial cells, non-specific cytoplasmic labelling was present. In the glandular part of the mucosa, non-specific PCNA-staining was absent.

For determination of the labelling index of ECL cells, at least 80 confocal images per rat were taken from the 3 slides at the 3 different levels. The ratio of double labelled cells (HDC + PCNA) and all HDC labelled cells yielded the labelling index of ECL cells.

Proliferation activity of ECL cells in the PPI group is expected to be increased compared with sham, GRA and GRA-PPI groups (Eissele, R., Patberg, H., Koop, H., Krack, W., Lorenz, W., McKnight, A.T., and Arnold, R. Effect of gastrin receptor blockade on endrocine cells in rats during achlorhydria. *Gastroenterology*, 103, 1596-1601, 1992). Increased proliferation by PPI will be completely blocked by GRA.

## Claims

1. Use of a compound of formula (I) 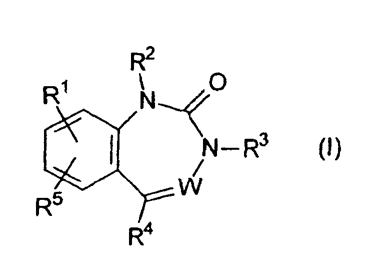 wherein:
W is N or N⁺-O⁻;
R¹ and R⁵ are independently H, C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino, (C₁ to C₆ alkyl)aminocarbonyl, di(C₁ to C₆ alkyl)aminocarbonyl, [*N*-Z)(C₁ to C₆ alkyl)carbonylamino, formyloxy, formamido, (C₁ to C₆ alkyl)aminosulfonyl, di(C₁ to C₆ alkyl)aminosulfonyl, [*N*-Z](C₁ to C₆ alkyl)sulfonylamino or cyano; or R¹ and R⁵ together form a methylenedioxy group;
R² is H or an optionally substituted C₁ to C₁₈ hydrocarbyl group wherein up to three C atoms may optionally be replaced by N, O and/or S atoms.
R³ is -(CR¹¹R¹²)ₘ-X-(CR¹³R¹⁴)ₚ-R⁹;
m is 0, 1, 2, 3 or 4;
p is 0, 1 or 2;
X is a bond, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 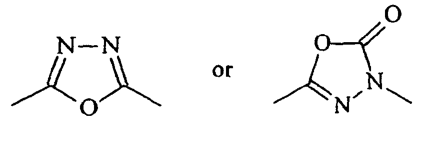
R⁹ is H; C₁ to C₆ alkyl; or phenyl, naphthyl, pyridyl, benzimidazolyl, indazolyl, quinolinyl, isoquinolinyl, tetrahydroisoquinolinyl, indolinyl, isoindolinyl, indolyl, isoindolyl or 2-pyridonyl, all optionally substituted with 1, 2 or 3 groups independently selected from -L-Q
wherein:
L is a bond, or a group of the formula -(CR¹⁷R¹⁸)ᵥ-Y-(CR¹⁷R¹⁸)_{w}, wherein v and w are independently 0, 1, 2 or 3, and Y is a bond, -CR¹⁵=CR¹⁶-, phenyl, furanyl, thiophenyl, pyrrolyl, thiazolyl, imidazolyl, oxazolyl, isoxazolyl, pyrazolyl, isoxazolonyl, piperazinyl, piperidinyl, morpholinyl, pyrrolidinyl, isothiazolyl, triazolyl, oxadiazolyl, thiadiazolyl, pyridyl or pyridazyl; and
Q is H, (C₁ to C₆ alkyl)oxy, [*N*-Z](C₁ to C₆ alkyl)oxy(C₁ to C₆ alkyl)amino, thio, (C₁ to C₆ alkyl)thio, carboxy(C₁ to C₆ alkyl)thio, carboxy, carboxy(C₁ to C₆ alkyl), carboxy(C₁ to C₆ alkenyl), [*N*-Z]carboxy(C₁ to C₆ alkyl)amino, carboxy(C₁ to C₆ alkyl)oxy, formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, amino, [*N*-Z](C₁ to C₆ alkyl)amino, aminocarbonyl, (C₁ to C₆ alkyl)aminocarbonyl, di(C₁ to C₆ alkyl)aminocarbonyl, [*N*-Z](C₁ to C₆ alkyl)carbonylamino, C₅ to C₈ cycloalkyl, [*N*-Z](C₁ to C₆ alkyl)carbonyl(C₁ to C₆ alkyl)amino, halo, halo(C₁ to C₆ alkyl), sulfamoyl, [*N*-Z](C₁ to C₆ alkyl)sulfonylamino, (C₁ to C₆ alkyl)sulfonylaminocarbonyl, carboxy(C₁ to C₆ alkyl)sulfonyl, carboxy(C₁ to C₆ alkyl)sulfinyl, tetrazolyl, [*N*-Z]tetrazolylamino, cyano, amidino, amidinothio, SO₃H, formyloxy, formamido, C₃ to C₈ cycloalkyl, (C₁ to C₆ alkyl)sulphamoyl, di(C₁ to C₆ alkyl)sulphamoyl, (C₁ to C₆ alkyl)carbonylaminosulfonyl, 5-oxo-2,5-dihydro[1,2,4]oxadiazolyl, carboxy(C₁ to C₆ alkyl)carbonylamino, tetrazolyl(C₁ to C₆ alkyl)thio, [*N*-Z]tetrazolyl(C₁ to C₆ alkyl)amino, 5-oxo-2,5-dihydro[1,2,4]thiadiazolyl, 5-oxo-1,2-dihydro[1,2,4]triazolyl, [*N*-Z](C₁ to C₆ alkyl)amino(C₁ to C₆ alkyl)amino, or a group of the formula 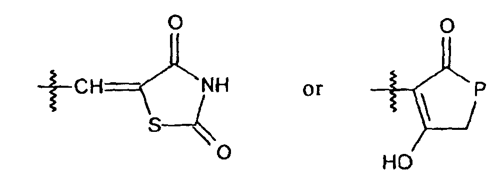
wherein P is O, S or NR¹⁹;
Z is H, C₁ to C₆ alkyl, t-butoxycarbonyl, acetyl, benzoyl or benzyl;
R⁴ is an optionally substituted C₁ to C₁₈ hydrocarbyl group wherein up to three C atoms may optionally be replaced by N, O and/or S atoms;
R¹¹, R¹², R¹³, R¹⁴, R¹⁵, R¹⁷, R¹⁸ and R¹⁹ are independently H or C₁ to C₃ alkyl; and
R¹⁶ is H, C₁ to C₃ alkyl, or acetylamino;
or a pharmaceutically acceptable salt thereof;
for the preparation of a medicament for the treatment of gastrin related disorders.

2. Use according to claim 1 wherein W is N.

3. Use according to claim 1 or claim 2 wherein R¹ and R⁵ are both H.

4. Use according to any preceding claim wherein R² is:
-(CH₂)ₛ-C(R⁶R⁷)ₙ-(CH₂)ₜ-R⁸
wherein:
R⁶ and R⁷ are independently selected from H, C₁ to C₆ alkyl or OH; or R⁶ and R⁷ together represent an =O group;
n is 0 or 1;
s is 0, 1, 2 or 3;
t is 0, 1, 2 or 3; and
R⁸ is selected from H, C₁ to C₁₂ alkyl, (C₁ to C₁₂ alkyl)oxy, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano).

5. Use according to claim 4 wherein s is 1, n is 1, and R⁶ and R⁷ together represent an =O group.

6. Use according to claims 4 or 5 wherein t is 0 and R⁸ is a C₃ to C₁₂ cycloalkyl group (optionally substituted with a methyl group) or a branched C₃ to C₁₂ alkyl group.

7. Use according to any one of claims 4 to 6 wherein R⁸ is a t-butyl, cyclohexyl, 1-methylcyclohexyl, 1-methylcyclopentyl or cyclopentyl group.

8. Use according to any one of the preceding claims wherein m is 1, R¹¹ is H and R¹² is H

9. Use according to any one of the preceding claims wherein p is 0.

10. Use according to any one of the preceding claims wherein X is C(O)NH

11. Use according to any one of the preceding claims wherein R⁹ is phenyl substituted with a carboxy, carboxy(C₁ to C₆ alkyl), tetrazolyl, tetrazolyl-*N*-(C₁ to C₆ alkyl)amino, carboxy(C₁ to C₆ alkyl)thio, carboxy(C₁ to C₆ alkyl)sulfonyl, (C₁ to C₆ alkyl)amino, or 5-oxo-2,5-dihydro[1,2,4]oxadiazolyl group; or R⁹ is a *N*-[carboxy(C₁ to C₆ alkyl)]indolinyl or *N*-[carboxy(C₁ to C₆ alkyl)]indolyl group.

12. Use according to claim 11 wherein the phenyl group is substituted at its 3-position.

13. Use according to any one of the preceding claims wherein R⁴ is
-(CH₂)_{q}-T-R¹⁰
wherein:
q is 0, 1, 2 or 3;
T is a bond, O, S, NH or N(C₁ to C₆ alkyl); and
R¹⁰ is C₁ to C₁₂ alkyl, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, C₃ to C₈ cycloalkyl, (C₃ to C₈ cycloalkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano).

14. Use according to claim 13 wherein q is 0, T is a bond and R¹⁰ is C₁ to C₁₂ alkyl, C₃ to C₁₂ cycloalkyl, pyridyl or phenyl (all optionally substituted with OMe, NMe₂, CF₃, Me, F, Cl, Br or I).

15. Use according to any one of the preceding claims wherein R⁴ is cyclohexyl.

16. A pharmaceutical composition comprising a proton pump inhibitor and a compound of formula (I), as defined in any one of claims 1 to 15, together with a pharmaceutically acceptable diluent or carrier.

17. A composition according to claim 16 wherein the proton pump inhibitor is selected from (RS)-rabeprazole, (RS)-omeprazole, lansoprazole, pantoprazole, (R)-omeprazole, (S)-omeprazole, perprazole, (R)-rabeprazole, (S)-rabeprazole, or the alkaline salts thereof.

18. A composition according to claim 16 or 17 wherein the proton pump inhibitor and the compound of formula (I) are each in an amount producing a therapeutically beneficial effect in patients suffering from gastrointestinal disorders.

19. A composition according to claim 18 wherein said therapeutically beneficial effect is a synergistic effect on the reduction of acid secretion in patients suffering from gastrointestinal disorders, or the prevention of gastrointestinal disorders in said patients, or the reduction of adverse effects associated with the one of the active ingredients by the other active ingredients.

20. A composition according to any one of claims 16 to 19 wherein the amount of each of the active ingredients is equal to or less than that which is approved or indicated in monotherapy with said active ingredient.

21. A kit containing as a first active ingredient a compound of formula (I), as defined in any one of claims I to 15, and as a second active ingredient a proton pump inhibitor, as a combined preparation for simultaneous, separate or sequential use in the treatment of patients suffering from gastrointestinal disorders.

22. Use of a composition according to any one of claims 16 to 20 or a kit according to claim 23 for the preparation of a medicament for the treatment of gastrointestinal disorders.

23. Use of a proton pump inhibitor for the preparation of a medicament for the treatment of gastrointestinal disorders, said treatment comprising the simultaneous or sequential administration of said proton pump inhibitor and a compound of formula (I), as defined in any one of claims 1 to 15, wherein said proton pump inhibitor enhances the effect of the compound of formula (I) on gastrin-related disorders in patients suffering from gastrointestinal disorders.

24. Use of a compound of formula (I), as defined in any one of claims I to 15, for the preparation of a medicament for the treatment of gastrointestinal disorders, said treatment comprising the simultaneous or sequential administration of said proton pump inhibitor and a compound of formula (I), wherein said compound of formula (I) enhances the effect of the proton pump inhibitor on the reduction of acid secretion in patients suffering from gastrointestinal disorders.

25. Use of a compound of formula (I), as defined in any one of claims 1 to 15, for the preparation of a medicament for reducing adverse effects associated with administration of proton pump inhibitors in patients suffering from gastrointestinal disorders.

26. Use according to claim 25 wherein the adverse effect is hyperplasia.

27. Use of a proton pump inhibitor for the preparation of a medicament for reducing adverse effects associated with administration of a compound of formula (I), as defined in any one of claims I to 15, in patients suffering from gastrointestinal disorders.

28. A method of making a pharmaceutical composition according to any one of claims 18 to 22, comprising mixing a compound of formula (I), as defined in any one of claims I to 15, and a proton pump inhibitor with a pharmaceutically acceptable diluent or carrier.

29. A compound of formula (IIa) 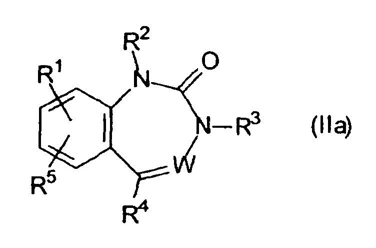 wherein:
W is N or N⁺-O⁻;
R¹ and R⁵ are independently H, C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino, (C₁ to C₆ alkyl)aminocarbonyl, di(C₁ to C₆ alkyl)aminocarbonyl, [*N*-Z](C₁ to C₆ alkyl)carbonylamino, formyloxy, formamido, (C₁ to C₆ alkyl)aminosulfonyl, di(C₁ to C₆ alkyl)aminosulfonyl, [*N*-Z](C₁ to C₆ alkyl)sulfonylamino or cyano; or R¹ and R⁵ together form a methylenedioxy group;
R² is -(CH₂)ₛ-C(O)-(CH₂)ₜ-R⁸
s is 0, 1, 2 or 3;
t is 0, 1, 2 or 3;
R⁸ is selected from H, C₁ to C₁₂ alkyl, (C₁ to C₁₂ alkyl)oxy, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano);
R³ is -(CR¹¹R¹²)ₘ-X-(CR¹³R¹⁴)ₚ-R⁹;
m is 0, 1, 2, 3 or 4;
p is 0, 1 or 2;
X is a bond, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 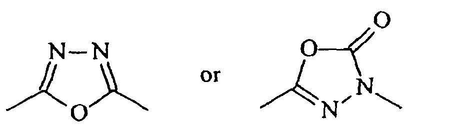
R⁹ is H; C₁ to C₆ alkyl; or phenyl, naphthyl, pyridyl, benzimidazolyl, indazolyl, quinolinyl, isoquinolinyl, tetrahydroisoquinolinyl, indolinyl, isoindolinyl, indolyl, isoindolyl or 2-pyridonyl, all optionally substituted with 1, 2 or 3 groups independently selected from -L-Q
wherein:
L is a bond, or a group of the formula -(CR¹⁷R¹⁸)ᵥ-Y-(CR¹⁷R¹⁸)_{w}, wherein v and w are independently 0, 1, 2 or 3, and Y is a bond, -CR¹⁵=CR¹⁶-, phenyl, furanyl, thiophenyl, pyrrolyl, thiazolyl, imidazolyl, oxazolyl, isoxazolyl, pyrazolyl, isoxazolonyl, piperazinyl, piperidinyl, morpholinyl, pyrrolidinyl, isothiazolyl, triazolyl, oxadiazolyl, thiadiazolyl, pyridyl or pyridazyl; and
Q is H, (C₁ to C₆ alkyl)oxy, [*N*-Z](C₁ to C₆ alkyl)oxy(C₁ to C₆ alkyl)amino, thio, (C₁ to C₆ alkyl)thio, carboxy(C₁ to C₆ alkyl)thio, carboxy, carboxy(C₁ to C₆ alkyl), carboxy(C₁ to C₆ alkenyl), [*N*-Z]carboxy(C₁ to C₆ alkyl)amino, carboxy(C₁ to C₆ alkyl)oxy, formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, amino, [*N*-Z)(C₁ to C₆ alkyl)amino, aminocarbonyl, (C₁ to C₆ alkyl)aminocarbonyl, di(C₁ to C₆ alkyl)aminocarbonyl, [*N*-Z](C₁ to C₆ alkyl)carbonylamino, C₅ to C₈ cycloalkyl, [*N*-Z](C₁ to C₆ alkyl)carbonyl(C₁ to C₆ alkyl)amino, halo, halo(C₁ to C₆ alkyl), sulfamoyl, [*N*-Z](C₁ to C₆ alkyl)sulfonylamino, (C₁ to C₆ alkyl)sulfonylaminocarbonyl, carboxy(C₁ to C₆ alkyl)sulfonyl, carboxy(C₁ to C₆ alkyl)sulfinyl, tetrazolyl, [*N*-Z]tetrazolylamino, cyano, amidino, amidinothio, SO₃H, formyloxy, formamido, C₃ to C₈ cycloalkyl, (C₁ to C₆ alkyl)sulphamoyl, di(C₁ to C₆ alkyl)sulphamoyl, (C₁ to C₆ alkyl)carbonylaminosulfonyl, 5-oxo-2,5-dihydro[1,2,4]oxadiazolyl, carboxy(C₁ to C₆ alkyl)carbonylamino, tetrazolyl(C₁ to C₆ alkyl)thio, [*N*-Z]tetrazolyl(C₁ to C₆ alkyl)amino, 5-oxo-2,5-dihydro[1,2,4]thiadiazolyl, 5-oxo-1,2-dihydro[1,2,4]triazolyl, [*N*-Z](C₁ to C₆ alkyl)amino(C₁ to C₆ alkyl)amino, or a group of the formula 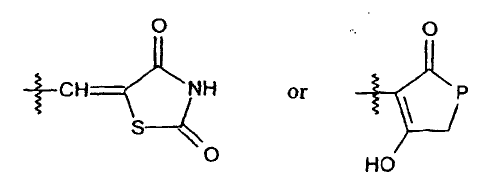
wherein P is O, S or NR¹⁹;
Z is H, C₁ to C₆ alkyl, t-butoxycarbonyl, acetyl, benzoyl or benzyl;
R⁴ is an optionally substituted C₁ to C₁₈ hydrocarbyl group wherein up to three C atoms may optionally be replaced by N, O and/or S atoms;
R¹¹, R¹², R¹³, R¹⁴, R¹⁵, R¹⁷, R¹⁸ and R¹⁹ are independently H or C₁ to C₃ alkyl; and
R¹⁶ is H or C₁ to C₃ alkyl, or acetylamino;
or a pharmaceutically acceptable salt thereof;
with the proviso that R² is not CH₂CO₂H or C(O)CH₃ when R⁴ is phenyl.

30. A compound of formula (IIb) 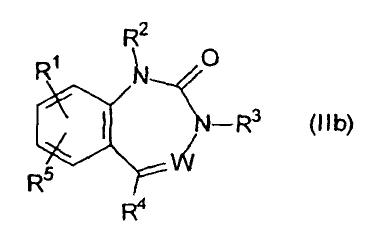 wherein:
W is N or N⁺-O⁻;
R¹ and R⁵ are independently H, C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino, (C₁ to C₆ alkyl)aminocarbonyl, di(C₁ to C₆ alkyl)aminocarbonyl, [*N*-Z](C₁ to C₆ alkyl)carbonylamino, formyloxy, formamido, (C₁ to C₆ alkyl)aminosulfonyl, di(C₁ to C₆ alkyl)aminosulfonyl, [*N*-Z](C₁ to C₆ alkyl)sulfonylamino or cyano; or R¹ and R⁵ together form a methylenedioxy group;
R² is an optionally substituted C₁ to C₁₈ hydrocarbyl group wherein up to three C atoms may optionally be replaced by N, O and/or S atoms;
R³ is -(CR¹¹R¹²)ₘ-X-(CR¹³R¹⁴)ₚ-R⁹;
m is 0, 1, 2, 3 or 4;
p is 0, 1 or 2;
X is a bond, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 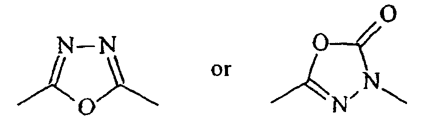
R⁹ is H; C₁ to C₆ alkyl; or phenyl, naphthyl, pyridyl, benzimidazolyl, indazolyl, quinolinyl, isoquinolinyl, tetrahydroisoquinolinyl, indolinyl, isoindolinyl, indolyl, isoindolyl or 2-pyridonyl, all optionally substituted with 1, 2 or 3 groups independently selected from -L-Q
wherein:
L is a bond, or a group of the formula -(CR¹⁷R¹⁸)ᵥ-Y-(CR¹⁷R¹⁸)_{w}, wherein v and w are independently 0, 1, 2 or 3, and Y is a bond, -CR¹⁵=CR¹⁶-, phenyl, furanyl, thiophenyl, pyrrolyl, thiazolyl, imidazolyl, oxazolyl, isoxazolyl, pyrazolyl, isoxazolonyl, piperazinyl, piperidinyl, morpholinyl, pyrrolidinyl, isothiazolyl, triazolyl, oxadiazolyl, thiadiazolyl, pyridyl or pyridazyl; and
Q is H, (C₁ to C₆ alkyl)oxy, [*N*-Z](C₁ to C₆ alkyl)oxy(C₁ to C₆ alkyl)amino, thio, (C₁ to C₆ alkyl)thio, carboxy(C₁ to C₆ alkyl)thio, carboxy, carboxy(C₁ to C₆ alkyl), carboxy(C₁ to C₆ alkenyl), [*N*-Z]carboxy(C₁ to C₆ alkyl)amino, carboxy(C₁ to C₆ alkyl)oxy, formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, amino, [*N*-Z](C₁ to C₆ alkyl)amino, aminocarbonyl, (C₁ to C₆ alkyl)aminocarbonyl, di(C₁ to C₆ alkyl)aminocarbonyl, [*N*-Z](C₁ to C₆ alkyl)carbonylamino, C₅ to C₈ cycloalkyl, [*N*-Z](C₁ to C₆ alkyl)carbonyl(C₁ to C₆ alkyl)amino, halo, halo(C₁ to C₆ alkyl), sulfamoyl, [*N*-Z](C₁ to C₆ alkyl)sulfonylamino, (C₁ to C₆ alkyl)sulfonylaminocarbonyl, carboxy(C₁ to C₆ alkyl)sulfonyl, carboxy(C₁ to C₆ alkyl)sulfinyl, tetrazolyl, [*N*-Z]tetrazolylamino, cyano, amidino, amidinothio, SO₃H, formyloxy, formamido, C₃ to C₈ cycloalkyl, (C₁ to C₆ alkyl)sulphamoyl, di(C₁ to C₆ alkyl)sulphamoyl, (C₁ to C₆ alkyl)carbonylaminosulfonyl, 5-oxo-2,5-dihydro[1,2,4]oxadiazolyl, carboxy(C₁ to C₆ alkyl)carbonylamino, tetrazolyl(C₁ to C₆ alkyl)thio, [*N*-Z]tetrazolyl(C₁ to C₆ alkyl)amino, 5-oxo-2,5-dihydro[1,2,4]thiadiazolyl, 5-oxo-1,2-dihydro[1,2,4]triazolyl, [*N*-Z](C₁ to C₆ alkyl)amino(C₁ to C₆ alkyl)amino, or a group of the formula 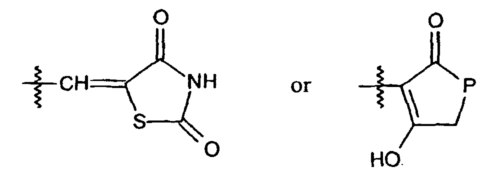
wherein P is O, S or NR¹⁹;
Z is H, C₁ to C₆ alkyl, t-butoxycarbonyl, acetyl, benzoyl or benzyl;
R⁴ is of formula
-(CH₂)_{q}-T-R¹⁰
wherein:
q is 0, 1, 2 or 3;
T is a bond, O, S, NH or N(C₁ to C₆ alkyl); and
R¹⁰ is C₁ to C₁₂ alkyl, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, C₃ to C₈ cycloalkyl, (C₃ to C₈ cycloalkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano);
R¹¹, R¹², R¹³, R¹⁴, R¹⁵, R¹⁷, R¹⁸ and R¹⁹ are independently H or C₁ to C₃ alkyl; and
R¹⁶ is H, C₁ to C₃ alkyl, or acetylamino;
or a pharmaceutically acceptable salt thereof;
with the proviso that R¹⁰ is not phenyl or substituted phenyl when q is 0 and T is a bond.

31. A compound of formula (IIa) or (IIb) according to claim 29 or 30 wherein W is N.

32. A compound of formula (IIa) or (IIb) according to any one of claims 29 to 31 wherein R¹ and R⁵ are both H.

33. A compound of formula (IIb) according to any of claims 30 to 32 wherein R² is:
-(CH₂)ₛ-C(R⁶R⁷)ₙ-(CH₂)ₜ-R⁸
wherein:
R⁶ and R⁷ are independently selected from H, C₁ to C₆ alkyl or OH; or R⁶ and R⁷ together represent an =O group;
n is 0 or 1;
s is 0, 1, 2 or 3;
t is 0, 1, 2 or 3; and
R⁸ is selected from H, C₁ to C₁₂ alkyl, (C₁ to C₁₂ alkyl)oxy, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano).

34. A compound of formula (IIb) according to claim 33 wherein s is 1, n is 1, and R⁶ and R⁷ together represent an =O group.

35. A compound of formula (IIb) according to claims 33 or 34 wherein t is 0 and R⁸ is a C₃ to C₁₂ cycloalkyl group or a branched C₃ to C₁₂ alkyl or group.

36. A compound of formula (IIa) according to claims 29, 31 or 32 wherein s is 1, t is 0 and R⁸ is a C₃ to C₁₂ cycloalkyl group or a branched C₃ to C₁₂ alkyl or group.

37. A compound of formula (IIa) or (IIb) according to claim 35 or 36 wherein R⁸ is a t-butyl or cyclopentyl group.

38. A compound of formula (IIa) or (IIb) according to any one of claims 29 to 37 wherein m is 1, R¹¹ is H and R¹² is H.

39. A compound of formula (IIa) or (IIb) according to any one of the claims 29 to 38 wherein p is 0.

40. A compound of formula (IIa) or (IIb) according to any one of claims 29 to 39 wherein X is C(O)NH.

41. A compound of formula (IIa) or (IIb) according to any one of claims 31 to 42 wherein R⁹ is phenyl substituted with a carboxy, carboxy(C₁ to C₆ alkyl), tetrazolyl, tetrazolyl-*N*-(C₁ to C₆ alkyl)amino, carboxy(C₁ to C₆ alkyl)thio, carboxy(C₁ to C₆ alkyl)sulfonyl, (C₁ to C₆ alkyl)amino, or 5-oxo-2,5-dihydro[1,2,4]oxadiazolyl group; or R⁹ is a *N*-[carboxy(C₁ to C₆ alkyl)]indolinyl or *N*-[carboxy(C₁ to C₆ alkyl)]indolyl group.

42. A compound of formula (IIa) or (IIb) according to claim 41 wherein the phenyl group is substituted at its 3-position.

43. A compound of formula (IIa) according to any one of claims 24, 31, 32 or 36-42 wherein R⁴ is
-(CH₂)_{q}-T-R¹⁰
wherein:
q is 0, 1, 2 or 3;
T is a bond, O, S, NH or N(C₁ to C₆ alkyl); and
R¹⁰ is C₁ to C₁₂ alkyl, C₃ to C₁₂ cycloalkyl, phenyl, naphthyl, pyridyl, pyrrolyl, imidazolyl, pyrazolyl, pyridazinyl, pyrimidinyl, triazolyl, furanyl, thienyl, furazanyl, oxazolyl, isoxazolyl, thiazolyl, thiazinyl, indolyl, indolinyl, isoindolyl, isoindolinyl, isoquinolinyl, quinolinyl, benzofuranyl, benzothienyl, piperazinyl, piperidinyl, pyrrolidinyl, pyrrolinyl, dihydropyranyl, tetrahydropyranyl, pyranyl, tetrahydrofuranyl, morpholinyl, thiazolidinyl, thiomorpholinyl or thioxanyl (all optionally substituted with 1, 2 or 3 groups independently selected from C₁ to C₆ alkyl, (C₁ to C₆ alkyl)oxy, C₃ to C₈ cycloalkyl, (C₃ to C₈ cycloalkyl)oxy, thio, (C₁ to C₆ alkyl)thio, carboxy, carboxy(C₁ to C₆ alkyl), formyl, (C₁ to C₆ alkyl)carbonyl, (C₁ to C₆ alkyl)oxycarbonyl, (C₁ to C₆ alkyl)carbonyloxy, nitro, trihalomethyl, hydroxy, hydroxy(C₁ to C₆ alkyl), amino, (C₁ to C₆ alkyl)amino, di(C₁ to C₆ alkyl)amino, aminocarbonyl, halo, halo(C₁ to C₆ alkyl), aminosulfonyl, (C₁ to C₆ alkyl)sulfonylamino or cyano).

44. A compound of formula (IIa) according to claim 43 wherein q is 0, T is a bond and R¹⁰ is C₁ to C₁₂ alkyl, C₃ to C₁₂ cycloalkyl, pyridyl or phenyl (all optionally substituted with OMe, NMe₂, CF₃, Me, F, Cl, Br or I).

45. A compound of formula (IIb) according to claims 30-34 or 37-42 wherein q is 0, T is a bond and R¹⁰ is C₁ to C₁₂ alkyl, C₃ to C₁₂ cycloalkyl or pyridyl (all optionally substituted with OMe, NMe₂, CF₃, Me, F, Cl, Br or I).

46. A compound of formula (IIa) or (IIb) according to claim 44 or 45 wherein R⁴ is C₃₋₁₂ cycloalkyl.

47. A compound of formula (IIa) or (IIb) according to any one of claims 44 to 46 wherein R⁴ is cyclohexyl.

48. A compound of formula (IIa) or (IIb) which is degraded *in vivo* to yield a compound according to any one of claims 29 to 47.

49. A pharmaceutical composition comprising a compound of formula (IIa) or (IIb) according to any of claims 29 to 48 together with a pharmaceutically acceptable diluent or carrier.

50. A compound of formula (IIa) or (IIb) according to any one of claims 29 to 48 or a composition according to claim 49 for use in medicine.

51. A method of making a pharmaceutical composition according to claim 49 comprising mixing a compound of formula (IIa) or (IIb) with a pharmaceutically acceptable diluent or carrier.

52. A method of making a compound according to formula (I), (IIa) or (IIb) comprising the steps of reacting a compound of formula (III) with NH₂NHR^{3'} and cyclising the resulting hydrazone compound with a bifunctional carbonyl reagent; 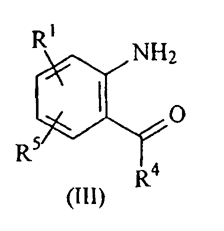 wherein R^{3'} is R³ or a suitable precursor thereof and R¹, R⁴ and R⁵ are as defined in claim 1.

## Patentansprüche

1. Verwendung einer Verbindung der Formel (I) 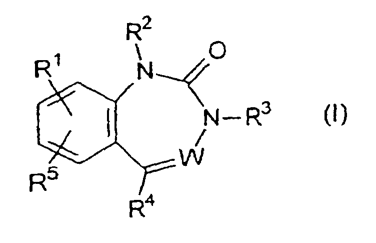 bei der
W gleich N oder N⁺-O⁻ ist;
R¹ und R⁵ sind unabhängig voneinander Wasserstoff, C₁-C₆-Alkyl (C₁-C₆-alkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino, (C₁-C₆-alkyl)Aminocarbonyl, di(C₁-C₆-alkyl)Aminocarbonyl, [*N-Z]* (C₁-C₆-alkyl)Carbonylamino, Formyloxy, Formamido, (C₁-C₆-alkyl)Aminosulfonyl, di(C₁-C₆-alkyl)Aminosulfonyl, [*N-Z]*(C₁-C₆-alkyl)Sulfonylamino oder Cyano; oder R¹ und R⁵ bilden zusammen eine Methylendioxygruppe;
R² ist Wasserstoff oder eine optional substituierte C₁-C₁₈-Kohlenwasserstoffgruppe, bei der bis zu drei Kohlenstoffatome optional durch N-, O- und/oder S-Atome ersetzt sein können,
R³ ist -(CR¹¹R¹²)ₘ-X-(CR¹³R¹⁴)ₚ-R⁹;
m ist 0, 1, 2, 3 oder 4;
p ist 0, 1 oder 2;
X ist eine Bindung, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 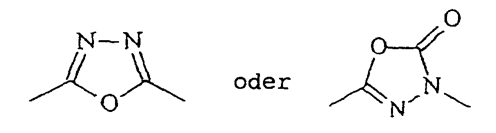 R⁹ ist Wasserstoff, C₁-C₆-Alkyl; oder Phenyl, Naphthyl, Pyridyl, Benzimidazolyl, Indazolyl, Chinolinyl, Isochinolinyl, Tetrahydroisochinolinyl, Indolinyl, Isoindolinyl, Indolyl, Isoindolyl oder 2-Pyridonyl, alle optional substituiert mit 1, 2 oder 3 Gruppen, die jeweils unabhängig voneinander ausgesucht sind aus -L-Q,
wobei
L eine Bindung ist, oder eine Gruppe der Formel -(CR¹⁷R¹⁸)ᵥ-Y-(CR¹⁷R¹⁸)_{w}, in der v und w unabhängig voneinander 0, 1, 2 oder 3 sind, und Y ist eine Bindung, -CR¹⁵=CR¹⁶, Phenyl, Furanyl, Thiophenyl, Pyrrolyl, Thiazolyl, Imidazolyl, Oxazolyl, Isoxazolyl, Pyrazolyl, Isoxazolonyl, Piperazinyl, Piperidinyl, Morpholinyl, Pyrrolidinyl, Isothiazolyl, Triazolyl, Oxadiazolyl, Thiadiazolyl, Pyridyl oder Pyridazyl; und
Q ist Wasserstoff, (C₁-C₆-alkyl)Oxy, [*N-Z]*(C₁-C₆alkyl)Oxy(C₁-C₆-alkyl)amino, Thio, (C₁-C₆-alkyl)Thio, Carboxy(C₁-C₆-alkyl)thio, Carboxy, Carboxy(C₁-C₆-alkyl), Carboxy(C₁-C₆alkenyl), [*N-Z]*Carboxy(C₁-C₆-alkyl)amino, Carboxy(C₁-C₆-alkyl)oxy, Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Amino, *[N-Z]*(C₁-C₆-alkyl)Amino, Aminocarbonyl, (C₁-C₆-alkyl)Aminocarbonyl, di(C₁-C₆-alkyl)Aminocarbonyl, [*N-Z]*(C₁-C₆-alkyl)Carbonylamino, C₅-C₈-Cycloalkyl, [*N*-*Z]* (C₁-C₆-alkyl)Carbonyl(C₁-C₆-alkyl)amino, Halo, Halo(C₁-C₆-alkyl), Sulfamoyl, *[N-Z]* (C₁-C₆-alkyl)Sulfonylamino, (C₁-C₆-alkyl)Sulfonylaminocarbonyl, Carboxy(C₁-C₆-alkyl)sulfonyl, Carboxy(C₁-C₆-alkyl)sulfinyl, Tetrazolyl, *[N*-*Z]*Tetrazolylamino, Cyano, Amidino, Amidinthio, SO₃H, Formyloxy, Formamido, C₃-C₈-Cycloalkyl, (C₁-C₆-alkyl)Sulfamoyl, di(C₁-C₆-alkyl)Sulfamayl, (C₁-C₆-alkyl)Carbonylaminosulfonyl, 5-Oxo-2,5-dihydro[1,2,4]oxadiazolyl, Carboxy(C₁-C₆-alkyl)carbonylamino, Tetrazolyl(C₁-C₆-alkyl)thio, *[N-Z]*Tetrazolyl(C₁-C₆-alkyl)amino, 5-Oxo-2,5-dihydro[1,2,4]thiadiazolyl, 5-Oxo-1,2-dihydro[1,2,4]triazolyl, *[N-Z]*(C₁-C₆-alkyl)Amino(C₁-C₆-alkyl)amino, oder eine Gruppe der Formel 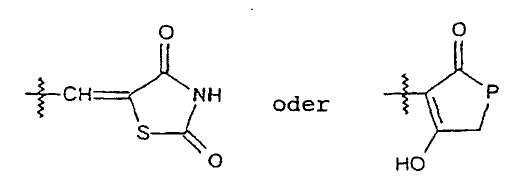 in der P gleich O, S oder NR¹⁹ ist;
Z ist Wasserstoff, C₁-C₆-Alkyl, t-Butoxycarbonyl, Acetyl, Benzoyl oder Benzyl;
R⁴ ist eine optional substituierte C₁-C₁₈-Kohlenwasserstoffgruppe, bei der bis zu drei Kohlenstoffatome optional durch N-, O- und/oder S-Atome ersetzt sein können;
R¹¹, R¹², R¹³, R¹⁴, R¹⁵, R¹⁷, R¹⁸ und R¹⁹ sind unabhängig voneinander Wasserstoff oder C₁-C₃-Alkyl; und
R¹⁶ ist Wasserstoff, C₁-C₃-Alkyl oder Acetylamino;
oder ein pharmazeutisch akzeptables Salz davon zur Herstellung eines Arzneimittels für die Behandlung von Krankheiten, die in Zusammenhang mit Gastrin stehen.

2. Verwendung nach Anspruch 1, bei der W gleich N ist.

3. Verwendung nach Anspruch 1 oder 2, bei der R¹ und R⁵ beide Wasserstoff sind.

4. Verwendung nach einem der vorhergehenden Ansprüche, bei der R² Folgendes ist:
-(CH₂)ₛ-C(R⁶R⁷)ₙ-(CH₂)ₜ-R⁸
in der:
R⁶ und R⁷ unabhängig voneinander ausgewählt sind aus Wasserstoff, C₁-C₆-Alkyl oder OH; oder R⁶ und R⁷ zusammen eine =O-Gruppe darstellen;
n ist 0 oder 1;
s ist 0, 1, 2 oder 3;
t ist 0, 1, 2 oder 3; und
R⁸ ist ausgewählt aus Wasserstoff, C₁-C₁₂-Alkyl, (C₁-C₁₂alkyl)Oxy, C₁-C₁₂-Cycloalkyl, Phenyl, Naphthyl, Pyridyl, Pyrrolyl, Imidazolyl, Pyrazolyl, Pyridazinyl, Pyrimidinyl, Triazolyl, Furanyl, Thienyl, Furazanyl, Oxazolyl, Isoxazolyl, Thiazolyl, Thiazinyl, Indolyl, Indolinyl, Isoindolyl, Isoindolinyl, Isochinolinyl, Chinolinyl, Benzofuranyl, Benzothienyl, Piperazinyl, Piperidinyl, Pyrrolidinyl, Pyrrolinyl, Dihydropyranyl, Tetrahydropyranyl, Pyranyl, Tetrahydrofuranyl, Morpholinyl, Thiazolidinyl, Thiomorpholinyl oder Thioxanyl (alle optional substituiert mit 1, 2 oder 3 Gruppen, die jeweils unabhängig voneinander ausgesucht sind aus C₁-C₆-Alkyl, (C₁-C₆-alkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonlyoxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino oder Cyano).

5. Verwendung nach Anspruch 4, bei der s gleich 1 ist, n gleich 1 ist, und R⁶ und R⁷ zusammen eine =O-Gruppe darstellen.

6. Verwendung nach Ansprüchen 4 oder 5, bei der t gleich 0 ist, und R⁸ eine C₃-C₁₂-Cycloalkylgruppe ist (optional substituiert mit einer Methylgruppe) oder eine verzweigte C₃-C₁₂-Alkylgruppe ist.

7. Verwendung nach einem der Ansprüche 4 bis 6, bei der R⁸ eine t-Butyl, Cyclohexyl, 1-Methylcyclohexyl, 1-Methylcyclopentyl oder Cyclopentylgruppe ist.

8. Verwendung nach einem der vorhergehenden Ansprüche, bei der m gleich 1 ist, R¹¹ gleich Wasserstoff ist und R¹² gleich Wasserstoff ist.

9. Verwendung nach einem der vorhergehenden Ansprüche, bei der p gleich 0 ist.

10. Verwendung nach einem der vorhergehenden Ansprüche, bei der X gleich C(O)NH ist.

11. Verwendung nach einem der vorhergehenden Ansprüche, bei der R⁹ Phenyl ist, substituiert mit einer der folgenden Gruppen, nämlich Carboxy, Carboxy(C₁-C₆-alkyl), Tetrazolyl, Tetrazolyl-N-(C₁-C₆-alkyl)amino, Carboxy(C₁-C₆-alkyl)thio, Carboxy(C₁-C₆-alkyl)sulfonyl, (C₁-C₆-alkyl)Amino, oder 5-Oxo-2,5-dihydro[1,2,4]oxadiazolyl; or R⁹ ist eine *N*-[Carboxy(C₁-C₆-alkyl)]indolinyl oder *N*-[Carboxy(C₁-C₆-alkyl)]indolyl-Gruppe.

12. Verwendung nach Anspruch 11, bei der die Phenylgruppe in deren 3-Stellung substituiert ist.

13. Verwendung nach einem der vorhergehenden Ansprüche, bei der R⁴ Folgendes ist, nämlich
-(CH₂)_{q}-T-R¹⁰
in der:
q gleich 0, 1, 2 oder 3 ist;
T ist eine Bindung, O, S, NH oder N(C₁-C₆-Alkyl); und
R¹⁰ ist C₁-C₁₂-Alkyl, C₃-C₁₂-Cycloalkyl, Phenyl, Naphthyl, Pyridyl, Pyrrolyl, Imidazolyl, Pyrazolyl, Pyridazinyl, Pyrimidinyl, Triazolyl, Furanyl, Thienyl, Furazanyl, Oxazolyl, Isoxazolyl, Thiazolyl, Thiazinyl, Indolyl, Indolinyl, Isoindolyl, Isoindolinyl, Isochinolinyl, Chinolinyl, Benzofuranyl, Benzothienyl, Piperazinyl, Piperidinyl, Pyrrolidinyl, Pyrrolinyl, Dihydropyranyl, Tetrahydropyranyl, Pyranyl, Tetrahydrofuranyl, Morpholinyl, Thiazolidinyl, Thiomorpholinyl oder Thioxanyl (alle optional substituiert mit 1, 2 oder 3 Gruppen, die jeweils unabhängig voneinander ausgesucht sind aus C₁-C₆-Alkyl, (C₁-C₆-alkyl)Oxy, C₃-C₈-Cycloalkyl, (C₃-C₈-cycloalkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino oder Cyano).

14. Verwendung nach Anspruch 13, bei der q gleich 0 ist, T eine Bindung ist, und R¹⁰ ist C₁-C₆-Alkyl, C₃-C₁₂-Cycloalkyl, Pyridyl oder Phenyl (alle optional substituiert mit OMe, NMe₂, CF₃, Me, F, Cl, Br oder J).

15. Verwendung nach einem der vorhergehenden Ansprüche, bei der R⁴ Cyclohexyl ist.

16. Pharmazeutische Zusammensetzung, mit einem Protonenpumpenhemmer und einer der Verbindungen der Formel (I), wie in einem der Ansprüche 1 bis 15 definiert, zusammen mit einem pharmazeutisch akzeptablen Verdünnungsmittel oder Träger.

17. Zusammensetzung nach Anspruch 16, bei der der Protonenpumpenhemmer ausgewählt ist aus (RS)-Rabeprazol, (RS)-Omeprazol, Lansoprazol, Pantoprazol, (R)-Omeprazol, (S)-Omeprazol, Perprazol, (R)-Rabeprazol, (S)-Rabeprazol, oder Alkalisalze davon.

18. Zusammensetzung nach Anspruch 16 oder 17, bei der der Protonenpumpenhemmer und die Verbindung der Formel (I) jeweils in einer Menge vorhanden sind, die eine therapeutisch nützliche Wirkung bei Patienten zeigt, die an gastrointestinalen Erkrankungen leiden.

19. Zusammensetzung nach Anspruch 18, bei der der therapeutisch nützliche Effekt ein synergistischer Effekt bezüglich der Reduktion von Säuresekretion bei Patienten, die an gastrointestinalen Erkrankungen leiden, ist, oder die Prävention von gastrointestinalen Erkrankungen bei solchen Patienten ist, oder die Reduktion von Nebeneffekten, die in Zusammenhang mit dem einen der aktiven Wirkstoffe auf Grund des anderen der aktiven Wirkstoffe stehen.

20. Zusammensetzung nach einem der Ansprüche 16 bis 19, bei der die Menge jedes der aktiven Wirkstoffe gleich oder geringer ist als diejenige Menge, die bei der Monotherapie mit diesen aktiven Wirkstoffen zugelassen oder angezeigt ist.

21. Kit, enthaltend als einen ersten aktiven Wirkstoff eine Verbindung der Formel (I), wie sie in einem der Ansprüche 1 bis 15 definiert, und als einen zweiten wirksamen Wirkstoff einen Protonenpumpenhemmer als Kombinationspräparat für simultane, getrennte oder schrittweise Anwendung in der Behandlung von Patienten, die an gastrointestinalen Erkrankungen leiden.

22. Verwendung einer Zusammensetzung nach einem der Ansprüche 16 bis 20 oder einem Kit nach Anspruch 23 zum Herstellen eines Arzneimittels zur Behandlung von gastrointestinalen Erkrankungen.

23. Verwendung eines Protonenpumpenhemmers zum Herstellen eines Arzneimittels zur Behandlung von gastrointestinalen Erkrankungen, wobei die Behandlung die simultane oder schrittweise Verabreichung des Protonenpumpenhemmers und einer Verbindung der Formel (I), wie in einem der Ansprüche 1 bis 15 beschrieben, aufweist, wobei der Protonenpumpenhemmer die Wirkung der Verbindung nach Formel (I) bei Erkrankungen, die mit Gastrin in Zusammenhang stehen, bei Patienten, die an gastrointestinalen Erkrankungen leiden, verstärkt.

24. Verwendung einer Verbindung der Formel (I), wie in einem der Ansprüche 1 bis 15 definiert, zur Herstellung eines Arzneimittels zur Behandlung von gastrointestinalen Erkrankungen, wobei die Behandlung die simultane oder schrittweise Verabreichung des Protonenpumpenhemmers und einer Verbindung der Formel (I) beinhaltet, wobei die Verbindung der Formel (I) die Wirkung des Protonenpumpenhemmers bezüglich der Reduktion der Säuresekretion bei Patienten, die an gastrointestinalen Erkrankungen leiden, erhöht.

25. Verwendung einer Verbindung der Formel (I), wie in einem der Ansprüche 1 bis 15 definiert, zur Herstellung eines Arzneimittels zur Reduzierung von Nebenwirkungen, die in Zusammenhang mit der Verabreichung von Protonenpumpenhemmern bei Patienten, die an gastrointestinalen Erkrankungen leiden, stehen.

26. Verwendung nach Anspruch 25, bei der die Nebenwirkung Hyperplasie ist.

27. Verwendung eines Protonenpumpenhemmers zur Herstellung eines Arzneimittels zur Reduzierung von Nebeneffekten, die mit der Verabreichung einer Verbindung der Formel (I), wie sie in einem der Ansprüche 1 bis 15 definiert ist, in Zusammenhang stehen, und zwar bei Patienten, die an gastrointestinalen Erkrankungen leiden.

28. Verfahren zum Herstellen einer pharmazeutischen Zusammensetzung nach einem der Ansprüche 18 bis 22, das Folgendes aufweist, Vermischen einer Verbindung der Formel (I), wie sie in einem der Ansprüche 1 bis 15 definiert ist, und eines Protonenpumpenhemmers mit einem pharmazeutisch akzeptablen Verdünnungsmittel oder Träger.

29. Verbindung der Formel (IIa) 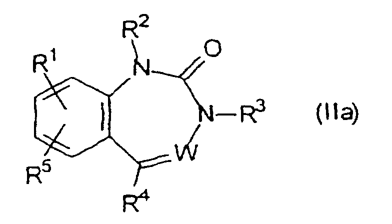 bei der
W gleich N oder N⁺-O⁻ ist;
R¹ und R⁵ sind unabhängig voneinander Wasserstoff, C₁-C₆-Alkyl (C₁-C₆-alkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino, (C₁-C₆-alkyl)Aminocarbonyl, di(C₁-C₆-alkyl)Aminocarbonyl, [*N-Z]*(C₁-C₆-alkyl)Carbonylamino, Formyloxy, Formamido, (C₁-C₆-alkyl)Aminosulfonyl, di(C₁-C₆-alkyl)Aminosulfonyl, [*N-Z]* (C₁-C₆-alkyl)Sulfonylamino oder Cyano; oder R¹ und R⁵ bilden zusammen eine Methylendioxygruppe;
R² ist -(CH₂)ₛ-C(O)-(CH₂)ₜ-R⁸;
s ist 0, 1, 2 oder 3;
t ist 0, 1, 2 oder 3; und
R⁸ ist ausgewählt aus Wasserstoff, C₁-C₁₂-Alkyl, (C₁-C₁₂-alkyl)Oxy, C₃-C₁₂-Cycloalkyl, Phenyl, Naphthyl, Pyridyl, Pyrrolyl, Imidazolyl, Pyrazolyl, Pyridazinyl, Pyrimidinyl, Triazolyl, Furanyl, Thienyl, Furazanyl, Oxazolyl, Isoxazolyl, Thiazolyl, Thiazinyl, Indolyl, Indolinyl, Isoindolyl, Isoindolinyl, Isochinolinyl, Chinolinyl, Benzofuranyl, Benzothienyl, Piperazinyl, Piperidinyl, Pyrrolidinyl, Pyrrolinyl, Dihydropyranyl, Tetrahydropyranyl, Pyranyl, Tetrahydrofuranyl, Morpholinyl, Thiazolidinyl, Thiomorpholinyl oder Thioxanyl (alle optional substituiert mit 1, 2 oder 3 Gruppen, die jeweils unabhängig voneinander ausgewählt sind aus C₁-C₆-Alkyl, (C₁-C₆-alkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino oder Cyano);
R³ ist -(CR¹¹R¹²)ₘ-X-(CR¹³R¹⁴)ₚ-R⁹;
m ist 0, 1, 2, 3 oder 4;
p ist 0, 1 oder 2;
X ist eine Bindung, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 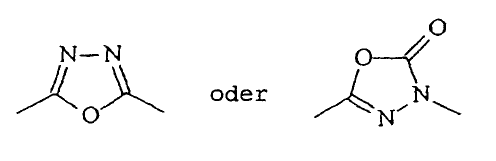 R⁹ ist Wasserstoff, C₁-C₆-Alkyl; oder Phenyl, Naphthyl, Pyridyl, Benzimidazolyl, Indazolyl, Chinolinyl, Isochinolinyl, Tetrahydroisochinolinyl, Indolinyl, Isoindolinyl, Indolyl, Isoindolyl oder 2-Pyridonyl, alle optional substituiert mit 1, 2 oder 3 Gruppen, die jeweils unabhängig voneinander ausgesucht sind aus -L-Q,
wobei
L eine Bindung ist, oder eine Gruppe der Formel -(CR¹⁷R¹⁸)ᵥ-Y-(CR¹⁷R¹⁸)_{w}, in der v und w unabhängig voneinander 0, 1, 2 oder 3 sind, und Y ist eine Bindung, -CR¹⁵=CR¹⁶, Phenyl, Furanyl, Thiophenyl, Pyrrolyl, Thiazolyl, Imidazolyl, Oxazolyl, Isoxazolyl, Pyrazolyl, Isoxazolonyl, Piperazinyl, Piperidinyl, Morpholinyl, Pyrrolidinyl, Isothiazolyl, Triazolyl, Oxadiazolyl, Thiadiazolyl, Pyridyl oder Pyridazyl; und
Q ist Wasserstoff, (C₁-C₆-alkyl)Oxy, [*N-Z]* (C₁-C₆-alkyl)Oxy(C₁-C₆-alkyl)amino, Thio, (C₁-C₆-alkyl)Thio, Carboxy(C₁-C₆-alkyl)thio, Carboxy, Carboxy(C₁-C₆-alkyl), Carboxy(C₁-C₆-alkenyl), [*N-Z]*Carboxy(C₁-C₆-alkyl)amino, Carboxy(C₁-C₆-alkyl)oxy, Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Amino, *[N-Z]*(C₁-C₆-alkyl)Amino, Aminocarbonyl, (C₁-C₆-alkyl)Aminocarbonyl, di(C₁-C₆-alkyl)Aminocarbonyl, [*N-Z]* (C₁-C₆-alkyl)Carbonylamino, C₅-C₈-Cycloalkyl, [*N-Z]* (C₁-C₆-alkyl)Carbonyl(C₁-C₆-alkyl)amino, Halo, Halo(C₁-C₆-alkyl), Sulfamoyl, *[N-Z]* (C₁-C₆-alkyl)Sulfonylamino, (C₁-C₆-alkyl)Sulfonylaminocarbonyl, Carboxy(C₁-C₆-alkyl)sulfonyl, Carboxy(C₁-C₆-alkyl)sulfinyl, Tetrazolyl, *[N-Z]*Tetrazolylamino, Cyano, Amidino, Amidinthio, SO₃H, Formyloxy, Formamido, C₃-C₈-Cycloalkyl, (C₁-C₆-alkyl)Sulfamoyl, di(C₁-C₆-alkyl)Sulfamoyl, (C₁-C₆-alkyl)Carbonylaminosulfonyl, 5-Oxo-2,5-dihydro[1,2,4]oxadiazolyl, Carboxy(C₁-C₆-alkyl)carbonylamino, Tetrazolyl(C₁-C₆-alkyl)thio, *[N-Z]*Tetrazolyl(C₁-C₆-alkyl)amino, 5-Oxo-2,5-dihydro[1,2,4]thiadiazolyl, 5-Oxo-1,2-dihydro[1,2,4]triazolyl, *[N-Z]*(C₁-C₆-alkyl)Amino(C₁-C₆-alkyl)amino, oder eine Gruppe der Formel 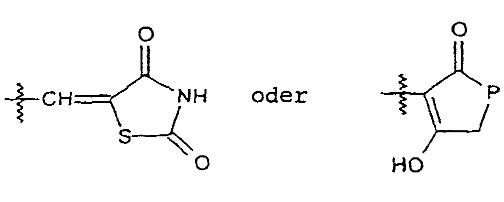 in der P gleich O, S oder NR¹⁹ ist;
Z ist Wasserstoff, C₁-C₆-Alkyl, t-Butoxycarbonyl, Acetyl, Benzoyl oder Benzyl;
R⁴ ist eine optional substituierte C₁-C₁₈-Kohlenwasserstoffgruppe, bei der bis zu drei Kohlenstoffatome optional durch N-, O- und/oder S-Atome ersetzt sein können;
R¹¹, R¹², R¹³, R¹⁴, R¹⁵, R¹⁷, R¹⁸ und R¹⁹ sind unabhängig voneinander Wasserstoff oder C₁-C₃-Alkyl; und
R¹⁶ ist Wasserstoff, C₁-C₃-Alkyl oder Acetylamino;
oder ein pharmazeutisch akzeptables Salz davon;
mit der Bedingung, dass R² nicht CH₂CO₂H oder C(O)CH₃ ist, falls R⁴ Phenyl ist.

30. Verbindung der Formel (IIb) 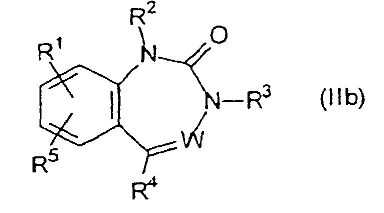 bei der
W gleich N oder N⁺-O⁻ ist;
R¹ und R⁵ sind unabhängig voneinander Wasserstoff, C₁-C₆-Alkyl (C₁-C₆-alkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino, (C₁-C₆-alkyl)Aminocarbonyl, di(C₁-C₆-alkyl)Aminocarbonyl, [*N*-*Z]* (C₁-C₆-alkyl)Carbonylamino, Formyloxy, Formamido, (C₁-C₆-alkyl)Aminosulfonyl, di(C₁-C₆-alkyl)Aminosulfonyl, [*N-Z*](C₁-C₆-alkyl)Sulfonylamino oder Cyano; oder R¹ und R⁵ bilden zusammen eine Methylendioxygruppe;
R² ist eine optional substituierte C₁-C₁₈-Kohlenwasserstoffgruppe, bei der bis zu drei Kohlenstoffatome optional durch N-, O- und/oder S-Atome ersetzt sein können;
R³ ist -(CR¹¹R¹²)ₘ-X-(CR¹³R¹⁴)ₚ-R⁹;
m ist 0, 1, 2, 3 oder 4;
p ist 0, 1 oder 2;
X ist eine Bindung, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 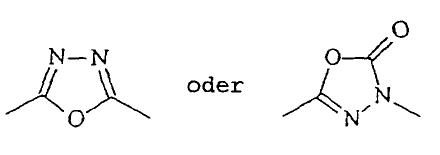 R⁹ ist Wasserstoff, C₁-C₆-Alkyl; oder Phenyl, Naphthyl, Pyridyl, Benzimidazolyl, Indazolyl, Chinolinyl, Isochinolinyl, Tetrahydroisochinolinyl, Indolinyl, Isoindolinyl, Indolyl, Isoindolyl oder 2-Pyridonyl, alle optional substituiert mit 1, 2 oder 3 Gruppen, die jeweils unabhängig voneinander ausgewählt sind aus -L-Q,
wobei
L eine Bindung ist, oder eine Gruppe der Formel -(CR¹⁷R¹⁸)ᵥ-Y-(CR¹⁷R¹⁸)_{w}, in der v und w unabhängig voneinander 0, 1, 2 oder 3 sind, und Y ist eine Bindung, -CR¹⁵=CR¹⁶, Phenyl, Furanyl, Thiophenyl, Pyrrolyl, Thiazolyl, Imidazolyl, Oxazolyl, Isoxazolyl, Pyrazolyl, Isoxazolonyl, Piperazinyl, Piperidinyl, Morpholinyl, Pyrrolidinyl, Isothiazolyl, Triazolyl, Oxadiazolyl, Thiadiazolyl, Pyridyl oder Pyridazyl; und
Q ist Wasserstoff, (C₁-C₆-alkyl)Oxy, [*N-Z]*(C₁-C₆-alkyl)Oxy(C₁-C₆-alkyl)amino, Thio, (C₁-C₆-alkyl)Thio, Carboxy(C₁-C₆-alkyl)thio, Carboxy, Carboxy(C₁-C₆-alkyl), Carboxy(C₁-C₆-alkenyl), [*N-Z]*Carboxy(C₁-C₆-alkyl)amino, Carboxy(C₁-C₆-alkyl)oxy, Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Amino, *[N-Z]*(C₁-C₆-alkyl)Amino, Aminocarbonyl, (C₁-C₆-alkyl)Aminocarbonyl, di(C₁-C₆-alkyl)Aminocarbonyl, [*N-Z]*(C₁-C₆-alkyl)Carbonylamino, C₅-C₈-Cycloalkyl, [*N-Z]*(C₁-C₆-alkyl)Carbonyl(C₁-C₆-alkyl)amino, Halo, Halo(C₁-C₆-alkyl), Sulfamoyl, *[N-Z]*(C₁-C₆-alkyl)Sulfonylamino, (C₁-C₆-alkyl)Sulfonylaminocarbonyl, Carboxy(C₁-C₆-alkyl)sulfonyl, Carboxy (C₁-C₆-alkyl)sulfinyl, Tetrazolyl, *[N-Z]*Tetrazolylamino, Cyano, Amidino, Amidinthio, SO₃H, Formyloxy, Formamido, C₃-C₈-Cycloalkyl, (C₁-C₆-alkyl)Sulfamoyl, di(C₁-C₆-alkyl)Sulfamoyl, (C₁-C₆-alkyl)Carbonylaminosulfonyl, 5-Oxo-2,5-dihydro[1,2,4]oxadiazolyl, Carboxy(C₁-C₆-alkyl)carbonylamino, Tetrazolyl(C₁-C₆-alkyl)thio, *[N-Z]*Tetrazolyl(C₁-C₆-alkyl)amino, 5-Oxo-2,5-dihydro[1,2,4]thiadiazolyl, 5-Oxo-1,2-dihydro[1,2,4]triazolyl, *[N-Z]*(C₁-C₆-alkyl)Amino(C₁-C₆-alkyl)amino, oder eine Gruppe der Formel 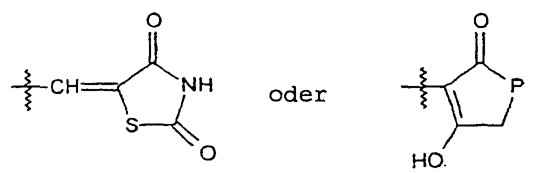 in der P gleich 0, S oder NR¹⁹ ist;
Z ist Wasserstoff, C₁-C₆-Alkyl, t-Butoxycarbonyl, Acetyl, Benzoyl oder Benzyl;
R⁴ ist die Formel
-(CH₂)_{q}-T-R¹⁰
in der
q gleich 0, 1, 2 oder 3 ist;
T ist eine Bindung, 0, S, NH oder N(C₁-C₆-Alkyl); und
R¹⁰ ist C₁-C₁₂-Alkyl, C₃-C₁₂-Cycloalkyl, Phenyl, Naphthyl, Pyridyl, Pyrrolyl, Imidazolyl, Pyrazolyl, Pyridazinyl, Pyrimidinyl, Triazolyl, Furanyl, Thienyl, Furazanyl, Oxazolyl, Isoxazolyl, Thiazolyl, Thiazinyl, Indolyl, Indolinyl, Isoindolyl, Isoindolinyl, Isochinolinyl, Chinolinyl, Benzofuranyl, Benzothienyl, Piperazinyl, Piperidinyl, Pyrrolidinyl, Pyrrolinyl, Dihydropyranyl, Tetrahydropyranyl, Pyranyl, Tetrahydrofuranyl, Morpholinyl, Thiazolidinyl, Thiomorpholinyl oder Thioxanyl (alle optional substituiert mit 1, 2 oder 3 Gruppen, die jeweils unabhängig voneinander auswählt sind aus C₁-C₆-Alkyl, (C₁-C₆-alkyl)Oxy, C₃-C₈-Cycloalkyl, (C₃-C₈-cycloalkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino oder Cyano);
R¹¹, R¹², R¹³, R¹⁴, R¹⁵, R¹⁷, R¹⁸ und R¹⁹ sind unabhängig voneinander Wasserstoff oder C₁-C₃-Alkyl; und
R¹⁶ ist Wasserstoff, C₁-C₃-Alkyl oder Acetylamino;
oder ein pharmazeutisch akzeptables Salz davon;
mit der Bedingung, dass R¹⁰ nicht Phenyl oder substituiertes Phenyl ist, wenn q gleich 0 ist und T eine Bindung ist.

31. Verbindung der Formel (IIa) oder (IIb) nach Anspruch 29 oder 30, bei der W gleich N ist.

32. Verbindung der Formel (IIa) oder (IIb) nach einem der Ansprüche 29 oder 31, bei der R¹ und R⁵ jeweils H sind.

33. Verbindung der Formel (IIa) oder (IIb) nach einem der Ansprüche 30 oder 32, bei der R² Folgendes ist:
-(CH₂)ₛ-C(R⁶R⁷)ₙ-(CH₂)ₜ-R⁸
in der:
R⁶ und R⁷ unabhängig voneinander ausgewählt sind aus Wasserstoff, C₁-C₆-Alkyl oder OH; oder R⁶ und R⁷ zusammen eine =O-Gruppe darstellen;
n ist 0 oder 1;
s ist 0, 1, 2 oder 3;
t ist 0, 1, 2 oder 3; und
R⁸ ist ausgewählt aus Wasserstoff, C₁-C₁₂-Alkyl, (C₁-C₁₂-alkyl)Oxy, C₁-C₁₂-Cycloalkyl, Phenyl, Naphthyl, Pyridyl, Pyrrolyl, Imidazolyl, Pyrazolyl, Pyridazinyl, Pyrimidinyl, Triazolyl, Furanyl, Thienyl, Furazanyl, Oxazolyl, Isoxazolyl, Thiazolyl, Thiazinyl, Indolyl, Indolinyl, Isoindolyl, Isoindolinyl, Isochinolinyl, Chinolinyl, Benzofuranyl, Benzothienyl, Piperazinyl, Piperidinyl, Pyrrolidinyl, Pyrrolinyl, Dihydropyranyl, Tetrahydropyranyl, Pyranyl, Tetrahydrofuranyl, Morpholinyl, Thiazolidinyl, Thiomorpholinyl oder Thioxanyl (alle optional substituiert mit 1, 2 oder 3 Gruppen, die jeweils unabhängig voneinander ausgewählt sind aus C₁-C₆-Alkyl, (C₁-C₆-alkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino oder Cyano).

34. Verbindung der Formel (IIb) nach Anspruch 33, bei der s gleich 1 ist, n gleich 1 ist, und R⁶ und R⁷ zusammen eine =O-Gruppe darstellen.

35. Verbindung der Formel (IIb) nach Anspruch 33 oder 34, bei der t gleich 0 ist, R⁸ eine C₃-C₁₂-Cycloalkylgruppe oder eine verzweigte C₃-C₁₂-Alkylgruppe ist.

36. Verbindung der Formel (IIa) nach den Ansprüchen 29, 31 oder 32, bei der s gleich 1 ist, t gleich 0 ist, und R⁸ eine C₃-C₁₂-Cycloalkylgruppe oder eine verzweigte C₃-C₁₂-Alkylgruppe ist.

37. Verbindung der Formel (IIa) oder (IIb) nach Anspruch 35 oder 36, bei der R⁸ eine t-Butyl- oder eine Cyclopentylgruppe ist.

38. Verbindung der Formel (IIa) oder (IIb) nach einem der Ansprüche 29 bis 37, bei der m gleich 1, R¹¹ gleich Wasserstoff und R¹² gleich Wasserstoff ist.

39. Verbindung der Formel (IIa) oder (IIb) nach einem der Ansprüche 29 bis 38, bei der p gleich 0 ist.

40. Verbindung der Formel (IIa) oder (IIb) nach einem der Ansprüche 29 bis 39, in der X gleich C(O)NH ist.

41. Verbindung der Formel (IIa) oder (IIb) nach einem der Ansprüche 31 bis 42, bei der R⁹ Phenyl ist, substituiert mit einer Carboxy-, Carboxy(C₁-C₆-alkyl)-, Tetrazolyl-, Tetrazolyl-*N*-(C₁-C₆-alkyl)amino-, Carboxy(C₁-C₆-alkyl)thio-, Carboxy(C₁-C₆-alkyl)sulfonyl-, (C₁-C₆-alkyl)Amino- oder 5-Oxo-2,5-dihydroxy[1,2,4]oxadiazolyl-Gruppe ist; oder R⁹ ist eine *N*-[Carboxy(C₁-C₆-alkyl)]indolinyl- oder *N*-[Carboxy(C₁-C₆-alkyl)]indolyl-Gruppe.

42. Verbindung der Formel (IIa) oder (IIb) nach Anspruch 41, bei der die Phenylgruppe an ihrer 3-Position substituiert ist.

43. Verbindung der Formel (IIa) nach einem der Ansprüche 24, 31, 32, oder 36 bis 42, in der R⁴ Folgendes ist
-(CH₂)_{q}-T-R¹⁰
in der
q gleich 0, 1, 2 oder 3 ist;
T eine Bindung ist, O, S, NH oder N(C₁-C₆-Alkyl); und
R¹⁰ ist C₁-C₁₂-Alkyl, C₃-C₁₂-Cycloalkyl, Phenyl, Naphthyl, Pyridyl, Pyrrolyl, Imidazolyl, Pyrazolyl, Pyridazinyl, Pyrimidinyl, Triazolyl, Furanyl, Thienyl, Furazanyl, Oxazolyl, Isoxazolyl, Thiazolyl, Thiazinyl, Indolyl, Indolinyl, Isoindolyl, Isoindolinyl, Isochinolinyl, Chinolinyl, Benzofuranyl, Benzothienyl, Piperazinyl, Piperidinyl, Pyrrolidinyl, Pyrrolinyl, Dihydropyranyl, Tetrahydropyranyl, Pyranyl, Tetrahydrofuranyl, Morpholinyl, Thiazolidinyl, Thiomorpholinyl oder Thioxanyl (alle optional substituiert mit 1, 2 oder 3 Gruppen, die jeweils unabhängig voneinander auswählt sind aus C₁-C₆-Alkyl, (C₁-C₆-alkyl)Oxy, C₃-C₈-Cycloalkyl, (C₃-C₈-cycloalkyl)Oxy, Thio, (C₁-C₆-alkyl)Thio, Carboxy, Carboxy(C₁-C₆-alkyl), Formyl, (C₁-C₆-alkyl)Carbonyl, (C₁-C₆-alkyl)Oxycarbonyl, (C₁-C₆-alkyl)Carbonyloxy, Nitro, Trihalomethyl, Hydroxy, Hydroxy(C₁-C₆-alkyl), Amino, (C₁-C₆-alkyl)Amino, di(C₁-C₆-alkyl)Amino, Aminocarbonyl, Halo, Halo(C₁-C₆-alkyl), Aminosulfonyl, (C₁-C₆-alkyl)Sulfonylamino oder Cyano).

44. Verbindung der Formel (IIa) nach Anspruch 43, in der q gleich 0 ist, T eine Bindung ist und R¹⁰ C₁-C₆-Alkyl, C₃-C₁₂-Cycloalkyl, Pyridyl oder Phenyl ist, alle optional substituiert mit OMe, NMe₂, CF₃, Me, F, Cl, Br oder J.

45. Verbindung der Formel (IIb) nach einem der Ansprüche 30 bis 35 oder 37 bis 42, in der q gleich 0 ist, T eine Bindung ist und R¹⁰ C₁-C₆-Alkyl, C₃-C₁₂-Cycloalkyl, Pyridyl oder Phenyl ist, alle optional substituiert mit OMe, NMe₂, CF₃, Me, F, Cl, Br oder J.

46. Verbindung der Formel (IIa) oder (IIb) nach Anspruch 44 oder 45, bei der R⁴ gleich C₃₋₁₂-Cycloalkyl ist.

47. Verbindung der Formel (IIa) oder (IIb) nach einem der Ansprüche 44 bis 46, in der R⁴ gleich Cyclohexyl ist.

48. Verbindung der Formel (IIa) oder (IIb), die *in vivo* abgebaut wird, um eine Verbindung nach einem der Ansprüche 29 bis 47 zu ergeben.

49. Pharmazeutische Zusammensetzung, die eine Verbindung der Formel (IIa) oder (IIb) nach einem der Ansprüche 29 bis 48 zusammen mit einem pharmazeutisch akzeptablen Verdünnungsmittel oder einem Träger aufweist.

50. Verbindung nach Formel (IIa) oder (IIb) nach einem der Ansprüche 29 bis 48 oder eine Zusammensetzung nach Anspruch 49 zur medizinischen Verwendung.

51. Verfahren zum Herstellen einer pharmazeutischen Zusammensetzung nach Anspruch 49, das Folgendes aufweist, nämlich Vermischen einer Verbindung der Formel (IIa) oder (IIb) mit einem pharmazeutisch verträglichen Verdünnungsmittel oder Träger.

52. Verfahren zum Herstellen einer Verbindung nach Formel (I), (IIa) oder (IIb) mit folgenden Schritten, nämlich Reagierenlassen einer Verbindung der Formel (III) mit NH₂NHR^{3'} und Cyclisieren der resultierenden Hydrazonverbindung mit einem bifunktionellen Carbonylreagens; 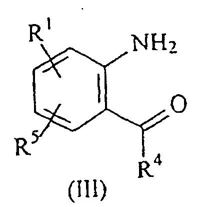 in der R^{3'} gleich R³ ist oder ein geeigneter Precursor davon, und R¹, R⁴ und R⁵ so wie in Anspruch 1 definiert sind.

## Revendications

1. Utilisation d'un composé de formule (I) : 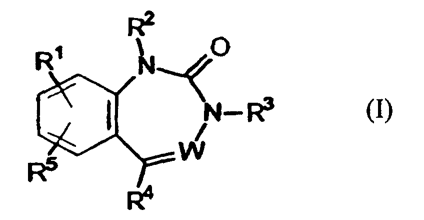 dans laquelle :
W représente N ou N⁺-O⁻ ;
R¹ et R⁵ représentent indépendamment H, un groupe alkyle en C₁ à C₆, (alkyle en C₁ à C₆)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, hydroxy(alkyle en C₁ à C₆), amino, (alkyle en C₁ à C₆)-amino, di(alkyle en C₁ à C₆)amino, aminocarbonyle, halo, halo(alkyle en C₁ à C₆), aminosulfonyle, (alkyle en C₁ à C₆)sulfonylamino, (alkyle en C₁ à C₆)aminocarbonyle, di(alkyle en C₁ à C₆)aminocarbonyle, [N-Z](alkyle en C₁ à C₆)carbonylamino, formyloxy, formamido, (alkyle en C₁ à C₆)-aminosulfonyle, di(alkyle en C₁ à C₆)aminosulfonyle, [N-Z](alkyle en C₁ à C₆)sulfonylamino ou cyano ; ou bien R¹ et R⁵ forment conjointement un groupe méthylènedioxy ;
R² représente H ou un groupe hydrocarbyle en C₁ à C₁₈ facultativement substitué dans lequel jusqu'à trois atomes de C peuvent facultativement être remplacés par des atomes de N, O et/ou S.
R³ représente -(CR¹¹R¹²)ₘ-X-(CR¹³R¹⁴)ₚ-R⁹ ;
m vaut 0, 1, 2, 3 ou 4 ;
p vaut 0, 1 ou 2 ;
X représente une liaison, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 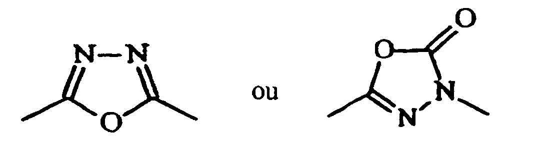
R⁹ représente H ; un groupe alkyle en C₁ à C₆ ; ou phényle, naphtyle, pyridyle, benzimidazolyle, indazolyle, quinolinyle, isoquinolinyle, tétrahydroisoquinolinyle, indolinyle, isoindolinyle, indolyle, isoindolyle ou 2-pyridonyle, tous étant facultativement substitués par 1, 2 ou 3 groupes choisis indépendamment à partir de -L-Q ;
où :
L est une liaison ou un groupe de formule -(CR¹⁷R¹⁸)ᵥ-Y-(CR¹⁷R¹⁸)_{w}, où v et w ont indépendamment une valeur de 0, 1, 2 ou 3, et Y est une liaison, -CR¹⁵=CR¹⁶, un groupe phényle, furannyle, thiophényle, pyrrolyle, thiazolyle, imidazolyle, oxazolyle, isoxazolyle, pyrazolyle, isoxazolonyle, pipérazinyle, pipéridinyle, morpholinyle, pyrrolidinyle, isothiazolyle, triazolyle, oxadiazolyle, thiadiazolyle, pyridyle ou pyridazyle ; et
Q représente H, un groupe (alkyle en C₁ à C₆)oxy, [N-Z](alkyle en C₁ à C₆)oxy(alkyle en C₁ à C₆)amino, thio, (alkyle en C₁ à C₆)thio, carboxy(alkyle en C₁ à C₆)thio, carboxy, carboxy(alkyle en C₁ à C₆), carboxy(alcényle en C₁ à C₆), [N-Z](carboxy(alkyle en C₁ à C₆)amino, carboxy(alkyle en C₁ à C₆)oxy, formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, amino, [N-Z](alkyle en C₁ à C₆)amino, aminocarbonyle, (alkyle en C₁ à C₆)aminocarbonyle, di(alkyle en C₁ à C₆)aminocarbonyle, [N-Z](alkyle en C₁ à C₆)carbonylamino, cycloalkyle en C₅ à C₈, [N-Z]-(alkyle en C₁ à C₆)carbonyl(alkyle en C₁ à C₆)amino, halo, halo(alkyle en C₁ à C₆), sulfamoyle, [N-Z](alkyle en C₁ à C₆)sulfonylamino, (alkyle en C₁ à C₆)sulfonylaminocarbonyle, carboxy(alkyle en C₁ à C₆)sulfonyle, carboxy(alkyle en C₁ à C₆)sulfinyle, tétrazolyle, [N-Z]tétrazolylamino, cyano, amidino, amidinothio, SO₃H, formyloxy, formamido, cycloalkyle en C₃ à C₈, (alkyle en C₁ à C₆)sulfamoyle, di(alkyle en C₁ à C₆)sulfamoyle, (alkyle en C₁ à C₆)carbonylaminosulfonyle, 5-oxo-2,5-dihydro[1,2,4]-oxadiazolyle, carboxy(alkyle en C₁ à C₆)carbonylamino, tétrazolyl(alkyle en C₁ à C₆)thio, [N-Z]tétrazolyl(alkyle en C₁ à C₆)amino, 5-oxo-2,5-dihydro[1,2,4]thiadiazolyle, 5-oxo-1,2-dihydro[1,2,4]triazolyle, [N-Z]-(alkyle en C₁ à C₆)amino(alkyle en C₁ à C₆)amino, ou un groupe de formule : 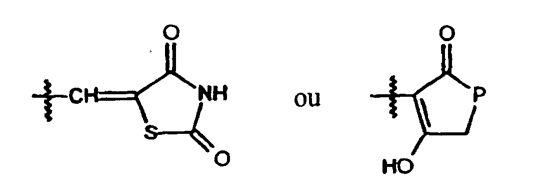
où P représente O, S ou NR¹⁹ ;
Z représente H, un groupe alkyle en C₁ à C₆, t-butoxycarbonyle, acétyle, benzoyle ou benzyle ;
R⁴ est un groupe hydrocarbyle en C₁ à C₁₈ facultativement substitué dans lequel jusqu'à trois atomes de C peuvent facultativement être remplacés par des atomes de N, O et/ou S ;
R¹¹, R¹², R¹³, R¹⁴, R¹⁵, R¹⁷, R¹⁸ et R¹⁹ représentent indépendamment H ou un groupe alkyle en C₁ à C₃ ; et
R¹⁶ représente H, un groupe alkyle en C₁ à C₃ ou acétylamino ;
ou un de ses sels pharmaceutiquement acceptables ;
pour la préparation d'un médicament pour le traitement de troubles liés à la gastrine.

2. Utilisation selon la revendication 1, dans laquelle W représente N.

3. Utilisation selon la revendication 1 ou la revendication 2, dans laquelle R¹ et R⁵ représentent tous deux H.

4. Utilisation selon l'une quelconque des revendications précédentes, dans laquelle R² répond à la formule :
-(CH₂)ₛ-C(R⁶R⁷)ₙ-(CH₂)ₜ-R⁸
dans laquelle :
R⁶ et R⁷ sont choisis indépendamment entre H, un groupe alkyle en C₁ à C₆ ou OH ; ou bien R⁶ et R⁷ représentent ensemble un groupe =O ;
n vaut 0 ou 1 ;
s vaut 0, 1, 2 ou 3 ;
t vaut 0, 1, 2 ou 3 ; et
R⁸ est choisi entre H, un groupe alkyle en C₁ à C₁₂, (alkyle en C₁ à C₁₂)oxy, cycloalkyle en C₃ à C₁₂, phényle, naphtyle, pyridyle, pyrrolyle, imidazolyle, pyrazolyle, pyridazinyle, pyrimidinyle, triazolyle, furannyle, thiényle, furazanyle, oxazolyle, isoxazolyle, thiazolyle, thiazinyle, indolyle, indolinyle, isoindolyle, isoindolinyle, isoquinolinyle, quinolinyle, benzofurannyle, benzothiényle, pipérazinyle, pipéridinyle, pyrrolidinyle, pyrrolinyle, dihydropyranyle, tétrahydropyranyle, pyranyle, tétrahydrofurannyle, morpholinyle, thiazolidinyle, thiomorpholinyle ou thioxanyle (tous ces groupes étant facultativement substitués par 1, 2 ou 3 groupes choisis indépendamment entre les groupes alkyle en C₁ à C₆, (alkyle en C₁ à C₆)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, hydroxy(alkyle en C₁ à C₆), amino, (alkyle en C₁ à C₆)-amino, di(alkyle en C₁ à C₆)amino, aminocarbonyle, halo, halo(alkyle en C₁ à C₆), aminosulfonyle, (alkyle en C₁ à C₆)sulfonylamino ou cyano).

5. Utilisation selon la revendication 4, dans laquelle s vaut 1, n vaut 1, et R⁶ et R⁷ représentent ensemble un groupe =O.

6. Utilisation selon la revendication 4 ou 5, dans laquelle t vaut 0, et R⁸ est un groupe cycloalkyle en C₃ à C₁₂ (facultativement substitué par un groupe méthyle) ou un groupe alkyle ramifié en C₃ à C₁₂.

7. Utilisation selon l'une quelconque des revendications 4 à 6, dans laquelle R⁸ est un groupe t-butyle, cyclohexyle, 1-méthylcyclohexyle, 1-méthylcyclopentyle ou cyclopentyle.

8. Utilisation selon l'une quelconque des revendications précédentes, dans laquelle m vaut 1, R¹¹ représente H et R¹² représente H.

9. Utilisation selon l'une quelconque des revendications précédentes, dans laquelle p vaut 0.

10. Utilisation selon l'une quelconque des revendications précédentes, dans laquelle X représente C(O)NH.

11. Utilisation selon l'une quelconque des revendications précédentes, dans laquelle R⁹ est un groupe phényle substitué par un groupe carboxy, carboxy(alkyle en C₁ à C₆), tétrazolyle, tétrazolyl-N-(alkyle en C₁ à C₆)amino, carboxy(alkyle en C₁ à C₆)thio, carboxy(alkyle en C₁ à C₆)-sulfonyle, (alkyle en C₁ à C₆)amino ou 5-oxo-2,5-dihydro[1,2,4]-oxadiazolyle ; ou bien R⁹ est un groupe N-[carboxy(alkyle en C₁ à C₆)]-indolinyle ou N-[carboxy(alkyle en C₁ à C₆)]indolyle.

12. Utilisation selon la revendication 11, dans laquelle le groupe phényle est substitué en sa position 3.

13. Utilisation selon l'une quelconque des revendications précédentes, dans laquelle R⁴ répond à la formule :
-(CH₂)_{q}-T-R¹⁰
dans laquelle :
q vaut 0, 1, 2 ou 3 ;
T représente une liaison, O, S, NH ou un groupe N(alkyle en C₁ à C₆); et
R¹⁰ est un groupe alkyle en C₁ à C₁₂, cycloalkyle en C₃ à C₁₂, phényle, naphtyle, pyridyle, pyrrolyle, imidazolyle, pyrazolyle, pyridazinyle, pyrimidinyle, triazolyle, furannyle, thiényle, furazanyle, oxazolyle, isoxazolyle, thiazolyle, thiazinyle, indolyle, indolinyle, isoindolyle, isoindolinyle, isoquinolinyle, quinolinyle, benzofurannyle, benzothiényle, pipérazinyle, pipéridinyle, pyrrolidinyle, pyrrolinyle, dihydropyranyle, tétrahydropyranyle, pyranyle, tétrahydrofurannyle, morpholinyle, thiazolidinyle, thiomorpholinyle ou thioxanyle (tous ces groupes étant facultativement substitués par 1, 2 ou 3 groupes choisis indépendamment parmi les groupes alkyle en C₁ à C₆, (alkyle en C₁ à C₆)oxy, cycloalkyle en C₃ à C₈, (cycloalkyle en C₃ à C₈)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)-carbonyloxy, nitro, trihalométhyle, hydroxy, hydroxy(alkyle en C₁ à C₆), amino, (alkyle en C₁ à C₆)amino, di(alkyle en C₁ à C₆)amino, aminocarbonyle, halo, halo(alkyle en C₁ à C₆), aminosulfonyle, (alkyle en C₁ à C₆)sulfonylamino ou cyano).

14. Utilisation selon la revendication 13, dans laquelle q vaut 0, T est une liaison et R¹⁰ est un groupe alkyle en C₁ à C₁₂, cycloalkyle en C₃ à C₁₂, pyridyle ou phényle (tous ceux-ci étant facultativement substitué par OMe, NMe₂, CF₃, Me, F, CI, Br ou I).

15. Utilisation selon l'une quelconque des revendications précédentes, dans laquelle R⁴ est un groupe cyclohexyle.

16. Composition pharmaceutique comprenant un inhibiteur de pompe à protons et un composé de formule (I), tel que défini dans l'une quelconque des revendications 1 à 15, conjointement avec un diluant ou support pharmaceutiquement acceptable.

17. Composition selon la revendication 16, dans laquelle l'inhibiteur de pompe à protons est choisi parmi le (RS)-rabéprazole, le (RS)-oméprazole, le lansoprazole, le pantoprazole, le (R)-oméprazole, le (S)-oméprazole, le perprazole, le (R)-rabéprazole, le (S)-rabéprazole, ou leurs sels alcalins.

18. Composition selon la revendication 16 ou 17, dans laquelle l'inhibiteur de pompe à protons et le composé de formule (I) sont présents chacun en une quantité produisant un effet thérapeutique bénéfique chez des patients souffrant de troubles gastrointestinaux.

19. Composition selon la revendication 18, dans laquelle ledit effet thérapeutique bénéfique est un effet synergique sur la réduction de la sécrétion d'acide chez des patients souffrant de troubles gastrointestinaux, ou la prévention de troubles gastrointestinaux chez lesdits patients, ou la réduction des effets néfastes associés à l'un des ingrédients actifs par les autres ingrédients actifs.

20. Composition selon l'une quelconque des revendication 16 à 19, dans laquelle la quantité de chacun des ingrédients actifs est égale ou inférieure à celle qui est autorisée ou indiquée dans une monothérapie avec ledit ingrédient actif.

21. Kit contenant comme premier ingrédient actif un composé de formule (I), tel que défini à l'une quelconque des revendications 1 à 15, et comme second ingrédient actif un inhibiteur de pompe à protons, sous forme d'une préparation combinée pour une utilisation simultanée, distincte ou séquentielle dans le traitement de patients souffrant de troubles gastrointestinaux.

22. Utilisation d'une composition selon l'une quelconque des revendications 16 à 20, ou d'un kit selon la revendication 21, pour la préparation d'un médicament pour le traitement de troubles gastrointestinaux.

23. Utilisation d'un inhibiteur de pompe à protons pour la préparation d'un médicament pour le traitement de troubles gastrointestinaux, ledit traitement comprenant l'administration simultanée ou séquentielle dudit inhibiteur de pompe à protons et d'un composé de formule (I), tel que défini à l'une quelconque des revendications 1 à 15, ledit inhibiteur de pompe à protons amplifiant l'effet du composé de formule (I) sur les troubles liés à la gastrine chez des patients souffrant de troubles gastrointestinaux.

24. Utilisation d'un composé de formule (I), tel que défini dans l'une quelconque des revendications 1 à 15, pour la préparation d'un médicament pour le traitement de troubles gastrointestinaux, ledit traitement comprenant l'administration simultanée ou séquentielle dudit inhibiteur de pompe à protons et d'un composé de formule (I), ledit composé de formule (I) amplifiant l'effet de l'inhibiteur de pompe à protons sur la réduction de la sécrétion d'acide chez des patients souffrant de troubles gastrointestinaux.

25. Utilisation d'un composé de formule (I), tel que défini dans l'une quelconque des revendications 1 à 15, pour la préparation d'un médicament pour réduire les effets néfastes associés à l'administration d'inhibiteurs de pompes à protons chez des patients souffrant de troubles gastrointestinaux.

26. Utilisation selon la revendication 25, dans laquelle l'effet néfaste est une hyperplasie.

27. Utilisation d'un inhibiteur de pompe à protons pour la préparation d'un médicament pour réduire les effets néfastes associés à l'administration d'un composé de formule (I), tel que défini dans l'une quelconque des revendications 1 à 15, chez des patients souffrant de troubles gastrointestinaux.

28. Procédé de préparation d'une composition pharmaceutique selon l'une quelconque des revendications 18 à 22, comprenant le mélange d'un composé de formule (I), tel que défini dans l'une quelconque des revendications 1 à 15, et d'un inhibiteur de pompe à protons avec un diluant ou support pharmaceutiquement acceptable.

29. Composé de formule (IIa) : 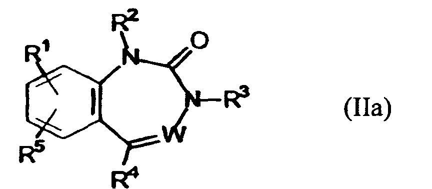 dans laquelle :
W représente N ou N⁺-O⁻ ;
R¹ et R⁵ représentent indépendamment H, un groupe alkyle en C₁ à C₆, (alkyle en C₁ à C₆)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, hydroxy(alkyle en C₁ à C₆), amino, (alkyle en C₁ à C₆)-amino, di(alkyle en C₁ à C₆)amino, aminocarbonyle, halo, halo(alkyle en C₁ à C₆), aminosulfonyle, (alkyle en C₁ à C₆)sulfonylamino, (alkyle en C₁ à C₆)aminocarbonyle, di(alkyle en C₁ à C₆)aminocarbonyle, [N-Z](alkyle en C₁ à C₆)carbonylamino, formyloxy, formamido, (alkyle en C₁ à C₆)-aminosulfonyle, di(alkyle en C₁ à C₆)aminosulfonyle, [N-Z](alkyle en C₁ à C₆)sulfonylamino ou cyano ; ou bien R¹ et R⁵ forment conjointement un groupe méthylènedioxy ;
R² est un groupe -(CH₂)ₛ-C(O)-(CH₂)ₜ-R⁸,
s vaut 0, 1, 2 ou 3 ;
t vaut 0, 1, 2 ou 3 ;
R⁸ est choisi entre H, un groupe alkyle en C₁ à C₁₂, (alkyle en C₁ à C₁₂)oxy, cycloalkyle en C₃ à C₁₂, phényle, naphtyle, pyridyle, pyrrolyle, imidazolyle, pyrazolyle, pyridazinyle, pyrimidinyle, triazolyle, furannyle, thiényle, furazanyle, oxazolyle, isoxazolyle, thiazolyle, thiazinyle, indolyle, indolinyle, isoindolyle, isoindolinyle, isoquinolinyle, quinolinyle, benzofurannyle, benzothiényle, pipérazinyle, pipéridinyle, pyrrolidinyle, pyrrolinyle, dihydropyranyle, tétrahydropyranyle, pyranyle, tétrahydrofurannyle, morpholinyle, thiazolidinyle, thiomorpholinyle ou thioxanyle (tous ces groupes étant facultativement substitués par 1, 2 ou 3 groupes choisis indépendamment parmi les groupes alkyle en C₁ à C₆, (alkyle en C₁ à C₆)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, hydroxy(alkyle en C₁ à C₆), amino, (alkyle en C₁ à C₆)-amino, di(alkyle en C₁ à C₆)amino, aminocarbonyle, halo, halo(alkyle en C₁ à C₆), aminosulfonyle, (alkyle en C₁ à C₆)sulfonylamino ou cyano).
R³ représente -(CR¹¹R¹²)ₘ-X-(CR¹³R¹⁴)ₚ-R⁹ ;
m vaut 0, 1, 2, 3 ou 4 ;
p vaut 0, 1 ou 2 ;
X représente une liaison, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 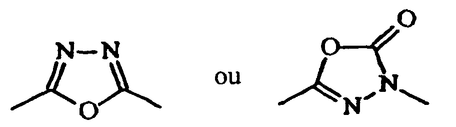
R⁹ représente H ; un groupe alkyle en C₁ à C₆ ; ou phényle, naphtyle, pyridyle, benzimidazolyle, indazolyle, quinolinyle, isoquinolinyle, tétrahydroisoquinolinyle, indolinyle, isoindolinyle, indolyle, isoindolyle ou 2-pyridonyle, tous ces groupes étant facultativement substitués par 1, 2 ou 3 groupes choisis indépendamment à partir de -L-Q ;
où :
L est une liaison ou un groupe de formule -(CR¹⁷R¹⁸)ᵥ-Y-(CR¹⁷R¹⁸)_{w}, dans laquelle v et w ont indépendamment une valeur de 0, 1, 2 ou 3, et Y est une liaison, -CR¹⁵=CR¹⁶-, un groupe phényle, furannyle, thiophényle, pyrrolyle, thiazolyle, imidazolyle, oxazolyle, isoxazolyle, pyrazolyle, isoxazolonyle, pipérazinyle, pipéridinyle, morpholinyle, pyrrolidinyle, isothiazolyle, triazolyle, oxadiazolyle, thiadiazolyle, pyridyle ou pyridazyle ; et
Q représente H, un groupe (alkyle en C₁ à C₆)oxy, [N-Z](alkyle en C₁ à C₆)oxy(alkyle en C₁ à C₆)amino, thio, (alkyle en C₁ à C₆)thio, carboxy(alkyle en C₁ à C₆)thio, carboxy, carboxy(alkyle en C₁ à C₆), carboxy(alcényle en C₁ à C₆), [N-Z](carboxy(alkyle en C₁ à C₆)amino, carboxy(alkyle en C₁ à C₆)oxy, formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, amino, [N-Z](alkyle en C₁ à C₆)amino, aminocarbonyle, (alkyle en C₁ à C₆)aminocarbonyle, di(alkyle en C₁ à C₆)aminocarbonyle, [N-Z](alkyle en C₁ à C₆)carbonylamino, cycloalkyle en C₅ à C₈, [N-Z]-(alkyle en C₁ à C₆)carbonyl(alkyle en C₁ à C₆)amino, halo, halo(alkyle en C₁ à C₆), sulfamoyle, [N-Z](alkyle en C₁ à C₆)sulfonylamino, (alkyle en C₁ à C₆)sulfonylaminocarbonyle, carboxy(alkyle en C₁ à C₆)sulfonyle, carboxy(alkyle en C₁ à C₆)sulfinyle, tétrazolyle, [N-Z]tétrazolylamino, cyano, amidino, amidinothio, SO₃H, formyloxy, formamido, cycloalkyle en C₃ à C₈, (alkyle en C₁ à C₆)sulfamoyle, di(alkyle en C₁ à C₆)sulfamoyle, (alkyle en C₁ à C₆)carbonylaminosulfonyle, 5-oxo-2,5-dihydro[1,2,4]-oxadiazolyle, carboxy(alkyle en C₁ à C₆)carbonylamino, tétrazolyl(alkyle en C₁ à C₆)thio, [N-Z]tétrazolyl(alkyle en C₁ à C₆)amino, 5-oxo-2,5-dihydro[1,2,4]thiadiazolyle, 5-oxo-1,2-dihydro[1,2,4]triazolyle, [N-Z]-(alkyle en C₁ à C₆)amino(alkyle en C₁ à C₆)amino, ou un groupe de formule : 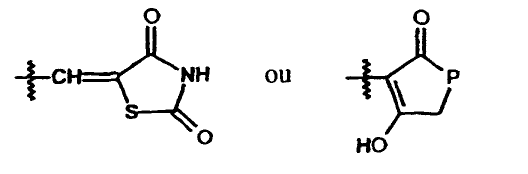
dans laquelle P représente O, S ou NR¹⁹ ;
Z représente H, un groupe alkyle en C₁ à C₆, t-butoxycarbonyle, acétyle, benzoyle ou benzyle ;
R⁴ est un groupe hydrocarbyle en C₁ à C₁₈ facultativement substitué dans lequel jusqu'à trois atomes de C peuvent facultativement être remplacés par des atomes de N, O et/ou S ;
R¹¹, R¹², R¹³, R¹⁴, R¹⁵, R¹⁷, R¹⁸ et R¹⁹ représentent indépendamment H ou un groupe alkyle en C₁ à C₃ ; et
R¹⁶ représente H, un groupe alkyle en C₁ à C₃ ou acétylamino ;
ou un de ses sels pharmaceutiquement acceptables ;
sous réserve que R² ne représente pas CH₂CO₂H ou C(O)CH₃ lorsque R⁴ est un groupe phényle.

30. Composé de formule (IIb) : 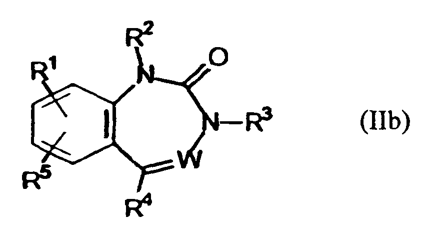 dans laquelle :
W représente N ou N⁺-O⁻ ;
R¹ et R⁵ représentent indépendamment H, un groupe alkyle en C_{1 6}, (alkyle en C₁ à C₆)oxy, thio, (alkyle en C₁ à C₆)thio, carboxy, carboxyalkyle en C₁ à C₆), formyle, (alkyle en C₁ à C₆)carbonyle, (alkyle en C₁ à C₆)xycarbonyle, (alkyle en C₁ à C₆)carbonyloxy, nitro, trihalométhyle, hydroxy, hydroxy(alkyle en C₁ à C₆), amino, (alkyle en C₁ à C₆)amino, di(alkyle en C₁ à C₆)amino, aminocarbonyle, halo, halo(alkyle en C₁ à C₆), aminosulfonyle, (alkyle en C₁ à C₆)sulfonylamino, (alkyle en C₁ à C₆)-aminocarbonyle, di(alkyle en C₁ à C₆)aminocarbonyle, [N-Z](alkyle en C₁ à C₆)carbonylamino, formyloxy, formamido, (alkyle en C₁ à C₆)aminosulfonyle, di(alkyle en C₁ à C₆)aminosulfonyle, [N-Z](alkyle en C₁ à C₆)-sulfonylamino ou cyano ; ou bien R¹ et R⁵ forment conjointement un groupe méthylènedioxy ;
R² est un groupe hydrocarbyle en C₁ à C₁₈ facultativement substitué dans lequel jusqu'à trois atomes de C peuvent facultativement être remplacés par des atomes de N, O et/ou S.
R³ représente -(CR¹¹R¹²)ₘ-X-(CR¹³R¹⁴)ₚ-R⁹ ;
m vaut 0, 1, 2, 3 ou 4 ;
p vaut 0, 1 ou 2 ;
X représente une liaison, -CR¹⁵=CR¹⁶-, -C≡C-, C(O)NH, NHC(O), C(O)NMe, NMeC(O), C(O)O, NHC(O)NH, NHC(O)O, OC(O)NH, NH, O, CO, SO₂, SO₂NH, C(O)NHNH, 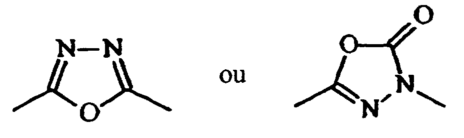
R⁹ représente H ; un groupe alkyle en C₁ à C₆ ; ou phényle, naphtyle, pyridyle, benzimidazolyle, indazolyle, quinolinyle, isoquinolinyle, tétrahydroisoquinolinyle, indolinyle, isoindolinyle, indolyle, isoindolyle ou 2-pyridonyle, tous étant facultativement substitués par 1, 2 ou 3 groupes choisis indépendamment à partir de -L-Q ;
où :
L est une liaison ou un groupe de formule -(CR¹⁷R¹⁸)ᵥ-Y-(CR¹⁷R¹⁸)_{w}, dans laquelle v et w ont indépendamment une valeur de 0, 1, 2 ou 3, et Y est une liaison, -CR¹⁵=CR¹⁶, un groupe phényle, furannyle, thiophényle, pyrrolyle, thiazolyle, imidazolyle, oxazolyle, isoxazolyle, pyrazolyle, isoxazolonyle, pipérazinyle, pipéridinyle, morpholinyle, pyrrolidinyle, isothiazolyle, triazolyle, oxadiazolyle, thiadiazolyle, pyridyle ou pyridazyle ; et
Q représente H, un groupe (alkyle en C₁ à C₆)oxy, [N-Z](alkyle en C₁ à C₆)oxy(alkyle en C₁ à C₆)amino, thio, (alkyle en C₁ à C₆)thio, carboxy(alkyle en C₁ à C₆)thio, carboxy, carboxy(alkyle en C₁ à C₆), carboxy(alcényle en C₁ à C₆), [N-Z](carboxy(alkyle en C₁ à C₆)amino, carboxy(alkyle en C₁ à C₆)oxy, formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, amino, [N-Z](alkyle en C₁ à C₆)amino, aminocarbonyle, (alkyle en C₁ à C₆)aminocarbonyle, di(alkyle en C₁ à C₆)aminocarbonyle, [N-Z](alkyle en C₁ à C₆)carbonylamino, cycloalkyle en C₅ à C₈, [N-Z](alkyle en C₁ à C₆)carbonyl(alkyle en C₁ à C₆)amino, halo, halo(alkyle en C₁ à C₆), sulfamoyle, [N-Z](alkyle en C₁ à C₆)sulfonylamino, (alkyle en C₁ à C₆)sulfonylaminocarbonyle, carboxy(alkyle en C₁ à C₆)sulfonyle, carboxy(alkyle en C₁ à C₆)sulfinyle, tétrazolyle, [N-Z]tétrazolylamino, cyano, amidino, amidinothio, SO₃H, formyloxy, formamido, cycloalkyle en C₃ à C₈, (alkyle en C₁ à C₆)sulfamoyle, di(alkyle en C₁ à C₆)sulfamoyle, (alkyle en C₁ à C₆)carbonylaminosulfonyle, 5-oxo-2,5-dihydro[1,2,4]-oxadiazolyle, carboxy(alkyle en C₁ à C₆)carbonylamino, tétrazolyl(alkyle en C₁ à C₆)thio, [N-Z]tétrazolyl(alkyle en C₁ à C₆)amino, 5-oxo-2,5-dihydro[1,2,4]thiadiazolyle, 5-oxo-1,2-dihydro[1,2,4]triazolyle, [N-Z]-(alkyle en C₁ à C₆)amino(alkyle en C₁ à C₆)amino, ou un groupe de formule : 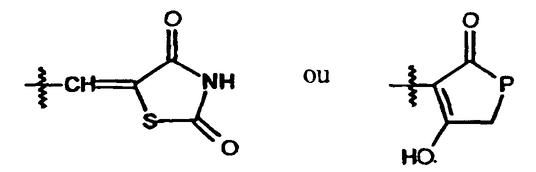
où P représente O, S ou NR¹⁹ ;
Z représente H, un groupe alkyle en C₁ à C₆, t-butoxycarbonyle, acétyle, benzoyle ou benzyle ;
R⁴ répond à la formule :
-(CH₂)_{q}-R-R¹⁰
dans laquelle :
q vaut 0, 1, 2 ou 3 ;
T représente une liaison, O, S, NH ou un groupe N(alkyle en C₁ à C₆) ; et
R¹⁰ est un groupe alkyle en C₁ à C₁₂, cycloalkyle en C₃ à C₁₂, phényle, naphtyle, pyridyle, pyrrolyle, imidazolyle, pyrazolyle, pyridazinyle, pyrimidinyle, triazolyle, furannyle, thiényle, furazanyle, oxazolyle, isoxazolyle, thiazolyle, thiazinyle, indolyle, indolinyle, isoindolyle, isoindolinyle, isoquinolinyle, quinolinyle, benzofurannyle, benzothiényle, pipérazinyle, pipéridinyle, pyrrolidinyle, pyrrolinyle, dihydropyranyle, tétrahydropyranyle, pyranyle, tétrahydrofurannyle, morpholinyle, thiazolidinyle, thiomorpholinyle ou thioxanyle (tous ces groupes étant facultativement substitués par 1, 2 ou 3 groupes choisis indépendamment parmi les groupes alkyle en C₁ à C₆, (alkyle en C₁ à C₆)-oxy, cycloalkyle en C₃ à C₈, (cycloalkyle en C₃ à C₈)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)-carbonyloxy, nitro, trihalométhyle, hydroxy, hydroxy(alkyle en C₁ à C₆), amino, (alkyle en C₁ à C₆)amino, di(alkyle en C₁ à C₆)amino, aminocarbonyle, halo, halo(alkyle en C₁ à C₆), aminosulfonyle, (alkyle en C₁ à C₆)sulfonylamino ou cyano) ;
R¹¹, R¹², R¹³, R¹⁴, R¹⁵, R¹⁷, R¹⁸ et R¹⁹ représentent indépendamment H ou un groupe alkyle en C₁ à C₃ ; et
R¹⁶ représente H, un groupe alkyle en C₁ à C₃ ou acétylamino ;
ou un de ses sels pharmaceutiquement acceptables ;
sous réserve que R¹⁰ ne soit pas un groupe phényle ou phényle substitué lorsque q vaut 0 et que T est une liaison.

31. Composé de formule (IIa) ou (IIb) selon la revendication 29 ou 30, dans lequel W représente N.

32. Composé de formule (IIa) ou (IIb) selon l'une quelconque des revendications 29 à 31, dans lequel R¹ et R⁵ représentent tous deux H.

33. Composé de formule (IIb) selon l'une quelconque des revendications 30 à 32, dans lequel R² répond à la formule :
-(CH₂)ₛ-C(R⁶R⁷)ₙ-(CH₂)ₜ-R⁸
dans laquelle :
R⁶ et R⁷ sont choisis indépendamment entre H, un groupe alkyle en C₁ à C₆ ou OH ; ou bien R⁶ et R⁷ représentent ensemble un groupe =O ;
n vaut 0 ou 1 ;
s vaut 0, 1, 2 ou 3 ;
t vaut 0, 1, 2 ou 3 ; et
R⁸ est choisi entre H, un groupe alkyle en C₁ à C₁₂, (alkyle en C₁ à C₁₂)oxy, cycloalkyle en C₃ à C₁₂, phényle, naphtyle, pyridyle, pyrrolyle, imidazolyle, pyrazolyle, pyridazinyle, pyrimidinyle, triazolyle, furannyle, thiényle, furazanyle, oxazolyle, isoxazolyle, thiazolyle, thiazinyle, indolyle, indolinyle, isoindolyle, isoindolinyle, isoquinolinyle, quinolinyle, benzofurannyle, benzothiényle, pipérazinyle, pipéridinyle, pyrrolidinyle, pyrrolinyle, dihydropyranyle, tétrahydropyranyle, pyranyle, tétrahydrofurannyle, morpholinyle, thiazolidinyle, thiomorpholinyle ou thioxanyle (tous ces groupes étant facultativement substitués par 1, 2 ou 3 groupes choisis indépendamment entre les groupes alkyle en C₁ à C₆, (alkyle en C₁ à C₆)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)carbonyloxy, nitro, trihalométhyle, hydroxy, hydroxy(alkyle en C₁ à C₆), amino, (alkyle en C₁ à C₆)-amino, di(alkyle en C₁ à C₆)amino, aminocarbonyle, halo, halo(alkyle en C₁ à C₆), aminosulfonyle, (alkyle en C₁ à C₆)sulfonylamino ou cyano).

34. Composé de formule (IIb) selon la revendication 33, dans lequel s vaut 1, n vaut 1, et R⁶ et R⁷ représentent ensemble un groupe =O.

35. Composé de formule (IIb) selon les revendications 33 ou 34, dans lequel t vaut 0 et R⁸ est un groupe cycloalkyle en C₃ à C₁₂ ou un groupe alkyle en C₃ à C₁₂ ramifié.

36. Composé de formule (IIb) selon la revendication 29, 31 ou 32, dans lequel s vaut 1, t vaut 0, et R⁸ est un groupe cycloalkyle en C₃ à C₁₂ ou un groupe alkyle en C₃ à C₁₂ ramifié.

37. Composé de formule (IIa) ou (IIb) selon la revendication 35 ou 36, dans lequel R⁸ est un groupe t-butyle ou cyclopentyle.

38. Composé de formule (IIa) ou (IIb) selon l'une quelconque des revendications 29 à 37, dans lequel m vaut 1, R¹¹ représente H et R¹² représente H.

39. Composé de formule (IIa) ou (IIb) selon l'une quelconque des revendications 29 à 38, dans lequel p vaut 0.

40. Composé de formule (IIa) ou (IIb) selon l'une quelconque des revendications 29 à 39, dans lequel X est un groupe C(O)NH.

41. Composé de formule (IIa) ou (IIb) selon l'une quelconque des revendications 31 à 42, dans lequel R⁹ est un groupe phényle substitué par un groupe carboxy, carboxy(alkyle en C₁ à C₆), tétrazolyle, tétrazolyl-N-(alkyle en C₁ à C₆)amino, carboxy(alkyle en C₁ à C₆)thio, carboxy(alkyle en C₁ à C₆)sulfonyle, (alkyle en C₁ à C₆)amino, ou 5-oxo-2,5-dihydro[1,2,4]-oxadiazolyle ; ou bien R⁹ est un groupe N-[carboxy(alkyle en C₁ à C₆)-indolinyle ou N-[carboxy(alkyle en C₁ à C₆)indolyle.

42. Composé de formule (IIa) ou (IIb) selon la revendication 41, dans lequel le groupe phényle est substitué en sa position 3.

43. Composé de formule (IIa) selon l'une quelconque des revendications 29, 31, 32 ou 36-42, dans lequel R⁴ répond à la formule :
-(CH₂)_{q}-T-R¹⁰
dans laquelle :
q vaut 0, 1, 2 ou 3 ;
T représente une liaison, O, S, NH ou un groupe N(alkyle en C₁ à C₆) ; et
R¹⁰ est un groupe alkyle en C₁ à C₁₂, cycloalkyle en C₃ à C₁₂, phényle, naphtyle, pyridyle, pyrrolyle, imidazolyle, pyrazolyle, pyridazinyle, pyrimidinyle, triazolyle, furannyle, thiényle, furazanyle, oxazolyle, isoxazolyle, thiazolyle, thiazinyle, indolyle, indolinyle, isoindolyle, isoindolinyle, isoquinolinyle, quinolinyle, benzofurannyle, benzothiényle, pipérazinyle, pipéridinyle, pyrrolidinyle, pyrrolinyle, dihydropyranyle, tétrahydropyranyle, pyranyle, tétrahydrofurannyle, morpholinyle, thiazolidinyle, thiomorpholinyle ou thioxanyle (tous ces groupes étant facultativement substitués par 1, 2 ou 3 groupes choisis indépendamment parmi les groupes alkyle en C₁ à C₆, (alkyle en C₁ à C₆)-oxy, cycloalkyle en C₃ à C₈, (cycloalkyle en C₃ à C₈)oxy, thio, (alkyle en C₁ à C₆)thio, carboxy, carboxy(alkyle en C₁ à C₆), formyle, (alkyle en C₁ à C₆)carbonyle, (alkyle en C₁ à C₆)oxycarbonyle, (alkyle en C₁ à C₆)-carbonyloxy, nitro, trihalométhyle, hydroxy, hydroxy(alkyle en C₁ à C₆), amino, (alkyle en C₁ à C₆)amino, di(alkyle en C₁ à C₆)amino, aminocarbonyle, halo, halo(alkyle en C₁ à C₆), aminosulfonyle, (alkyle en C₁ à C₆)sulfonylamino ou cyano).

44. Composé de formule (IIa) selon la revendication 43, dans lequel q vaut 0, T est une liaison et R¹⁰ est un groupe alkyle en C₁ à C₁₂, cycloalkyle en C₃ à C₁₂, pyridyle ou phényle (tous ces groupes étant facultativement substitué par OMe, NMe₂, CF₃, Me, F, CI, Br ou I).

45. Composé de formule (IIb) selon les revendications 30-34 ou 37-42, dans lequel q vaut 0, T est une liaison et R¹⁰ est un groupe alkyle en C₁ à C₁₂, cycloalkyle en C₃ à C₁₂ ou pyridyle (tous ces groupes étant facultativement substitués par OMe, NMe₂, CF₃, Me, F, CI, Br ou I).

46. Composé de formule (IIa) ou (IIb) selon la revendication 44 ou 45, dans lequel R⁴ est un groupe cycloalkyle en C₃ à C₁₂·

47. Composé de formule (IIa) ou (IIb) selon l'une quelconque des revendications 44 à 46, dans lequel R⁴ est un groupe cyclohexyle.

48. Composé de formule (IIa) ou (IIb) qui est dégradé *in vivo* pour donner un composé selon l'une quelconque des revendications 29 à 47.

49. Composition pharmaceutique comprenant un composé de formule (IIa) ou (IIb) selon l'une quelconque des revendications 29 à 48, conjointement avec un diluant ou support pharmaceutiquement acceptable.

50. Composé de formule (IIa) ou (IIb) selon l'une quelconque des revendications 29 à 48, ou composition selon la revendication 49, pour une utilisation en médecine.

51. Procédé de préparation d'une composition pharmaceutique selon la revendication 49, comprenant le mélange d'un composé de formule (IIa) ou (IIb) avec un diluant ou support pharmaceutiquement acceptable.

52. Procédé de préparation d'un composé selon la formule (I), (IIa) ou (IIb), comprenant les étapes consistant à faire réagir un composé de formule (III) avec NH₂NHR^{3'}, et à cycliser le composé d'hydrazone résultant avec un réactif carbonyle bifonctionnel : 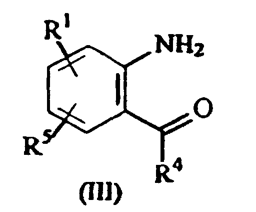 où R^{3'} représente R³ ou un précurseur approprié de celui-ci, et R¹, R⁴ et R⁵ sont tels que définis dans la revendication 1.
